# EUROPEAN PATENT APPLICATION

(11) **EP 4 687 407 A1**
(43) Date of publication of application: **04.02.2026**
(21) Application number: 25190216.9
(22) Date of filing: 17.07.2025
(51) Int. Cl.: H10K 85/60, H10K 50/16

(54) **LIGHT-EMITTING DEVICE INCLUDING HETEROCYCLIC COMPOUND, ELECTRONIC APPARATUS INCLUDING THE LIGHT-EMITTING DEVICE, AND THE HETEROCYCLIC COMPOUND**

(30) Priority: 31.07.2024 KR 20240102048
(71) Applicant: Samsung Display Co., Ltd., Yongin-si, Gyeonggi-do 17113 (KR)
(72) Inventor: KIM, Dongjun, 17113 Yongin-si (KR); KIM, Chaeyeong, 17113 Yongin-si (KR); RYU, Hwasook, 17113 Yongin-si (KR); PAK, Hankyu, 17113 Yongin-si (KR); HAN, Sanghyun, 17113 Yongin-si (KR)
(74) Representative: Marks & Clerk LLP

(57) **Abstract**

A light-emitting device includes a first electrode (110), a second electrode (150) opposite to the first electrode, an interlayer (130) between the first electrode and the second electrode, and a heterocyclic compound represented by Formula 1. In addition, there are provided an electronic apparatus including the light-emitting device, and the heterocyclic compound represented by Formula 1:

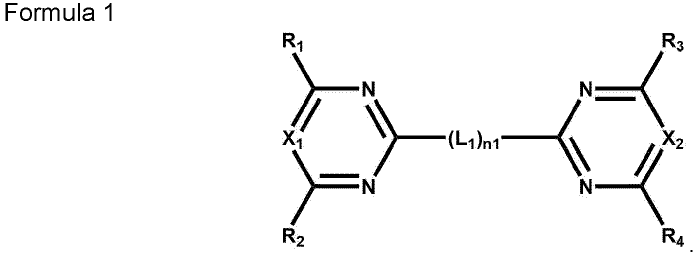

## Description

### BACKGROUND

### 1. Field

One or more embodiments of the present disclosure relate to a light-emitting device including a heterocyclic compound, an electronic apparatus including the light-emitting device, and the heterocyclic compound.

### 2. Description of the Related Art

Among light-emitting devices, self-emissive devices (e.g., organic light-emitting devices) are notable for their relatively wide viewing angles, high contrast ratios, short response times, and/or excellent or desirable (suitable) characteristics in terms of luminance, driving voltage, and/or response speed. In other words, self-emissive devices, such as organic light-emitting devices, stand out due to these advantageous properties.

In a light-emitting device, a first electrode is arranged on a substrate and followed sequentially by a hole transport region, an emission layer, an electron transport region, and a second electrode. Holes provided from the first electrode move toward the emission layer through the hole transport region, while electrons provided from the second electrode move toward the emission layer through the electron transport region. These carriers, namely the holes and electrons, recombine in the emission layer to produce excitons. The excitons may transition and decay from an excited state to a ground state, thereby generating light.

### SUMMARY

One or more aspects of embodiments of the present disclosure are directed toward a light-emitting device including a heterocyclic compound, an electronic apparatus including the light-emitting device, and the heterocyclic compound.

Additional aspects will be set forth in part in the description which follows and, in part, will be apparent from the description, or may be learned by practice of the presented embodiments of the disclosure.

According to one or more embodiments of the present disclosure, a light-emitting device includes:
a first electrode;
a second electrode opposite to (e.g., facing) the first electrode;
an interlayer between the first electrode and the second electrode and including an emission layer; and
a heterocyclic compound represented by Formula 1: 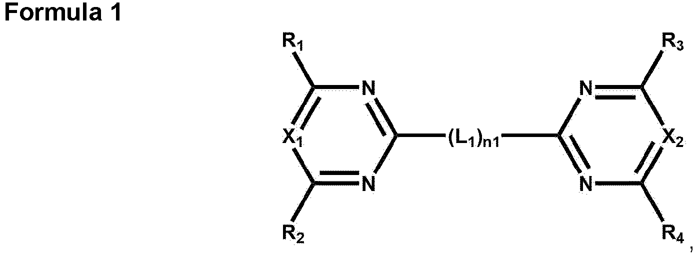
wherein, in Formula 1,
   X₁ is C(R₅) or N,
   X₂ is C(R₆) or N,
   L₁ is a C₃-C₆₀ carbocyclic group unsubstituted or substituted with at least one R₁₀ₐ or a C₁-C₆₀ heterocyclic group unsubstituted or substituted with at least one R₁₀ₐ,
   n1 is an integer from 1 to 5,
   R₁ to R₆ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -C(Q₁)(Q₂)(Q₃), - Si(Q₁)(Q₂)(Q₃), -N(Q₁)(Q₂), -B(Q₁)(Q₂), -C(=O)(Q₁), -S(=O)₂(Q₁), -P(=O)(Q₁)(Q₂), or a group represented by Formula 2: 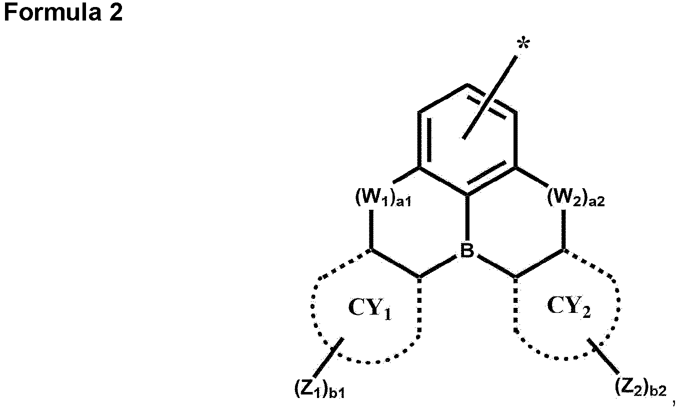
   wherein, in Formula 2,
      ring CY₁ and ring CY₂ are each independently a C₃-C₆₀ carbocyclic group unsubstituted or substituted with at least one R₁₀ₐ or a C₁-C₆₀ heterocyclic group unsubstituted or substituted with at least one R₁₀ₐ,
      W₁ is a single bond, O, S, N(Z₃), C(Z₃)(Z₄), or Si(Z₃)(Z₄),
      W₂ is a single bond, O, S, N(Z₅), C(Z₅)(Z₆), or Si(Z₅)(Z₆),
      a1 and a2 are each independently 0 or 1,
      if (e.g., when) a1 is 0, *-(W₁)ₐ₁-*' does not exist (e.g., is not present),
      if (e.g., when) a2 is 0, *-(W₂)ₐ₂-*' does not exist (e.g., is not present),
      b1 and b2 are each independently an integer from 0 to 10,
      Z₁ to Z₆ are each independently be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -C(Q₁)(Q₂)(Q₃), - Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂),
      * indicates a binding site to a neighboring carbon atom,
      at least one selected from among R₁ to R₄ in Formula 1 is a C₃-C₁₀ alkyl group unsubstituted or substituted with at least one R₁₀ₐ, a C₃-C₁₀ cycloalkyl group unsubstituted or substituted with at least one R₁₀ₐ, or a group represented by Formula 2,
      R₁₀ₐ is:
         deuterium, -F, -Cl, -Br, -I, a hydroxyl group, a cyano group, or a nitro group;
         a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
         a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), - C(=O)(Q₂₁), -S(=O)₂(Q₂₁), -P(=O)(Q₂₁)(Q₂₂), or any combination thereof; or
      -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
      Q₁ to Q₃, Q₁₁ to Q₁₃, Q₂₁ to Q₂₃, and Q₃₁ to Q₃₃ are each independently: hydrogen; deuterium; -F; -Cl; -Br; -I; a hydroxyl group; a cyano group; a nitro group; or a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

According to one or more embodiments of the present disclosure, an electronic apparatus and electronic equipment each include the light-emitting device.

According to one or more embodiments of the present disclosure, provided is a heterocyclic compound represented by Formula 1.

At least some of the above and other features of the invention are set out in the claims.

### BRIEF DESCRIPTION OF DRAWINGS

The accompanying drawings are included to provide a further understanding of the present disclosure and are incorporated in and constitute a part of the present disclosure. The drawings illustrate embodiments of the present disclosure and, together with the description, serve to explain principles of the present disclosure. The above and other aspects, features, and advantages of certain embodiments of the disclosure will be more apparent from the following description taken in conjunction with the accompanying drawings, in which:
FIG. 1 is a schematic view of a structure of a light-emitting device according to one or more embodiments of the present disclosure;
FIG. 2 is a schematic view of a structure of a light-emitting apparatus according to one or more embodiments of the present disclosure;
FIG. 3 is a schematic view of a structure of a light-emitting apparatus according to one or more embodiments of the present disclosure;
FIG. 4 is a schematic view of electronic equipment according to one or more embodiments of the present disclosure;
FIG. 5 is a diagram schematically illustrating an exterior of a vehicle as electronic equipment including a light-emitting device according to one or more embodiments of the present disclosure; and
FIGS. 6A - 6C are each a diagram schematically illustrating an interior of a vehicle according to one or more embodiments of the present disclosure.

### DETAILED DESCRIPTION

Reference will now be made in more detail to one or more embodiments, examples of which are illustrated in the accompanying drawings, wherein like reference numerals refer to like elements throughout the present disclosure, and duplicative descriptions thereof may not be provided for conciseness. In this regard, the presented embodiments may have different forms and should not be construed as being limited to the descriptions set forth herein. Accordingly, embodiments of the present disclosure are merely described in more detail, by referring to the drawings, to explain aspects of the present disclosure. As used herein, the term "and/or" or "or" may include any and all combinations of one or more of the associated listed items. Throughout the disclosure, the expressions such as "at least one of," "one of," and "selected from," when preceding a list of elements, modify the entire list of elements and do not modify the individual elements of the list. For example, "at least one of a, b, or c", "at least one selected from a, b, and c", "at least one selected from among a to c", etc., may indicate only a, only b, only c, both (e.g., simultaneously) a and b, both (e.g., simultaneously) a and c, both (e.g., simultaneously) b and c, all of a, b, and c, or variations thereof.

According to one or more embodiments of the present disclosure, a light-emitting device includes: a first electrode; a second electrode opposite to (e.g., facing) the first electrode; an interlayer between the first electrode and the second electrode and including an emission layer; and a heterocyclic compound represented by Formula 1: 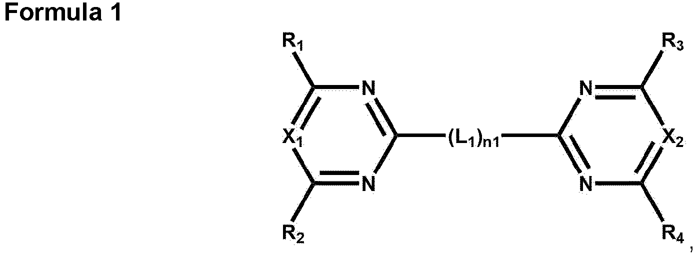 wherein, in Formula 1,
X₁ is C(R₅) or N,
X₂ is C(R₆) or N,
L₁ is a C₃-C₆₀ (*e.g.*, C₃-C₃₀) carbocyclic group unsubstituted or substituted with at least one R₁₀ₐ or a C₁-C₆₀ (*e.g.*, C₁-C₂₀) heterocyclic group unsubstituted or substituted with at least one R₁₀ₐ,
n1 is an integer from 1 to 5,
R₁ to R₆ are each independently hydrogen, deuterium, -F, -Cl, -Br, -I, a hydroxyl group, a cyano group, a nitro group, a C₁-C₆₀ (*e.g.*, C₁-C₂₀) alkyl group unsubstituted or substituted with at least one R₁₀ₐ, a C₂-C₆₀ (*e.g.*, C₂-C₂₀) alkenyl group unsubstituted or substituted with at least one R₁₀ₐ, a C₂-C₆₀ (*e.g.*, C₂-C₂₀) alkynyl group unsubstituted or substituted with at least one R₁₀ₐ, a C₁-C₆₀ (*e.g.*, C₁-C₂₀) alkoxy group unsubstituted or substituted with at least one R₁₀ₐ, a C₃-C₆₀ (*e.g.*, C₃-C₃₀) carbocyclic group unsubstituted or substituted with at least one R₁₀ₐ, a C₁-C₆₀ (*e.g.*, C₁-C₂₀) heterocyclic group unsubstituted or substituted with at least one R₁₀ₐ, -C(Q₁)(Q₂)(Q₃), - Si(Q₁)(Q₂)(Q₃), -N(Q₁)(Q₂), -B(Q₁)(Q₂), -C(=O)(Q₁), -S(=O)₂(Q₁), -P(=O)(Q₁)(Q₂), or a group represented by Formula 2: 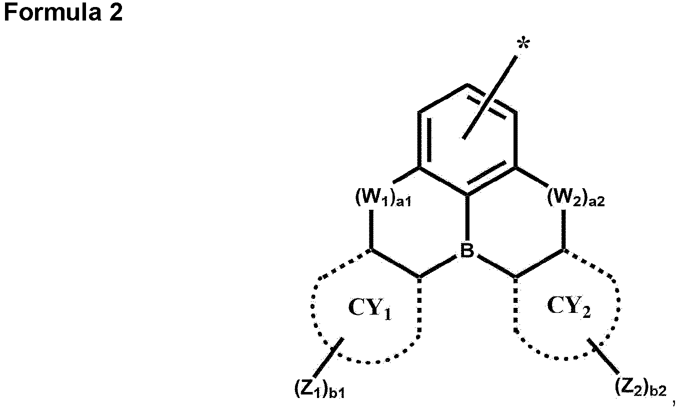
wherein, in Formula 2,
   ring CY₁ and ring CY₂ are each independently a C₃-C₆₀ (*e.g*., C₃-C₃₂) carbocyclic group unsubstituted or substituted with at least one R₁₀ₐ or a C₁-C₆₀ (*e.g.*, C₁-C₂₀) heterocyclic group unsubstituted or substituted with at least one R₁₀ₐ,
   W₁ is a single bond, O, S, N(Z₃), C(Z₃)(Z₄), or Si(Z₃)(Z₄),
   W₂ is a single bond, O, S, N(Z₅), C(Z₅)(Z₆), or Si(Z₅)(Z₆),
   a1 and a2 are each independently 0 or 1,
   if (e.g., when) a1 is 0, *-(W₁)ₐ₁-*' does not exist (e.g., is not present),
   if (e.g., when) a2 is 0, *-(W₂)ₐ₂-*' does not exist (e.g., is not present),
   b1 and b2 are each independently an integer from 0 to 10,
   Z₁ to Z₆ are each independently hydrogen, deuterium, -F, -Cl, -Br, -I, a hydroxyl group, a cyano group, a nitro group, a C₁-C₆₀ (*e.g.*, C₁-C₂₀) alkyl group unsubstituted or substituted with at least one R₁₀ₐ, a C₂-C₆₀ (*e.g.*, C₂-C₂₀) alkenyl group unsubstituted or substituted with at least one R₁₀ₐ, a C₂-C₆₀ (*e.g.*, C₂-C₂₀) alkynyl group unsubstituted or substituted with at least one R₁₀ₐ, a C₁-C₆₀ (*e.g.*, C₁-C₂₀) alkoxy group unsubstituted or substituted with at least one R₁₀ₐ, a C₃-C₆₀ (*e.g.*, C₃-C₃₀) carbocyclic group unsubstituted or substituted with at least one R₁₀ₐ, a C₁-C₆₀ (*e.g.*, C₁-C₂₀) heterocyclic group unsubstituted or substituted with at least one R₁₀ₐ, -C(Q₁)(Q₂)(Q₃), - Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂), * indicates a binding site to a neighboring carbon atom,
   at least one selected from among R₁ to R₄ in Formula 1 is a C₃-C₁₀ alkyl group unsubstituted or substituted with at least one R₁₀ₐ, a C₃-C₁₀ cycloalkyl group unsubstituted or substituted with at least one R₁₀ₐ, or a group represented by Formula 2,
   R₁₀ₐ is:
      deuterium, -F, -Cl, -Br, -I, a hydroxyl group, a cyano group, or a nitro group;
      a C₁-C₆₀ (*e.g.*, C₁-C₂₀) alkyl group, a C₂-C₆₀ (*e.g.*, C₂-C₂₀) alkenyl group, a C₂-C₆₀ (*e.g.*, C₂-C₂₀) alkynyl group, or a C₁-C₆₀ (*e.g.*, C₁-C₂₀) alkoxy group, each unsubstituted or substituted with deuterium, -F, -Cl, -Br, -I, a hydroxyl group, a cyano group, a nitro group, a C₃-C₆₀ (*e*.*g*., C₃-C₃₀) carbocyclic group, a C₁-C₆₀ (*e.g*., C₁-C₂₀) heterocyclic group, a C₆-C₆₀ (*e.g*., C₆-C₃₀) aryloxy group, a C₆-C₆₀ (*e.g*., C₆-C₃₀) arylthio group, a C₇-C₆₀ (*e.g.*, C₇-C₃₀) arylalkyl group, a C₂-C₆₀ (*e.g*., C₂-C₂₀) heteroarylalkyl group, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), - P(=O)(Q₁₁)(Q₁₂), or any combination thereof;
      a C₃-C₆₀ (*e.g*., C₃-C₃₀) carbocyclic group, a C₁-C₆₀ (*e.g.*, C₁-C₂₀) heterocyclic group, a C₆-C₆₀ (*e.g.*, C₆-C₃₀) aryloxy group, a C₆-C₆₀ (*e.g*., C₆-C₃₀) arylthio group, a C₇-C₆₀ (*e.g*., C₇-C₃₀) arylalkyl group, or a C₂-C₆₀ (*e.g*., C₂-C₂₀) heteroarylalkyl group, each unsubstituted or substituted with deuterium, -F, -Cl, -Br, -I, a hydroxyl group, a cyano group, a nitro group, a C₁-C₆₀ (*e.g.*, C₁-C₂₀) alkyl group, a C₂-C₆₀ (*e.g*., C₂-C₂₀) alkenyl group, a C₂-C₆₀ (*e.g*., C₂-C₂₀) alkynyl group, a C₁-C₆₀ (*e.g*., C₁-C₂₀) alkoxy group, a C₃-C₆₀ (*e.g*., C₃-C₃₀) carbocyclic group, a C₁-C₆₀ (*e.g.*, C₁-C₂₀) heterocyclic group, a C₆-C₆₀ (*e.g.*, C₆-C₃₀) aryloxy group, a C₆-C₆₀ (*e.g*., C₆-C₃₀) arylthio group, a C₇-C₆₀ (*e.g*., C₇-C₃₀) arylalkyl group, a C₂-C₆₀ (*e.g.*, C₂-C₂₀) heteroarylalkyl group, - Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), - P(=O)(Q₂₁)(Q₂₂), or any combination thereof; or
      -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
      Q₁ to Q₃, Q₁₁ to Q₁₃, Q₂₁ to Q₂₃, and Q₃₁ to Q₃₃ are each independently: hydrogen; deuterium; -F; -Cl; -Br; -I; a hydroxyl group; a cyano group; a nitro group; or a C₁-C₆₀ (*e.g*., C₁-C₂₀) alkyl group, a C₂-C₆₀ (*e.g*., C₂-C₂₀) alkenyl group, a C₂-C₆₀ (*e.g*., C₂-C₂₀) alkynyl group, a C₁-C₆₀ (*e.g*., C₁-C₂₀) alkoxy group, a C₃-C₆₀ (*e.g*., C₃-C₃₀) carbocyclic group, or a C₁-C₆₀ (*e.g.*, C₁-C₂₀) heterocyclic group, each unsubstituted or substituted with deuterium, -F, a cyano group, a C₁-C₆₀ (*e.g*., C₁-C₂₀) alkyl group, a C₁-C₆₀ (*e.g*., C₁-C₂₀) alkoxy group, a phenyl group, a biphenyl group, or any combination thereof.

In one or more embodiments, the heterocyclic compound may have a symmetrical structure.

In one or more embodiments, the heterocyclic compound may have an asymmetric structure.

In one or more embodiments, the heterocyclic compound may include at least one of deuterium (D), -F, a cyano group, a methyl group, a tert-butyl group, a carbazolyl group, a norbornanyl group, or any combination thereof.

In one or more embodiments, X₁ and X₂ may each be N.

In one or more embodiments, L₁ may be a divalent C₃-C₆₀ (*e.g.*, C₃-C₃₀) carbocyclic group unsubstituted or substituted with at least one R₁₀ₐ or a divalent C₁-C₆₀ (*e.g*., C₁-C₂₀) heterocyclic group unsubstituted or substituted with at least one R₁₀ₐ.

In one or more embodiments, L₁ may be a C₃-C₁₀ cycloalkylene group, a C₁-C₁₀ heterocycloalkylene group, a C₃-C₁₀ cycloalkenylene group, a C₁-C₁₀ heterocycloalkenylene group, a C₆-C₆₀ (*e.g*., C₆-C₃₀) arylene group, a C₁-C₆₀ (*e.g.*, C₁-C₂₀) heteroarylene group, a divalent non-aromatic condensed polycyclic group, or a divalent non-aromatic condensed heteropolycyclic group, each unsubstituted or substituted with at least one R₁₀ₐ.

In one or more embodiments, L₁ may be a C₆-C₃₀ (*e*.*g*., C₆-C₂₀) arylene group unsubstituted or substituted with at least one R₁₀ₐ.

In one or more embodiments, L₁ may be a phenylene group, a naphthylene group, or an anthracenylene group, each unsubstituted or substituted with at least one R₁₀ₐ.

In one or more embodiments, L₁ may be a group represented by any one selected from among Formulae 3-1 to 3-24: 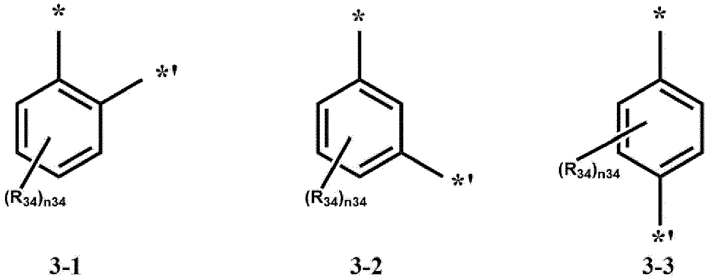 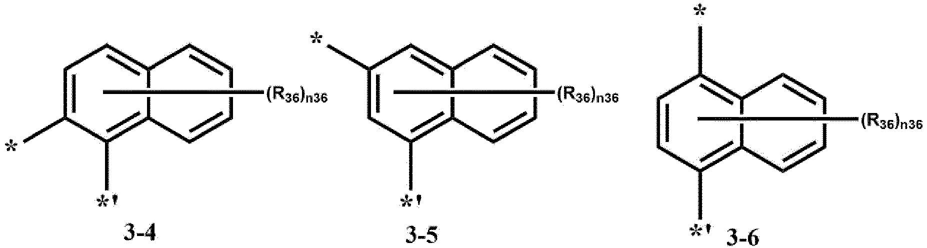 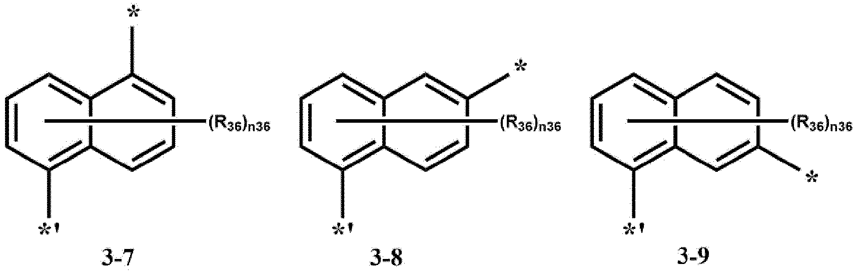 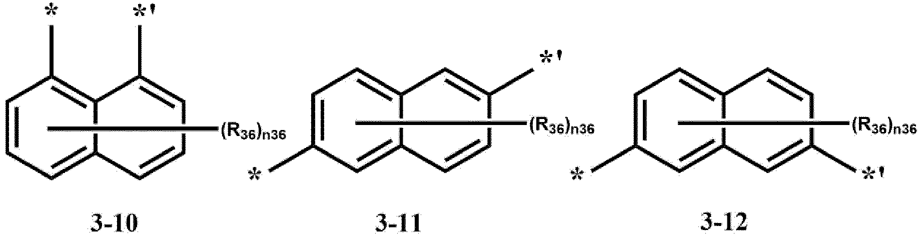 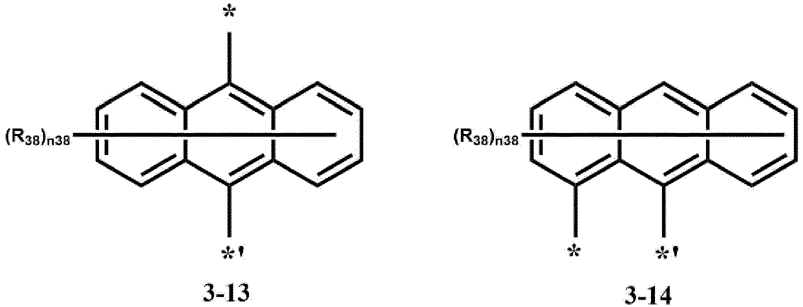 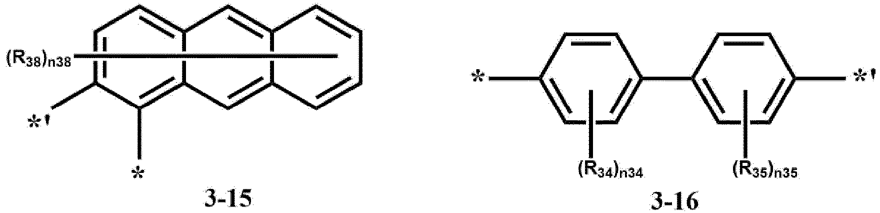 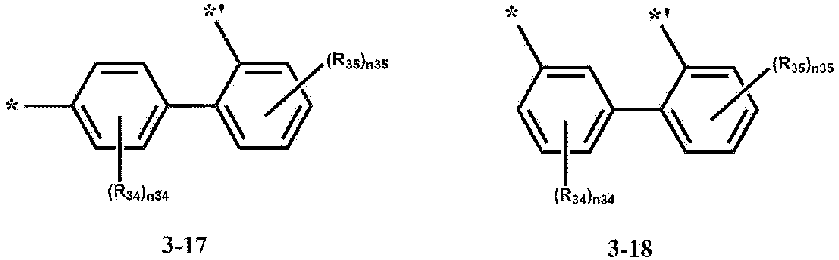 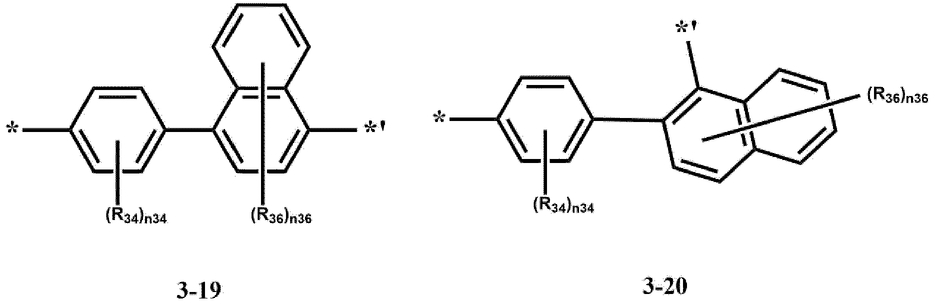 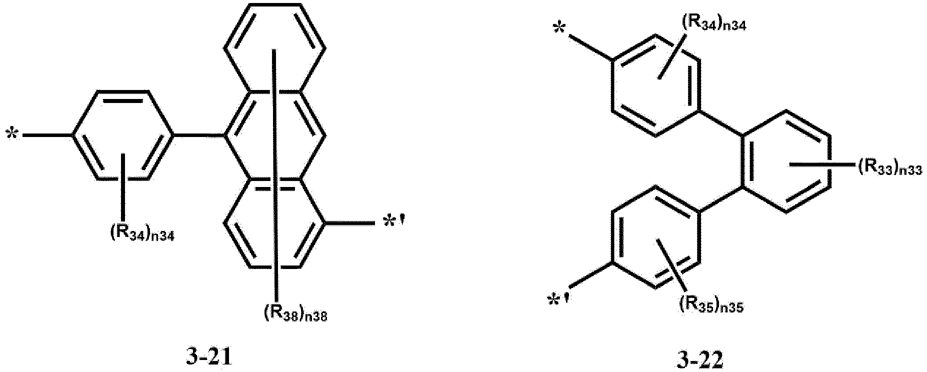 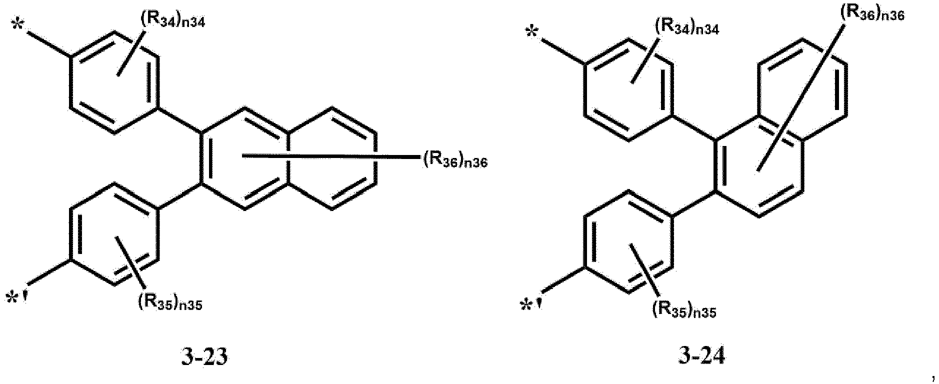 wherein, in Formulae 3-1 to 3-24,
R₃₃ to R₃₆ and R₃₈ are each independently the same as described with respect to R₁,
n33 to n35 are each independently be an integer from 0 to 4,
n36 is an integer from 0 to 6,
n38 is an integer from 0 to 8, and
* and *' each indicate a binding site to a neighboring carbon atom.

In one or more embodiments, n1 may be an integer from 1 to 3.

In one or more embodiments, ring CY₁ and ring CY₂ may each independently be a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, an indenoanthracene group, a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, or an azadibenzofuran group, each unsubstituted or substituted with at least one R₁₀ₐ.

In one or more embodiments, ring CY₁ and ring CY₂ may each independently be a benzene group, a pentalene group, a naphthalene group, an azulene group, an indacene group, an acenaphthylene group, a phenalene group, a phenanthrene group, an anthracene group, a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, or an azadibenzofuran group, each unsubstituted or substituted with at least one R₁₀ₐ.

In one or more embodiments, a1 and a2 may each be 0, or a1 and a2 may each be 1.

In one or more embodiments, W₁ may be O or N(Z₃), and W₂ may be O or N(Z₅).

In one or more embodiments, the group represented by Formula 2 may be a group represented by any one selected from among Formulae 2-1 to 2-4.

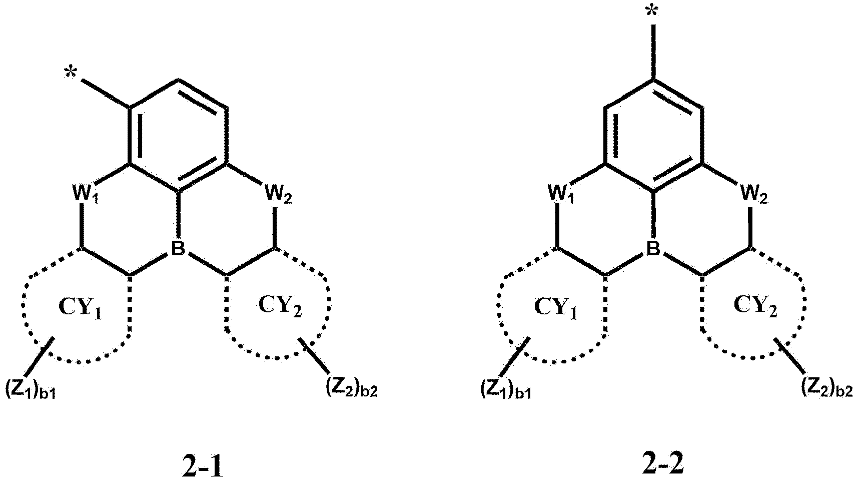

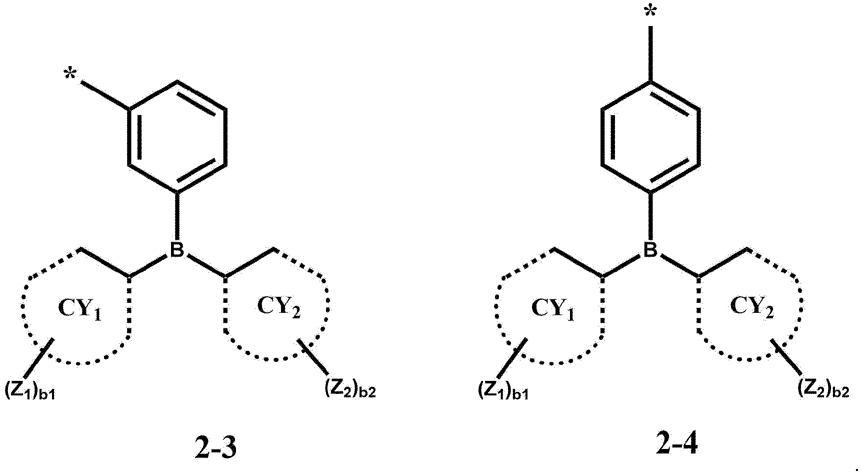

In one or more embodiments, two selected from among R₁ to R₄ may each independently be selected from among a C₃-C₁₀ alkyl group unsubstituted or substituted with at least one R₁₀ₐ, a C₃-C₁₀ cycloalkyl group unsubstituted or substituted with at least one R₁₀ₐ, and a group represented by Formula 2, and
the other groups (e.g., the remaining groups) may not be selected from among C₃-C₁₀ alkyl group unsubstituted or substituted with at least one R₁₀ₐ, a C₃-C₁₀ cycloalkyl group unsubstituted or substituted with at least one R₁₀ₐ, and a group represented by Formula 2.

In one or more embodiments, three selected from among R₁ to R₄ may each independently be selected from among a C₃-C₁₀ alkyl group unsubstituted or substituted with at least one R₁₀ₐ, a C₃-C₁₀ cycloalkyl group unsubstituted or substituted with at least one R₁₀ₐ, and a group represented by Formula 2, and
the other group (e.g., the remaining group) may not be selected from among a C₃-C₁₀ alkyl group unsubstituted or substituted with at least one R₁₀ₐ, a C₃-C₁₀ cycloalkyl group unsubstituted or substituted with at least one R₁₀ₐ, and a group represented by Formula 2.

In one or more embodiments, R₁ to R₄ may each independently be a C₃-C₁₀ alkyl group unsubstituted or substituted with at least one R₁₀ₐ, a C₃-C₁₀ cycloalkyl group unsubstituted or substituted with at least one R₁₀ₐ, or a group represented by Formula 2.

In one or more embodiments, at least one selected from among R₁ to R₄ may be: an *n*-propyl group, an iso-propyl group, an *n*-butyl group, a *sec*-butyl group, an isobutyl group, a *tert*-butyl group, an *n*-pentyl group, a *tert*-pentyl group, a neopentyl group, an isopentyl group, a *sec*-pentyl group, a 3-pentyl group, a *sec*-isopentyl group, an *n*-hexyl group, an iso-hexyl group, a *sec*-hexyl group, a *tert*-hexyl group, an *n*-heptyl group, an iso-heptyl group, a *sec*-heptyl group, a *tert*-heptyl group, an *n*-octyl group, an iso-octyl group, a *sec*-octyl group, a *tert*-octyl group, an *n*-nonyl group, an iso-nonyl group, a *sec*-nonyl group, a *tert*-nonyl group, an *n*-decyl group, an iso-decyl group, a sec-decyl group, a *tert*-decyl group, a cyclopropyl group, a cyclobutyl group, a cyclopentyl group, a cyclohexyl group, a cycloheptyl group, a cyclooctyl group, an adamantanyl group, a norbornanyl group, a bicyclo[1.1.1]pentyl group, a bicyclo[2.1.1]hexyl group, or a bicyclo[2.2.2]octyl group, each unsubstituted or substituted with at least one R₁₀ₐ; or a group represented by Formula 2.

In one or more embodiments, at least one selected from among R₁ to R₄ may be: an *iso*-propyl group, a *sec*-butyl group, an isobutyl group, a *tert*-butyl group, a *tert*-pentyl group, a neopentyl group, an isopentyl group, a *sec*-pentyl group, a 3-pentyl group, a *sec*-isopentyl group, an *iso*-hexyl group, a *sec*-hexyl group, a *tert*-hexyl group, an *iso*-heptyl group, a *sec*-heptyl group, a *tert*-heptyl group, an *iso*-octyl group, a *sec-*octyl group, a *tert*-octyl group, an *iso*-nonyl group, a *sec*-nonyl group, a *tert*-nonyl group, an *iso*-decyl group, a *sec*-decyl group, a *tert*-decyl group, a cyclohexyl group, a cycloheptyl group, a cyclooctyl group, or an adamantanyl group, each unsubstituted or substituted with at least one R₁₀ₐ; or a group represented by Formula 2.

In one or more embodiments, at least one selected from among R₁ to R₄ may be: an *iso*-propyl group, a *sec*-butyl group, an isobutyl group, a *tert*-butyl group, or a cyclohexyl group, each unsubstituted or substituted with at least one R₁₀ₐ; or a group represented by Formula 2.

In one or more embodiments, R₁ to R₆ may each independently be hydrogen, deuterium, -F, a cyano group, a C₁-C₂₀ (*e.g*., C₁-C₁₀) alkyl group unsubstituted or substituted with at least one R₁₀ₐ, a C₃-C₆₀ (*e.g*., C₃-C₃₀) carbocyclic group unsubstituted or substituted with at least one R₁₀ₐ, or a C₁-C₆₀ (*e.g.*, C₁-C₂₀) heterocyclic group unsubstituted or substituted with at least one R₁₀ₐ.

In one or more embodiments, R₁ to R₆ may each independently be: hydrogen, deuterium, -F, or a cyano group;
a C₁-C₂₀ (*e.g.*, C₁-C₁₀) alkyl group unsubstituted or substituted with deuterium, -F, a cyano group, or any combination thereof; or
a benzene group, a naphthalene group, a biphenyl group, a terphenyl group, a cyclohexane group, a pyridine group, a dibenzofuran group, a dibenzothiophene group, a carbazole group, an azadibenzofuran group, an azadibenzothiophene group, or an azacarbazole group, each unsubstituted or substituted with deuterium, -F, a cyano group, a C₁-C₂₀ (*e.g*., C₁-C₁₀) alkyl group, a deuterated C₁-C₂₀ (*e.g.*, C₁-C₁₀) alkyl group, a fluorinated C₁-C₂₀ (*e.g*., C₁-C₁₀) alkyl group, a phenyl group, a deuterated phenyl group, a fluorinated phenyl group, a (C₁-C₂₀ alkyl)phenyl group, a biphenyl group, a terphenyl group, a dibenzofuranyl group, a dibenzothiophenyl group, a carbazolyl group, a fluorenyl group, a dibenzosilolyl group, a tetralin group, or any combination thereof.

In one or more embodiments, R₁ to R₆ may each independently be:
hydrogen, deuterium, -F, a cyano group;
a methyl group, an ethyl group, an *n*-propyl group, an isopropyl group, an *n-*butyl group, an isobutyl group, a *sec*-butyl group, a *tert*-butyl group, an n-pentyl group, an isopentyl group, a *sec*-pentyl group, a *tert*-pentyl group, a 2-methylbutyl group, a 2,2-dimethylpropyl group, a 1-ethylpropyl group, or a 1,2-dimethylpropyl group, each unsubstituted or substituted with deuterium, -F, -Cl, -Br, -I, a hydroxyl group, a cyano group, or a nitro group; or
a benzene group, a naphthalene group, a biphenyl group, a terphenyl group, a cyclohexane group, a pyridine group, a dibenzofuran group, a dibenzothiophene group, a carbazole group, an azadibenzofuran group, an azadibenzothiophene group, or an azacarbazole group, each unsubstituted or substituted with deuterium, -F, -Cl, - Br, -I, a hydroxyl group, a cyano group, a nitro group, a methyl group, an ethyl group, an *n*-propyl group, an *iso*-propyl group, an *n*-butyl group, an isobutyl group, a *sec*-butyl group, a *tert*-butyl group, an *n*-pentyl group, an isopentyl group, a *sec*-pentyl group, a *tert*-pentyl group, a 2-methylbutyl group, a 2,2-dimethylpropyl group, a 1-ethylpropyl group, a 1,2-dimethylpropyl group, a phenyl group, a biphenyl group, a terphenyl group, a tetralin group, or any combination thereof.

In one or more embodiments, R₁ to R₆ may each independently be: hydrogen, deuterium, -F, -Cl, -Br, -I, a cyano group, or a C₁-C₂₀ (*e.g*., C₁-C₁₀) alkyl group;
a C₁-C₂₀ (*e.g*., C₁-C₁₀) alkyl group substituted with at least one selected from among deuterium, -F, -Cl, -Br, -I, -CD₃, -CD₂H, -CDH₂, -CF₃, -CF₂H, -CFH₂, a cyano group, a nitro group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a naphthyl group, a pyridinyl group, and a pyrimidinyl group;
a phenyl group, a naphthyl group, a biphenyl group, a terphenyl group, a cyclohexyl group, a pyridine group, a dibenzofuranyl group, a dibenzothiophenyl group, a carbazolyl group, an azadibenzofuranyl group, an azadibenzothiophenyl group, or an azacarbazolyl group, each unsubstituted or substituted with deuterium, -F, -Cl, -Br, -I, - CD₃, -CD₂H, -CDH₂, -CF₃, -CF₂H, -CFH₂, a tert-butyl group, a cyano group, a C₁-C₂₀ (*e.g.*, C₁-C₁₀) alkyl group, a phenyl group, a biphenyl group, a terphenyl group, a tetralin group, a (C₁-C₁₀ alkyl)phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, -Si(Q₃₁)(Q₃₂)(Q₃₃), -N(Q₃₁)(Q₃₂), -B(Q₃₁)(Q₃₂), or any combination thereof; or
-Si(Q₁)(Q₂)(Q₃), -N(Q₁)(Q₂), or -B(Q₁)(Q₂), and
Q₁ to Q₃ and Q₃₁ to Q₃₃ may each independently be:
-CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂; or
an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, a naphthyl group, a pyridinyl group, a pyrimidinyl group, a pyridazinyl group, a pyrazinyl group, or a triazinyl group, each unsubstituted or substituted with at least one of deuterium, a C₁-C₁₀ alkyl group, a phenyl group, a biphenyl group, a pyridinyl group, a pyrimidinyl group, a pyridazinyl group, a pyrazinyl group, or a triazinyl group.

In one or more embodiments, R₁ to R₆ may each independently be:
hydrogen, deuterium, -F, or a cyano group;
a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a tert-pentyl group, a 2-methylbutyl group, a 2,2-dimethylpropyl group, a 1-ethylpropyl group, or a 1,2-dimethylpropyl group, each unsubstituted or substituted with deuterium, -F, -Cl, -Br, -I, a hydroxyl group, a cyano group, or a nitro group; or
a benzene group, a naphthalene group, a biphenyl group, a terphenyl group, a cyclohexane group, a pyridine group, a dibenzofuran group, a dibenzothiophene group, a carbazole group, an azadibenzofuran group, an azadibenzothiophene group, or an azacarbazole group, each unsubstituted or substituted with deuterium, -F, -Cl, - Br, -I, a hydroxyl group, a cyano group, a nitro group, a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, a 2-methylbutyl group, a 2,2-dimethylpropyl group, a 1-ethylpropyl group, a 1, 2-dimethylpropyl group, a phenyl group, a biphenyl group, a terphenyl group, a tetralin group, or any combination thereof.

In one or more embodiments, Z₁ to Z₆ may each independently be hydrogen, deuterium, -F, a cyano group, a C₁-C₂₀ (*e.g*., C₁-C₁₀) alkyl group unsubstituted or substituted with at least one R₁₀ₐ, a C₃-C₆₀ (*e.g.*, C₃-C₃₀) carbocyclic group unsubstituted or substituted with at least one R₁₀ₐ, or a C₁-C₆₀ (*e.g*., C₁-C₂₀) heterocyclic group unsubstituted or substituted with at least one R₁₀ₐ.

In one or more embodiments, Z₁ to Z₆ may each independently be:
hydrogen, deuterium, -F, or a cyano group;
a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a tert-pentyl group, a 2-methylbutyl group, a 2,2-dimethylpropyl group, a 1-ethylpropyl group, or a 1,2-dimethylpropyl group, each unsubstituted or substituted with deuterium, -F, -Cl, -Br, -I, a hydroxyl group, a cyano group, or a nitro group; or
a C₃-C₆₀ (*e.g*., C₃-C₃₀) carbocyclic group or a C₁-C₆₀ (*e.g*., C₁-C₂₀) heterocyclic group, each unsubstituted or substituted with deuterium, -F, -Cl, -Br, -I, a hydroxyl group, a cyano group, a nitro group, a methyl group, an ethyl group, an *n-*propyl group, an *iso*-propyl group, an *n*-butyl group, an isobutyl group, a *sec*-butyl group, a *tert*-butyl group, an n-pentyl group, an isopentyl group, a *sec*-pentyl group, a *tert*-pentyl group, a 2-methylbutyl group, a 2,2-dimethylpropyl group, a 1-ethylpropyl group, a 1,2-dimethylpropyl group, a phenyl group, a biphenyl group, a terphenyl group, a tetralin group, or any combination thereof.

In one or more embodiments, the heterocyclic compound may be any one selected from among Compounds 1 to 468.

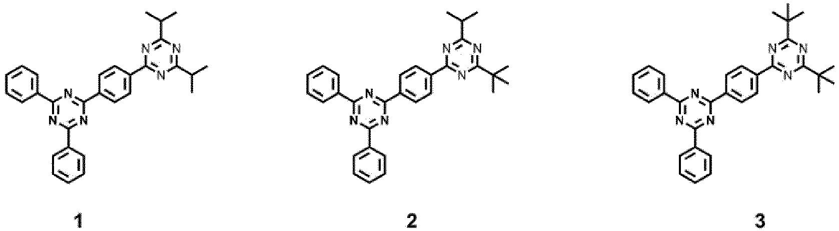

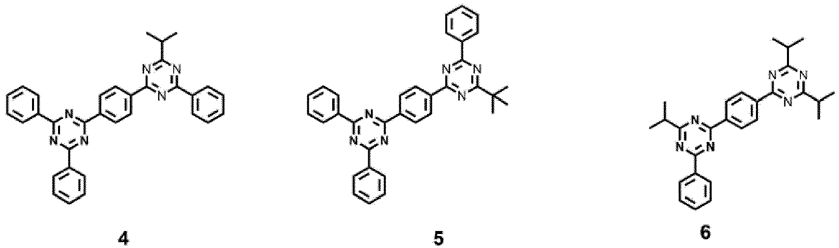

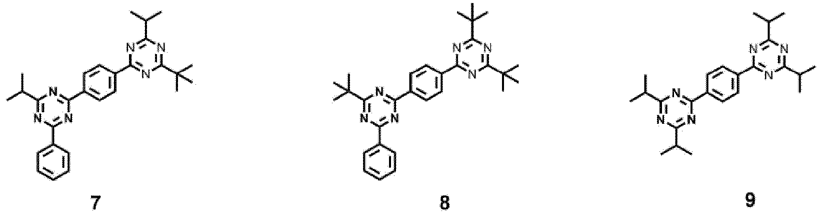

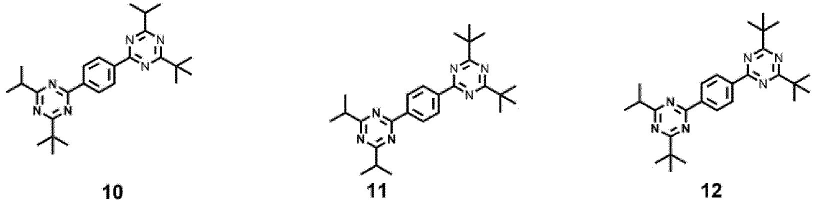

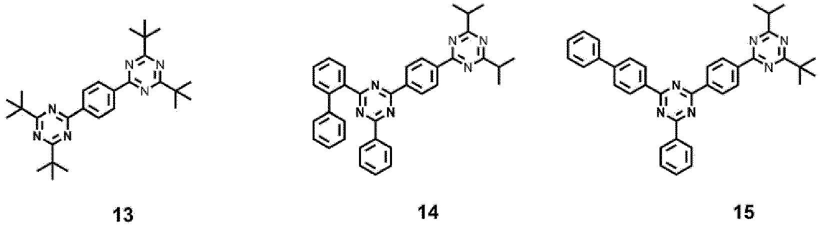

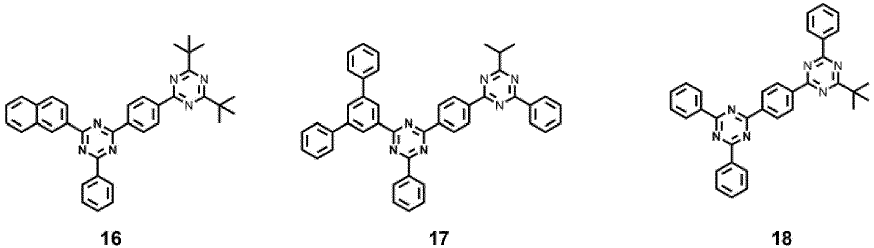

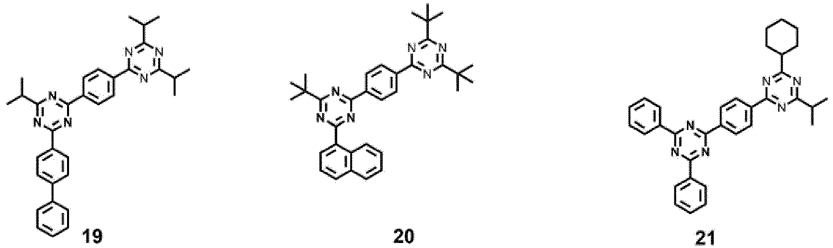

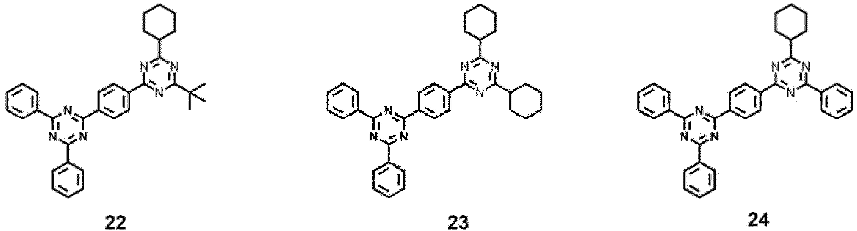

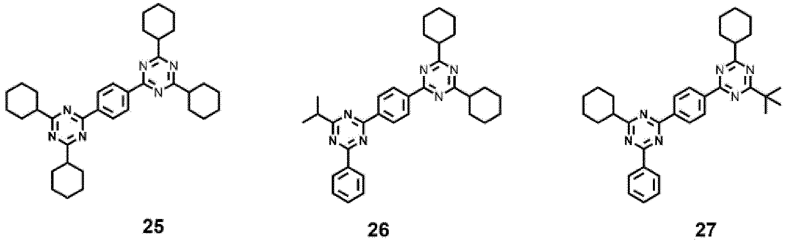

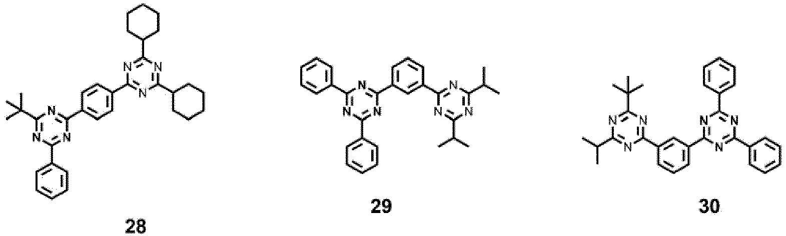

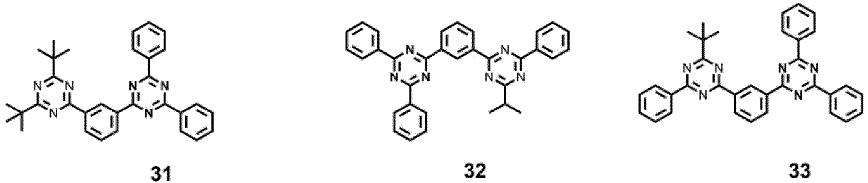

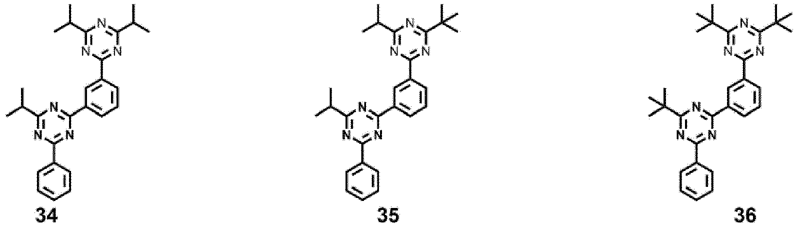

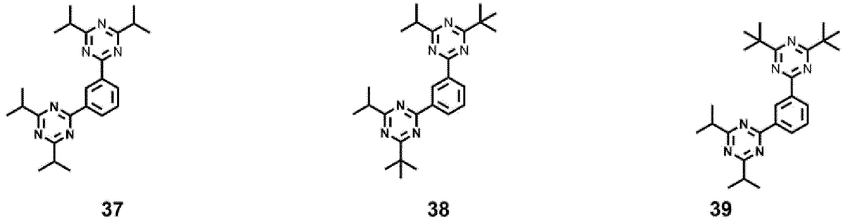

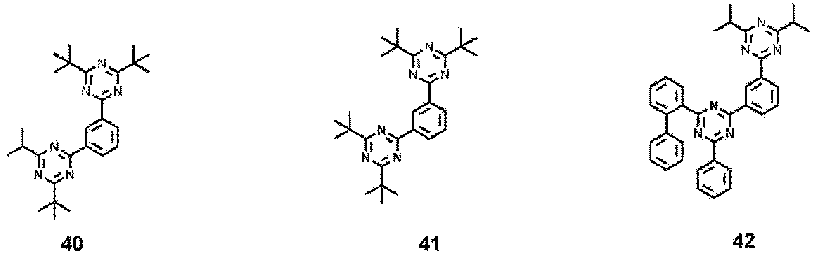

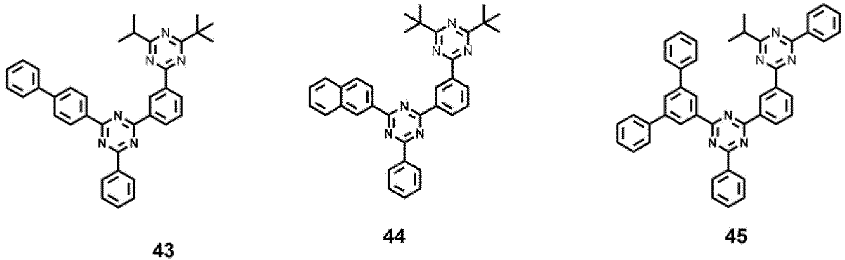

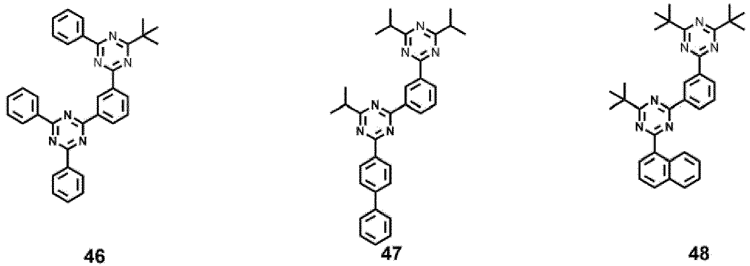

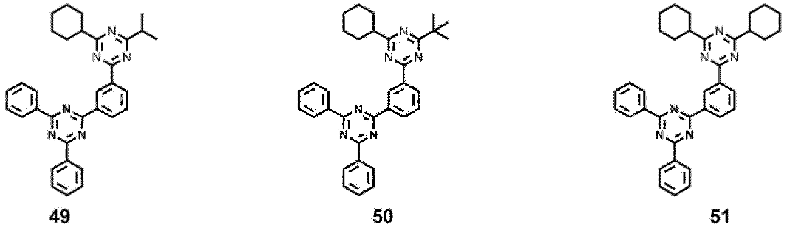

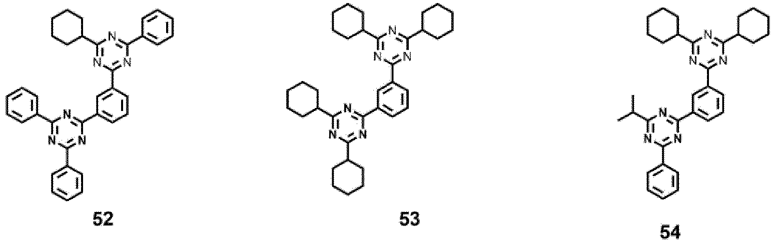

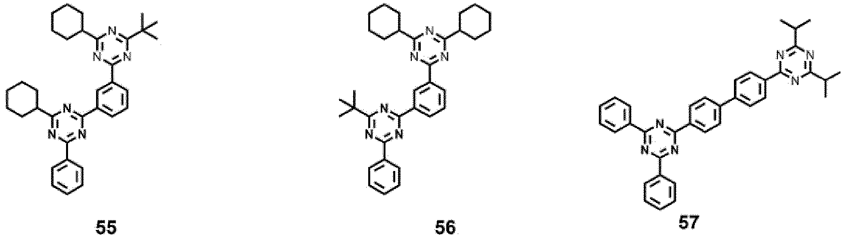

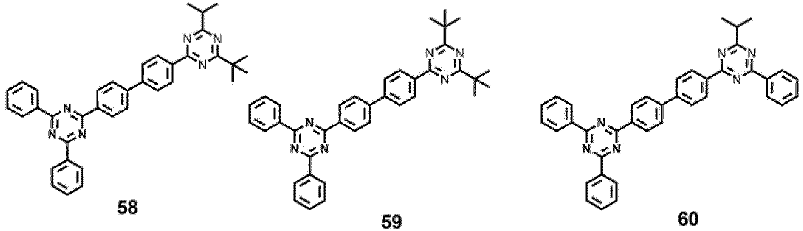

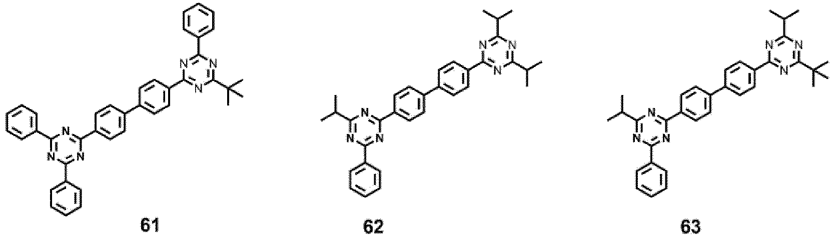

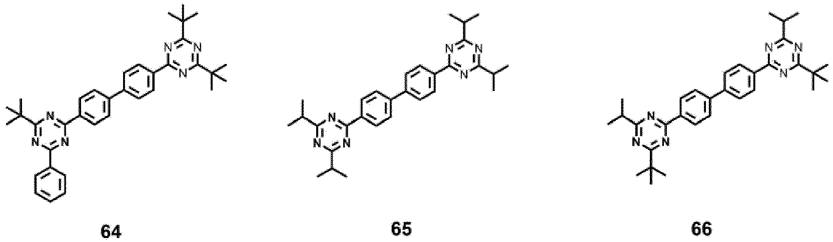

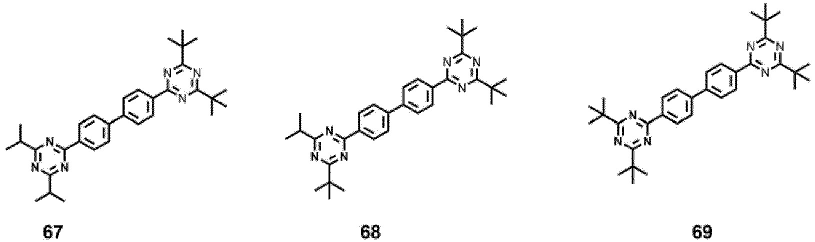

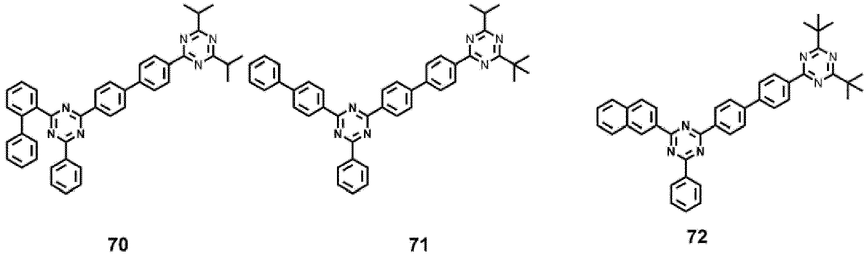

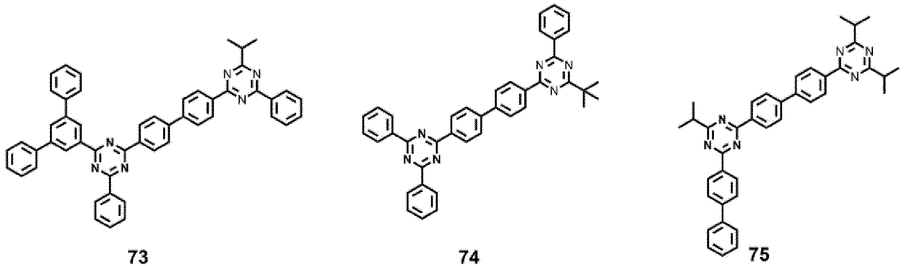

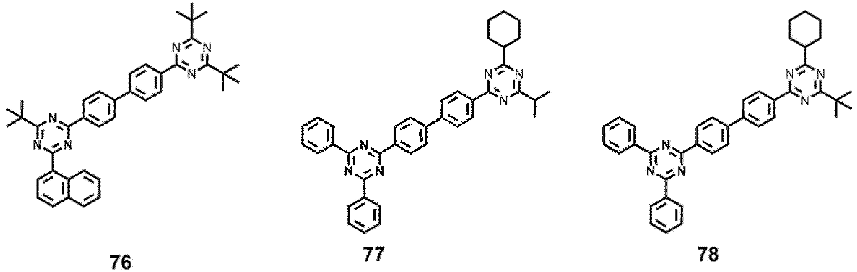

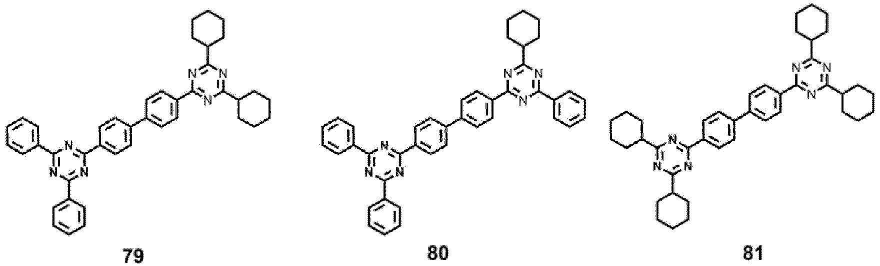

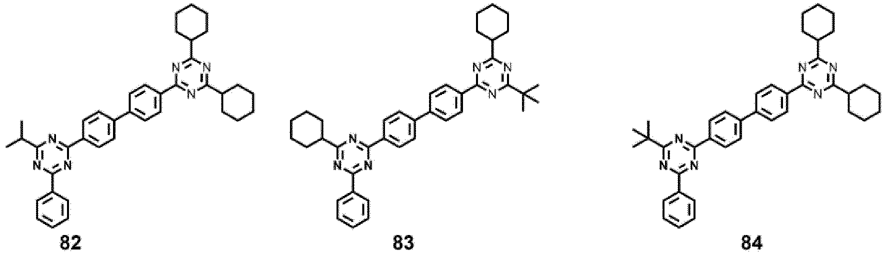

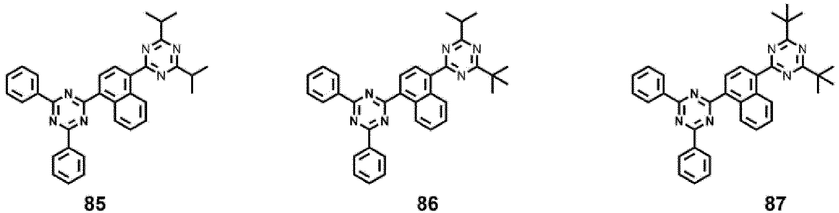

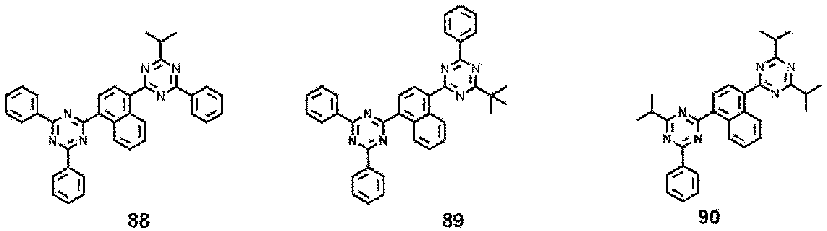

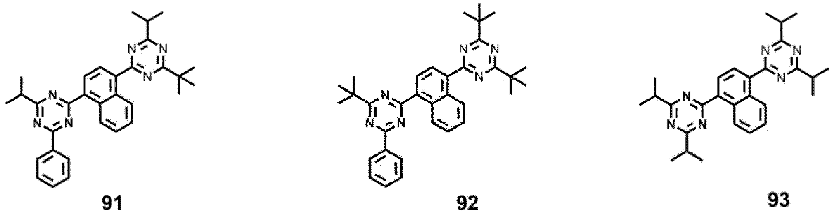

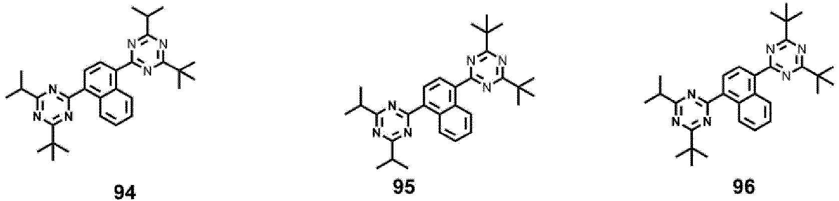

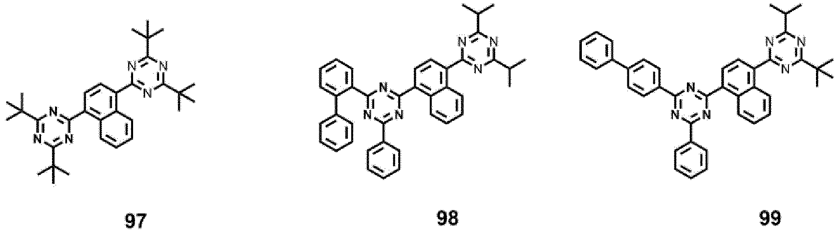

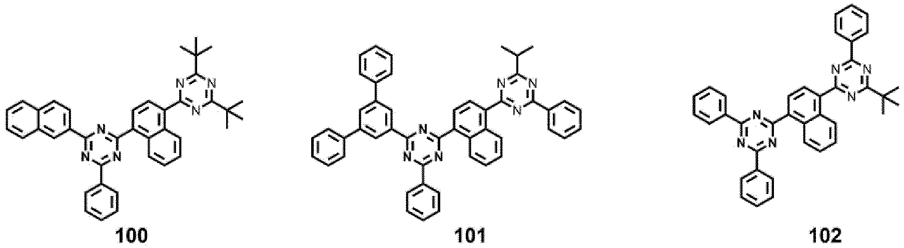

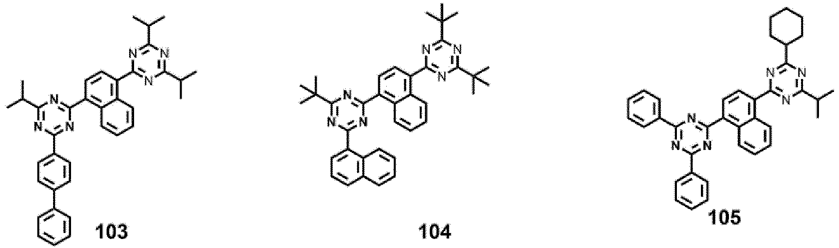

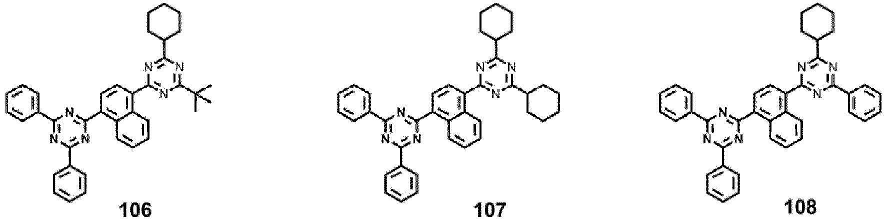

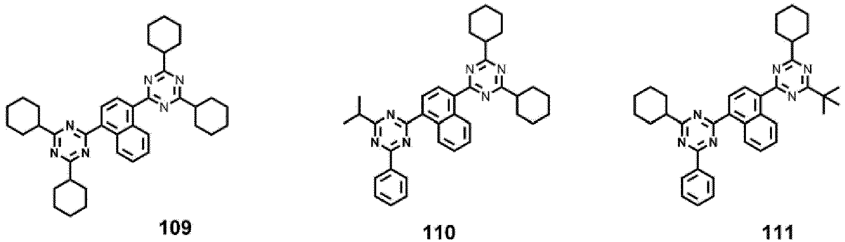

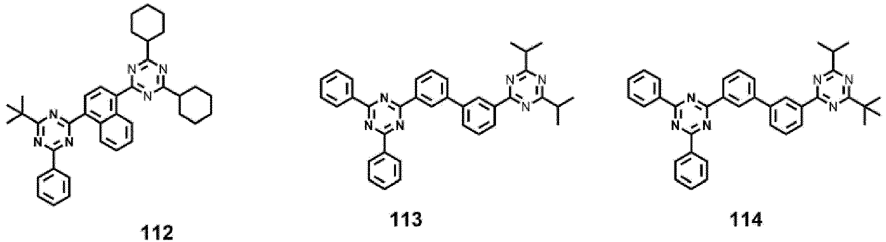

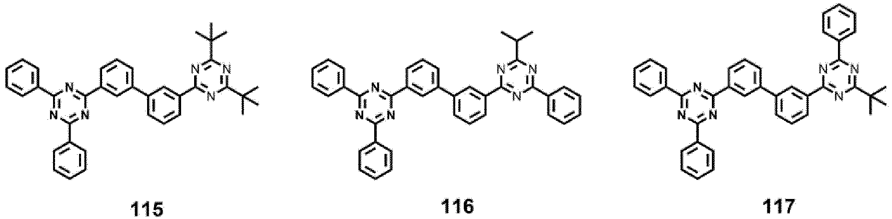

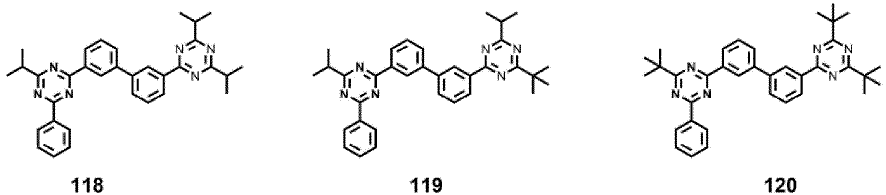

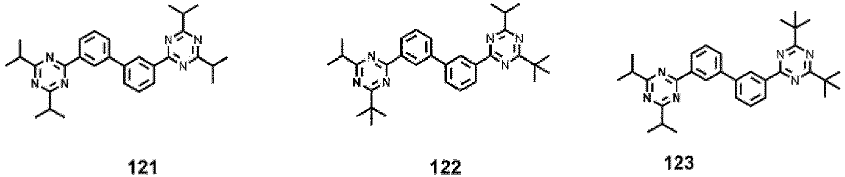

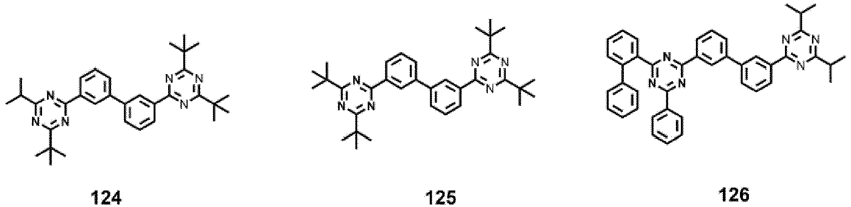

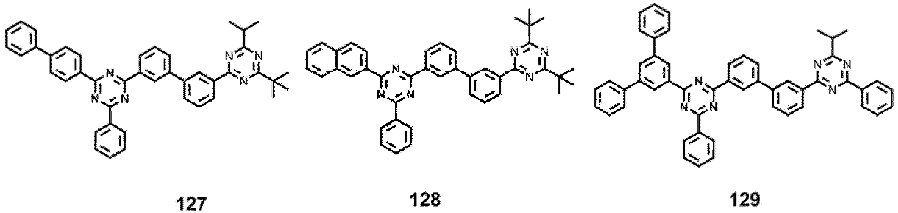

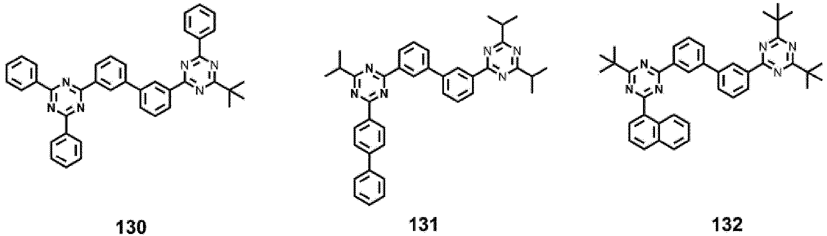

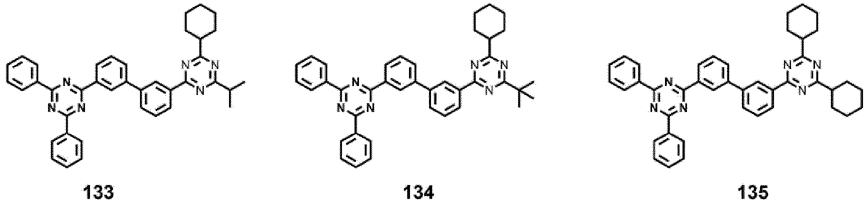

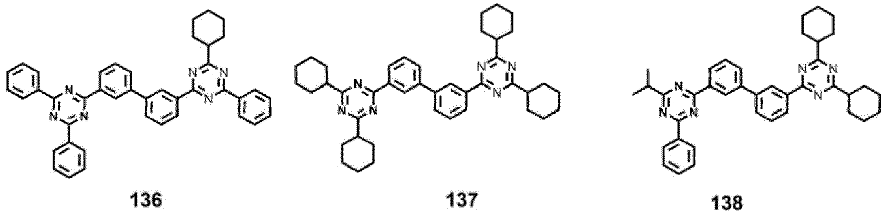

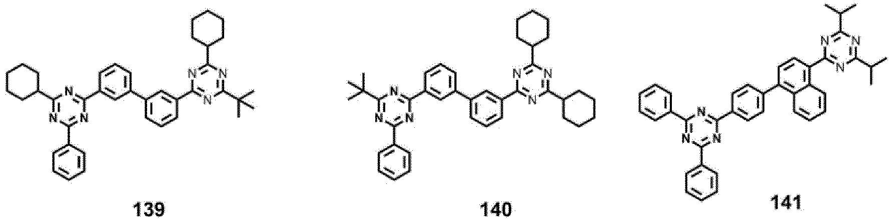

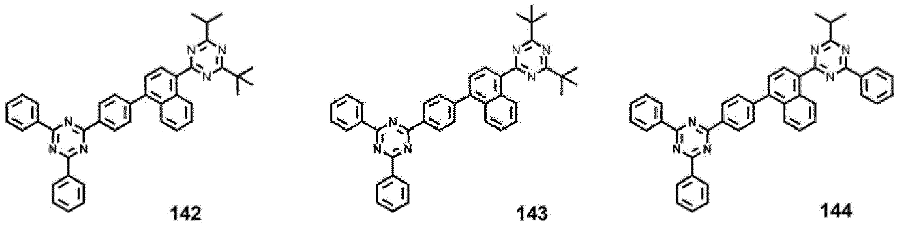

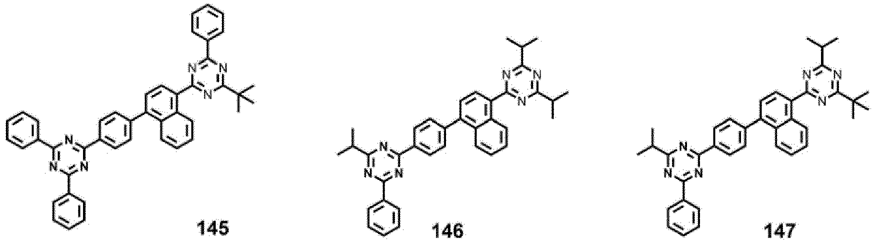

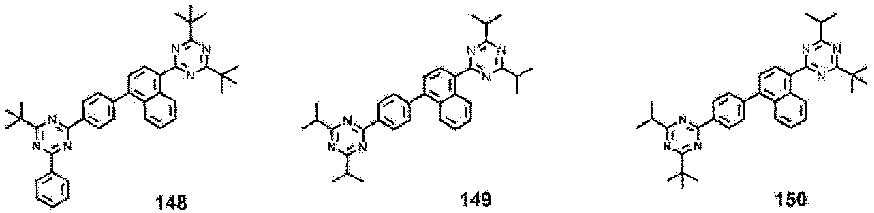

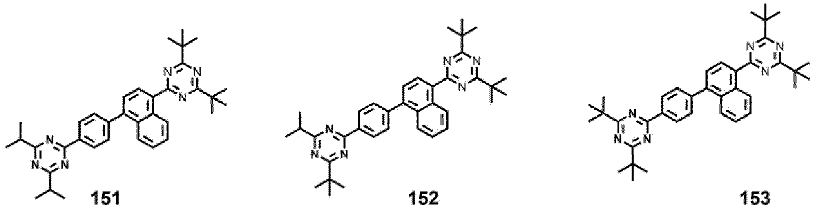

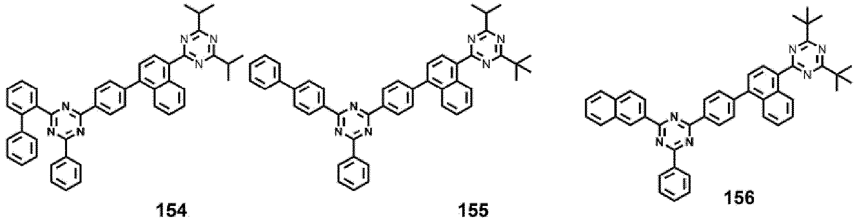

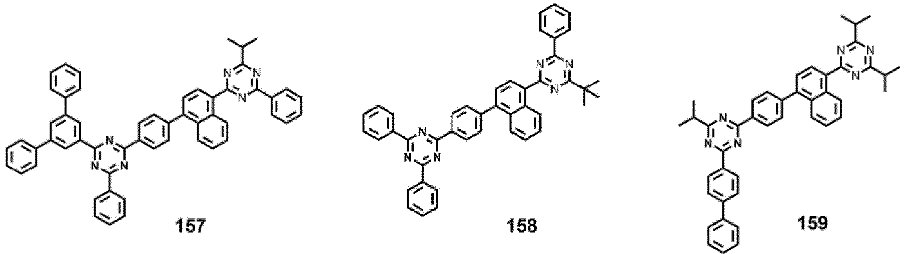

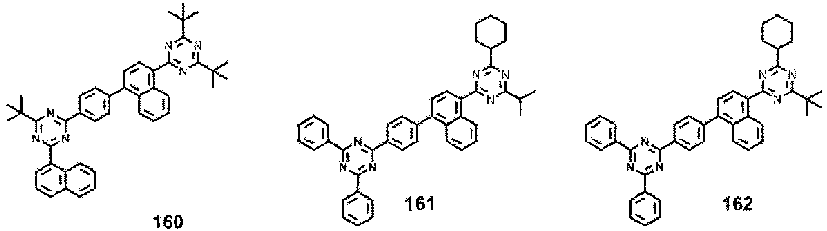

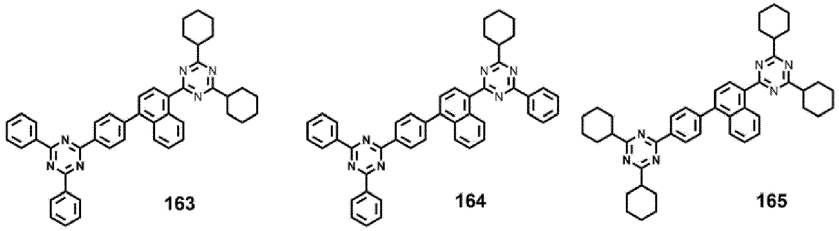

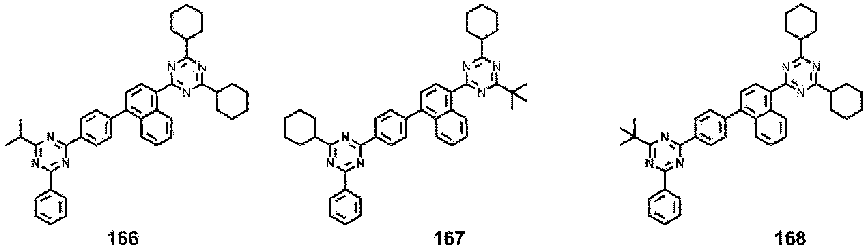

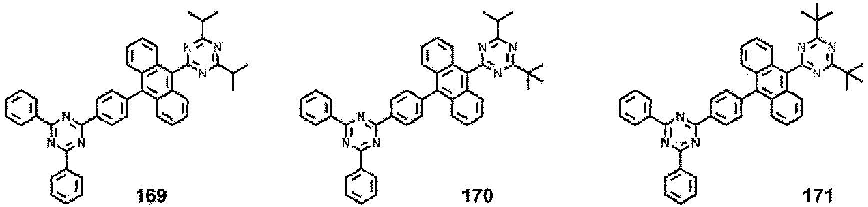

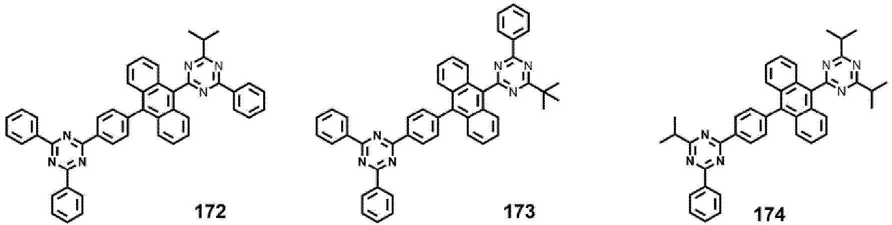

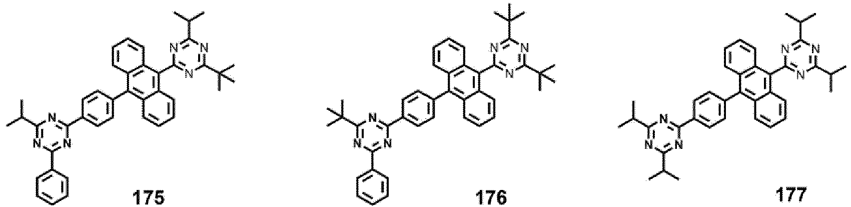

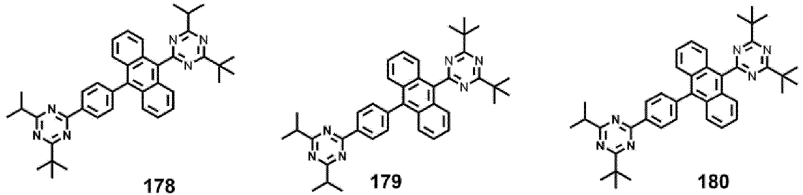

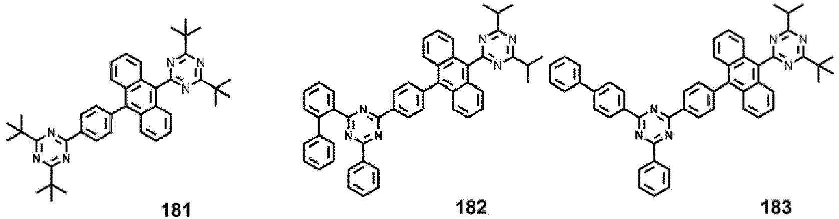

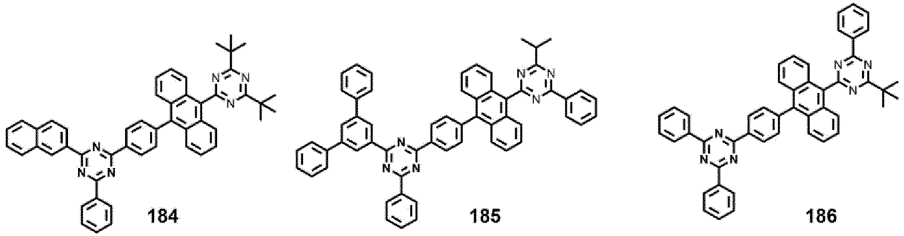

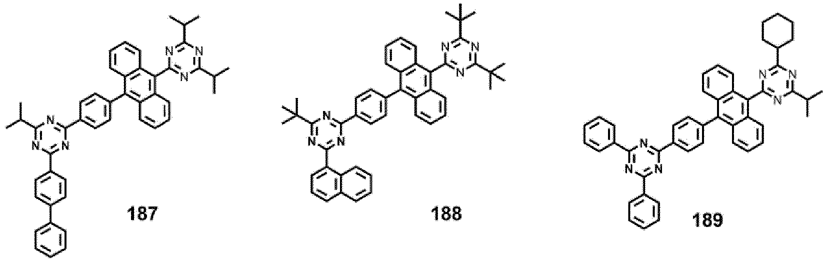

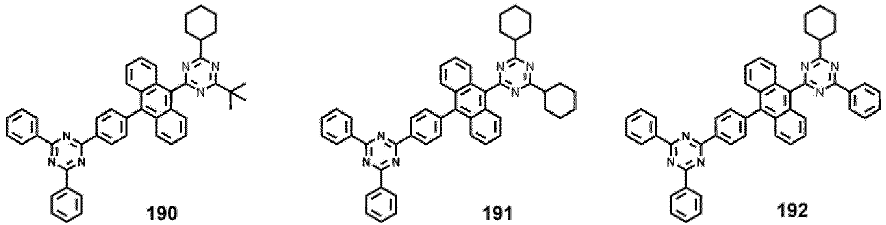

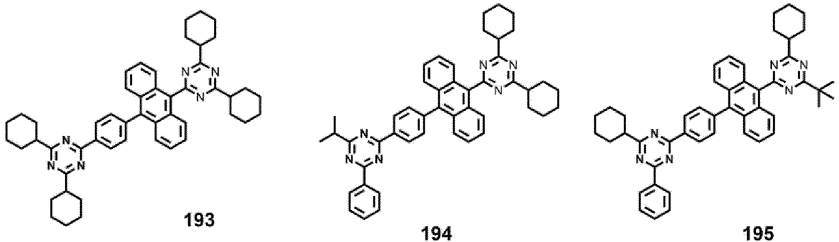

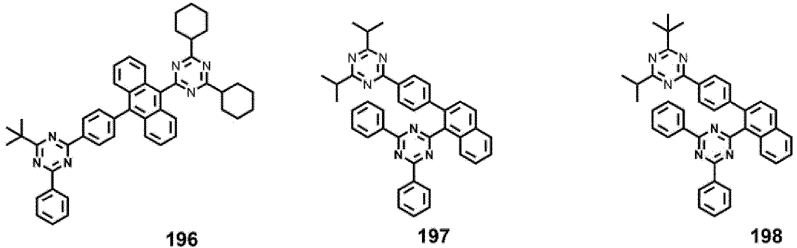

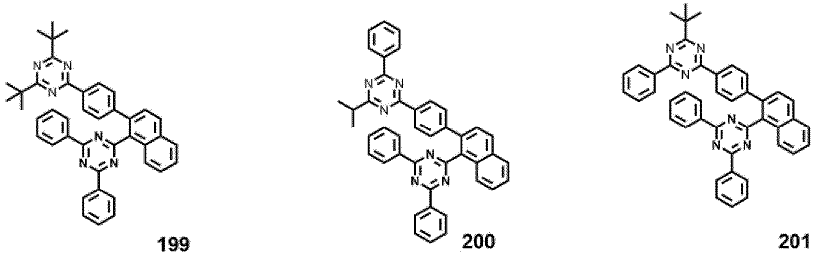

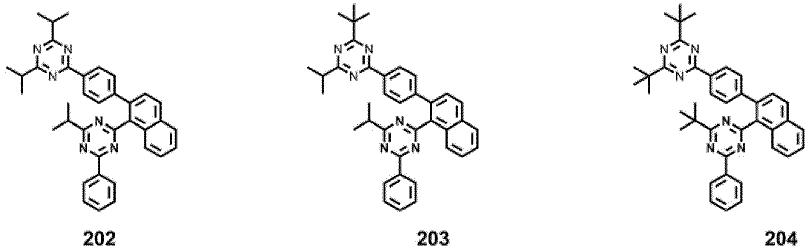

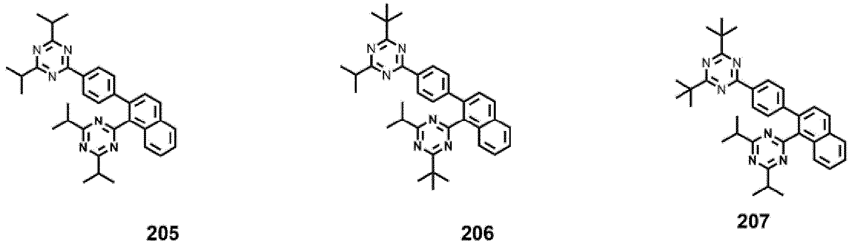

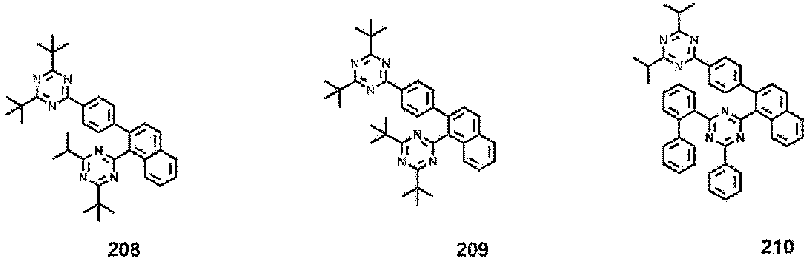

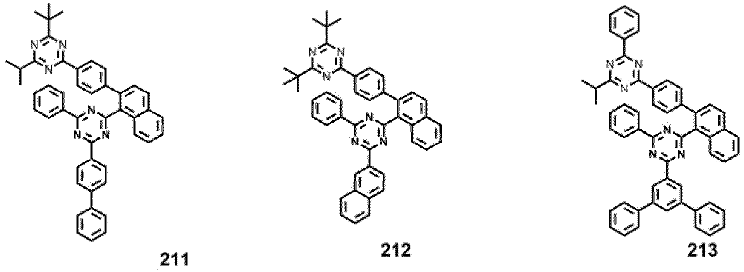

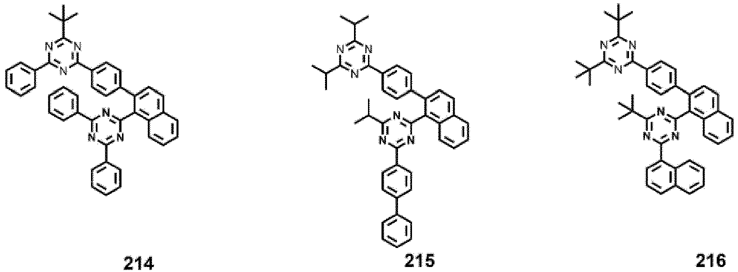

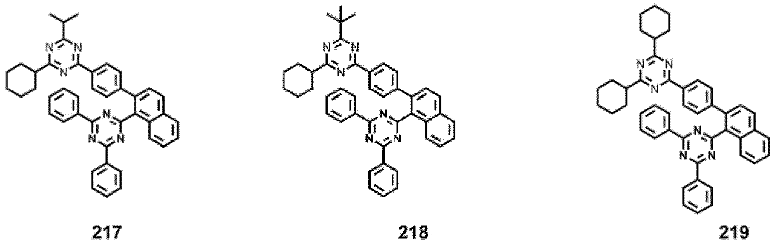

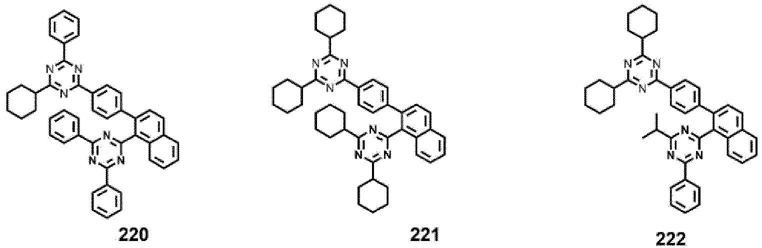

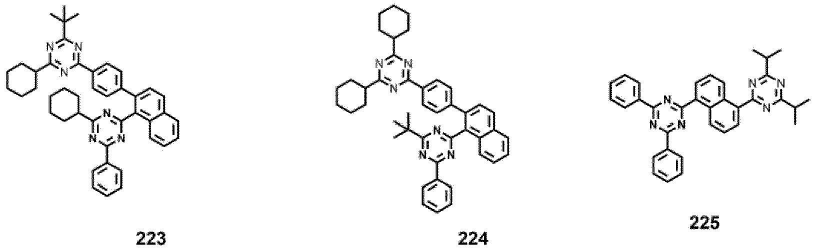

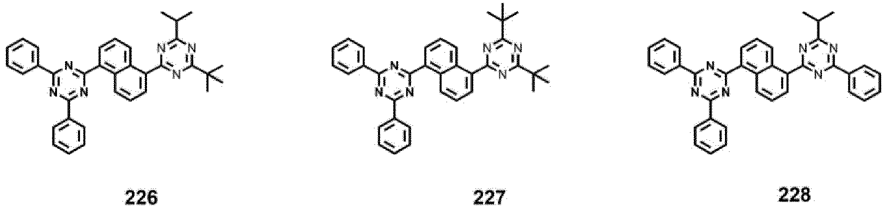

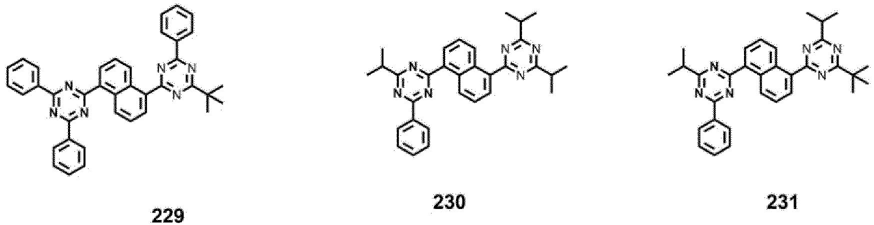

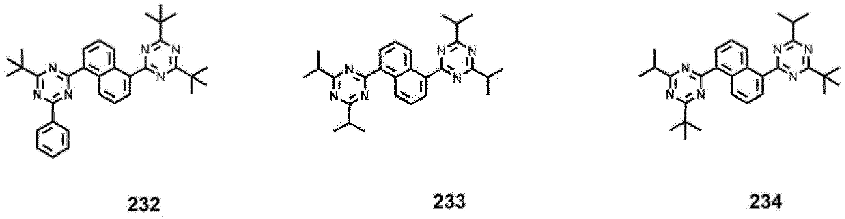

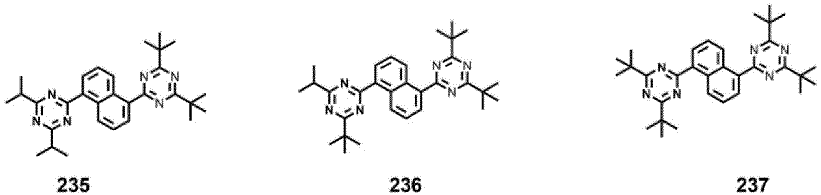

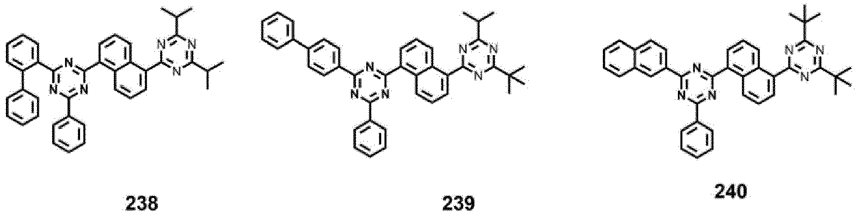

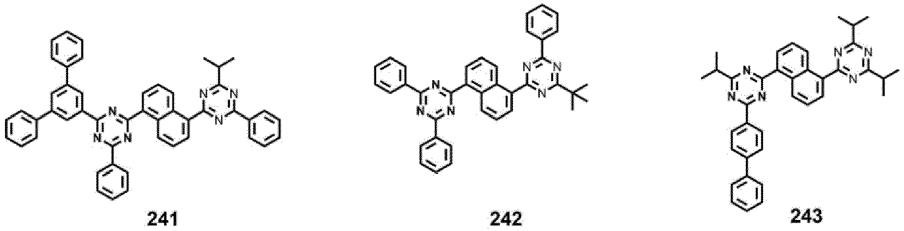

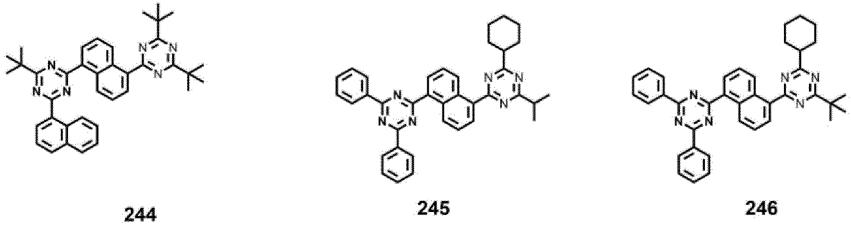

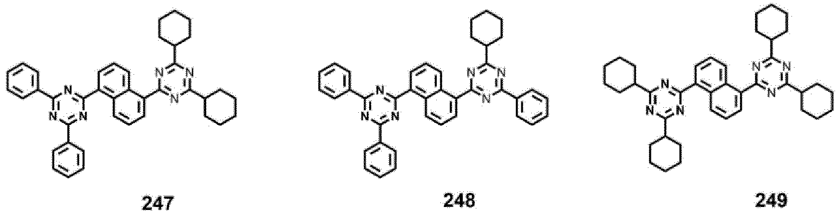

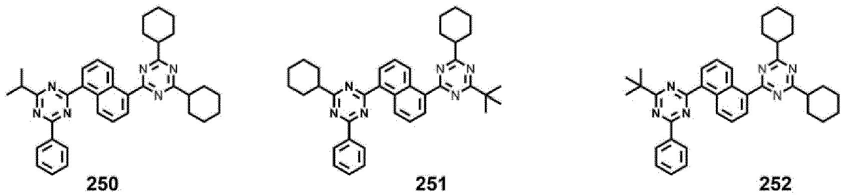

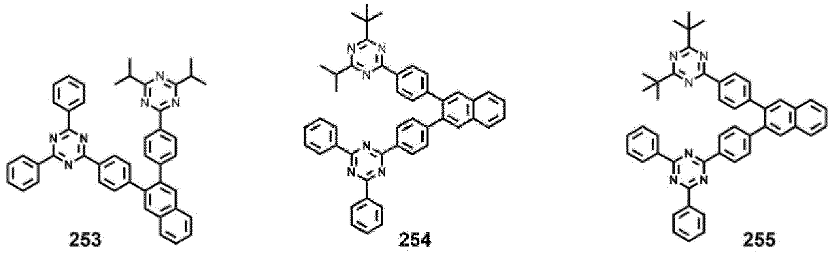

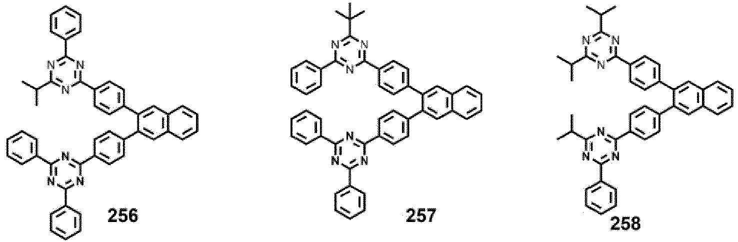

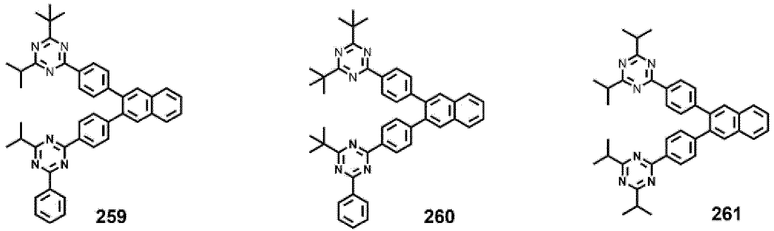

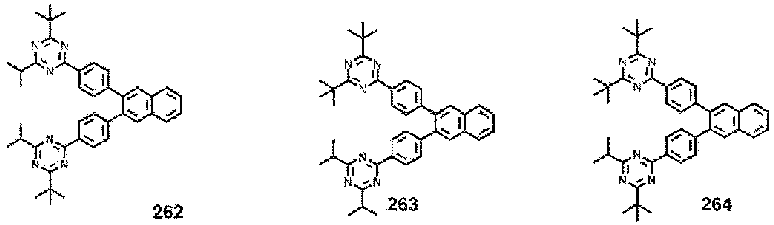

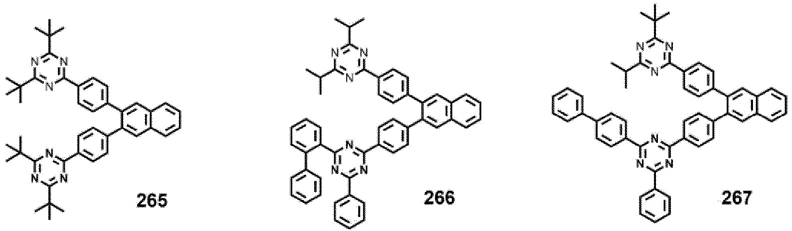

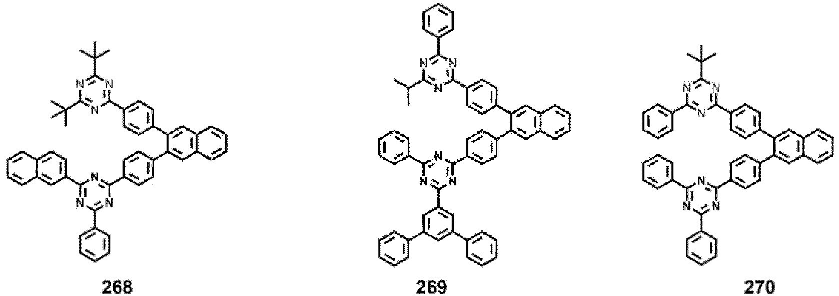

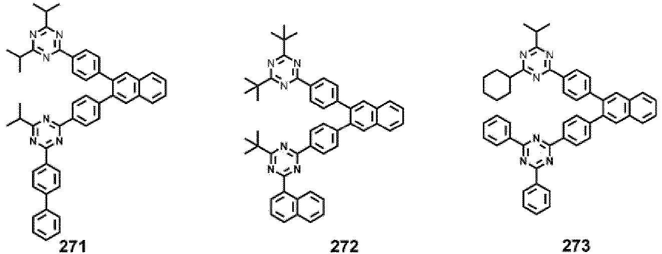

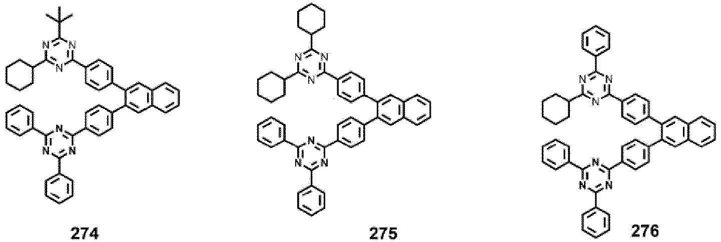

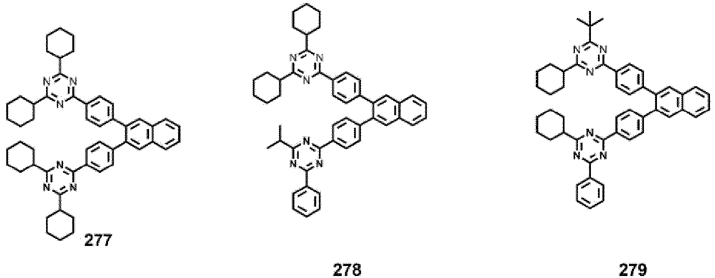

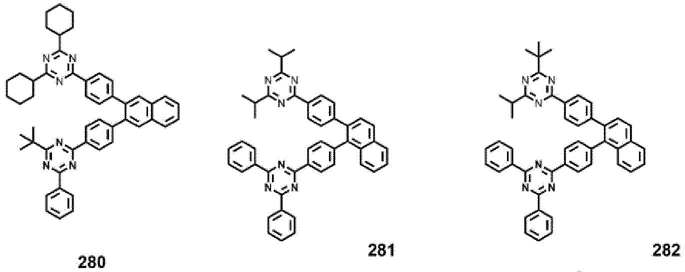

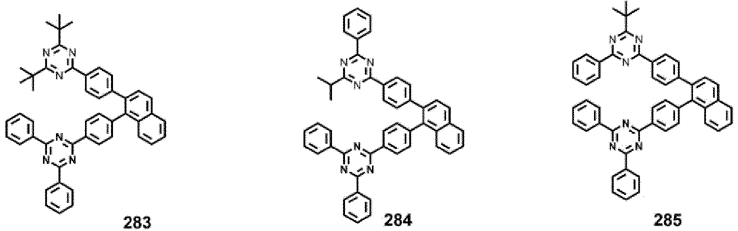

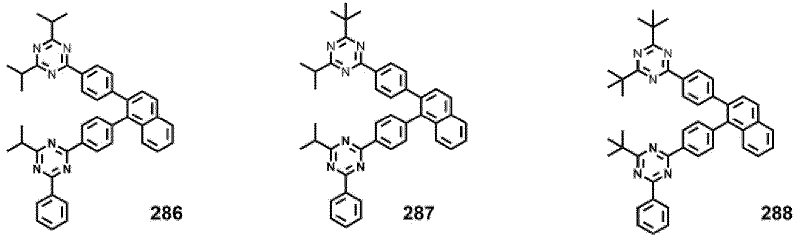

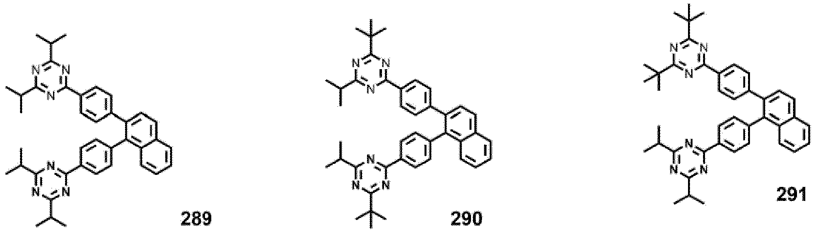

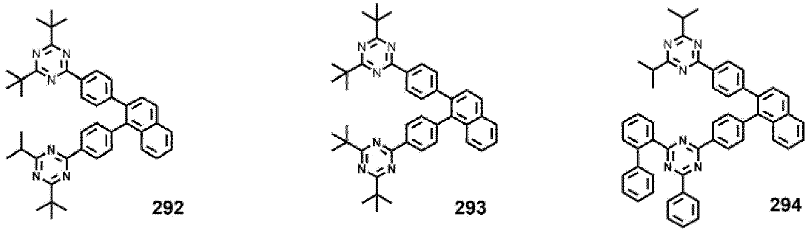

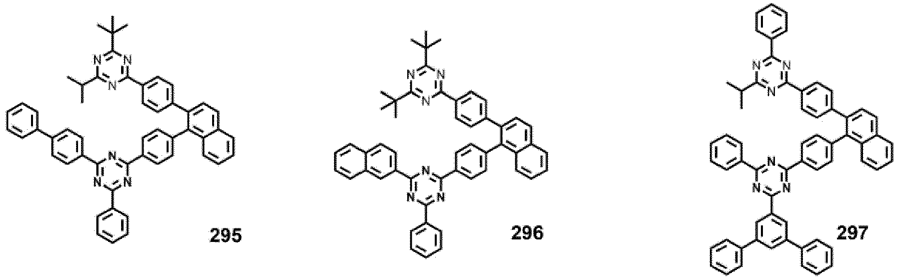

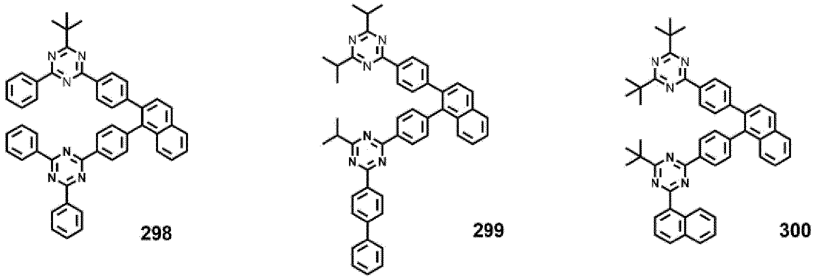

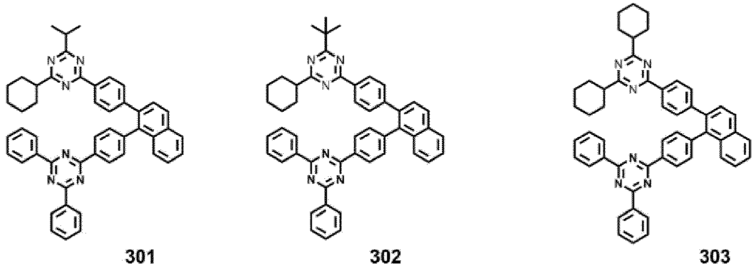

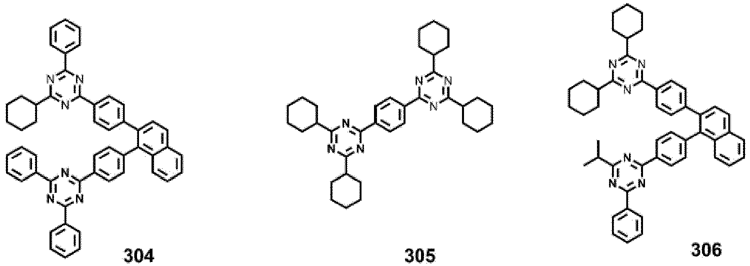

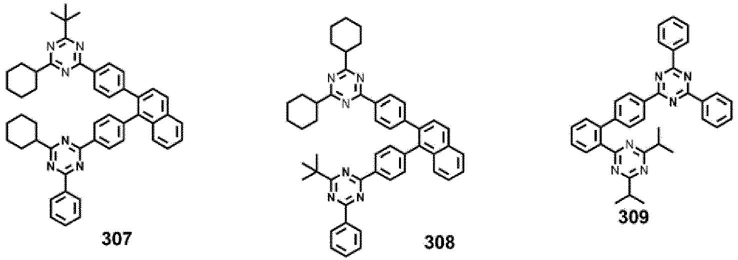

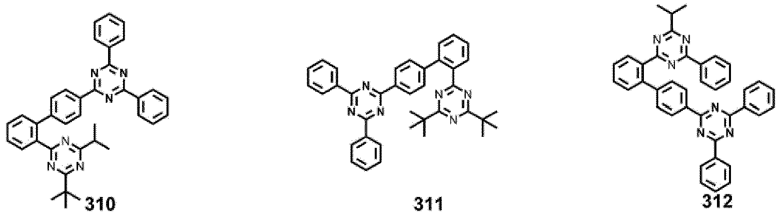

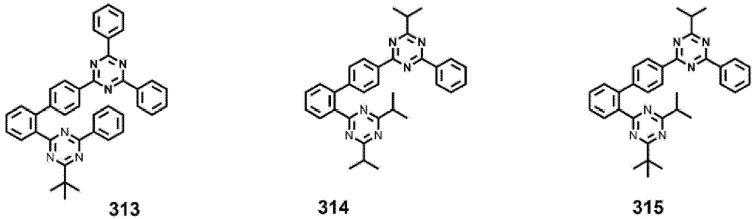

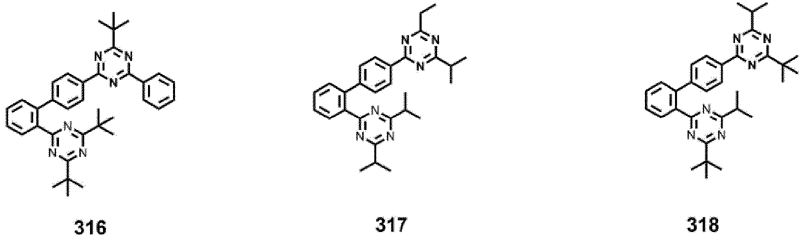

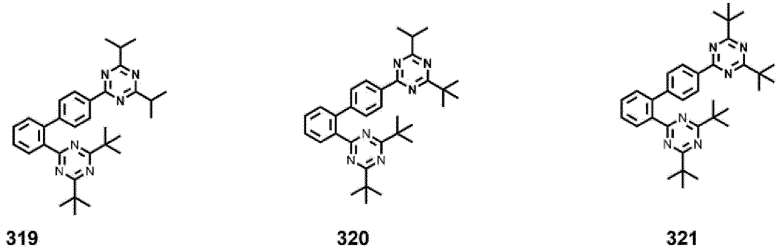

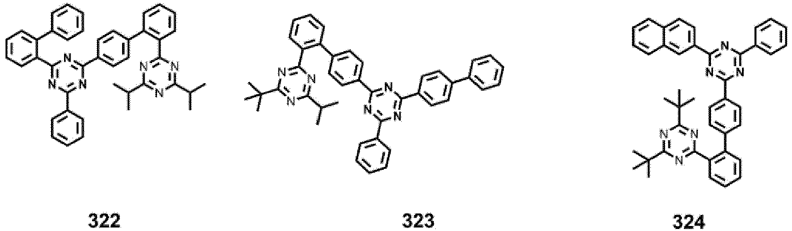

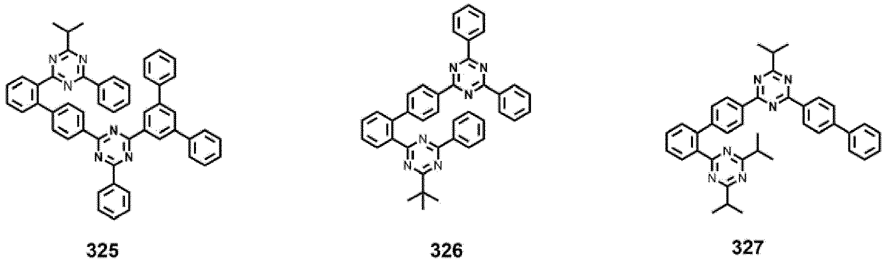

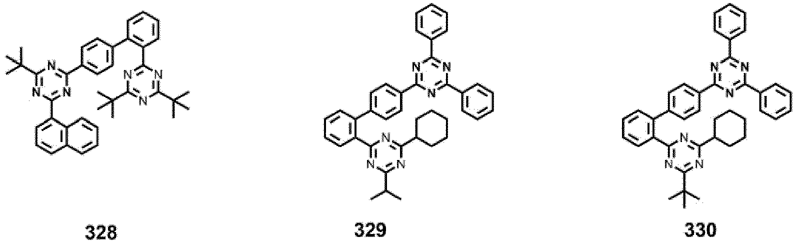

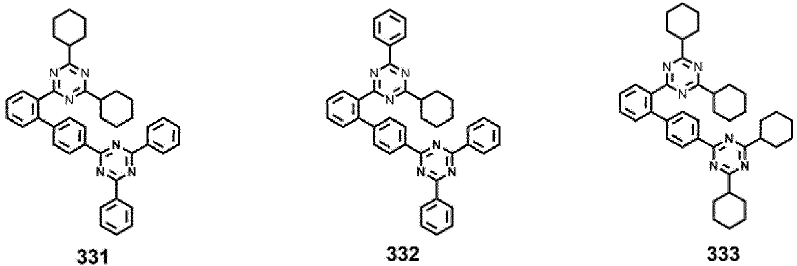

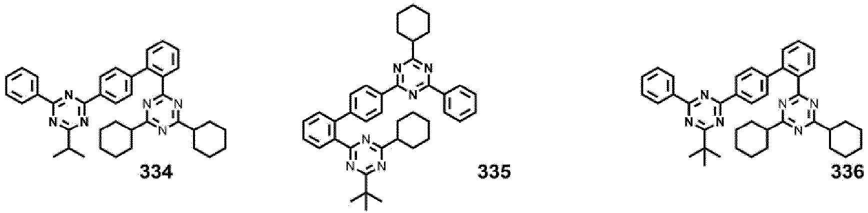

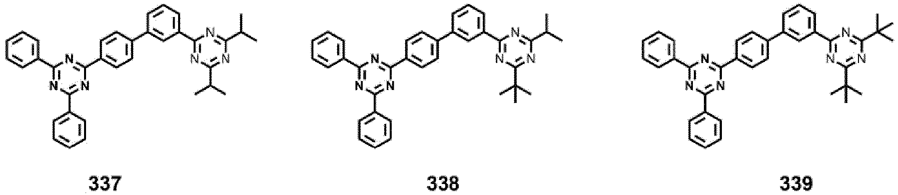

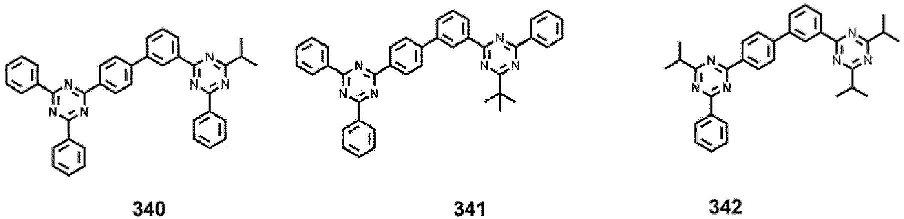

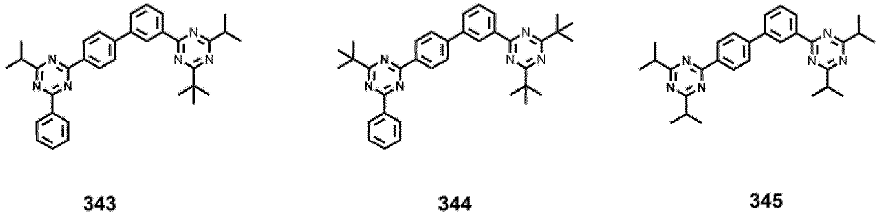

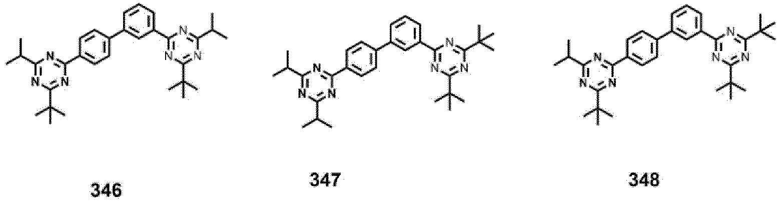

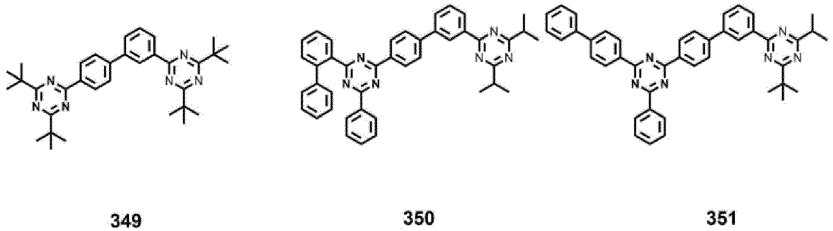

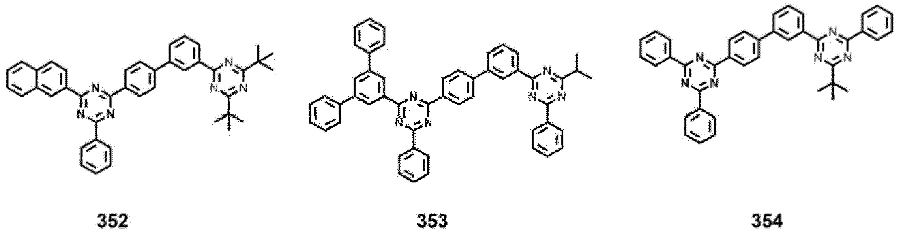

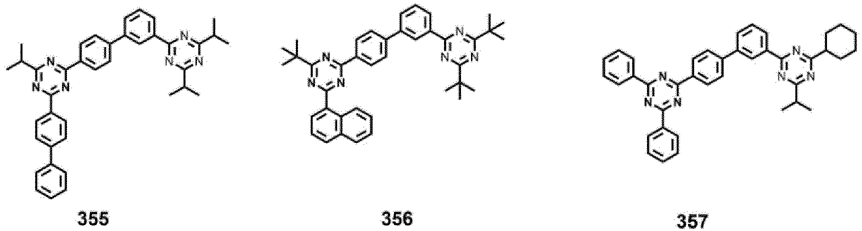

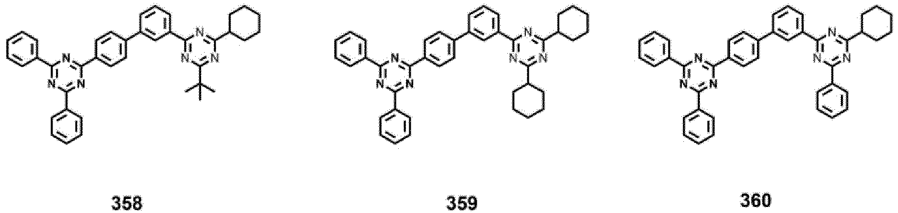

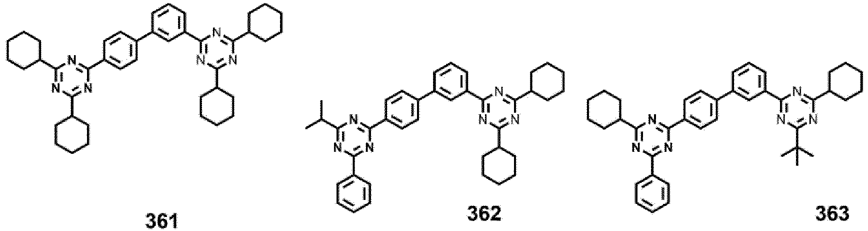

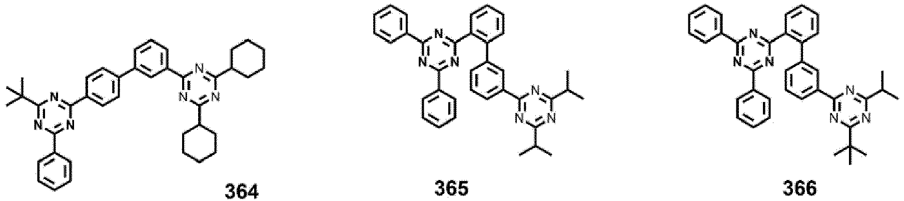

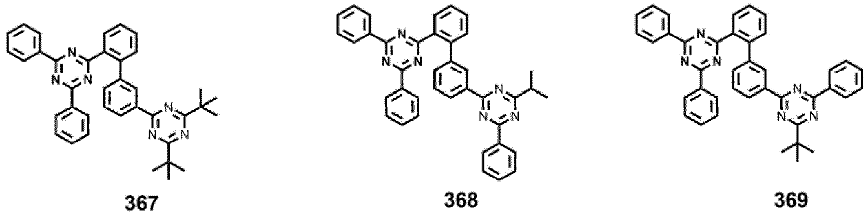

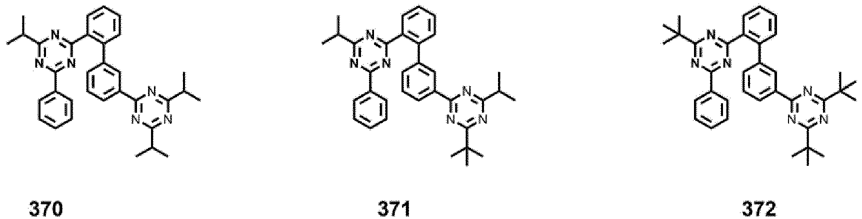

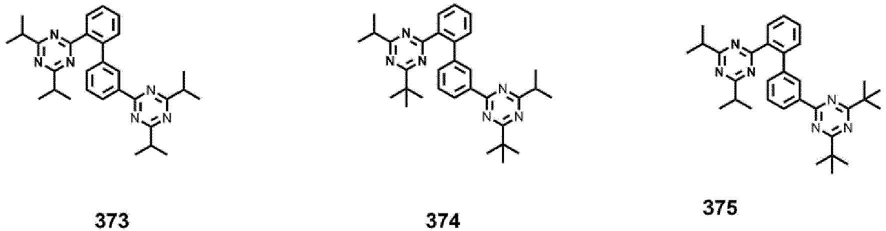

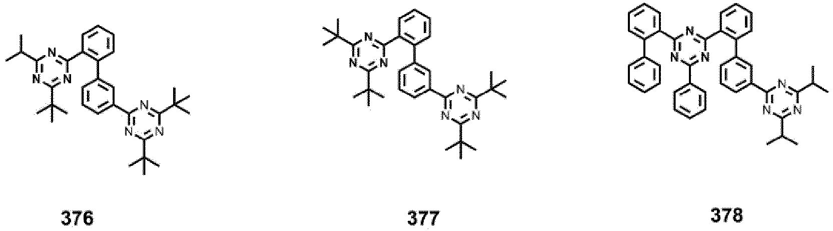

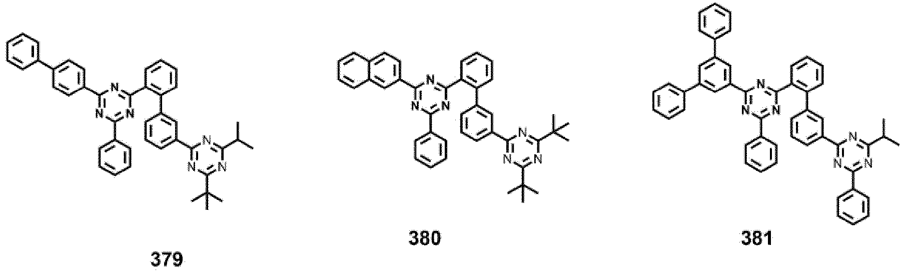

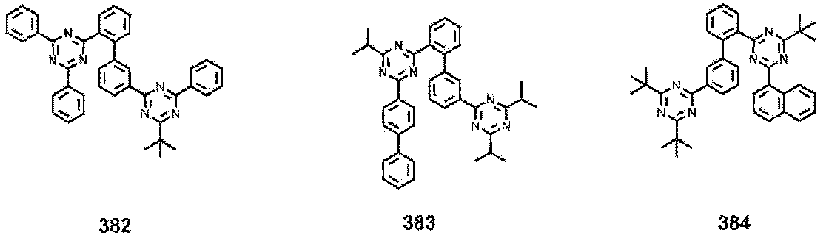

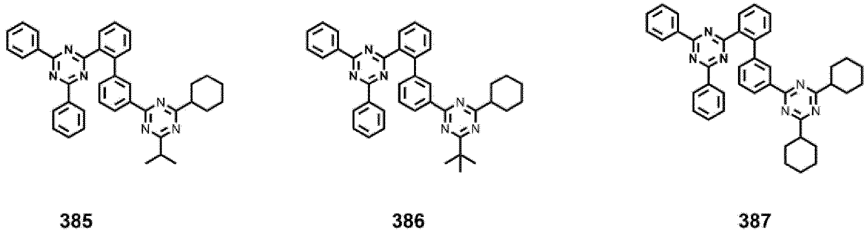

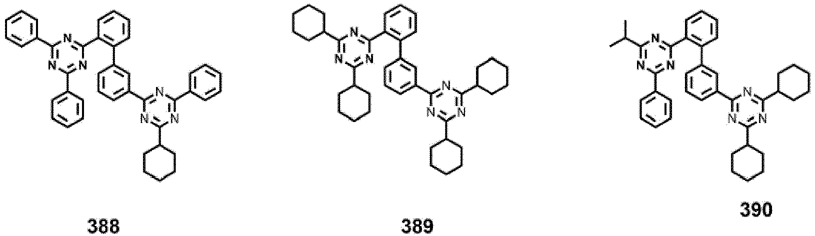

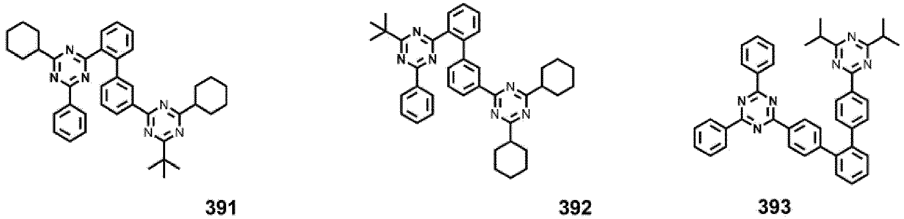

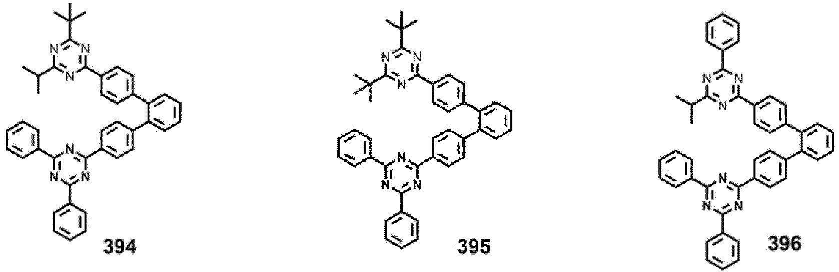

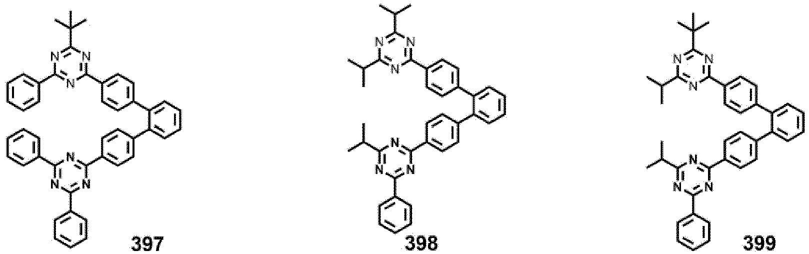

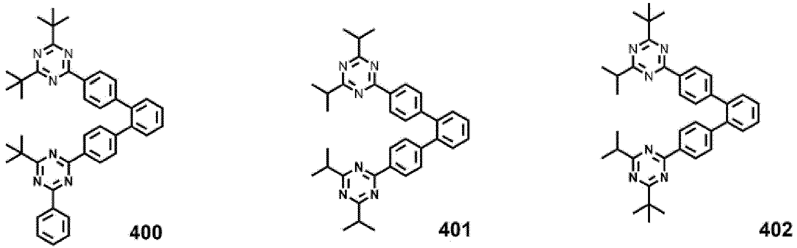

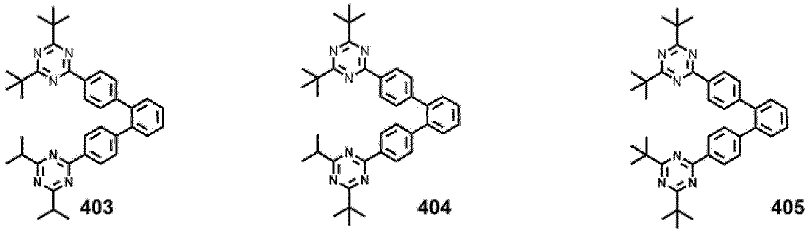

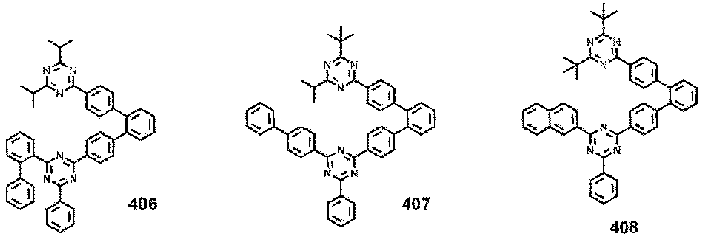

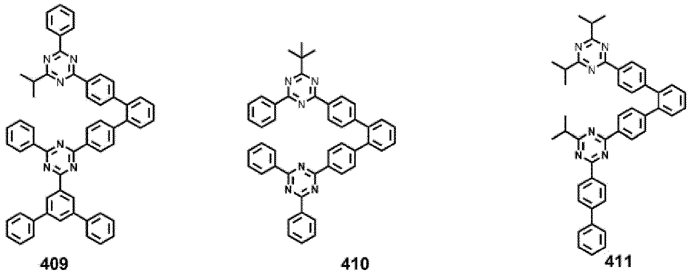

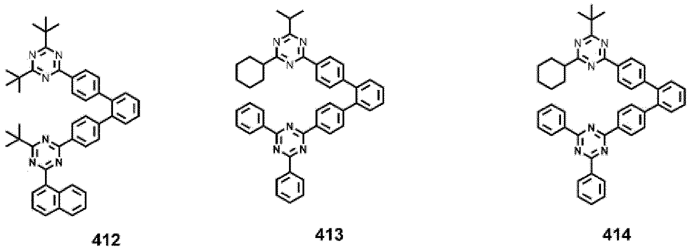

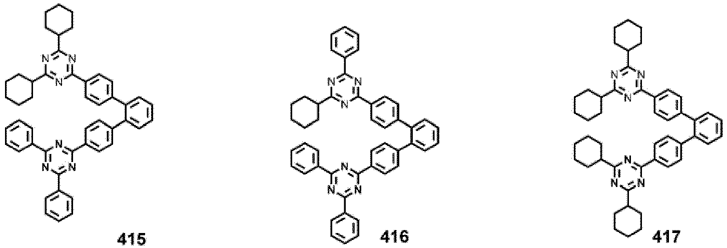

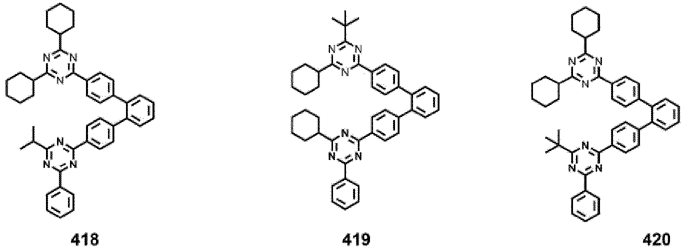

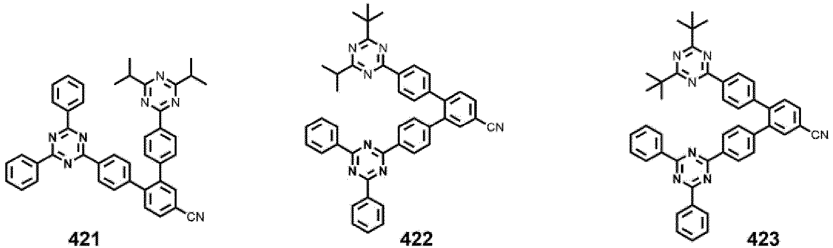

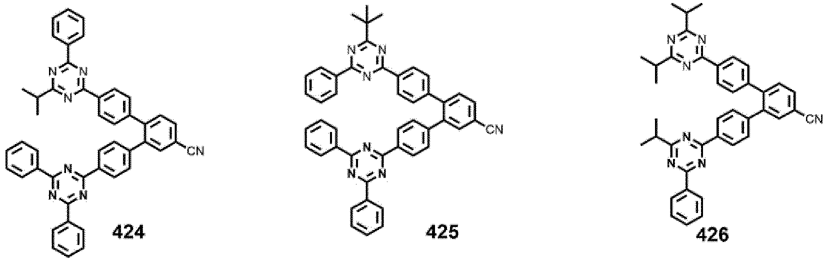

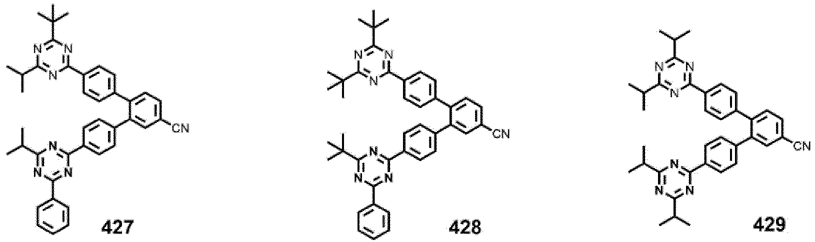

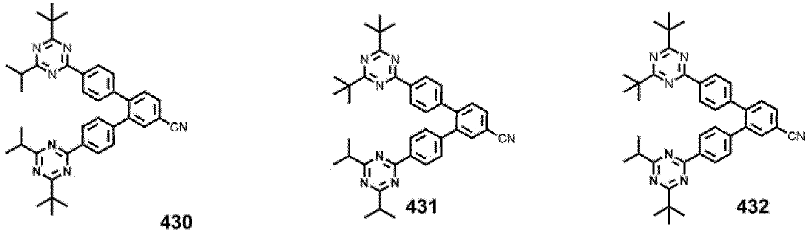

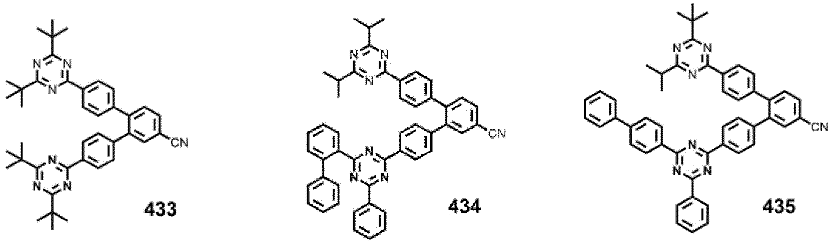

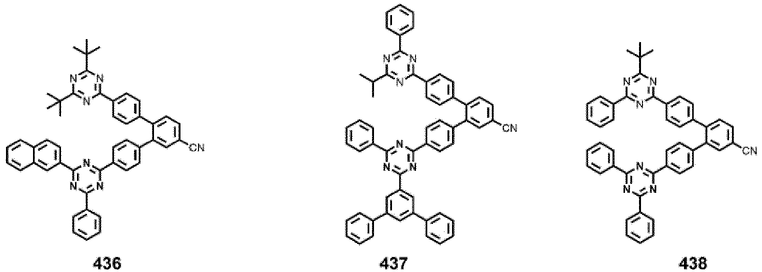

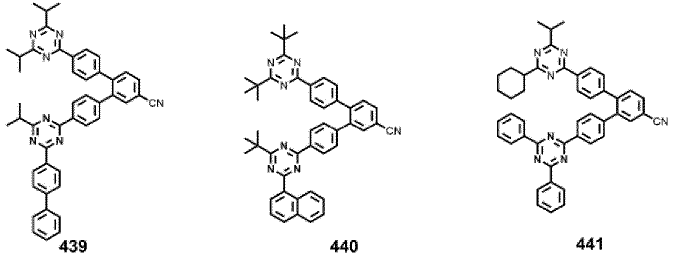

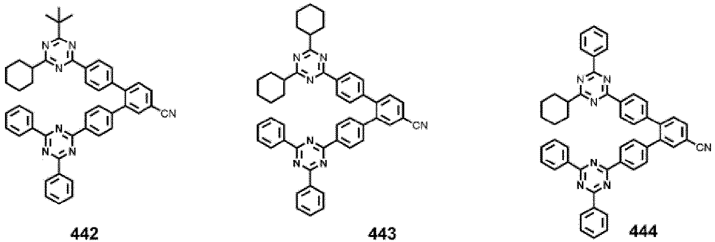

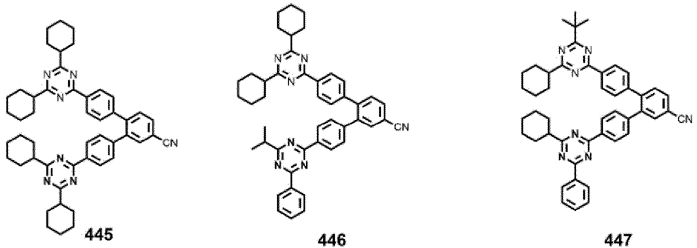

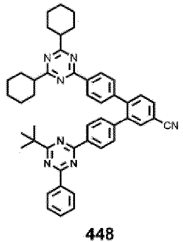

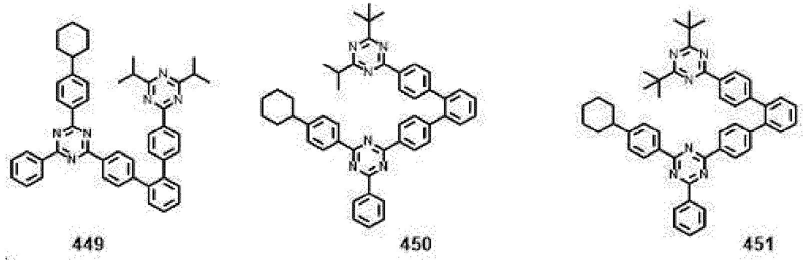

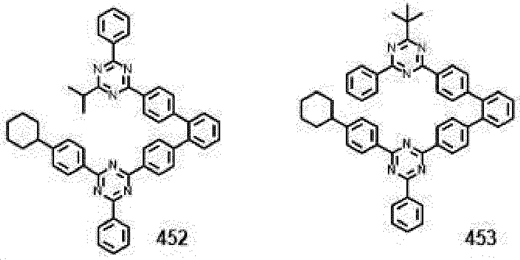

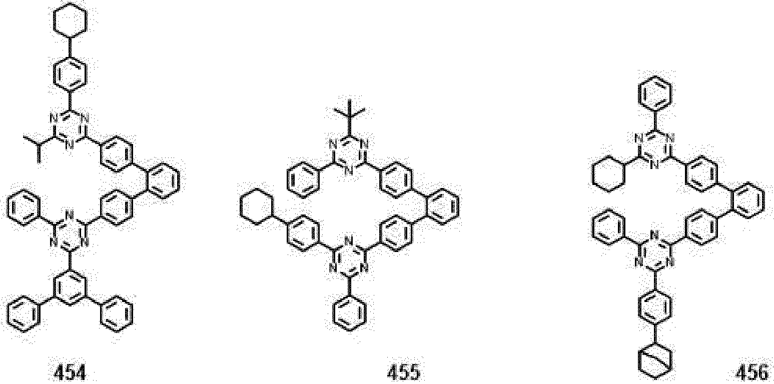

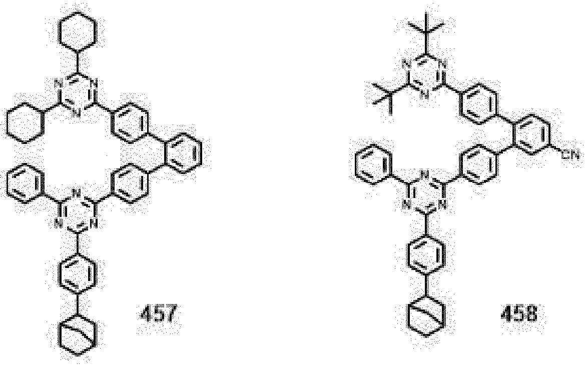

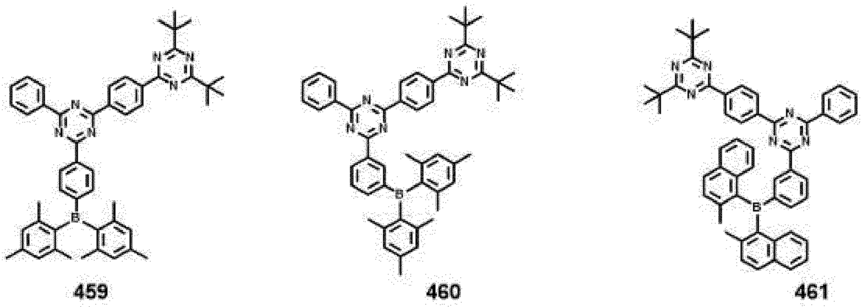

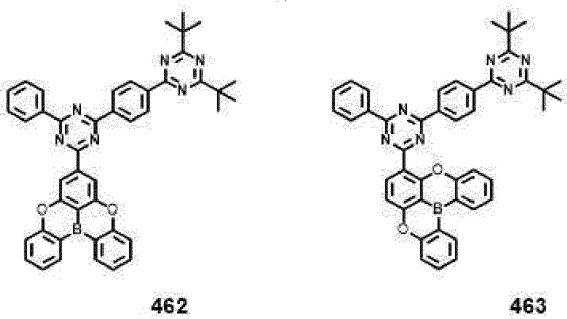

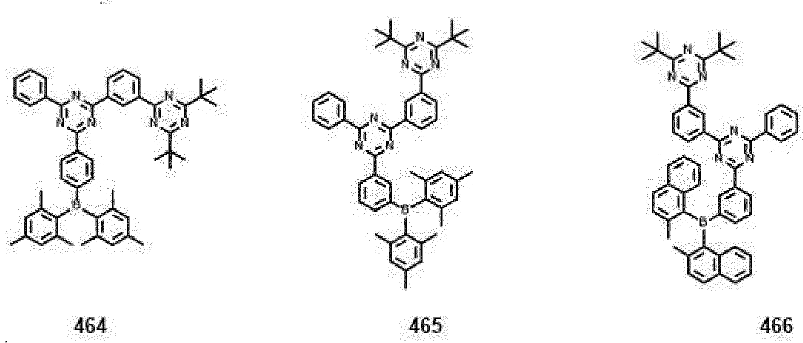

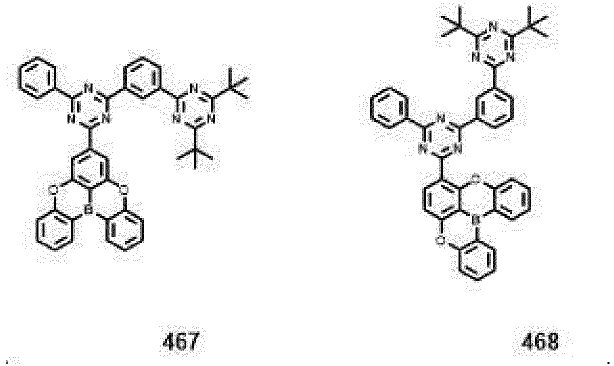

The heterocyclic compound may improve the charge balance in an emission layer by modulating the electron transfer ability due to the inclusion of a C₃-C₁₀ alkyl group unsubstituted or substituted with at least one R₁₀ₐ, a C₃-C₁₀ cycloalkyl group unsubstituted or substituted with at least one R₁₀ₐ, or a group represented by Formula 2 each directly bonded to a diazine or triazine moiety in the compound.

Accordingly, a light-emitting device incorporating the heterocyclic compound may facilitate energy transfer within the device, and thus improve luminescence efficiency and lifespan characteristics.

Synthesis methods of the heterocyclic compound represented by Formula 1 may be recognizable by one of ordinary skill in the art by referring to Synthesis Examples and/or Examples described herein.

At least one heterocyclic compound represented by Formula 1 may be used in a light-emitting device (for example, an organic light-emitting device). Accordingly, provided is a light-emitting device including: a first electrode; a second electrode opposite to (e.g., facing) the first electrode; and an interlayer located between the first electrode and the second electrode and including an emission layer, wherein the interlayer includes the heterocyclic compound represented by Formula 1.

In one or more embodiments,
the first electrode of the light-emitting device may be an anode,
the second electrode of the light-emitting device may be a cathode,
the interlayer may further include a hole transport region between the first electrode and the emission layer and an electron transport region between the emission layer and the second electrode,
the hole transport region may include a hole injection layer, a hole transport layer, an emission auxiliary layer, an electron-blocking layer, or any combination thereof, and
the electron transport region may include a hole-blocking layer, an electron transport layer, an electron injection layer, an electron control layer, or any combination thereof.

In one or more embodiments, the heterocyclic compound represented by Formula 1 may be included in the interlayer.

In one or more embodiments, the heterocyclic compound represented by Formula 1 may be included in the electron transport region.

In one or more embodiments, the emission layer may include a host and a dopant.

In one or more embodiments, the emission layer may further include a sensitizer.

In one or more embodiments, the emission layer may be to emit blue light.

In one or more embodiments, the dopant may be a phosphorescent dopant or a delayed fluorescence dopant.

In one or more embodiments, the emission layer may include a first host and a second host, wherein the first host may be a hole-transporting compound including at least one electron-donating group, and the second host may be an electron-transporting compound including at least one electron-withdrawing group.

In one or more embodiments, the emission layer may further include a third compound, and the third compound may be a metal-containing compound.

In one or more embodiments, the third compound may serve as a sensitizer such as a phosphorescent sensitizer.

In one or more embodiments, the third compound may not emit light.

In one or more embodiments, the emission layer may further include at least one of an auxiliary dopant or a sensitizer.

In one or more embodiments, the auxiliary dopant and the sensitizer may each independently be an organometallic compound including Pt and a tetradentate ligand bonded to Pt, wherein the tetradentate ligand may include a carbene moiety chemically bonded to the Pt. For example, the auxiliary dopant and/or the sensitizer may include the third compound.

In one or more embodiments, the first host and the second host may serve as an exciplex host.

The term "electron-donating group" as used herein refers to any moiety having ability to donate electrons, for example, may be a π electron-rich C₃-C₆₀ cyclic group or an amine group, but embodiments of the present disclosure are not limited thereto. The electron-donating group may refer to a cyclic group other than a π electron-deficient nitrogen-containing C₁-C₆₀ cyclic group.

The term "electron-withdrawing group" as used herein refers to any moiety having ability to withdraw electrons, for example, may be -F, -CFH₂, -CF₂H, -CF₃, -CN, -NO₂, a π electron-deficient nitrogen-containing C₁-C₆₀ cyclic group, or any combination thereof. However, embodiments of the present disclosure are not limited thereto.

Regarding a luminescence pathway in the light-emitting device according to one or more embodiments, the first host and the second host may form an exciton (first process), the energy of the exciton may be transferred to the third compound (second process), and the energy may be transferred from the third compound to an emitter (e.g., a dopant) such as an organometallic compound (third process).

In one or more embodiments, the amount of the third compound may be more than 0 parts by weight and less than 50 parts by weight based on the total weight of 100 parts by weight of the emission layer.

In one or more embodiments, the first host may include at least one carbazole moiety, and the second host may include at least one azine moiety.

In one or more embodiments, the first host may be a compound represented by Formula 301-1A or 301-2A: 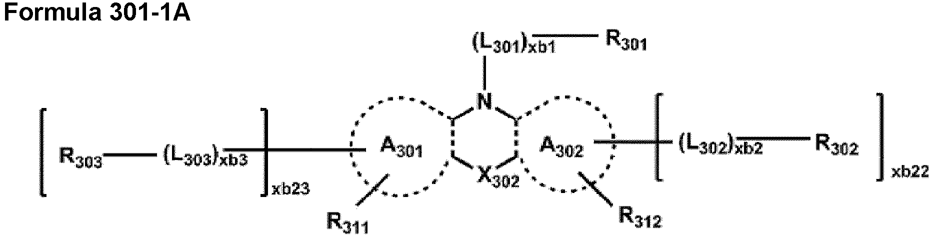 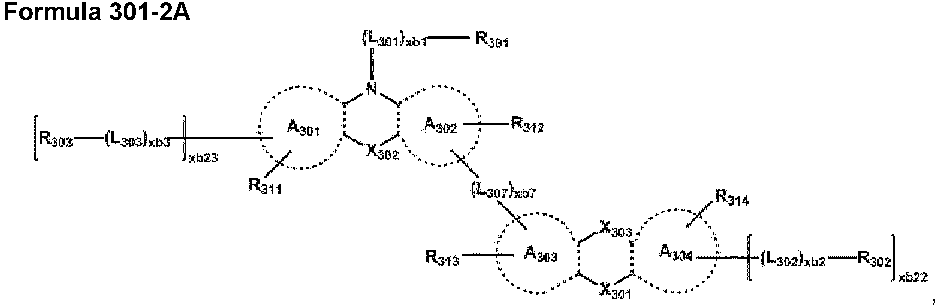 wherein, in Formulae 301-1A and 301-2A,
ring A₃₀₁ to ring A₃₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
X₃₀₁ may be O, S, N[(L₃₀₄)_{xb4}-R₃₀₄ₐ], C(R₃₀₄ₐ)(R_{304b}), or Si(R₃₀₄ₐ)(R_{304b}),
X₃₀₂ may be a single bond, O, S, N[(L₃₀₅)_{xb5}-R₃₀₅ₐ], C(R₃₀₅ₐ)(R_{305b}), or Si(R₃₀₅ₐ)(R_{305b}),
X₃₀₃ may be a single bond, O, S, N[(L₃₀₆)_{xb6}-R₃₀₆ₐ], C(R₃₀₆ₐ)(R_{306b}), or Si(R₃₀₆ₐ)(R_{306b}),
xb22 and xb23 may each independently be an integer from 0 to 10,
L₃₀₁ to L₃₀₇ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xb1 to xb7 may each independently be an integer from 0 to 5,

R₃₀₁ to R₃₀₃, R₃₀₄ₐ to R₃₀₆ₐ, R_{304b} to R_{306b}, and R₃₁₁ to R₃₁₄ may each independently be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), or -P(=O)(Q₃₀₁)(Q₃₀₂), and

Q₃₀₁ to Q₃₀₃ are each the same as described with respect to Q₁.

In one or more embodiments, the first host may be one of (e.g., any one selected from among) Compounds HTH1 to HTH56 and Compounds HTH1' to HTH40', but embodiments of the present disclosure are not limited thereto:

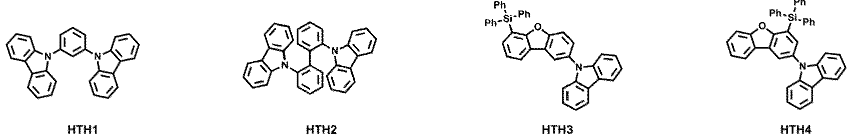

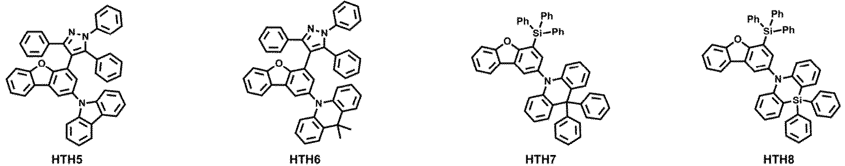

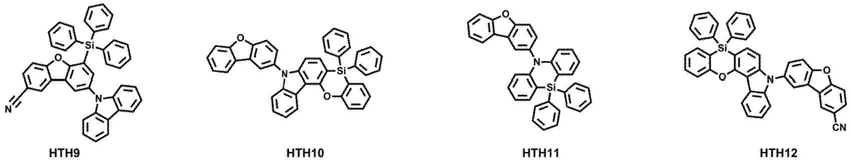

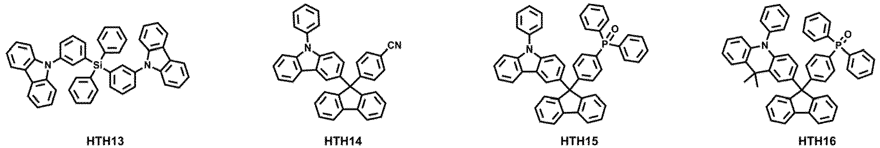

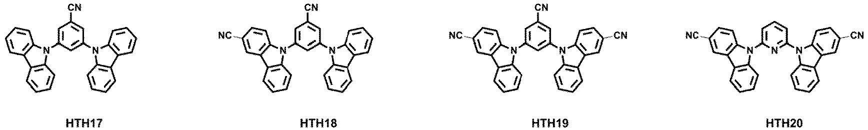

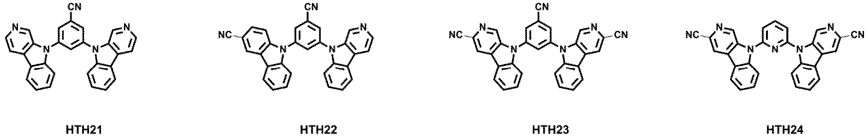

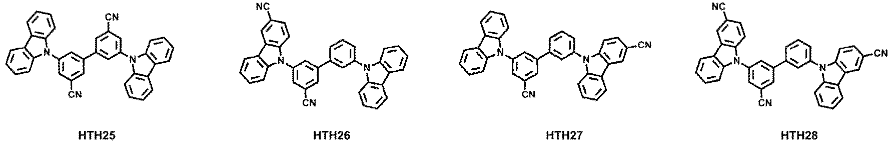

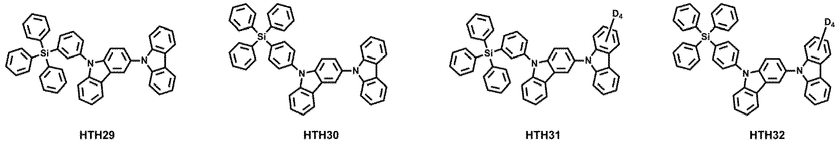

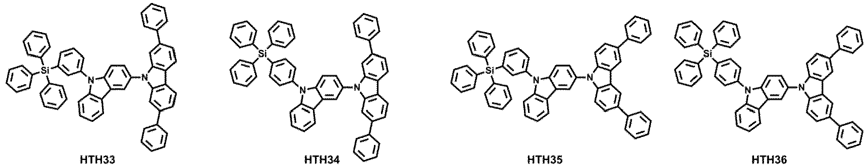

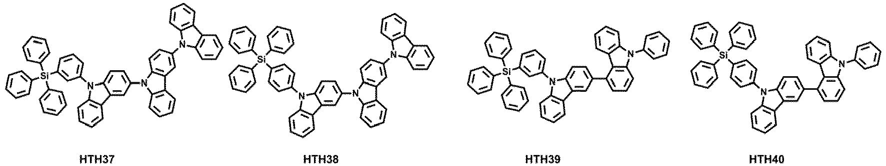

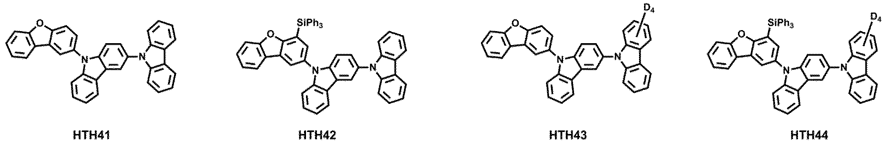

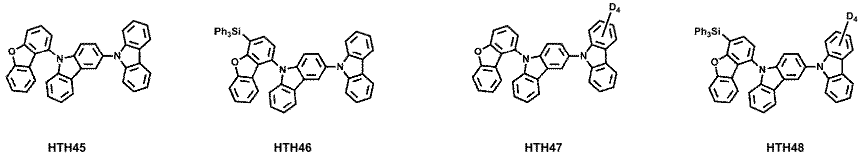

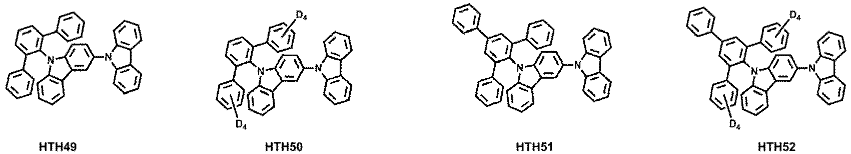

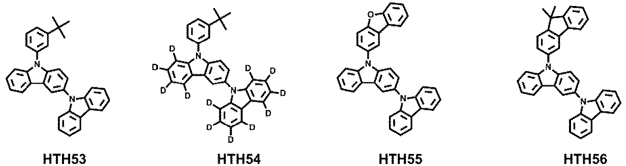

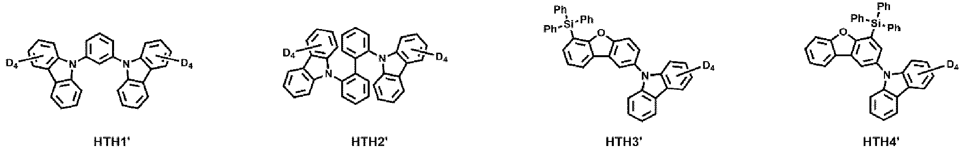

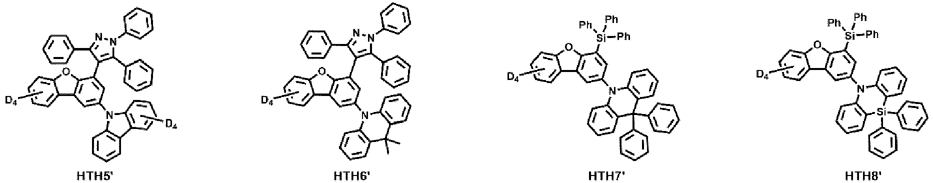

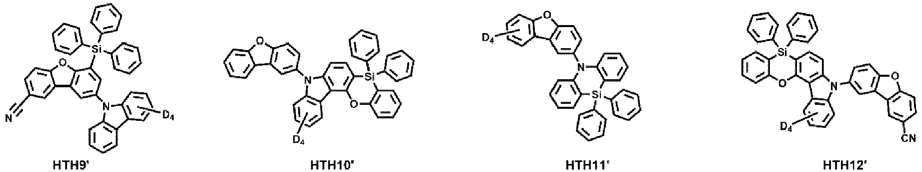

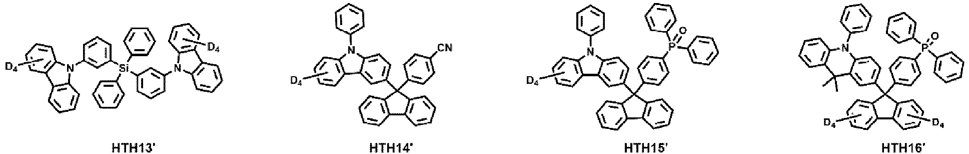

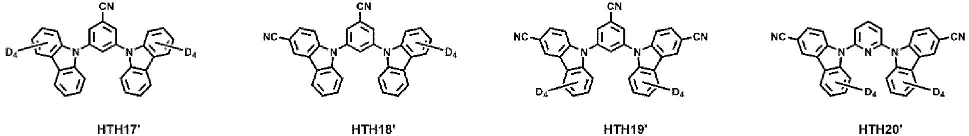

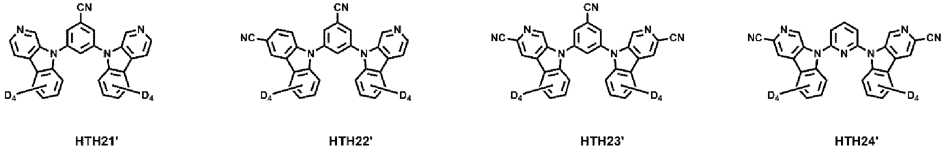

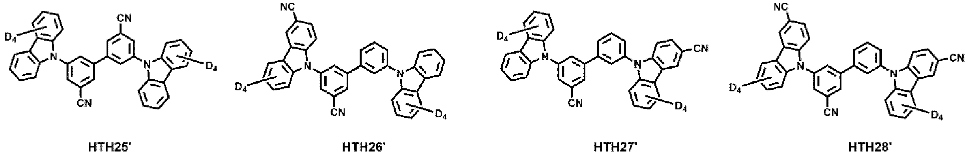

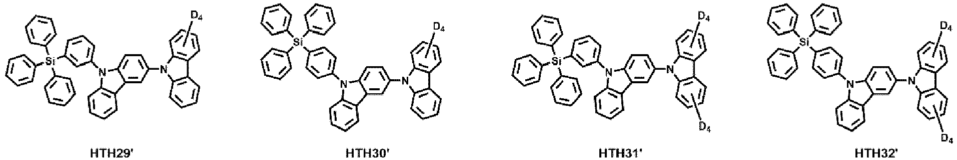

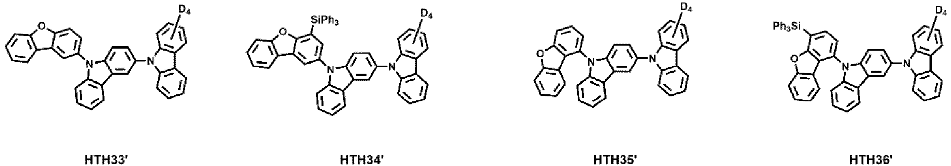

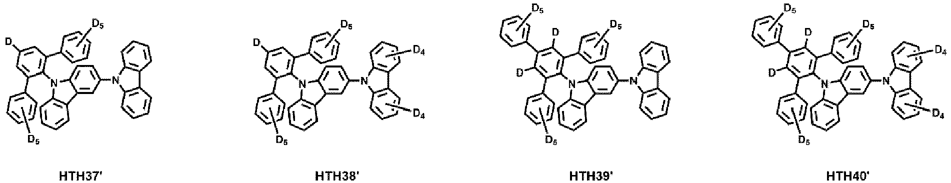

In one or more embodiments, the second host may be a compound represented by Formula 302: 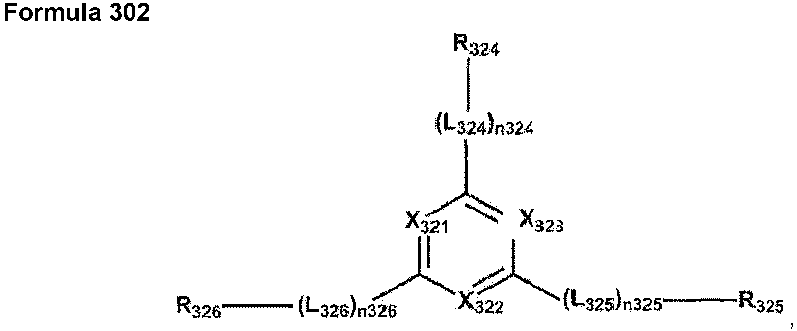 wherein, in Formula 302,
X₃₂₁ may be C(R₃₂₁) or N,
X₃₂₂ may be C(R₃₂₂) or N,
X₃₂₃ may be C(R₃₂₃) or N,
at least one selected from among X₃₂₁ to X₃₂₃ may be N,
L₃₂₄ to L₃₂₆ may each independently be a single bond, a C₃-C₆₀ carbocyclic group unsubstituted or substituted with at least one R₁₀ₐ, C₁-C₆₀ heterocyclic group unsubstituted or substituted with at least one R₁₀ₐ, *-C(Q₃₂₁₎(Q₃₂₂)-*, *-Si(Q₃₂₁)(Q₃₂₂)-*', *-B(Q₃₂₁)-*', or *-N(Q₃₂₁)-*',
n324 to n326 may each independently be an integer from 1 to 5,
R₃₂₁ to R₃₂₆ may each independently be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, - Si(Q₃₂₃)(Q₃₂₄)(Q₃₂₅), -N(Q₃₂₃)(Q₃₂₄), -B(Q₃₂₃)(Q₃₂₄), -C(=O)(Q₃₂₃), -S(=O)₂(Q₃₂₃), or - P(=O)(Q₃₂₃)(Q₃₂₄),
two or more neighboring groups selected from among Q₃₂₁ to Q₃₂₅ and R₃₂₁ to R₃₂₆ may optionally be bonded to each other to form a C₅-C₃₀ carbocyclic group unsubstituted or substituted with at least one R₁₀ₐ or a C₂-C₃₀ heterocyclic group unsubstituted or substituted with at least one R₁₀ₐ,
* and *' each indicate a binding site to a neighboring atom,
R₁₀ₐ is the same as described herein, and
Q₃₂₁ to Q₃₂₅ may each be the same as described with respect to Q₁.

In one or more embodiments, the second host may be one of (e.g., any one selected from among) Compounds ETH1 to ETH86 and Compounds ETH1' to ETH32', but embodiments of the present disclosure are not limited thereto:

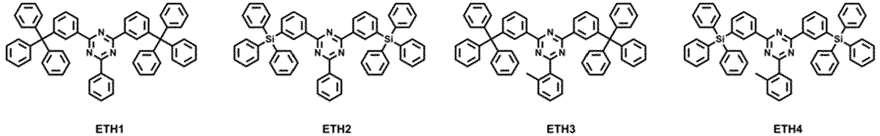

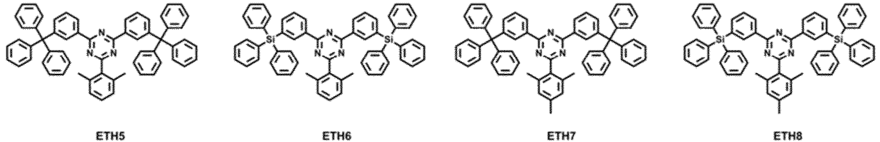

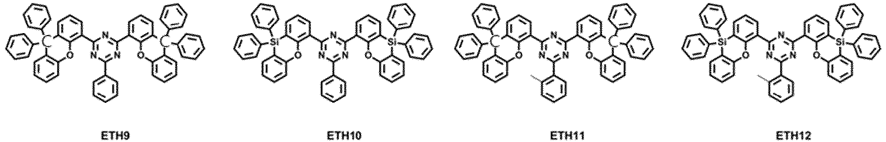

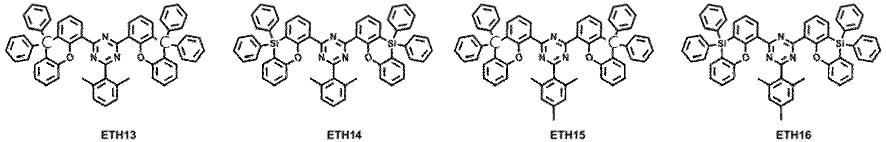

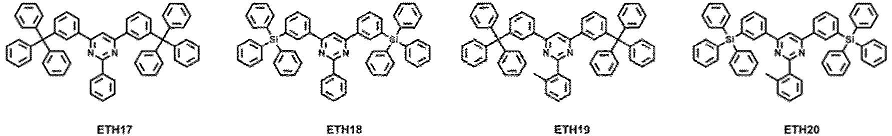

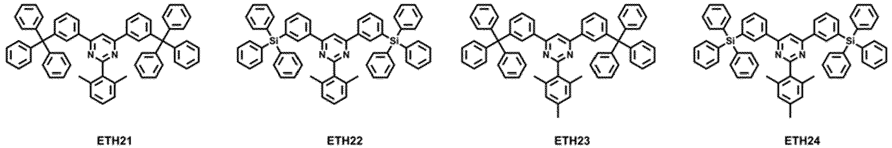

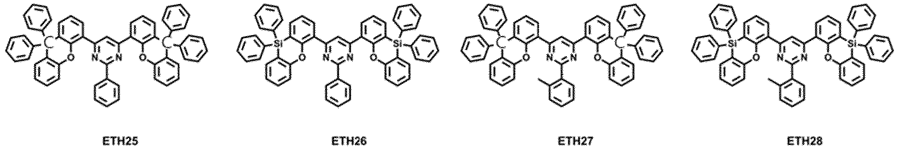

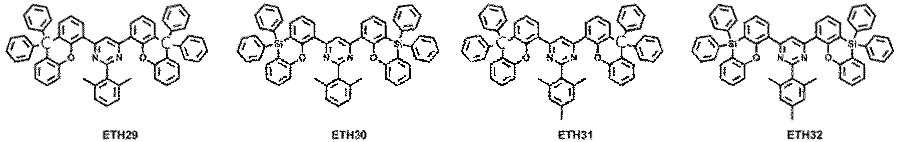

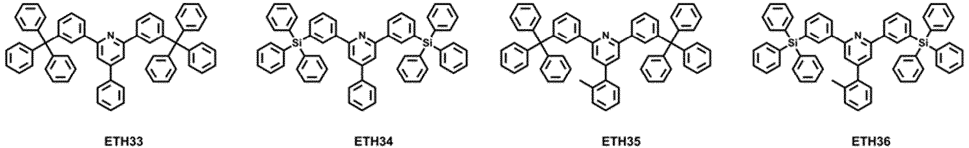

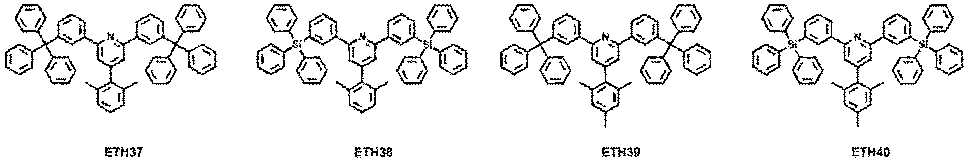

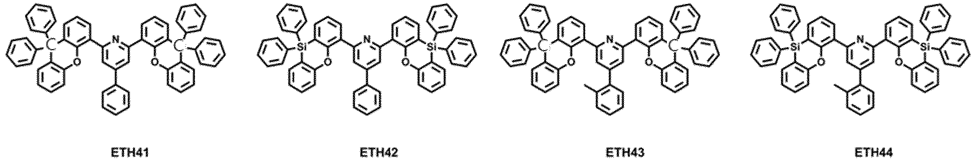

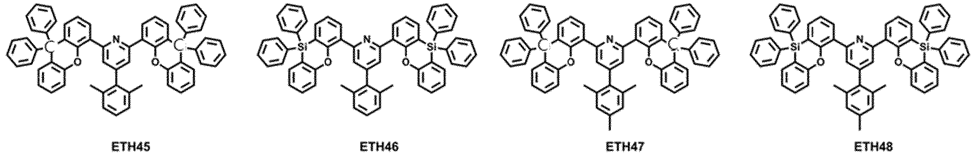

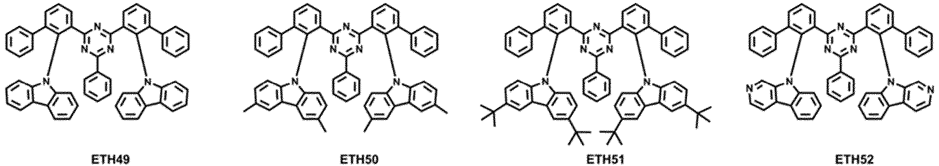

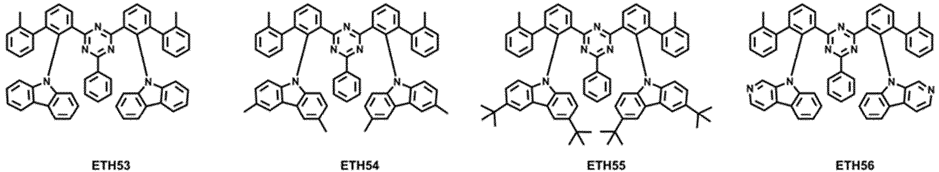

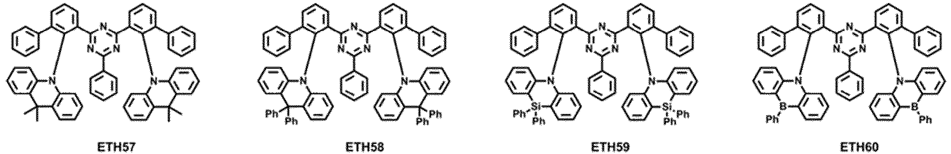

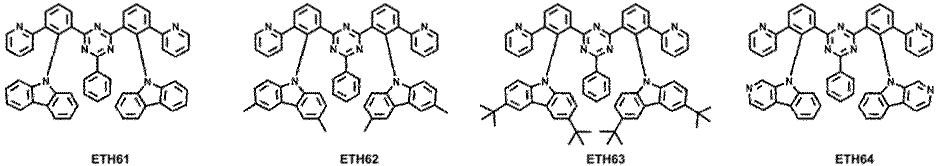

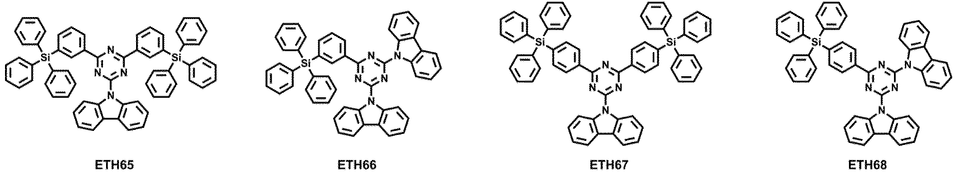

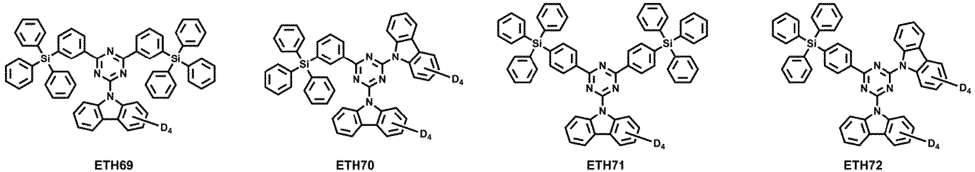

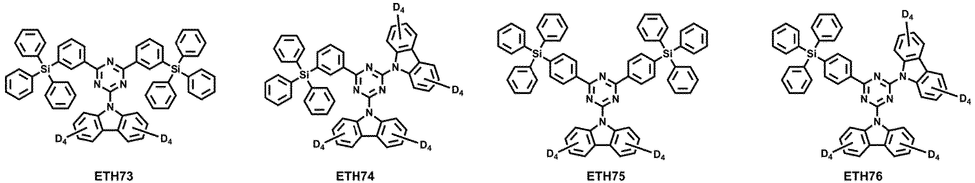

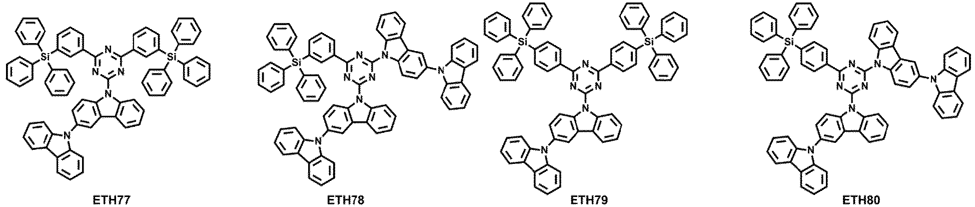

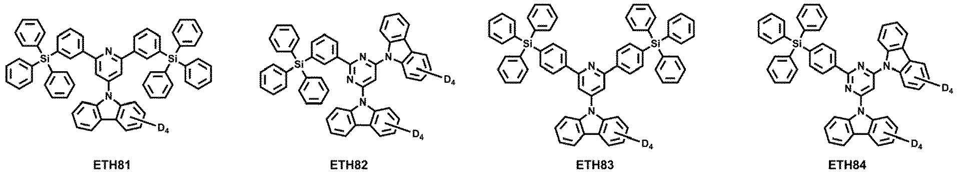

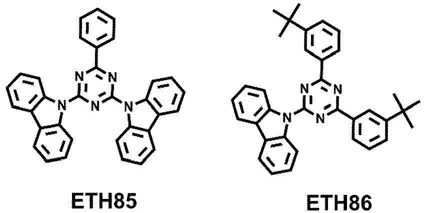

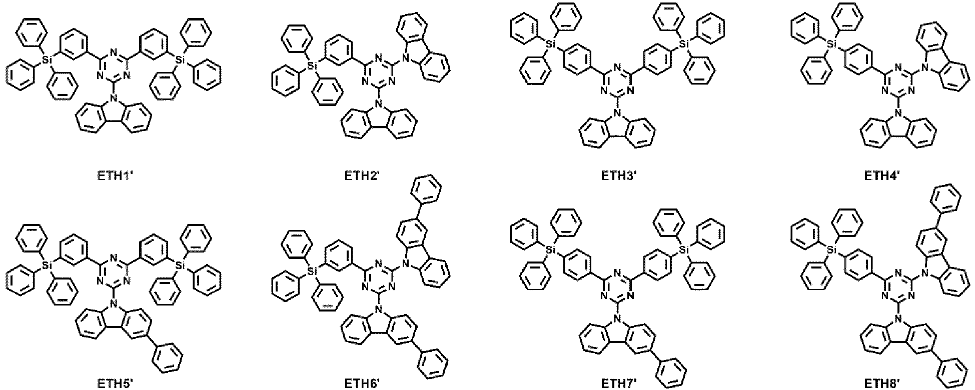

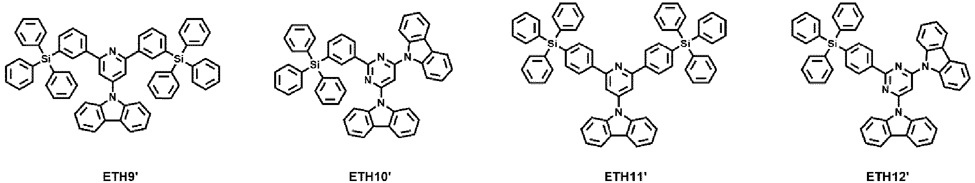

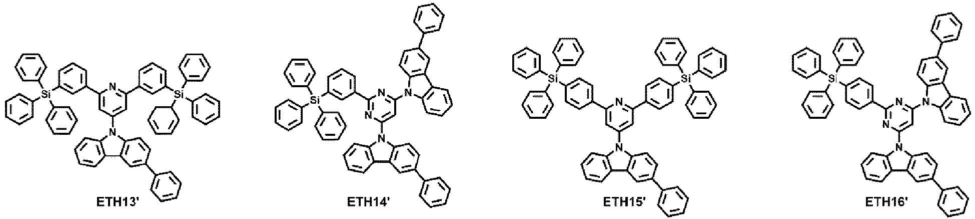

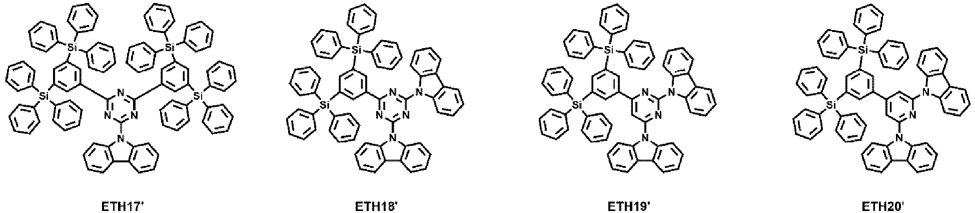

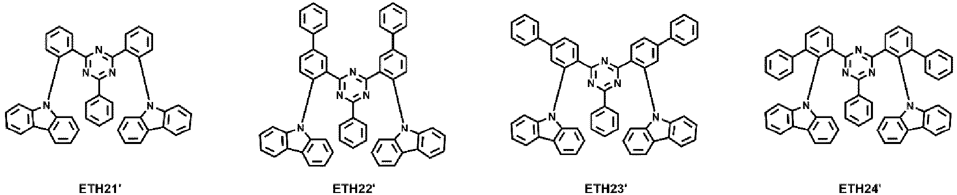

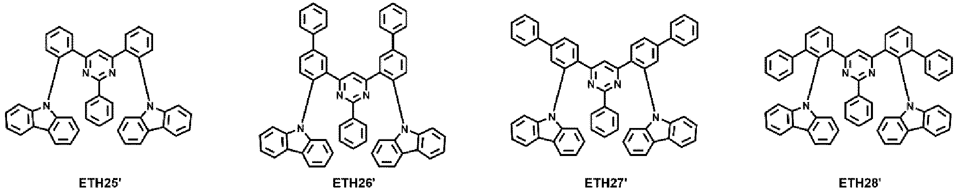

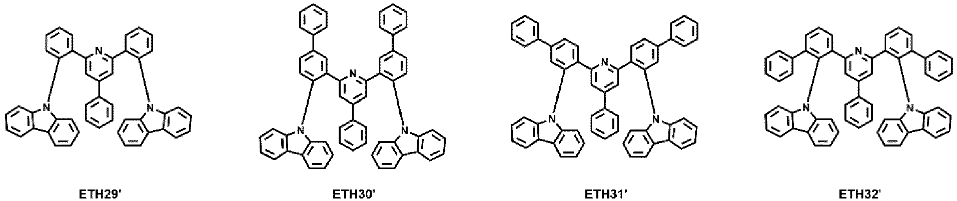

In one or more embodiments, the third compound may be represented by Formula 401A:

**Formula 401A** **M₄₀₁(L₄₀₁)ₙ₄₀₁(L₄₀₂)ₙ₄₀₂**

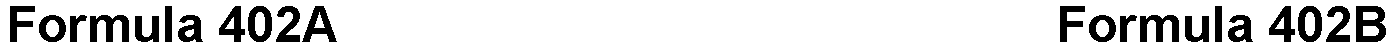

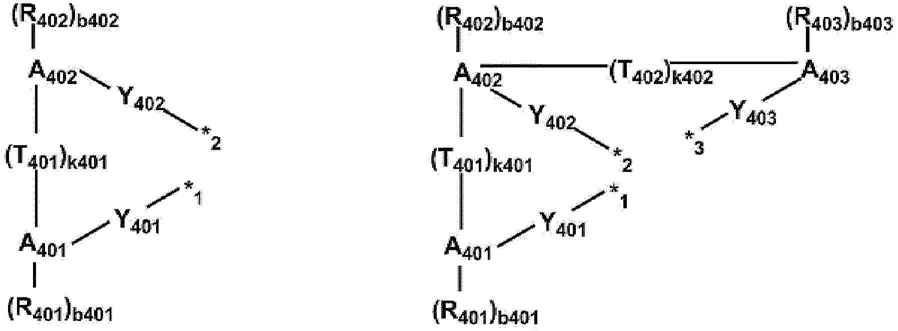

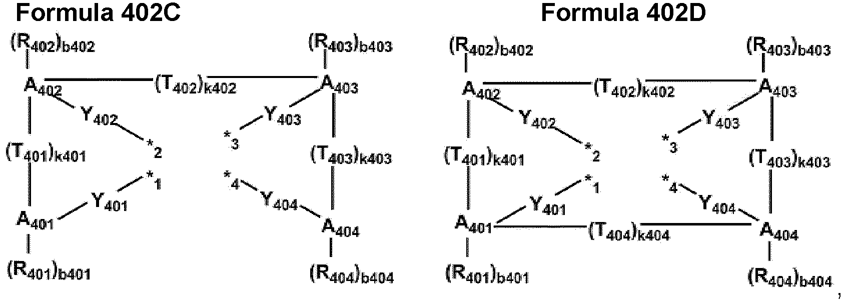

wherein, in Formulae 401A and 402A to 402D,
M₄₀₁ may be a first-row transition metal of the Periodic Table of Elements, a second-row transition metal of the Periodic Table of Elements, or a third-row transition metal of the Periodic Table of Elements,
L₄₀₁ may be a ligand represented by one selected from among Formulae 402A to 402D,
L₄₀₂ may be a monodentate ligand, a bidentate ligand, or a tridentate ligand,
n401 may be 1 or 2,
n402 may be an integer from 0 to 4,
A₄₀₁ to A₄₀₄ may each independently be a C₅-C₃₀ carbocyclic group or a C₁-C₃₀ heterocyclic group,
T₄₀₁ to T₄₀₄ may each independently be a single bond, a double bond, *-O-*', *-S-*', *-C(=O)-*', *-S(=O)-*', *-C(R₄₀₅)(R₄₀₆)-*', *-C(R₄₀₅)=C(R₄₀₆)-*', *-C(R₄₀₅)=*', *-Si(R₄₀₅)(R₄₀₆)-*', *-B(R₄₀₅)-*', *-N(R₄₀₅)-*', or *-P(R₄₀₅)-*',
k401 to k404 may each independently be 1, 2, or 3,
Y₄₀₁ to Y₄₀₄ may each independently be a single bond (e.g., a covalent bond or a coordinate bond), *-O-*', *-S-*', *-C(R₄₀₇)(R₄₀₈)-*', *-Si(R₄₀₇)(R₄₀₈)-*', *-B(R₄₀₇)-*', *-N(R₄₀₇)-*', or *-P(R₄₀₇)-*',
*₁, *₂, *₃, and *₄ each indicate a binding site to M₄₀₁,
R₄₀₁ to R₄₀₈ may each independently be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -Si(Q₁)(Q₂)(Q₃), -N(Q₁)(Q₂), - B(Q₁)(Q₂), -C(=O)(Q₁), -S(=O)₂(Q₁), or -P(=O)(Q₁)(Q₂),
R₄₀₁ to R₄₀₈ may optionally be bonded to each other to form a C₅-C₆₀ carbocyclic group unsubstituted or substituted with at least one R₁₀ₐ or a C₁-C₆₀ heterocyclic group unsubstituted or substituted with at least one R₁₀ₐ,
b401 to b404 may each independently be an integer from 0 to 10,
* and *' each indicate a binding site to a neighboring atom, and
Q₁ to Q₃ and R₁₀ₐ are each the same as described herein.

In one or more embodiments, the compound represented by Formula 401A may be a carbene complex.

The term "carbene complex" as used herein refers to a complex which includes a metal and a ligand bonded to the metal, wherein at least one bond between the metal and the ligand is a bond between the metal and carbon of carbene.

In one or more embodiments, the sensitizer may include the compound represented by Formula 401A.

In one or more embodiments, the third compound may include one of (e.g., may include at least one or be any one selected from among) Compounds PD1 to PD41, but embodiments of the present disclosure are not limited thereto:

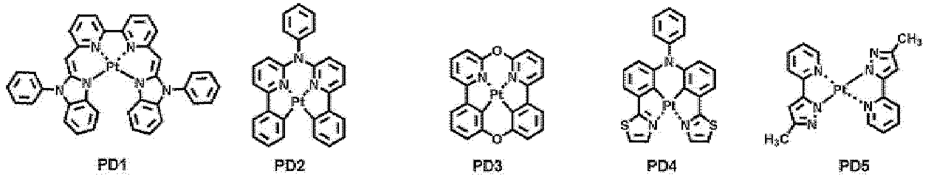

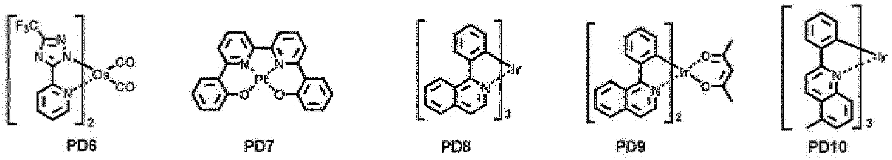

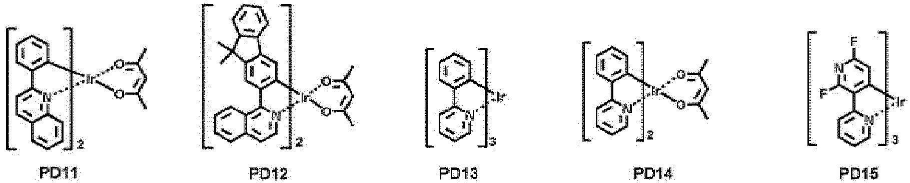

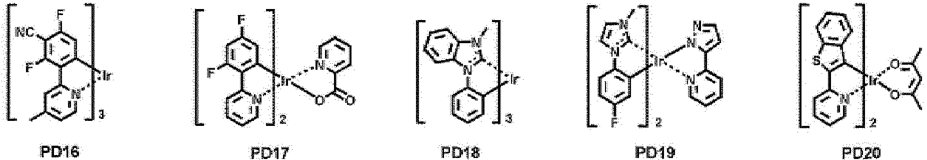

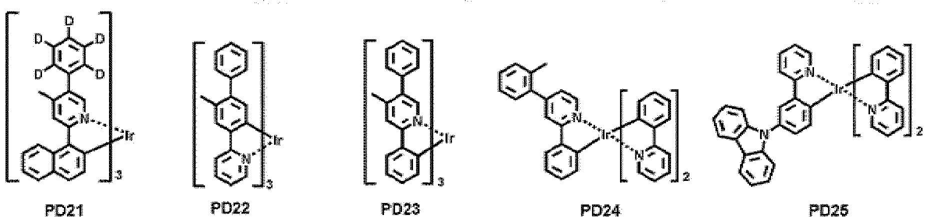

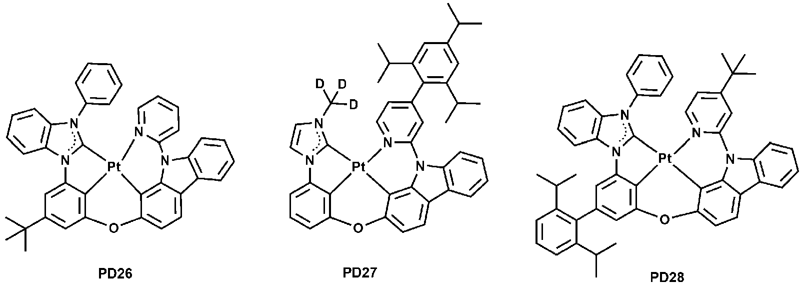

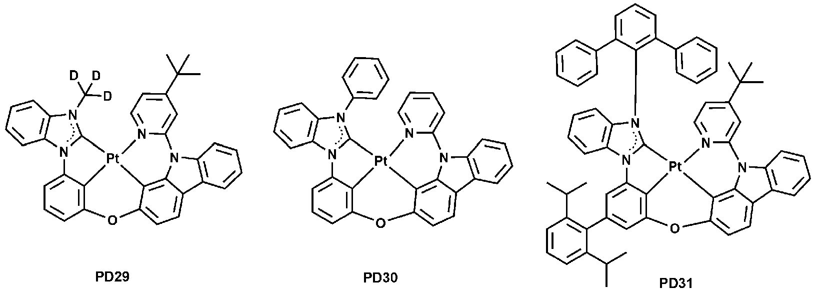

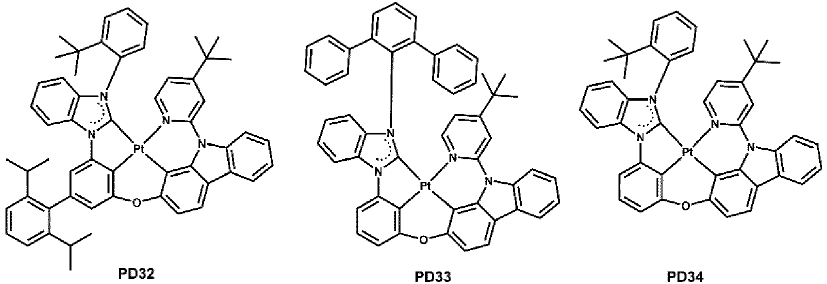

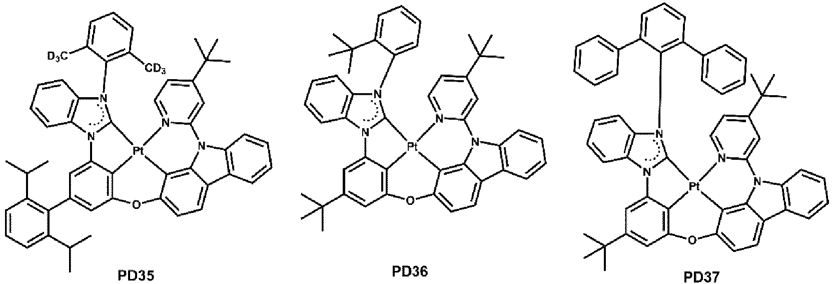

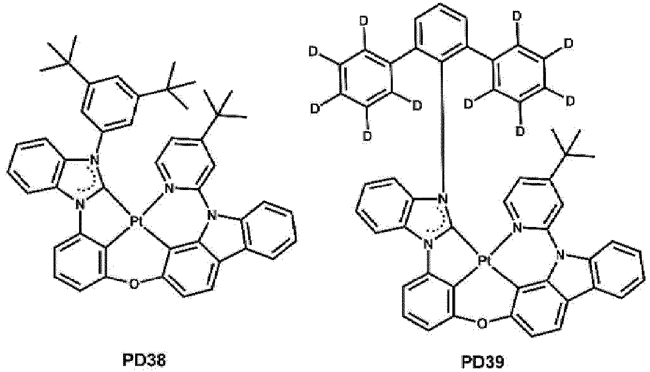

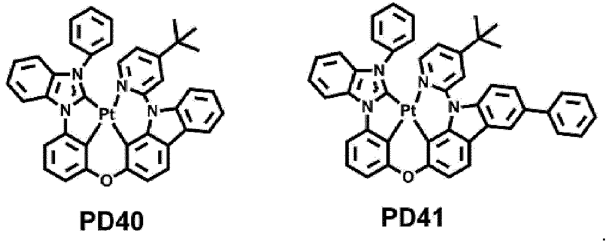

In one or more embodiments, R₃₀₁ to R₃₀₃, R₃₀₄ₐ to R₃₀₆ₐ, R_{304b} to R_{306b}, and R₃₁₁ to R₃₁₄ in Formulae 301-1A and 301-2A, R₃₂₁ to R₃₂₆ in Formula 302, and R₄₀₁ to R₄₀₈ in Formulae 401A and 402A to 402D may each independently be: hydrogen, deuterium, -F, -Cl, -Br, -I, a hydroxyl group, a cyano group, a nitro group, an amidino group, a hydrazine group, a hydrazone group, a C₁-C₂₀ alkyl group, or a C₁-C₂₀ alkoxy group;
a C₁-C₂₀ alkyl group or a C₁-C₂₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group, a hydrazone group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a (C₁-C₁₀ alkyl)phenyl group, a naphthyl group, a tetrahydronaphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a benzoisoquinolinyl group, a phthalazinyl group, a naphthylidinyl group, a quinoxalinyl group, a benzoquinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a thiadiazolyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, an azafluorenyl group, or an azadibenzosilolyl group, each unsubstituted or substituted with deuterium, -F, -Cl, -Br, - I, -CD₃, -CD₂H, -CDH₂, -CF₃, -CF₂H, -CFH₂, a hydroxyl group, a cyano group, a nitro group, an amidino group, a hydrazine group, a hydrazone group, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a (C₁-C₁₀ alkyl)phenyl group, a naphthyl group, a tetrahydronaphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a benzoisoquinolinyl group, a phthalazinyl group, a naphthylidinyl group, a quinoxalinyl group, a benzoquinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a thiadiazolyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, an azafluorenyl group, an azadibenzosilolyl group, -Si(Q₃₁)(Q₃₂)(Q₃₃), -B(Q₃₁)(Q₃₂), -P(Q₃₁)(Q₃₂), -C(=O)(Q₃₁), - S(=O)₂(Q₃₁), -P(=O)(Q₃₁)(Q₃₂), or any combination thereof; or
-C(Q₁)(Q₂)(Q₃), -S(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂), and
Q₁ to Q₃ and Q₃₁ to Q₃₃ are each the same as described herein.

In one or more embodiments, R₃₀₁ to R₃₀₃, R₃₀₄ₐ to R₃₀₆ₐ, R_{304b} to R_{306b}, and R₃₁₁ to R₃₁₄ in Formulae 301-1A to 301-2A, R₃₂₁ to R₃₂₆ in Formula 302, and R₄₀₁ to R₄₀₈ in Formulae 401A and 402A to 402D may each independently be:
hydrogen, deuterium, -F, -Cl, -Br, -I, -CD₃, -CD₂H, -CDH₂, -CF₃, -CF₂H, - CFH₂, a cyano group, a nitro group, a C₁-C₆₀ alkyl group, a C₂-C₆₀ alkenyl group, a C₂-C₆₀ alkynyl group, or a C₁-C₆₀ alkoxy group;
a group represented by one (e.g., any one) selected from among Formulae 9-1 to 9-61 or a group represented by one selected from among Formulae 10-1 to 10-348; or
-C(Q₁)(Q₂)(Q₃), -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂): 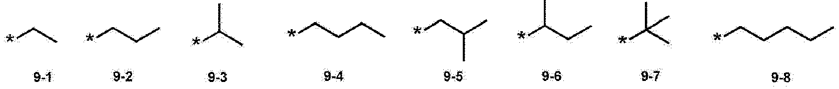 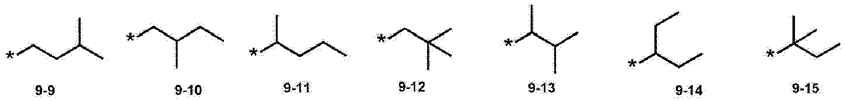 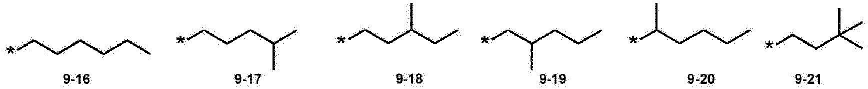 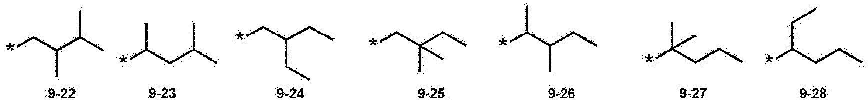 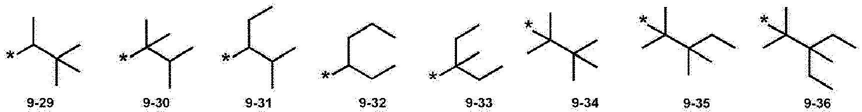 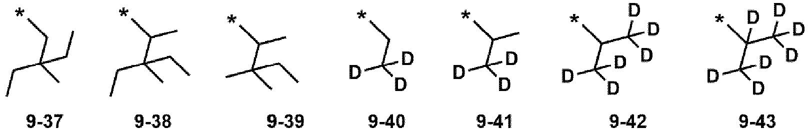 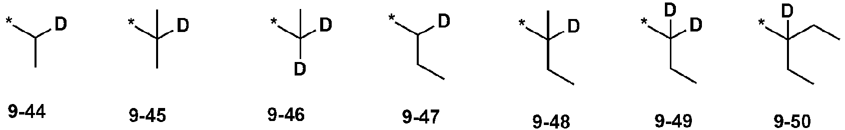 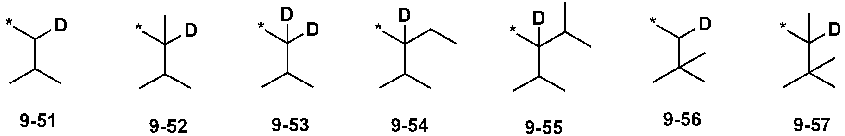 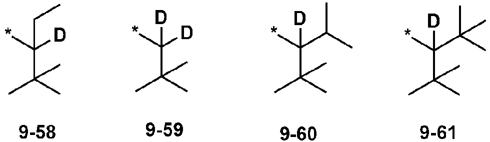 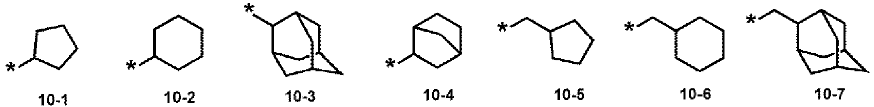 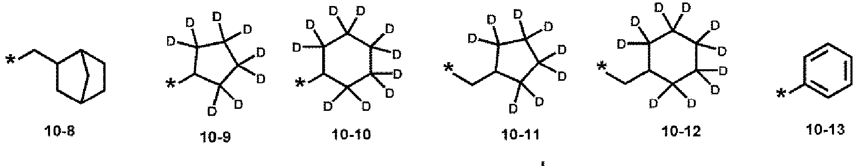 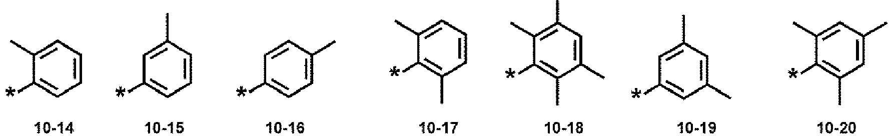 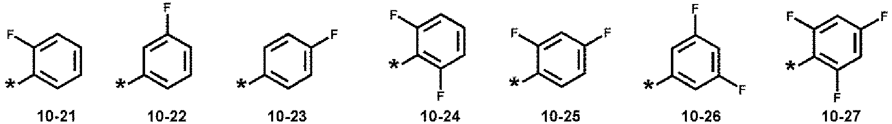 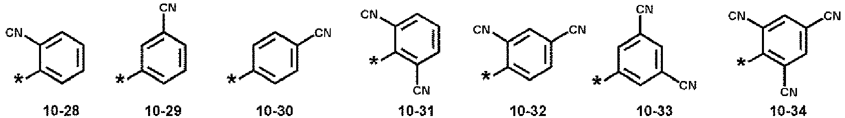 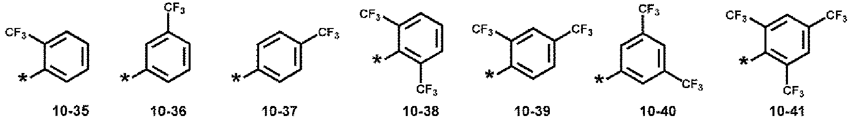 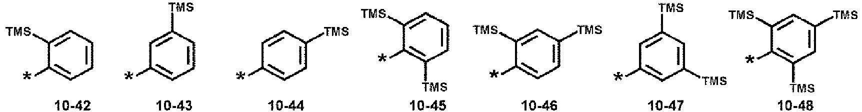 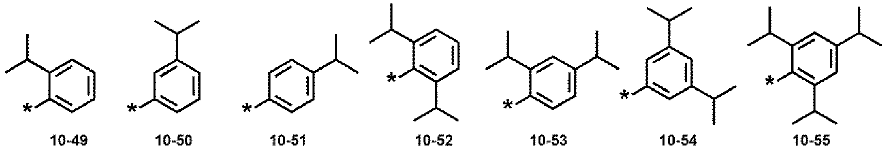 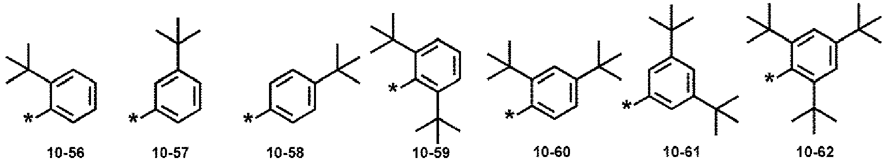 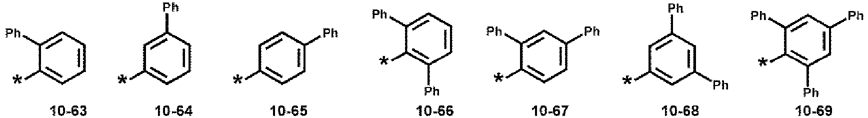 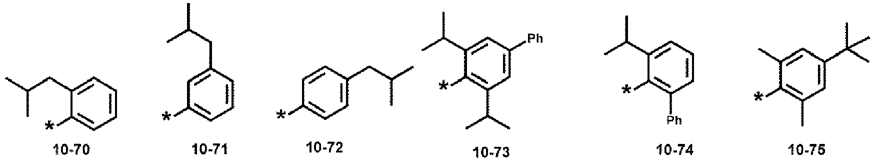 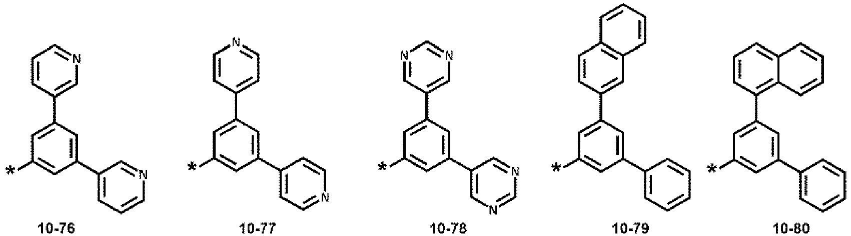 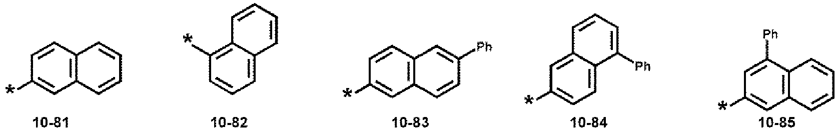 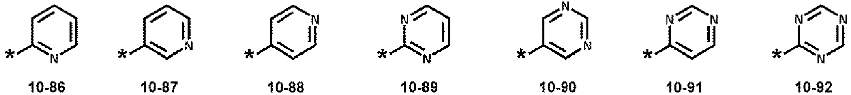 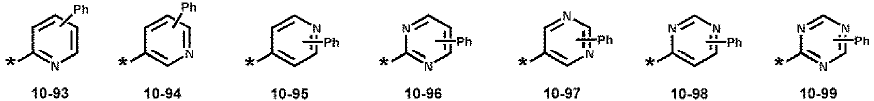 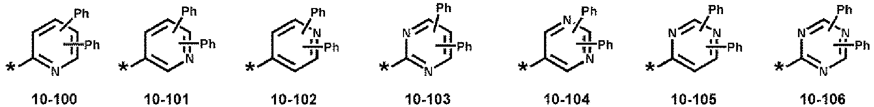 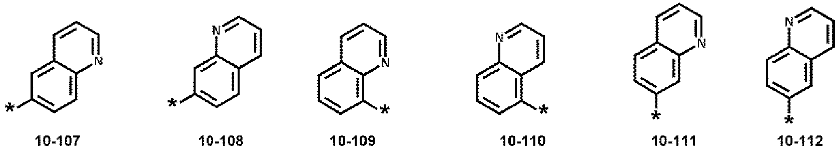 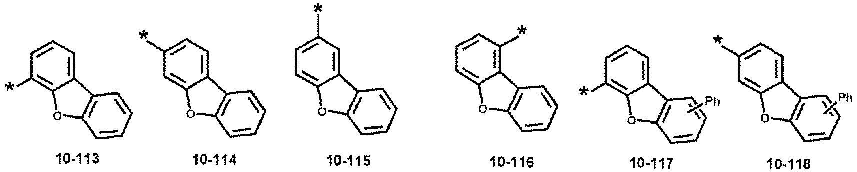 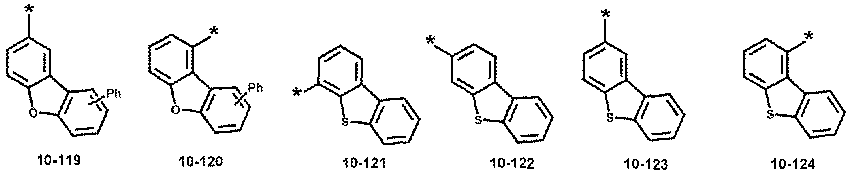 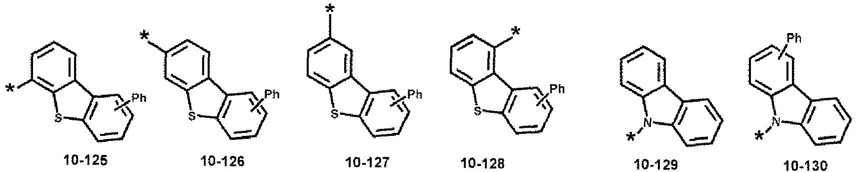 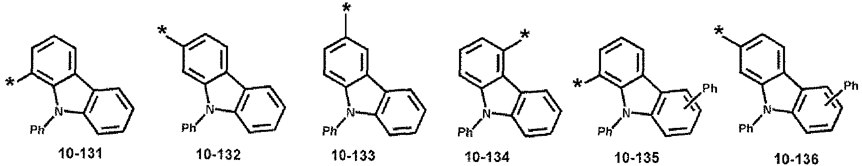 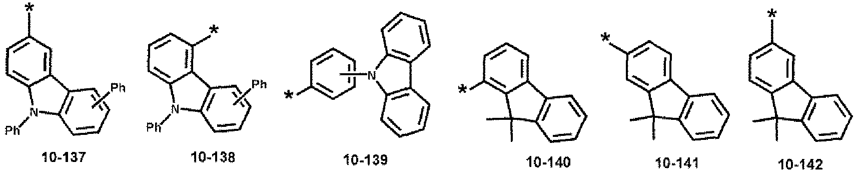 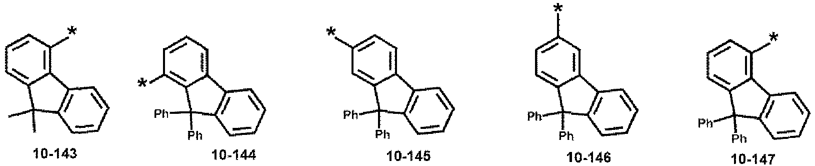 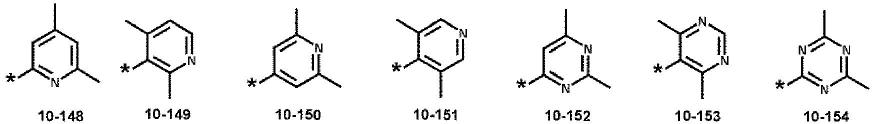 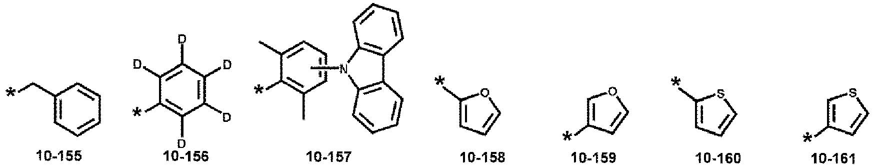 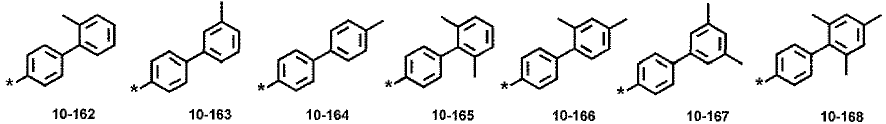 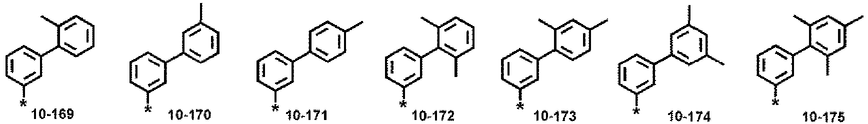 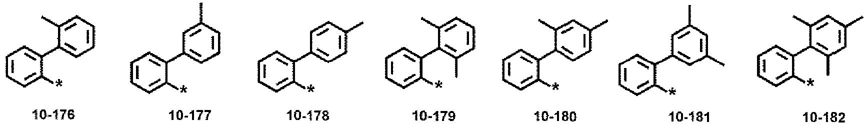 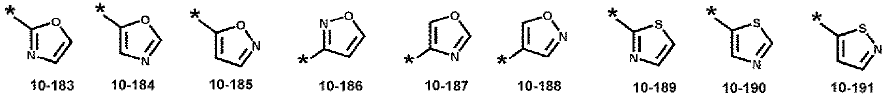 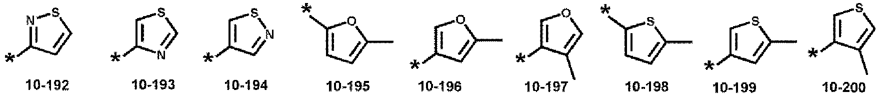 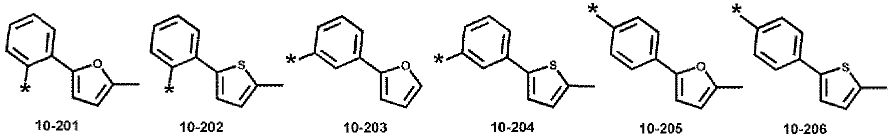 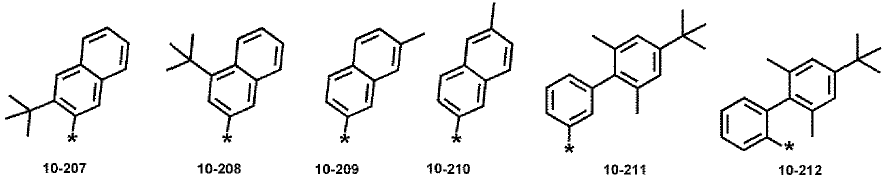 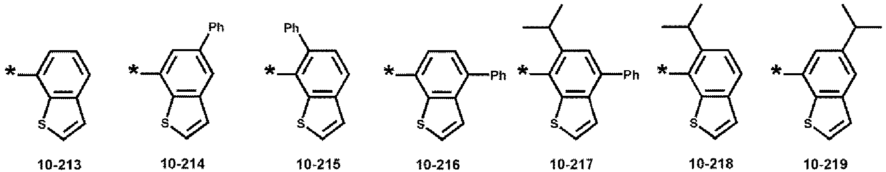 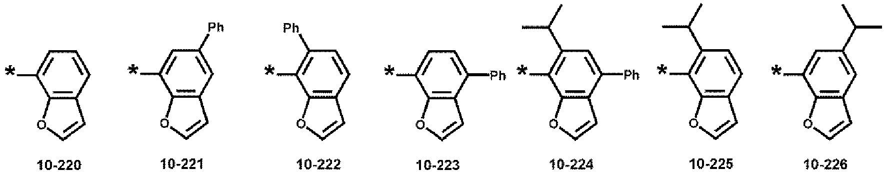 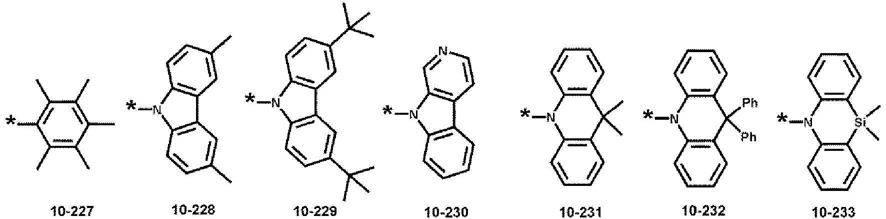 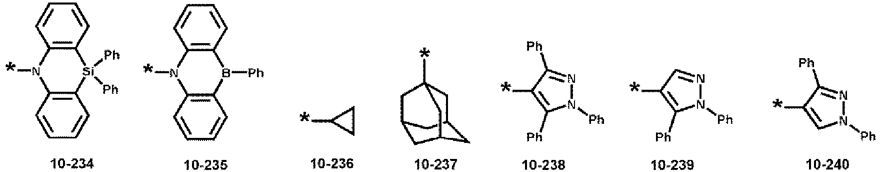 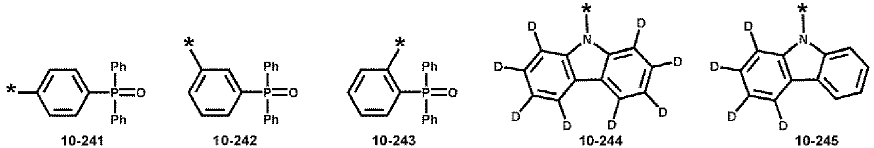 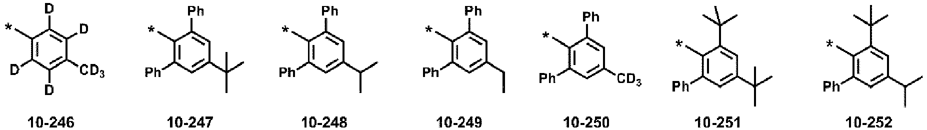 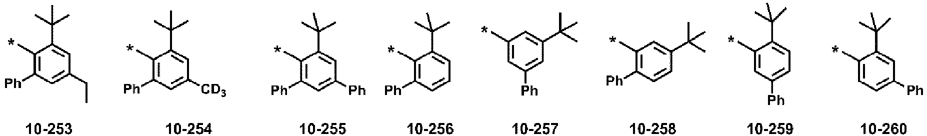 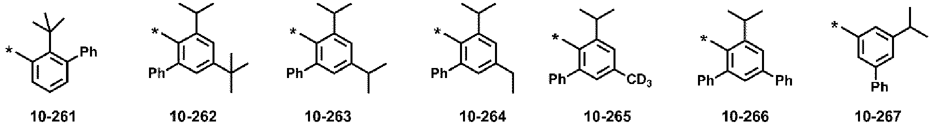 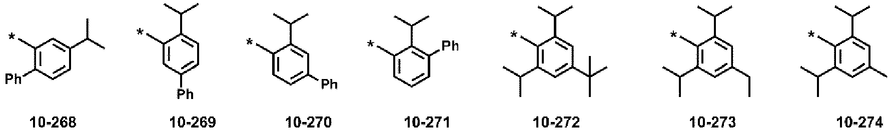 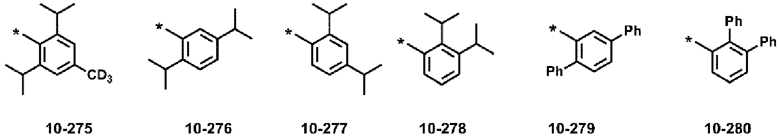 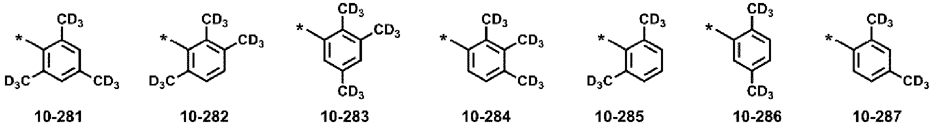 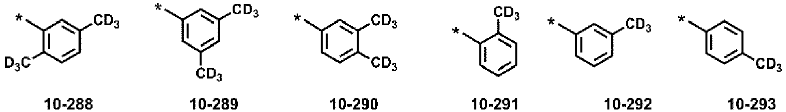 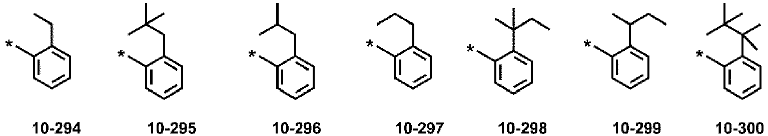 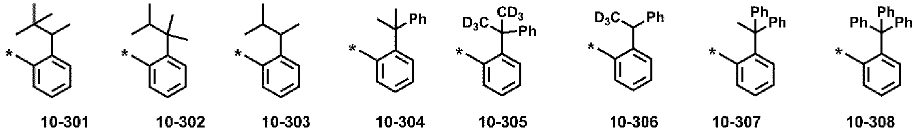 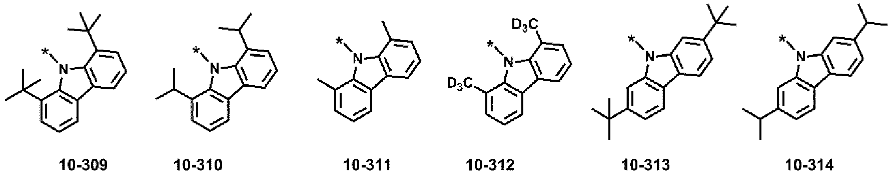 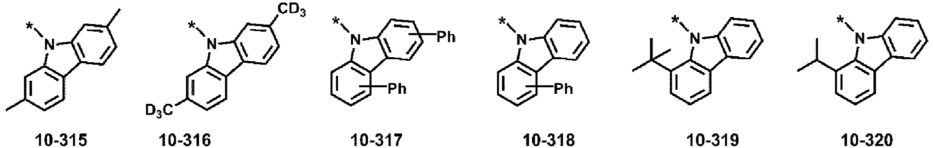 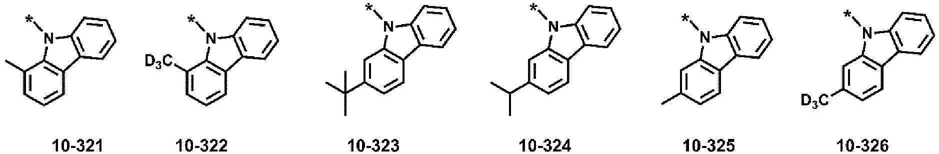 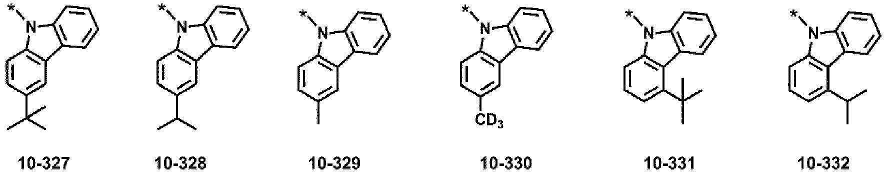 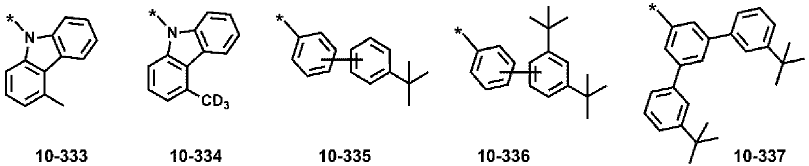 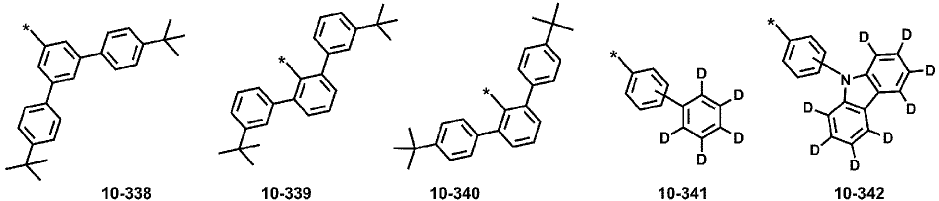 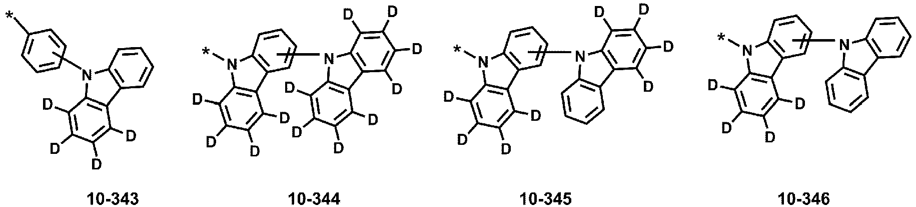 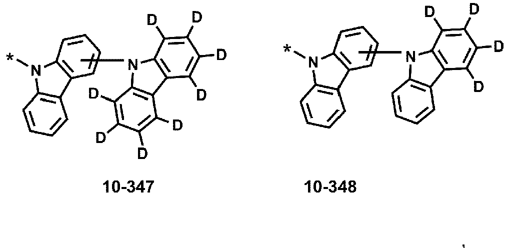
wherein, in Formulae 9-1 to 9-61 and 10-1 to 10-348, * indicates a binding site to a neighboring atom, "Ph" represents a phenyl group, "D" represents deuterium, and "TMS" represents a trimethylsilyl group.
Q₁ to Q₃ are each the same as described herein.

In one or more embodiments, the electron transport region of the light-emitting device may include a hole-blocking layer, and the hole-blocking layer may include a phosphine oxide-containing compound, a silicon-containing compound, or any combination thereof. In one or more embodiments, the hole-blocking layer may directly contact the emission layer.

In one or more embodiments, the light-emitting device may further include a capping layer arranged outside (e.g., on) the first electrode or outside (e.g., on) the second electrode.

In one or more embodiments, the light-emitting device may further include at least one of a first capping layer located outside (e.g., on) the first electrode or a second capping layer located outside (e.g., on) the second electrode, and at least one of the first capping layer and/or the second capping layer may include the heterocyclic compound represented by Formula 1. More details on the first capping layer and/or the second capping layer may be referred to the descriptions provided herein.

In one or more embodiments, the light-emitting device may further include a first capping layer on (e.g., arranged on) the outside of the first electrode. For example, the first capping layer may include the heterocyclic compound represented by Formula 1.

In one or more embodiments, the light-emitting device may further include a second capping layer on (e.g., arranged on) the outside of the second electrode. For example, the second capping layer may include the heterocyclic compound represented by Formula 1.

In one or more embodiments, the light-emitting device may further include a first capping layer on (e.g., arranged outside) the first electrode and a second capping layer on (e.g., arranged outside) the second electrode. For example, at least one of the first capping layer and/or the second capping layer may include the heterocyclic compound represented by Formula 1.

The expression "(an interlayer and/or a capping layer) includes at least one heterocyclic compound" as used herein may include embodiments in which "(an interlayer and/or a capping layer) includes identical heterocyclic compounds represented by Formula 1" and embodiments in which "(an interlayer and/or a capping layer) includes two or more different heterocyclic compounds each represented by Formula 1."

For example, in one or more embodiments, the interlayer and/or the capping layer may include, as the heterocyclic compound, Compound 1 only. In this regard, Compound 1 may be present in the emission layer of the light-emitting device. In one or more embodiments, the interlayer may include, as the heterocyclic compound, Compound 1 and Compound 2. In this regard, Compound 1 and Compound 2 may be present in substantially the same layer (for example, both (e.g., simultaneously) Compound 1 and Compound 2 may be present in the emission layer), or may be present in different layers (for example, Compound 1 may be present in the emission layer, and Compound 2 may be present in the electron transport region).

The term "interlayer" as used herein refers to a single layer and/or all layers between the first electrode and the second electrode of the light-emitting device.

One or more aspects of embodiments of the disclosure are directed toward an electronic apparatus including the light-emitting device. The electronic apparatus may further include a thin-film transistor. For example, in one or more embodiments, the electronic apparatus may further include a thin-film transistor including a source electrode and a drain electrode, wherein the first electrode of the light-emitting device may be electrically connected to the source electrode or the drain electrode of the thin-film transistor. In one or more embodiments, the electronic apparatus may further include a color filter, a color conversion layer, a touch screen layer, a polarizing layer, or any combination thereof. More details on the electronic apparatus may be referred to the descriptions provided herein.

One or more aspects of embodiments of the present disclosure are directed toward an electronic equipment including the electronic apparatus, and the electronic equipment may be at least one of a flat panel display, a curved display, a computer monitor, a medical monitor, a television, a billboard, an indoor light, an outdoor light, a light for signaling, a head-up display, a fully transparent display, a partially transparent display, a flexible display, a rollable display, a foldable display, a stretchable display, a laser printer, a telephone, a mobile phone, a tablet, a phablet, a personal digital assistant (PDA), a wearable device, a laptop computer, a digital camera, a camcorder, a viewfinder, a micro display, a 3D display, a virtual reality display, an augmented reality display, a vehicle, a video wall including multiple displays tiled together, a theater screen, a stadium screen, a phototherapy device, or a signboard.

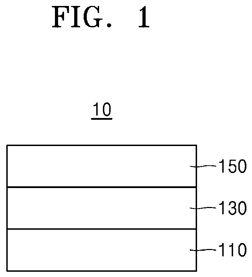

### Description of FIG. 1

FIG. 1 is a schematic cross-sectional view of a light-emitting device 10 according to one or more embodiments of the present disclosure. The light-emitting device 10 may include a first electrode 110, an interlayer 130, and a second electrode 150.

Hereinafter, a structure of the light-emitting device 10 according to one or more embodiments and a method of manufacturing the light-emitting device 10 will be described in more detail with reference to FIG. 1.

### First electrode 110

In FIG. 1, in one or more embodiments, a substrate may be additionally provided and arranged under the first electrode 110 and/or on the second electrode 150. As the substrate, a glass substrate or a plastic substrate may be used. In one or more embodiments, the substrate may be a flexible substrate and may include plastics with excellent or suitable heat resistance and durability, such as polyimide, polyethylene terephthalate (PET), polycarbonate, polyethylene naphthalate, polyarylate (PAR), polyetherimide, or any combination thereof.

The first electrode 110 may be formed by, for example, depositing or sputtering a material for forming the first electrode 110 on the substrate. When the first electrode 110 is an anode, a material for forming the first electrode 110 may be a high-work function material that facilitates injection of holes.

The first electrode 110 may be a reflective electrode, a transflective electrode, or a transmissive electrode. In one or more embodiments, If (e.g., when) the first electrode 110 is a transmissive electrode, a material for forming the first electrode 110 may include indium tin oxide (ITO), indium zinc oxide (IZO), tin oxide (SnO₂), zinc oxide (ZnO), or any combination thereof. In one or more embodiments, if (e.g., when) the first electrode 110 is a transflective electrode or a reflective electrode, a material for forming the first electrode 110 may include magnesium (Mg), silver (Ag), aluminium (Al), aluminium-lithium (Al-Li), calcium (Ca), magnesium-indium (Mg-In), magnesium-silver (Mg-Ag), or any combination thereof.

The first electrode 110 may have a single-layer structure including (e.g., consisting of) a single layer or a multilayer structure including a plurality of layers. In one or more embodiments, the first electrode 110 may have a three-layer structure of ITO/Ag/ITO.

### Interlayer 130

The interlayer 130 may be arranged above (e.g., on) the first electrode 110. The interlayer 130 may include an emission layer.

The interlayer 130 may further include a hole transport region between the first electrode 110 and the emission layer, and an electron transport region between the emission layer and the second electrode 150.

In one or more embodiments, the interlayer 130 may further include, in addition to one or more suitable organic materials, a metal-containing compound such as an organometallic compound, an inorganic material such as quantum dots, and/or the like.

In one or more embodiments, the interlayer 130 may include, i) two or more emitting units sequentially stacked between the first electrode 110 and the second electrode 150, and ii) a charge generation layer between adjacent emitting units among the two or more emitting units. When the interlayer 130 includes the two or more emitting units and the charge generation layer as described herein, the light-emitting device 10 may be a tandem light-emitting device.

### Hole transport region in interlayer 130

The hole transport region may have: i) a single-layer structure including (e.g., consisting of) a single layer including (e.g., consisting of) a single material, ii) a single-layer structure including (e.g., consisting of) a single layer including (e.g., consisting of) a plurality of materials that are different from each other, or iii) a multilayer structure including a plurality of layers including a plurality of materials that are different from each other.

The hole transport region may include a hole injection layer, a hole transport layer, an emission auxiliary layer, an electron-blocking layer, or any combination thereof.

For example, in one or more embodiments, the hole transport region may have a multi-layer structure including a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron-blocking layer structure, wherein constituent layers in each structure are sequentially stacked from the first electrode 110 in the stated order.

In one or more embodiments, the hole transport region may include a compound represented by Formula 201, a compound represented by Formula 202, or any combination thereof: 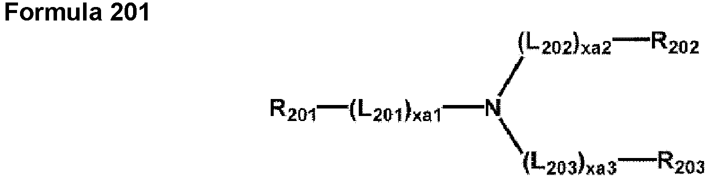 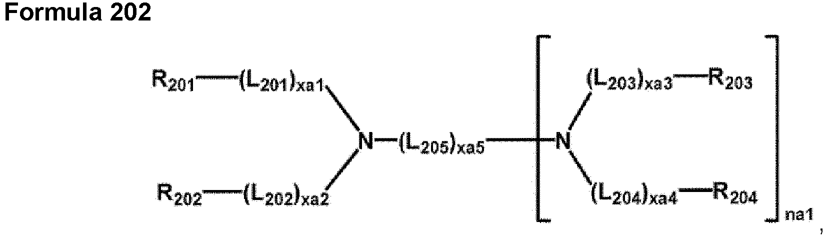 wherein, in Formulae 201 and 202,
L₂₀₁ to L₂₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
L₂₀₅ may be *-O-*', *-S-*', *-N(Q₂₀₁)-*', a C₁-C₂₀ alkylene group unsubstituted or substituted with at least one R₁₀ₐ, a C₂-C₂₀ alkenylene group unsubstituted or substituted with at least one R₁₀ₐ, a C₃-C₆₀ carbocyclic group unsubstituted or substituted with at least one R₁₀ₐ, or a C₁-C₆₀ heterocyclic group unsubstituted or substituted with at least one R₁₀ₐ,
xa1 to xa4 may each independently be an integer from 0 to 5,
xa5 may be an integer from 1 to 10,
R₂₀₁ to R₂₀₄ and Q₂₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
R₂₀₁ and R₂₀₂ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ, to form a C₆-C₆₀ polycyclic group (for example, a carbazole group) unsubstituted or substituted with at least one R₁₀ₐ (for example, see Compound HT16),
R₂₀₃ and R₂₀₄ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ, to form a C₆-C₆₀ polycyclic group unsubstituted or substituted with at least one R₁₀ₐ, and
na1 may be an integer from 1 to 4.

In one or more embodiments, each of Formulae 201 and 202 may include at least one selected from among groups represented by Formulae CY201 to CY217: 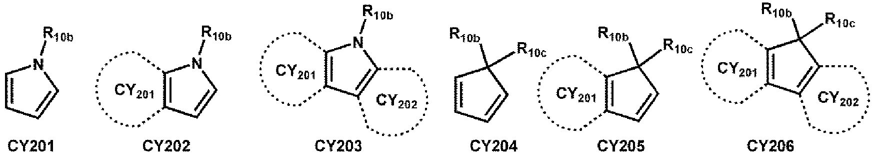 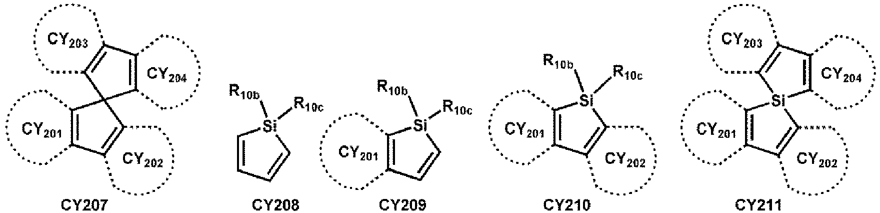 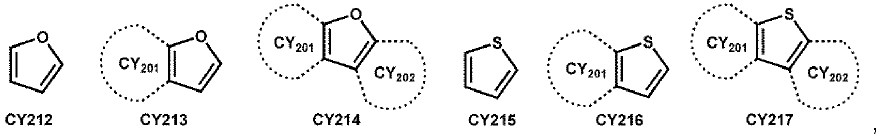 wherein, in Formulae CY201 to CY217, R_{10b} and R_{10c} may each be the same as described with respect to R₁₀ₐ, ring CY₂₀₁ to ring CY₂₀₄ may each independently be a C₃-C₂₀ carbocyclic group or a C₁-C₂₀ heterocyclic group, and at least one hydrogen in Formulae CY201 to CY217 may be unsubstituted or substituted with R₁₀ₐ.

In one or more embodiments, in Formulae CY201 to CY217, ring CY₂₀₁ to ring CY₂₀₄ may each independently be a benzene group, a naphthalene group, a phenanthrene group, or an anthracene group.

In one or more embodiments, each of Formulae 201 and 202 may include at least one selected from among the groups represented by Formulae CY201 to CY203.

In one or more embodiments, Formula 201 may include at least one selected from among the groups represented by Formulae CY201 to CY203 and at least one selected from among the groups represented by Formulae CY204 to CY217.

In one or more embodiments, in Formula 201, xa1 may be 1, R₂₀₁ may be a group represented by any one selected from among Formulae CY201 to CY203, xa2 may be 0, and R₂₀₂ may be a group represented by any one selected from among Formulae CY204 to CY207.

In one or more embodiments, each of Formulae 201 and 202 may not include (e.g., may exclude) any of the groups represented by Formulae CY201 to CY203.

In one or more embodiments, each of Formulae 201 and 202 may not include (e.g., may exclude) any of the groups represented by Formulae CY201 to CY203 and may include at least one selected from among the groups represented by Formulae CY204 to CY217.

In one or more embodiments, each of Formulae 201 and 202 may not include (e.g., may exclude) any of the groups represented by Formulae CY201 to CY217.

In one or more embodiments, the hole transport region may include one of (e.g., selected from among) Compounds HT1 to HT46, 4,4',4"-[tris(3-methylphenyl)phenylamino] triphenylamine (m-MTDATA), 4,4',4"-tris(N,N-diphenylamino)triphenylamine (TDATA), 4,4',4"-tris[N-(2-naphthyl)-N-phenylamino]-triphenylamine (2-TNATA), N,N'-di(naphthalen-1-yl)-N,N'-diphenyl-benzidine (NPB(NPD)), β-NPB, N,N'-bis(3-methylphenyl)-N,N'-diphenyl-[1,1'-biphenyl]-4,4'-diamine (TPD), Spiro-TPD, Spiro-NPB, methylated NPB, 4,4'-cyclohexylidene bis[N,N-bis(4-methylphenyl)benzenamine] (TAPC), 4,4'-bis[N,N'-(3-tolyl)amino]-3,3'-dimethylbiphenyl (HMTPD), 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), or any combination thereof:

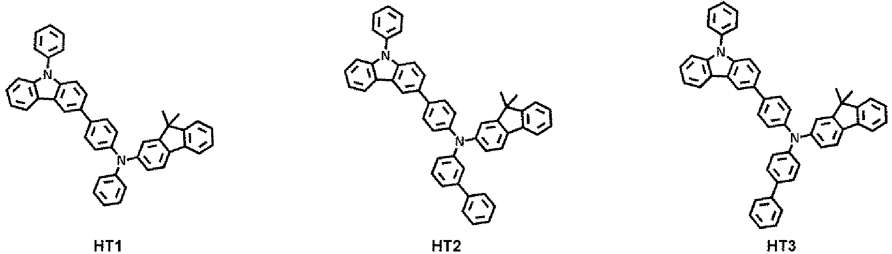

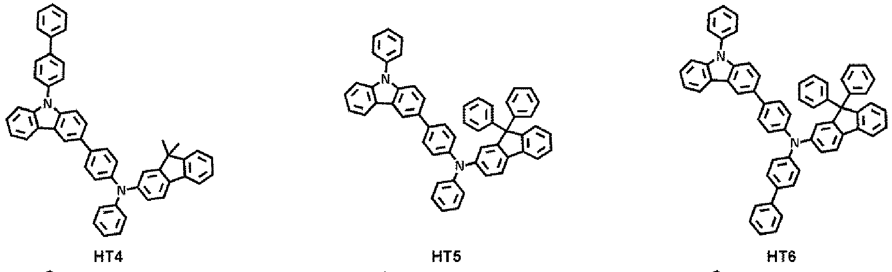

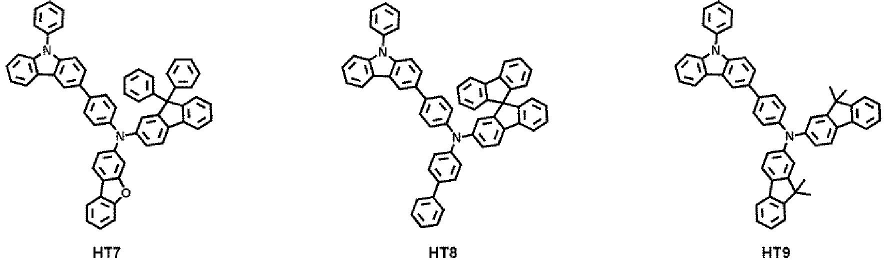

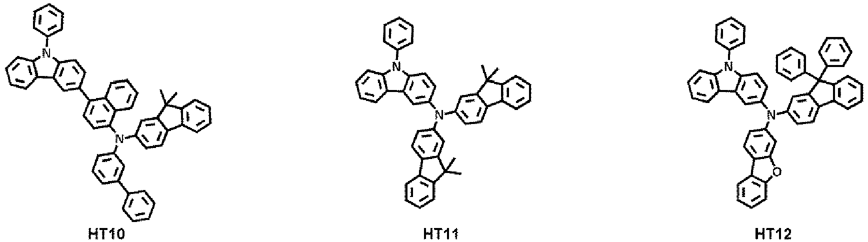

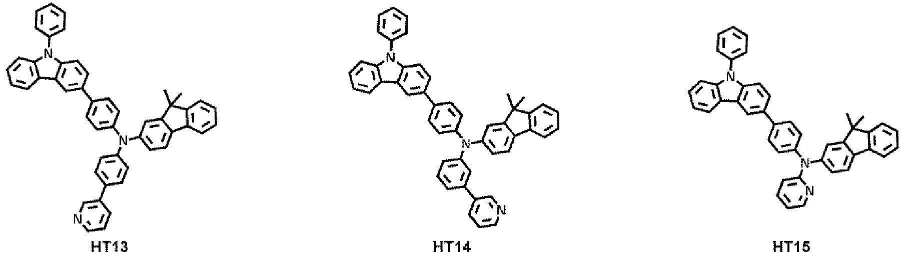

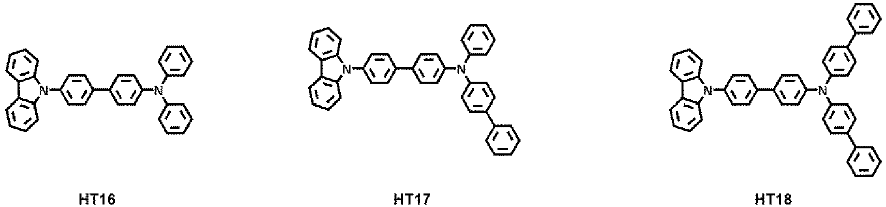

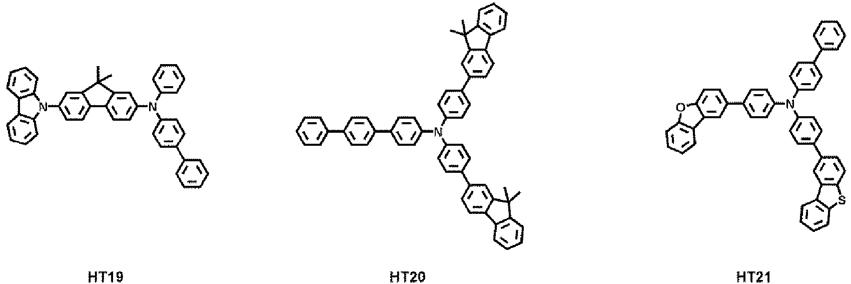

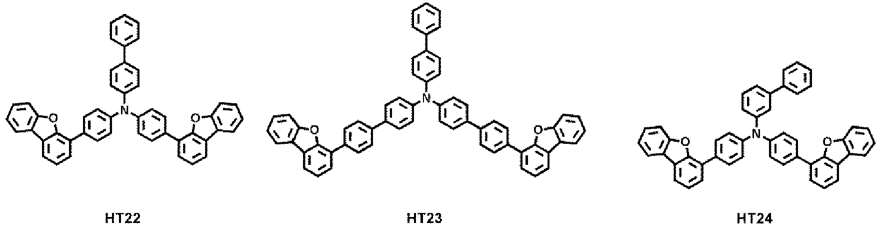

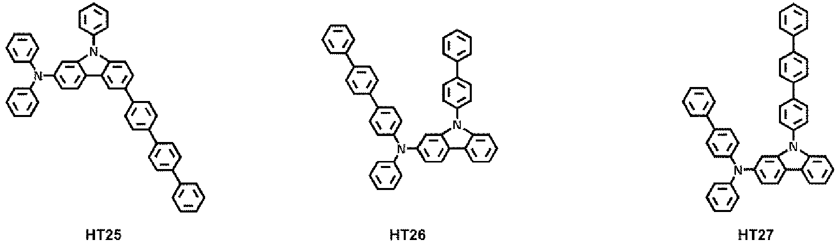

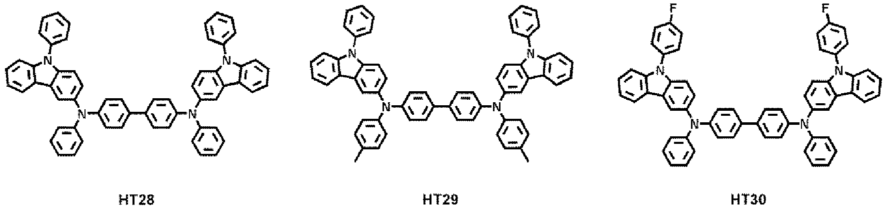

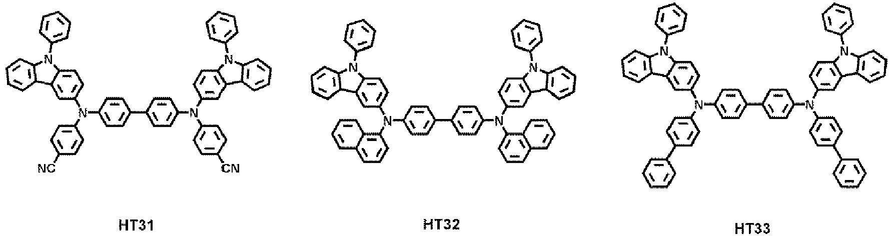

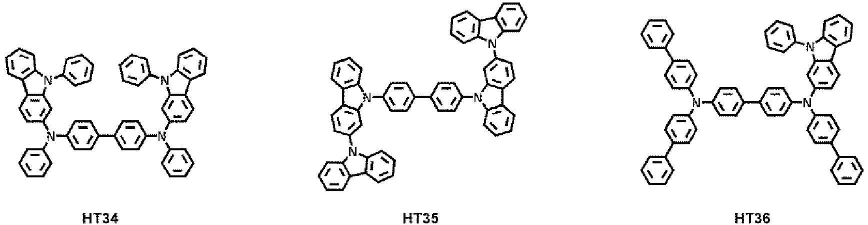

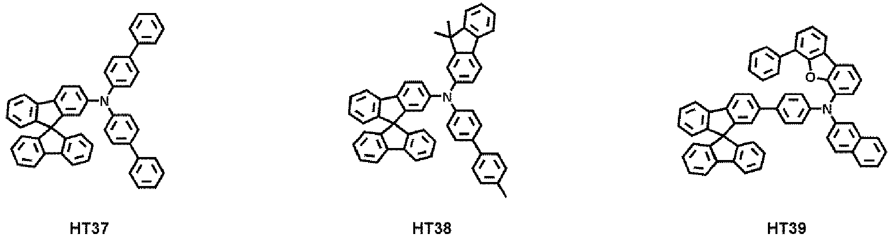

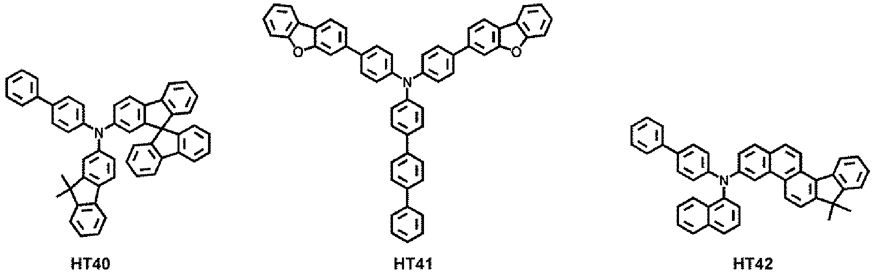

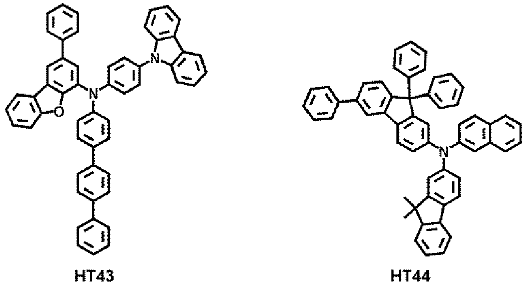

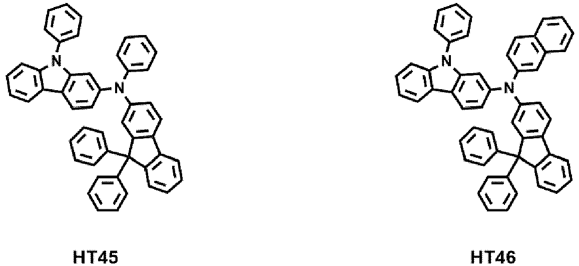

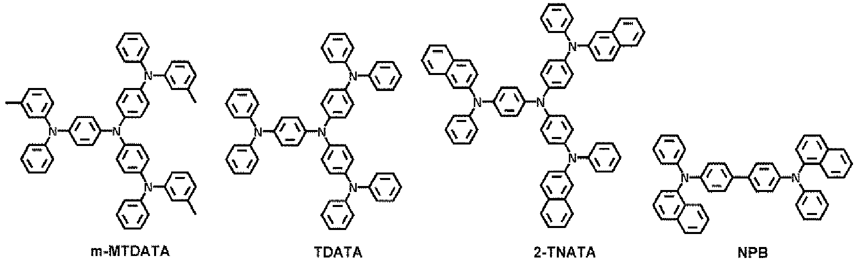

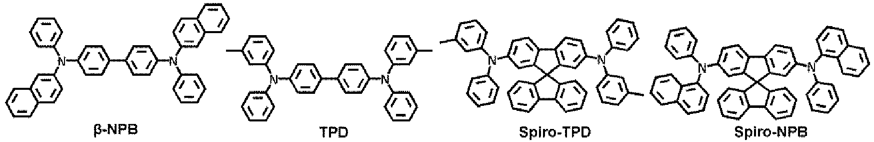

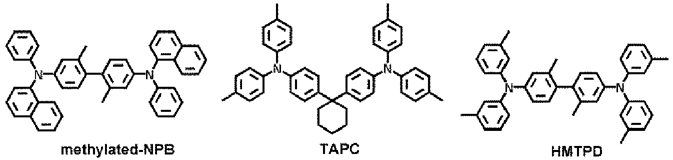

A thickness of the hole transport region may be about 50 Angstroms (Å) to about 10,000 Å, for example, about 100 Å to about 4,000 Å. When the hole transport region includes a hole injection layer, a hole transport layer, or any combination thereof, a thickness of the hole injection layer may be about 100 Å to about 9,000 Å, for example, about 100 Å to about 1,000 Å, and a thickness of the hole transport layer may be about 50 Å to about 2,000 Å, for example, about 100 Å to about 1,500 Å. When the thicknesses of the hole transport region, the hole injection layer, and the hole transport layer are within the ranges described above, satisfactory hole transporting characteristics may be obtained without a substantial increase in driving voltage.

The emission auxiliary layer may increase light emission efficiency by compensating for an optical resonance distance according to the wavelength of light emitted by the emission layer, and the electron-blocking layer may block the leakage of electrons from the emission layer to the hole transport region. Materials that may be included in the hole transport region may be included in the emission auxiliary layer and the electron-blocking layer.

### p-dopant

In one or more embodiments, the hole transport region may further include, in addition to one or more of these aforementioned materials, a charge-generation material for the improvement of conductive properties. The charge-generation material may be uniformly (e.g., substantially uniformly) or non-uniformly dispersed in the hole transport region (for example, in the form of a single layer including (e.g., consisting of) a charge-generation material).

The charge-generation material may be, for example, a p-dopant.

For example, the lowest unoccupied molecular orbital (LUMO) energy level of the p-dopant may be less than or equal to -3.5 eV.

In one or more embodiments, the p-dopant may include a quinone derivative, a cyano group-containing compound, a compound including an element EL1 and an element EL2, or any combination thereof.

Non-limiting examples of the quinone derivative may include tetracyanoquinodimethane (TCNQ) and 2,3,5,6-tetrafluoro-7,7,8,8-tetracyanoquinodimethane (F4-TCNQ).

Non-limiting examples of the cyano group-containing compound may include dipyrazino[2,3-f: 2',3'-h] quinoxaline-2,3,6,7,10,11-hexacarbonitrile (HAT-CN) and a compound represented by Formula 221.

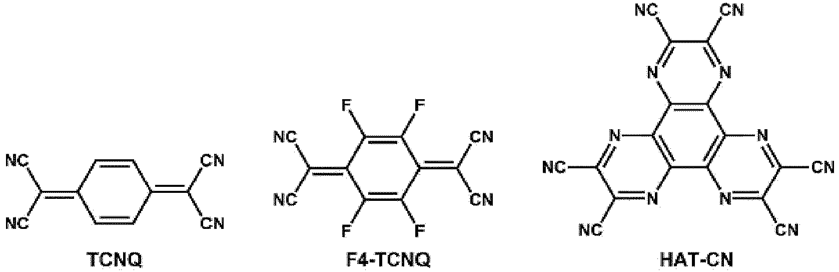

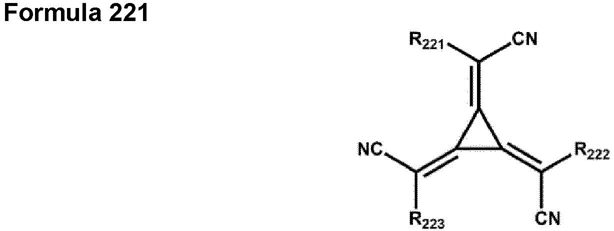

In Formula 221,
R₂₂₁ to R₂₂₃ may each independently be a C₃-C₆₀ carbocyclic group unsubstituted or substituted with at least one R₁₀ₐ or a C₁-C₆₀ heterocyclic group unsubstituted or substituted with at least one R₁₀ₐ, and
at least one selected from among R₂₂₁ to R₂₂₃ may each independently be a C₃-C₆₀ carbocyclic group or a C₁-C₆₀ heterocyclic group, each substituted with: a cyano group; -F; -Cl; -Br; -I; a C₁-C₂₀ alkyl group substituted with a cyano group, -F, -Cl, -Br, - I, or any combination thereof; or any combination thereof.

In the compound including the element EL1 and the element EL2, the element EL1 may be a metal, a metalloid, or a (e.g., any suitable) combination thereof, and the element EL2 may be a non-metal, a metalloid, or a (e.g., any suitable)combination thereof.

Non-limiting examples of the metal may include an alkali metal (for example, lithium (Li), sodium (Na), potassium (K), rubidium (Rb), cesium (Cs), and/or the like); an alkaline earth metal (for example, beryllium (Be), magnesium (Mg), calcium (Ca), strontium (Sr), barium (Ba), and/or the like); a transition metal (for example, titanium (Ti), zirconium (Zr), hafnium (Hf), vanadium (V), niobium (Nb), tantalum (Ta), chromium (Cr), molybdenum (Mo), tungsten (W), manganese (Mn), technetium (Tc), rhenium (Re), iron (Fe), ruthenium (Ru), osmium (Os), cobalt (Co), rhodium (Rh), iridium (Ir), nickel (Ni), palladium (Pd), platinum (Pt), copper (Cu), silver (Ag), gold (Au), and/or the like); a post-transition metal (for example, zinc (Zn), indium (In), tin (Sn), and/or the like); and a lanthanide metal (for example, lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), and/or the like).

Non-limiting examples of the metalloid may include silicon (Si), antimony (Sb), and/or tellurium (Te).

Non-limiting examples of the non-metal may include oxygen (O) and/or a halogen (for example, F, Cl, Br, I, and/or the like).

Non-limiting examples of the compound including the element EL1 and the element EL2 may include a metal oxide, a metal halide (for example, a metal fluoride, a metal chloride, a metal bromide, a metal iodide, and/or the like), a metalloid halide (for example, a metalloid fluoride, a metalloid chloride, a metalloid bromide, a metalloid iodide, and/or the like), a metal telluride, or any combination thereof.

Non-limiting examples of the metal oxide may include a tungsten oxide (for example, WO, W₂O₃, WO₂, WO₃, W₂O₅, and/or the like), a vanadium oxide (for example, VO, V₂O₃, VO₂, V₂O₅, and/or the like), a molybdenum oxide (MoO, Mo₂O₃, MoO₂, MoO₃, Mo₂O₅, and/or the like), and/or a rhenium oxide (for example, ReO₃, and/or the like).

Non-limiting examples of the metal halide may include an alkali metal halide, an alkaline earth metal halide, a transition metal halide, a post-transition metal halide, and/or a lanthanide metal halide.

Non-limiting examples of the alkali metal halide may include LiF, NaF, KF, RbF, CsF, LiCl, NaCl, KCI, RbCl, CsCl, LiBr, NaBr, KBr, RbBr, CsBr, Lil, Nal, KI, Rbl, and/or CsI.

Non-limiting examples of the alkaline earth metal halide may include BeF₂, MgF₂, CaF₂, SrF₂, BaF₂, BeCl₂, MgCl₂, CaCl₂, SrCl₂, BaCl₂, BeBr₂, MgBr₂, CaBr₂, SrBr₂, BaBr₂, BeI₂, Mgl₂, Cal₂, SrI₂, and/or BaI₂.

Non-limiting examples of the transition metal halide may include a titanium halide (for example, TiF₄, TiCl₄, TiBr₄, TiI₄, and/or the like), a zirconium halide (for example, ZrF₄, ZrCl₄, ZrBr₄, ZrI₄, and/or the like), a hafnium halide (for example, HfF₄, HfCl₄, HfBr₄, HfI₄, and/or the like), a vanadium halide (for example, VF₃, VCl₃, VBr₃, VI₃, and/or the like), a niobium halide (for example, NbF₃, NbCl₃, NbBr₃, NbI₃, and/or the like), a tantalum halide (for example, TaF₃, TaCl₃, TaBr₃, TaI₃, and/or the like), a chromium halide (for example, CrF₃, CrCl₃, CrBr₃, CrI₃, and/or the like), a molybdenum halide (for example, MoF₃, MoCl₃, MoBr₃, MoI₃, and/or the like), a tungsten halide (for example, WF₃, WCl₃, WBr₃, WI₃, and/or the like), a manganese halide (for example, MnF₂, MnCl₂, MnBr₂, MnI₂, and/or the like), a technetium halide (for example, TcF₂, TcCl₂, TcBr₂, TcI₂, and/or the like), a rhenium halide (for example, ReF₂, ReCl₂, ReBr₂, ReI₂, and/or the like), an iron(II) halide (for example, FeF₂, FeCl₂, FeBr₂, FeI₂, and/or the like), a ruthenium halide (for example, RuF₂, RuCl₂, RuBr₂, Rul₂, and/or the like), an osmium halide (for example, OsF₂, OsCl₂, OsBr₂, OsI₂, and/or the like), a cobalt halide (for example, CoF₂, CoCl₂, CoBr₂, CoI₂, and/or the like), a rhodium halide (for example, RhF₂, RhCl₂, RhBr₂, RhI₂, and/or the like), an iridium halide (for example, IrF₂, IrCl₂, IrBr₂, IrI₂, and/or the like), a nickel halide (for example, NiF₂, NiCl₂, NiBr₂, NiI₂, and/or the like), a palladium halide (for example, PdF₂, PdCl₂, PdBr₂, PdI₂, and/or the like), a platinum halide (for example, PtF₂, PtCl₂, PtBr₂, PtI₂, and/or the like), a copper(I) halide (for example, CuF, CuCl, CuBr, Cul, and/or the like), a silver halide (for example, AgF, AgCl, AgBr, AgI, and/or the like), and/or a gold halide (for example, AuF, AuCl, AuBr, Aul, and/or the like).

Non-limiting examples of the post-transition metal halide may include a zinc halide (for example, ZnF₂, ZnCl₂, ZnBr₂, ZnI₂, and/or the like), an indium halide (for example, InI₃, and/or the like), and/or a tin halide (for example, SnI₂, and/or the like).

Non-limiting examples of the lanthanide metal halide may include YbF, YbF₂, YbF₃, SmF₃, YbCl, YbCl₂, YbCl₃, SmCl₃, YbBr, YbBr₂, YbBr₃, SmBr₃, Ybl, YbI₂, YbI₃, and SmI₃.

Non-limiting examples of the metalloid halide may include an antimony halide (for example, SbCl₅, and/or the like).

Non-limiting examples of the metal telluride may include an alkali metal telluride (for example, Li₂Te, Na₂Te, K₂Te, Rb₂Te, Cs₂Te, and/or the like), an alkaline earth metal telluride (for example, BeTe, MgTe, CaTe, SrTe, BaTe, and/or the like), a transition metal telluride (for example, TiTe₂, ZrTe₂, HfTe₂, V₂Te₃, Nb₂Te₃, Ta₂Te₃, Cr₂Te₃, Mo₂Te₃, W₂Te₃, MnTe, TcTe, ReTe, FeTe, RuTe, OsTe, CoTe, RhTe, IrTe, NiTe, PdTe, PtTe, Cu₂Te, CuTe, Ag₂Te, AgTe, Au₂Te, and/or the like), a post-transition metal telluride (for example, ZnTe, and/or the like), and/or a lanthanide metal telluride (for example, LaTe, CeTe, PrTe, NdTe, PmTe, EuTe, GdTe, TbTe, DyTe, HoTe, ErTe, TmTe, YbTe, LuTe, and/or the like).

### Emission layer in interlayer 130

When the light-emitting device 10 is a full-color light-emitting device, the emission layer may be patterned into a red emission layer, a green emission layer, and/or a blue emission layer, according to a sub-pixel. In one or more embodiments, the emission layer may have a stacked structure of two or more layers selected from among a red emission layer, a green emission layer, and a blue emission layer, in which the two or more layers contact each other or are separated from each other, to emit white light (e.g., combined white light). In one or more embodiments, the emission layer may include two or more materials selected from among a red light-emitting material, a green light-emitting material, and a blue light-emitting material, in which the two or more materials are mixed with each other in a single layer, to emit white light (e.g., combined white light).

The emission layer may include a host and a dopant. The dopant may include a phosphorescent dopant, a fluorescent dopant, or any combination thereof.

An amount of the dopant in the emission layer may be from about 0.01 part by weight to about 15 parts by weight based on 100 parts by weight of the host.

In one or more embodiments, the emission layer may include a quantum dot.

In one or more embodiments, the emission layer may include a delayed fluorescence material. The delayed fluorescence material may act as a host or a dopant in the emission layer.

A thickness of the emission layer may be about 100 Å to about 1,000 Å, for example, about 200 Å to about 600 Å. When the thickness of the emission layer is within this range, excellent or suitable luminescence characteristics may be obtained without a substantial increase in driving voltage.

### Host

In one or more embodiments, the host may include a compound represented by Formula 301:

**Formula 301** **[Ar₃₀₁]_{xb11}-[(L₃₀₁)_{xb1}-R₃₀₁]xb21,**

wherein, in Formula 301,
Ar₃₀₁ and L₃₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xb11 may be 1, 2, or 3,
xb1 may be an integer from 0 to 5,
R₃₀₁ may be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), or -P(=O)(Q₃₀₁)(Q₃₀₂),
xb21 may be an integer from 1 to 5, and
Q₃₀₁ to Q₃₀₃ are each the same as described with respect to Q₁.

In one or more embodiments, if (e.g., when) xb11 in Formula 301 is 2 or more, two or more of Ar₃₀₁(s) may be linked to each other via a single bond.

In one or more embodiments, the host may include a compound represented by Formula 301-1, a compound represented by Formula 301-2, or any combination thereof: 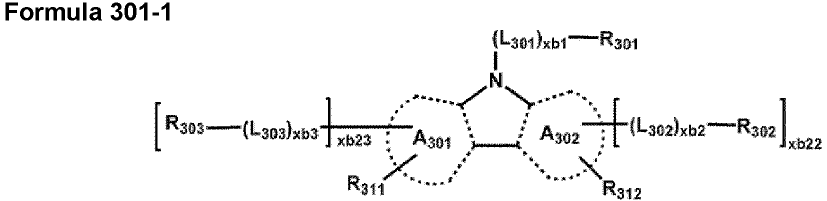 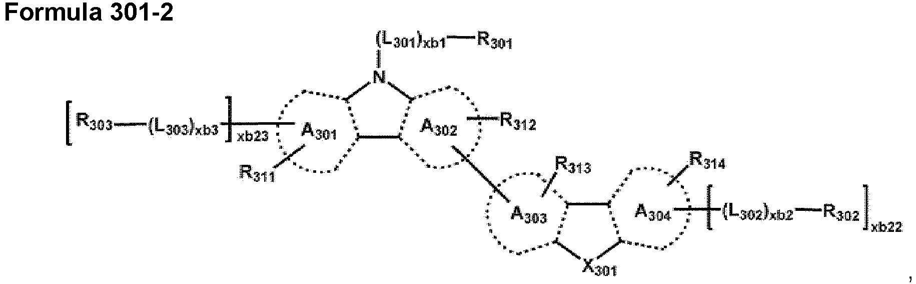 wherein, in Formulae 301-1 and 301-2,
ring A₃₀₁ to ring A₃₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
X₃₀₁ may be O, S, N[(L₃₀₄)_{xb4}-R₃₀₄], C(R₃₀₄)(R₃₀₅), or Si(R₃₀₄)(R₃₀₅),
xb22 and xb23 may each independently be 0, 1, or 2,
L₃₀₁, xb1, and R₃₀₁ are each the same as described herein,
L₃₀₂ to L₃₀₄ may each independently be the same as described with respect to L₃₀₁,
xb2 to xb4 may each independently be the same as described with respect to xb1, and
R₃₀₂ to R₃₀₅ and R₃₁₁ to R₃₁₄ are each the same as described with respect to R301.

In one or more embodiments, the host may include an alkaline earth metal complex, a post-transition metal complex, or any combination thereof. In one or more embodiments, the host may include a Be complex (for example, Compound H55), an Mg complex, a Zn complex, or any combination thereof.

In one or more embodiments, the host may include: one of (e.g., include at least one or be any one selected from among) Compounds H1 to H128; 9,10-di(2-naphthyl)anthracene (ADN); 2-methyl-9,10-bis(naphthalen-2-yl)anthracene (MADN); 9,10-di-(2-naphthyl)-2-t-butyl-anthracene (TBADN); 4,4'-bis(N-carbazolyl)-1,1'-biphenyl (CBP);1,3-di(carbazol-9-yl)benzene (mCP); 1,3,5-tri(carbazol-9-yl)benzene (TCP); or any combination thereof:

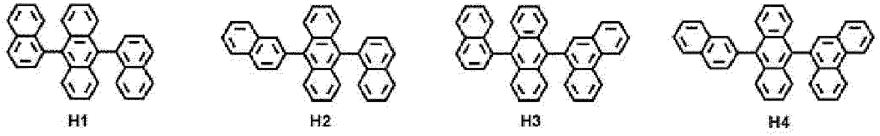

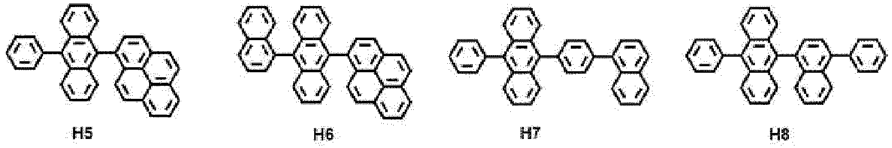

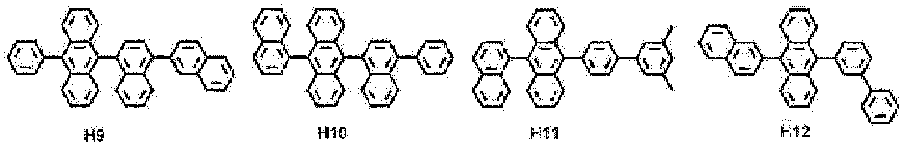

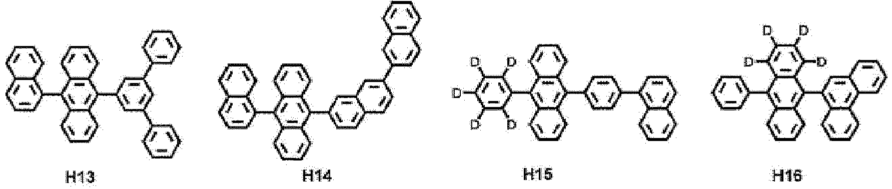

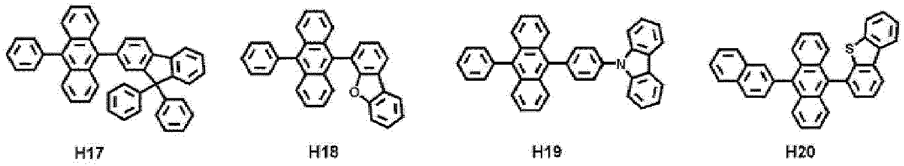

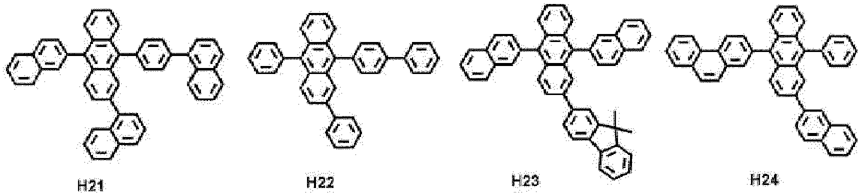

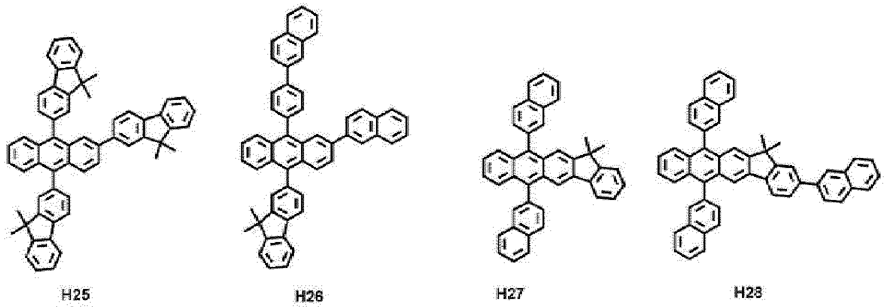

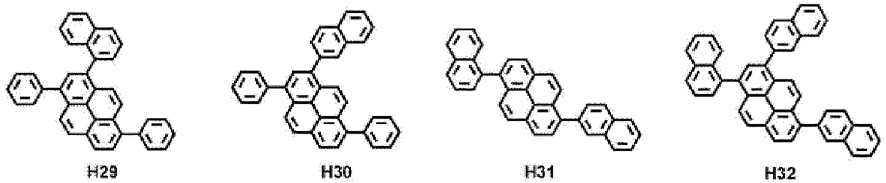

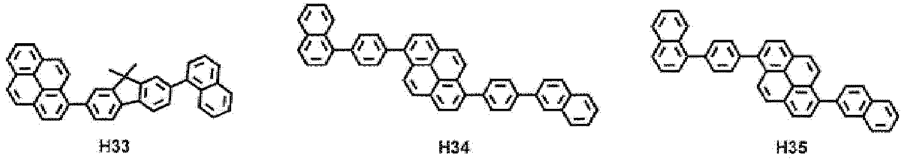

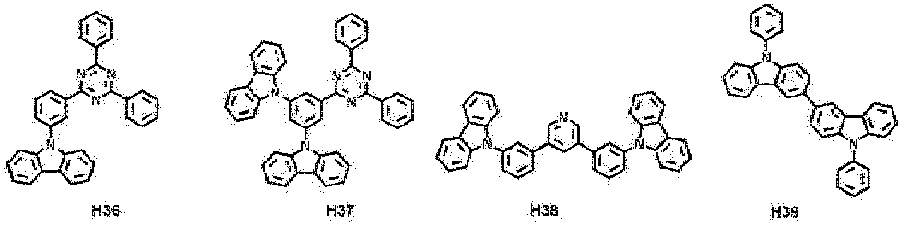

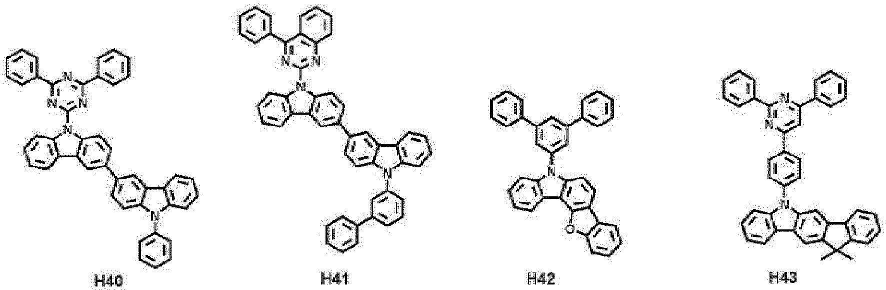

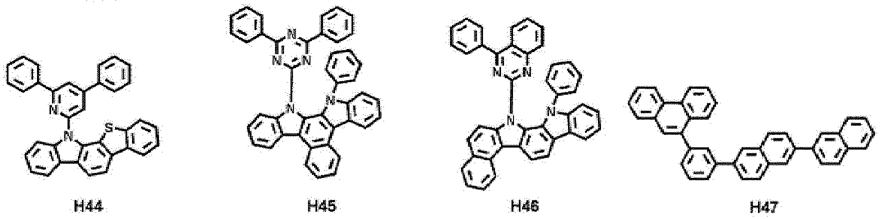

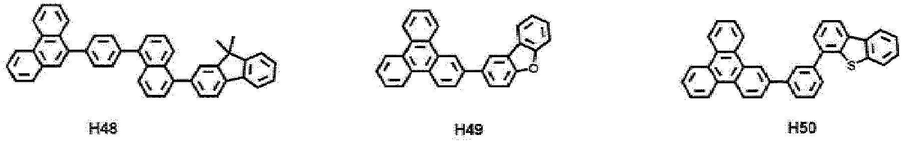

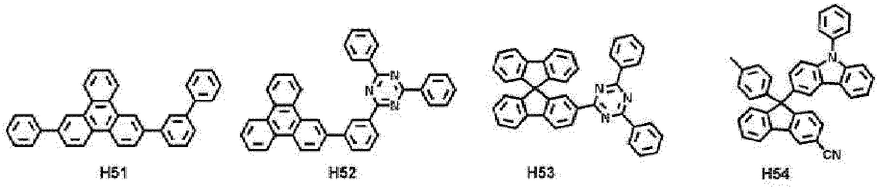

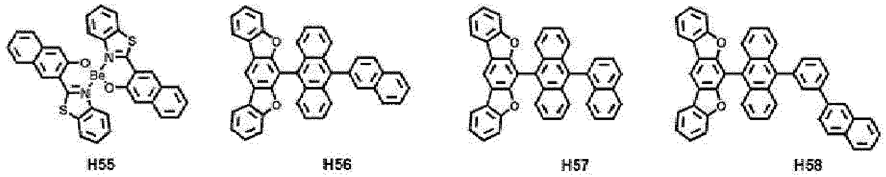

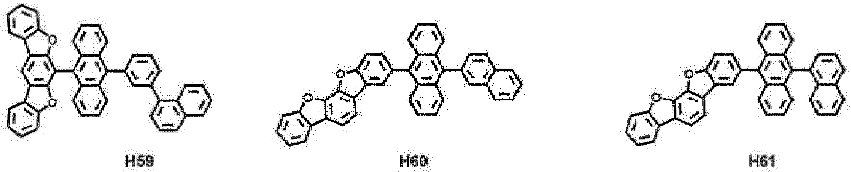

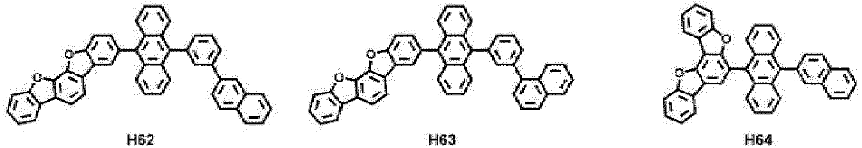

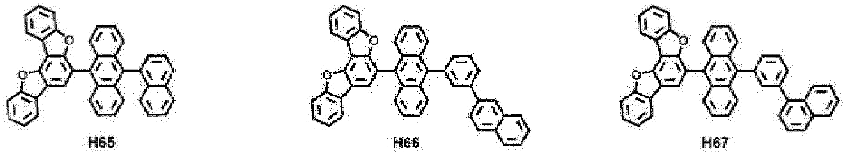

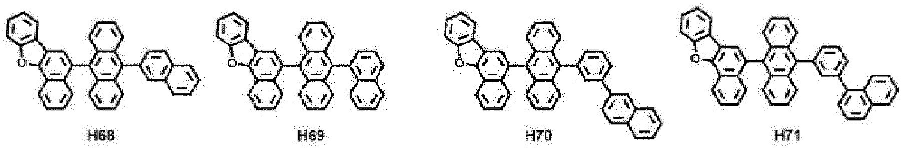

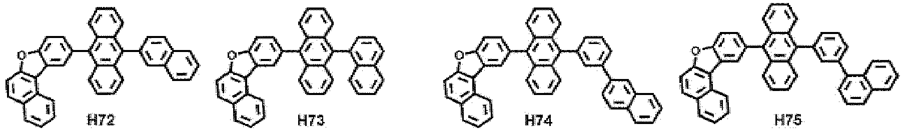

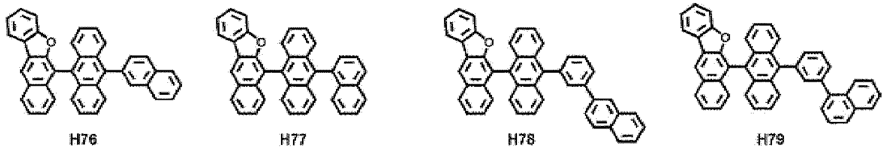

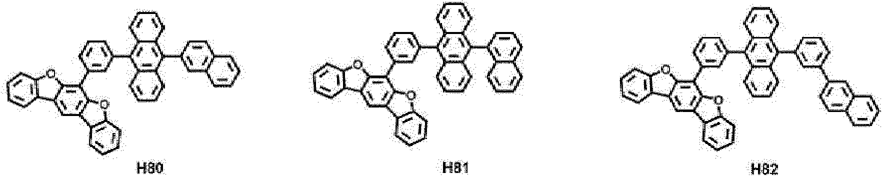

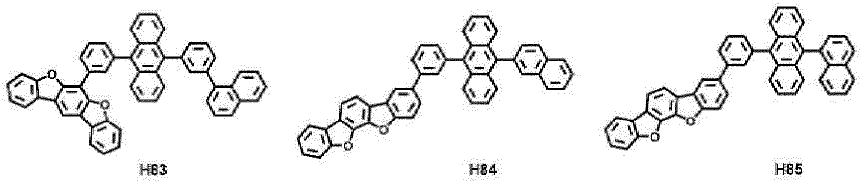

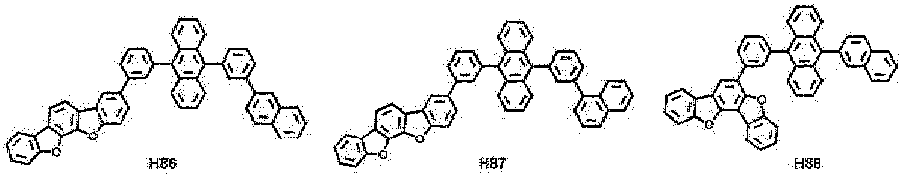

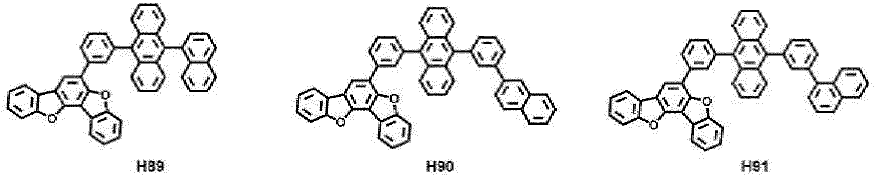

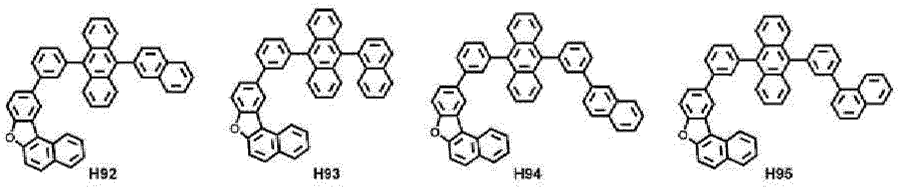

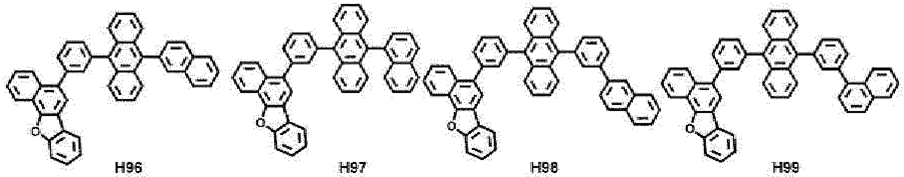

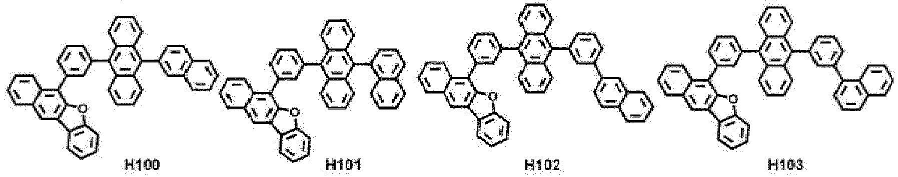

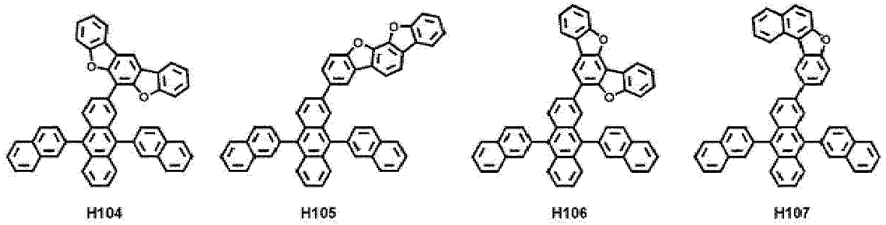

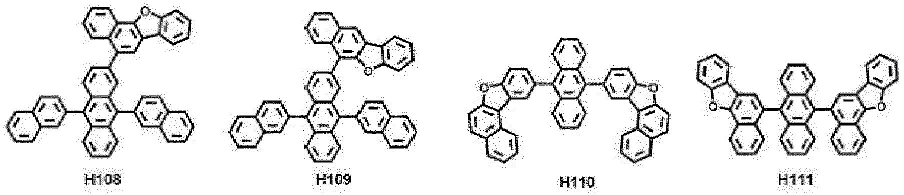

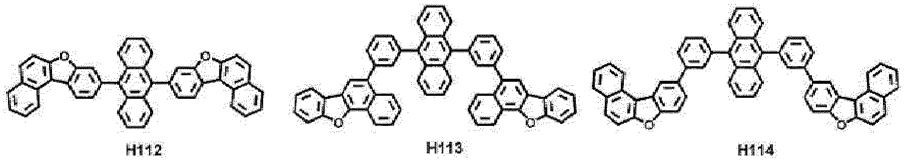

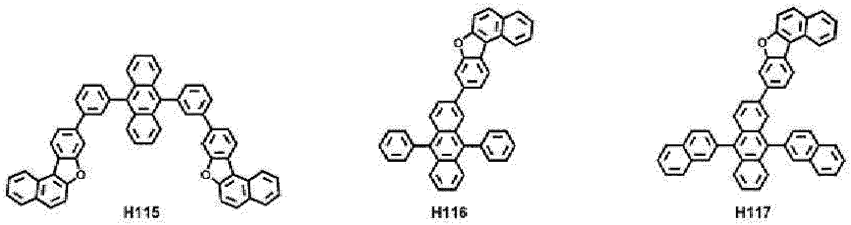

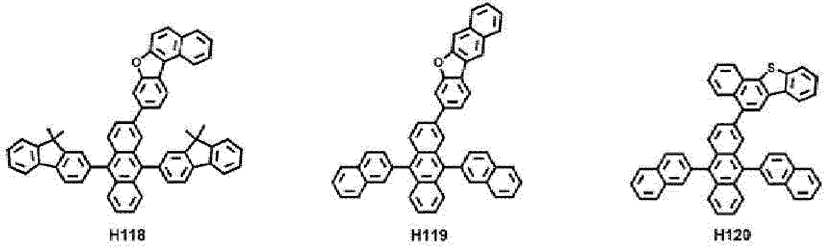

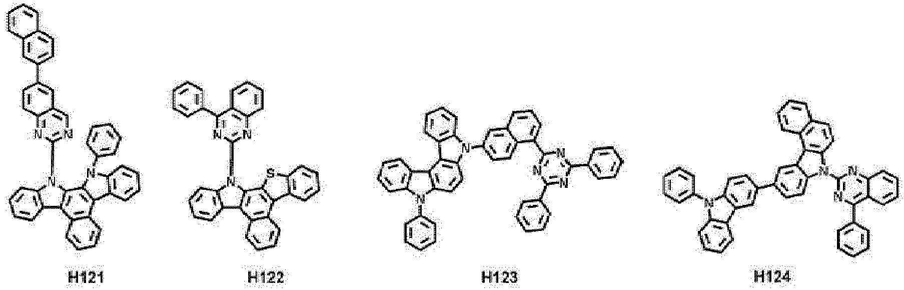

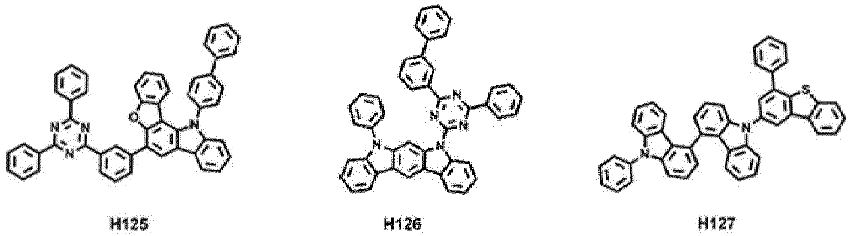

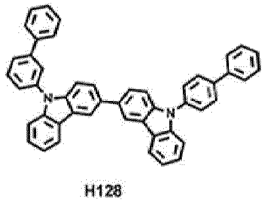

### Phosphorescent dopant

The phosphorescent dopant may include at least one transition metal as a central metal.

The phosphorescent dopant may include a monodentate ligand, a bidentate ligand, a tridentate ligand, a tetradentate ligand, a pentadentate ligand, a hexadentate ligand, or any combination thereof.

The phosphorescent dopant may be electrically neutral.

In one or more embodiments, the phosphorescent dopant may include an organometallic compound represented by Formula 401:

**Formula 401** **M(L₄₀₁)_{xc1}(L₄₀₂)_{xc2}**

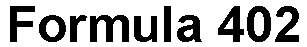

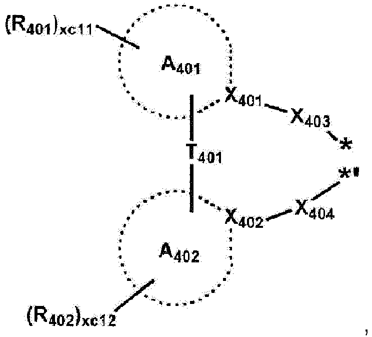

wherein, in Formulae 401 and 402,
M may be a transition metal (e.g., Ir, Pt, Pd, Os, Ti, Au, Hf, Eu, Tb, Rh, Re, or Tm),
L₄₀₁ may be a ligand represented by Formula 402, and xc1 is 1, 2, or 3, wherein, if (e.g., when) xc1 is 2 or more, two or more of L₄₀₁(s) may be identical to or different from each other,
L₄₀₂ may be an organic ligand, and xc2 may be 0, 1, 2, 3, or 4, wherein, if (e.g., when) xc2 is 2 or more, two or more of L₄₀₂(s) may be identical to or different from each other,
X₄₀₁ and X₄₀₂ may each independently be nitrogen or carbon,
ring A₄₀₁ and ring A₄₀₂ may each independently be a C₃-C₆₀ carbocyclic group or a C₁-C₆₀ heterocyclic group,
T₄₀₁ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C(Q₄₁₁)-*',
X₄₀₃ and X₄₀₄ may each independently be a chemical bond (for example, a covalent bond or a coordinate bond), O, S, N(Q₄₁₃), B(Q₄₁₃), P(Q₄₁₃), C(Q₄₁₃)(Q₄₁₄), or Si(Q₄₁₃)(Q₄₁₄),
Q₄₁₁ to Q₄₁₄ are each the same as described with respect to Q₁,
R₄₀₁ and R₄₀₂ may each independently be hydrogen, deuterium, -F, -Cl, -Br, - I, a hydroxyl group, a cyano group, a nitro group, a C₁-C₂₀ alkyl group unsubstituted or substituted with at least one R₁₀ₐ, a C₁-C₂₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), - S(=O)₂(Q₄₀₁), or -P(=O)(Q₄₀₁)(Q₄₀₂),
Q₄₀₁ to Q₄₀₃ are each the same as described with respect to Q₁,
xc11 and xc12 may each independently be an integer from 0 to 10, and
* and *' in Formula 402 each indicates a binding site to M in Formula 401.

In one or more embodiments, in Formula 402, i) X₄₀₁ may be nitrogen, and X₄₀₂ may be carbon, or ii) each of X₄₀₁ and X₄₀₂ may be nitrogen.

In one or more embodiments, if (e.g., when) xc1 in Formula 402 is 2 or more, two ring A₄₀₁(s) in two or more of L₄₀₁(s) may optionally be linked to each other via T₄₀₂, which is a linking group, and/or two ring A₄₀₂(s) may optionally be linked to each other via T₄₀₃, which is a linking group (see Compounds PD1 to PD4 and PD7). T₄₀₂ and T₄₀₃ are each the same as described with respect to T₄₀₁.

L₄₀₂ in Formula 401 may be an organic ligand. In one or more embodiments, L₄₀₂ may include a halogen, a diketone group (for example, an acetylacetonate group), a carboxylic acid group (for example, a picolinate group), -C(=O), an isonitrile group, a -CN group, a phosphorus-containing group (for example, a phosphine group, a phosphite group, and/or the like), or any combination thereof.

In one or more embodiments, the phosphorescent dopant may include, for example, one of (e.g., include at least one or be any one selected from among) compounds PD1 to PD39, or any combination thereof:

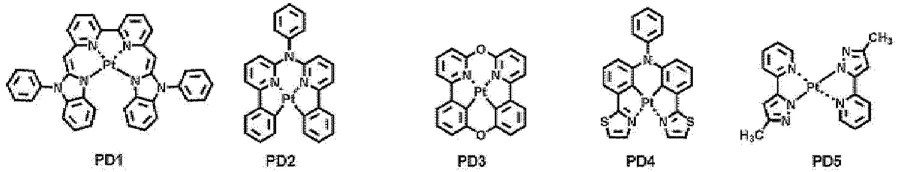

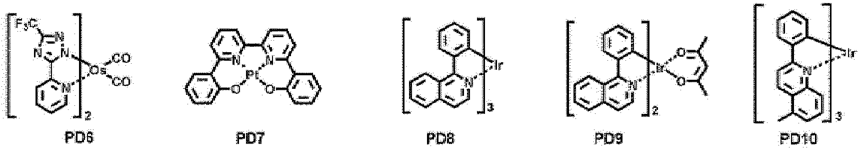

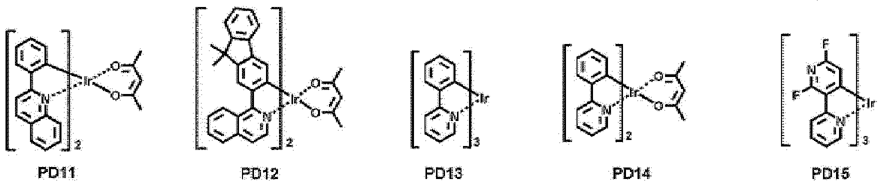

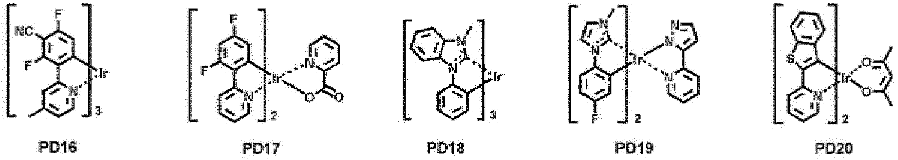

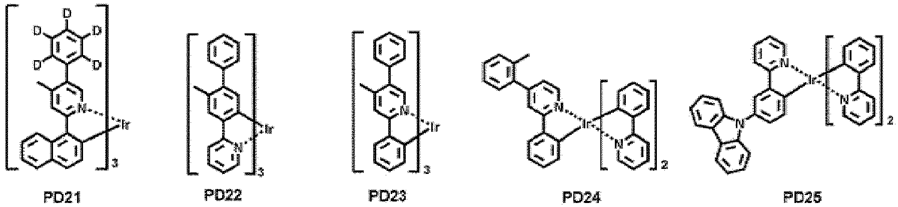

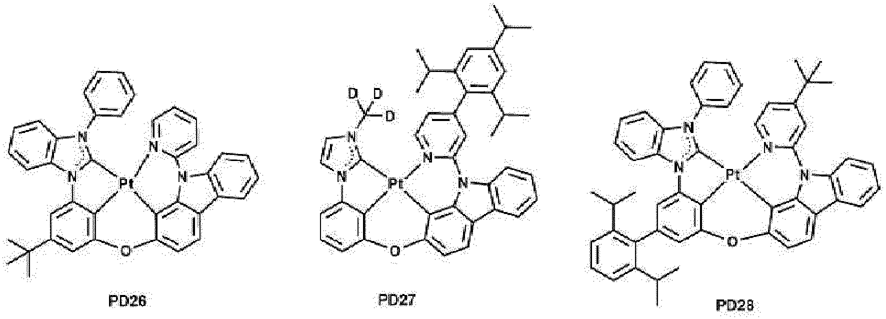

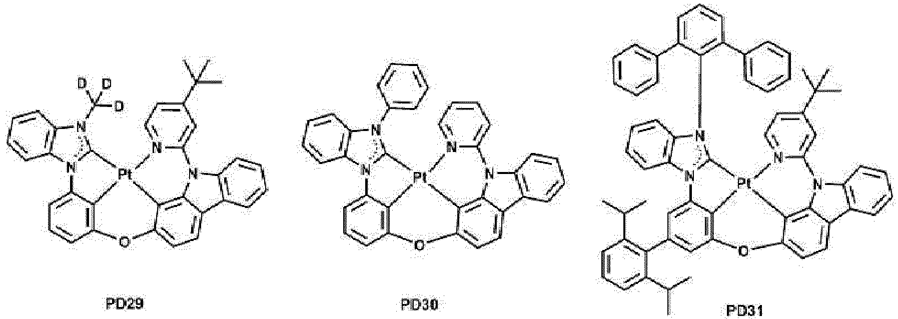

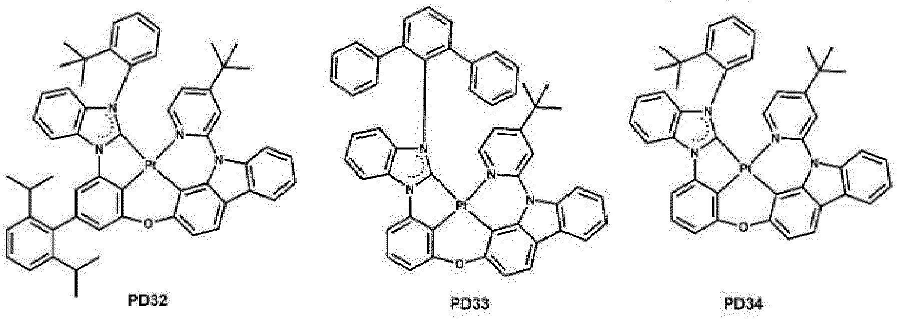

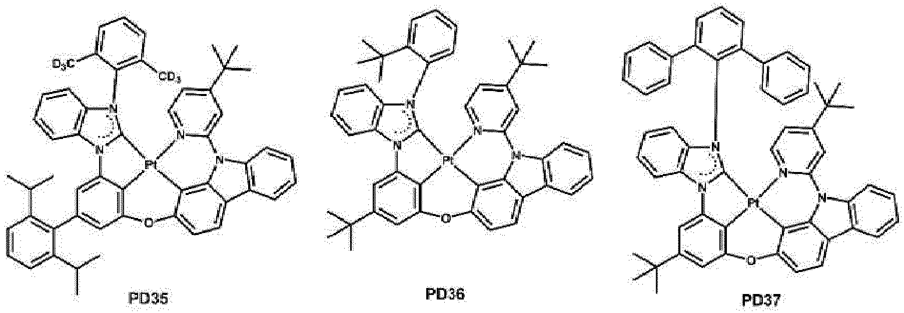

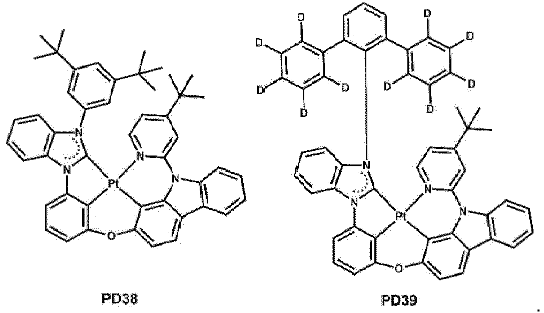

### Fluorescent dopant

The fluorescent dopant may include an amine group-containing compound, a styryl group-containing compound, or any combination thereof.

For example, in one or more embodiments, the fluorescent dopant may include a compound represented by Formula 501: 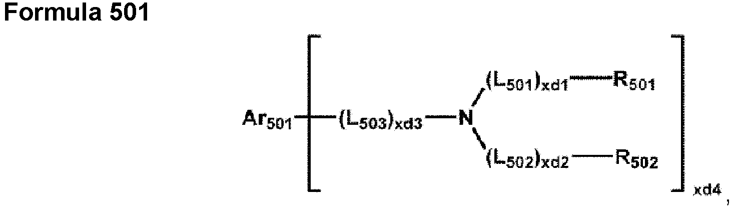 wherein, in Formula 501,
Ar₅₀₁, L₅₀₁ to L₅₀₃, R₅₀₁, and R₅₀₂ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xd1 to xd3 may each independently be 0, 1, 2, or 3, and
xd4 may be 1, 2, 3, 4, 5, or 6.

In one or more embodiments, Ar₅₀₁ in Formula 501 may be a condensed cyclic group (for example, an anthracene group, a chrysene group, a pyrene group, and/or the like) in which three or more monocyclic groups are condensed together.

In one or more embodiments, xd4 in Formula 501 may be 2.

In one or more embodiments, the fluorescent dopant may include: one of (e.g., include at least one or be any one selected from among) Compounds FD1 to FD37; 4,4'-bis(2,2-diphenylvinyl)-1,1'-biphenyl (DPVBi); 4,4'-bis[4-(N,N-diphenylamino)styryl]biphenyl (DPAVBi); or any combination thereof:

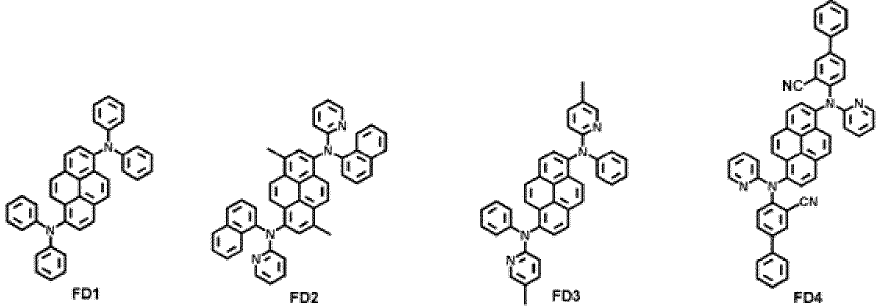

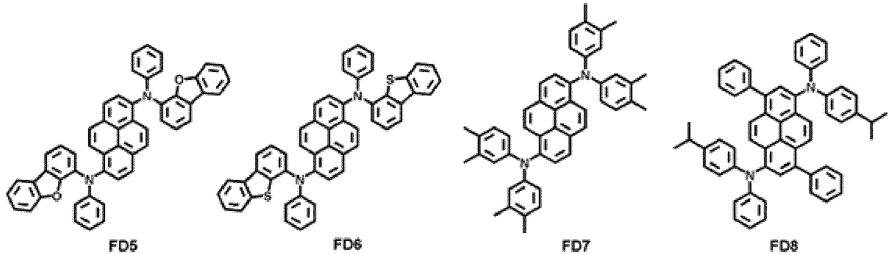

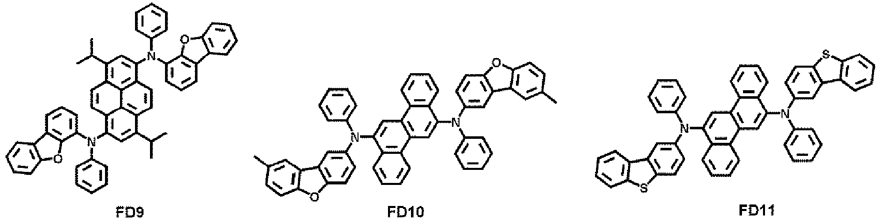

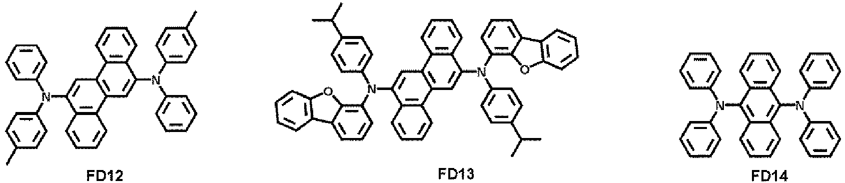

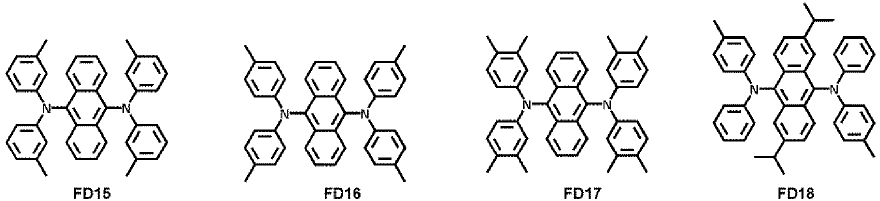

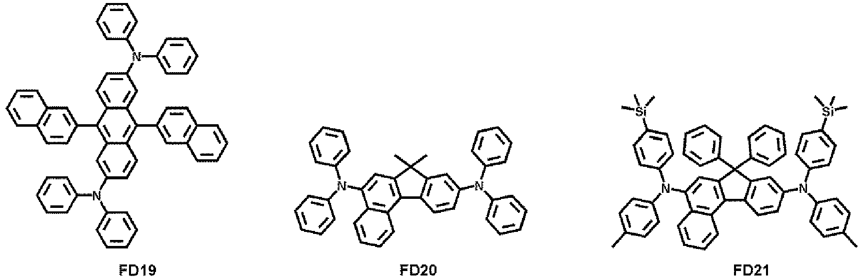

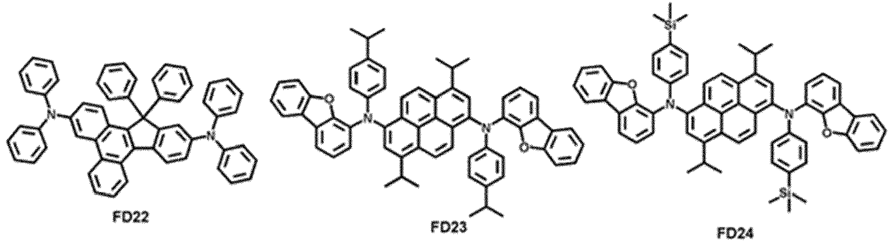

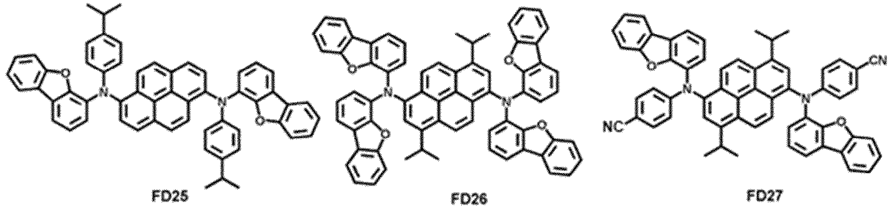

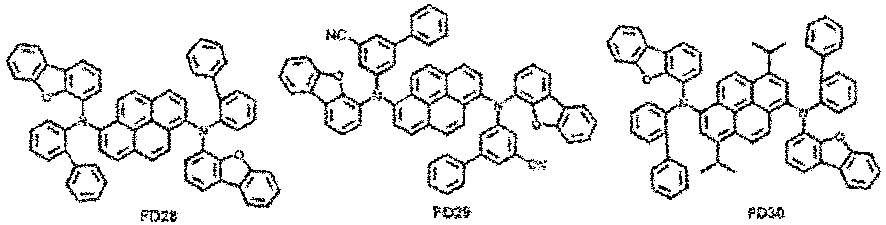

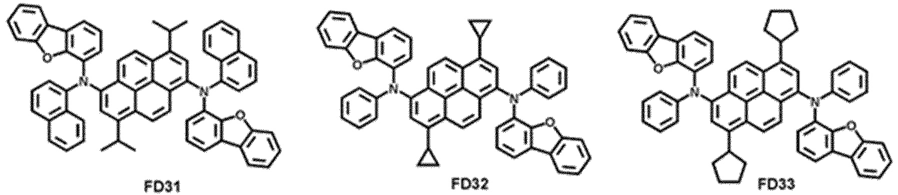

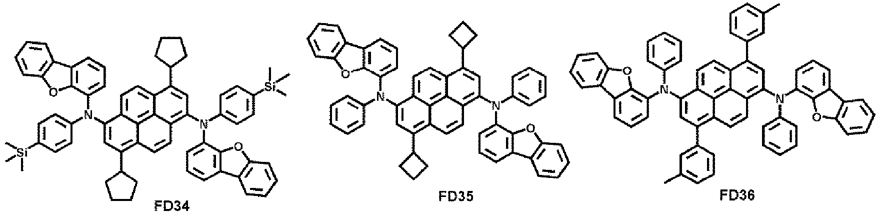

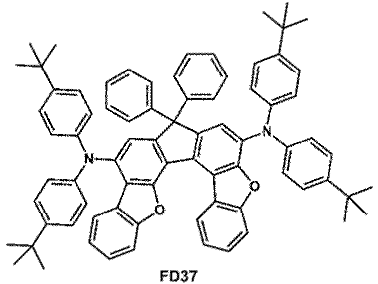

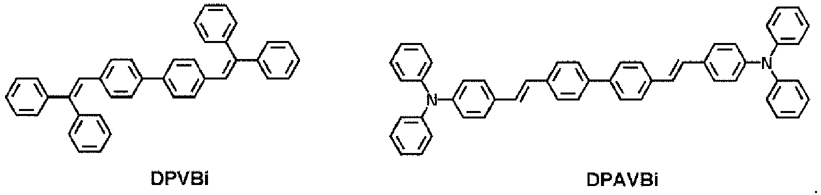

### Delayed fluorescence material

In one or more embodiments, the emission layer may include a delayed fluorescence material.

Herein, the delayed fluorescence material may be selected from among compounds capable of emitting delayed fluorescence based on a delayed fluorescence emission mechanism.

The delayed fluorescence material included in the emission layer may act as a host or a dopant depending on the type (kind) of other materials included in the emission layer.

In one or more embodiments, a difference (e.g., an absolute value of the difference) between a triplet energy level (eV) of the delayed fluorescence material and the singlet energy level (eV) of the delayed fluorescence material may be at least about 0 eV and not more than about 0.5 eV. When the difference between the triplet energy level (eV) of the delayed fluorescence material and the singlet energy level (eV) of the delayed fluorescence material satisfies the above-described range, up-conversion from the triplet state to the singlet state of the delayed fluorescence materials may effectively occur, and thus, the luminescence efficiency of the light-emitting device 10 may be improved.

In one or more embodiments, the delayed fluorescence material may include: i) a material including at least one electron donor (for example, a π electron-rich C₃-C₆₀ cyclic group such as a carbazole group) and at least one electron acceptor (for example, a sulfoxide group, a cyano group, a π electron-deficient nitrogen-containing C₁-C₆₀ cyclic group, and/or the like), and/or ii) a material including a C₈-C₆₀ polycyclic group including at least two cyclic groups that are condensed with each other while sharing boron (B).

In one or more embodiments, the delayed fluorescence material may be represented by Formula 701 or Formula 702: 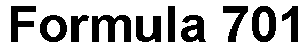 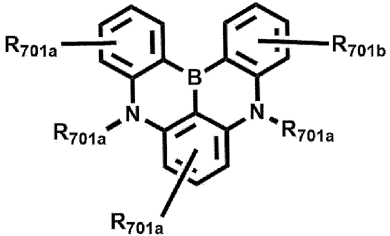 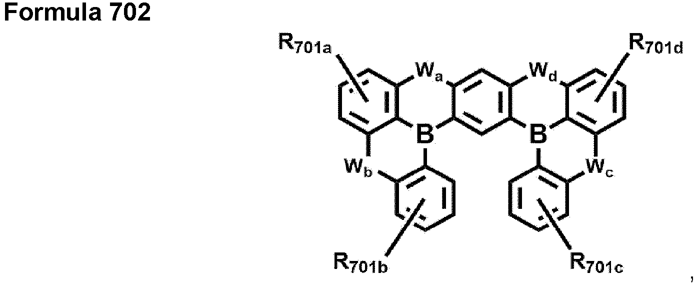 wherein, in Formulae 701 and 702,
Wₐ to W_{d} may each independently be selected from among N(R₇₀₁ₑ), C(R₇₀₁ₑ)(R_{701f}), O, and S,
R₇₀₁ₐ to R_{701f} may each independently be hydrogen, deuterium, -F, -Cl, -Br, - I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -C(Q₁)(Q₂)(Q₃), - Si(Q₁)(Q₂)(Q ₃), -N(Q₁)(Q₂), -B(Q₁)(Q₂), -C(=O)(Q₁), -S(=O)₂(Q₁), or -P(=O)(Q₁)(Q₂),
R₁₀ₐ may be:
   deuterium, -F, -Cl, -Br, -I, a hydroxyl group, a cyano group, or a nitro group;
   a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
   a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), - C(=O)(Q₂₁), -S(=O)₂(Q₂₁), -P(=O)(Q₂₁)(Q₂₂), or any combination thereof; or
   **-Si(Q₃₁)(Q₃₂)(Q₃₃), -N(Q₃₁)(Q₃₂),** -B(Q₃₁)(Q₃₂), -C(=O)(Q₃₁), -S(=O)₂(Q₃₁), or - P(=O)(Q₃₁)(Q₃₂), and
   Q₁ to Q₃, Q₁₁ to Q₁₃, Q₂₁ to Q₂₃, and Q₃₁ to Q₃₃ may each independently be: hydrogen; deuterium; -F; -Cl; -Br; -I; a hydroxyl group; a cyano group; a nitro group; or a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

Non-limiting examples of the delayed fluorescence material may include at least one selected from among Compounds DF1 to DF14 and Compounds DFD1 to DFD29:

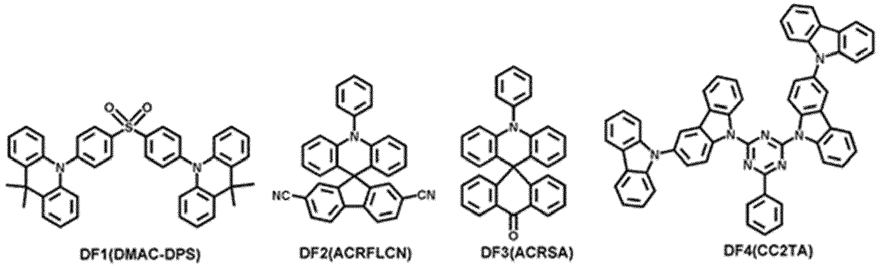

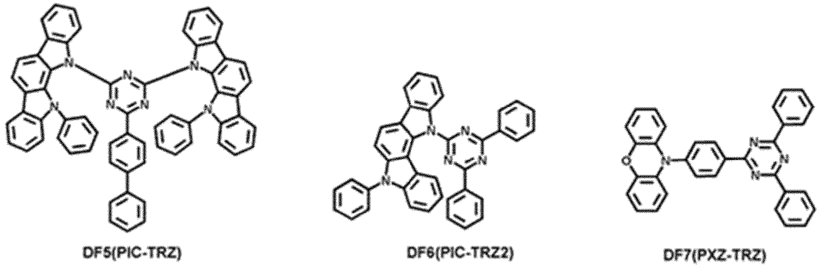

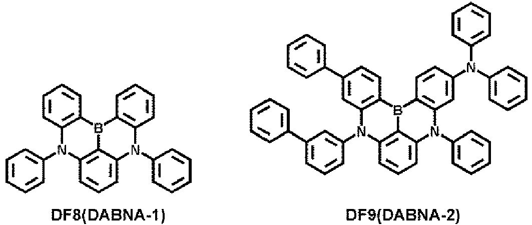

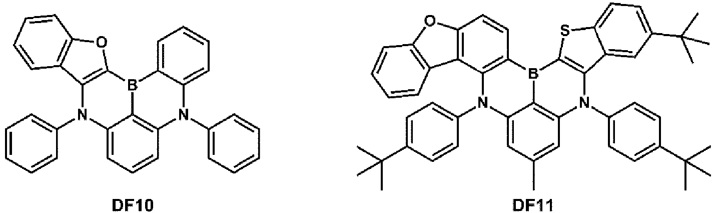

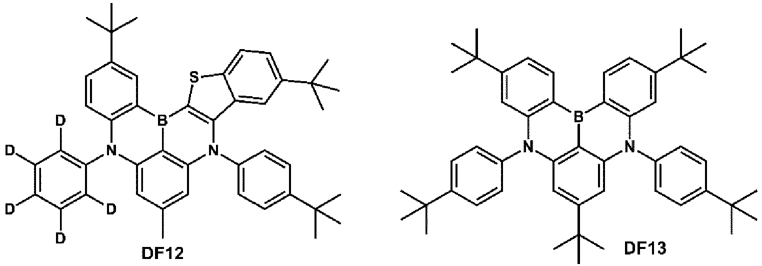

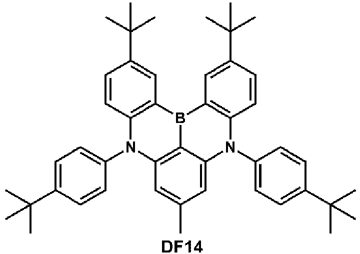

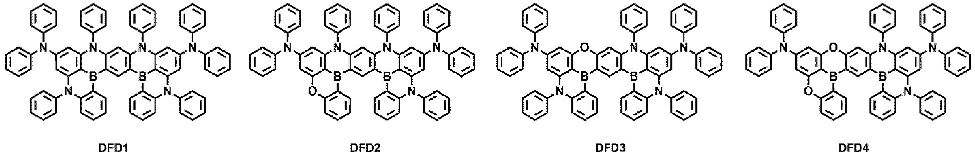

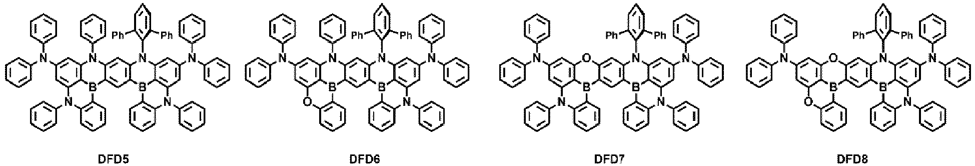

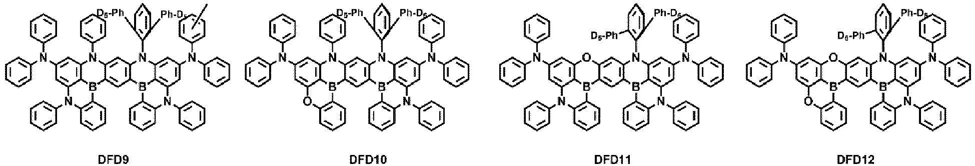

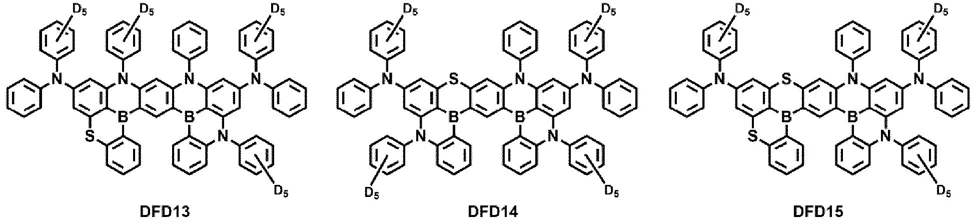

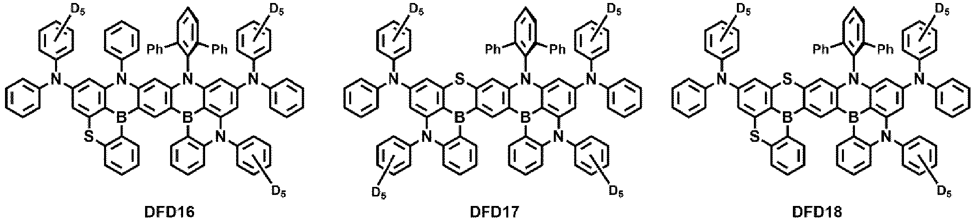

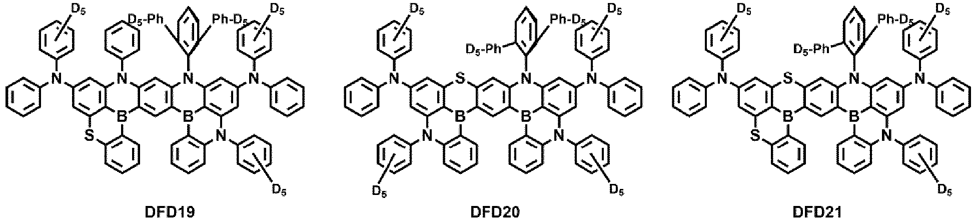

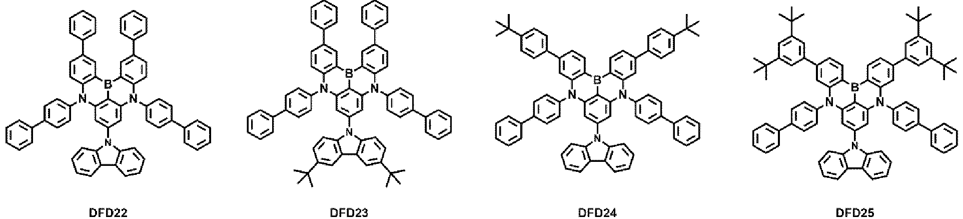

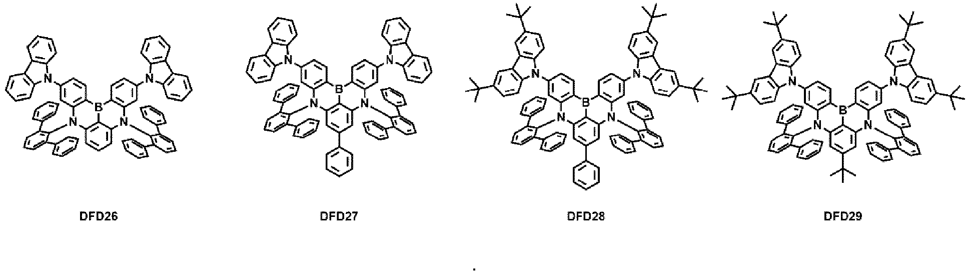

**Quantum dot**

In one or more embodiments, the emission layer may include a quantum dot.

The term "quantum dot" as used herein refers to a crystal of a semiconductor compound, and may include any material capable of emitting light of one or more suitable emission wavelengths according to the size of the crystal.

A diameter of the quantum dot may be, for example, in a range of about 1 nanometer (nm) to about 10 nm. In the present disclosure, when quantum dots or quantum dot particles are spherical, "diameter" indicates a particle diameter or an average particle diameter, and when the particles are non-spherical, the "diameter" indicates a major axis length or an average major axis length. The diameter of the particles may be measured utilizing a scanning electron microscope or a particle size analyzer. As the particle size analyzer, for example, HORIBA, LA-950 laser particle size analyzer, may be utilized. When the size of the particles is measured utilizing a particle size analyzer, the average particle diameter is referred to as D50. D50 refers to the average diameter of particles whose cumulative volume corresponds to 50 vol% in the particle size distribution (e.g., cumulative distribution), and refers to the value of the particle size corresponding to 50% from the smallest particle when the total number of particles is 100% in the distribution curve accumulated in the order of the smallest particle size to the largest particle size.

The quantum dot may be synthesized by a wet chemical process, a metal organic chemical vapor deposition process, a molecular beam epitaxy process, or any process similar thereto.

The wet chemical process is a method including mixing a precursor material of a quantum dot with an organic solvent and then growing a quantum dot particle crystal. When the crystal grows, the organic solvent naturally acts as a dispersant coordinated on the surface of the quantum dot crystal and controls the growth of the crystal so that the growth of quantum dot particles may be controlled or selected through a process which costs lower, and is easier than vapor deposition methods, such as metal organic chemical vapor deposition (MOCVD) or molecular beam epitaxy (MBE).

The quantum dot may include Group II-VI semiconductor compounds, Group III-V semiconductor compounds, Group III-VI semiconductor compounds, Group I-III-VI semiconductor compounds, Group IV-VI semiconductor compounds, Group IV elements or compounds, and/or a (e.g., any suitable) combination thereof.

Non-limiting examples of the Group II-VI semiconductor compound may include (e.g., be): a binary compound, such as CdS, CdSe, CdTe, ZnS, ZnSe, ZnTe, ZnO, HgS, HgSe, HgTe, MgSe, and/or MgS; a ternary compound, such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, and/or MgZnS; a quaternary compound, such as CdZnSeS, CdZnSeTe, CdZnSTe, CdHgSeS, CdHgSeTe, CdHgSTe, HgZnSeS, HgZnSeTe, and/or HgZnSTe; and/or a (e.g., any suitable) combination thereof.

Non-limiting examples of the Group III-V semiconductor compound may include (e.g., be): a binary compound, such as GaN, GaP, GaAs, GaSb, AIN, AlP, AlAs, AlSb, InN, InP, InAs, InSb, and/or the like; a ternary compound, such as GaNP, GaNAs, GaNSb, GaPAs, GaPSb, AINP, AlNAs, AlNSb, AlPAs, AlPSb, InGaP, InNP, InAlP, InNAs, InNSb, InPAs, InPSb, and/or the like; a quaternary compound, such as GaAlNP, GaAlNAs, GaAINSb, GaAlPAs, GaAlPSb, GalnNP, GalnNAs, GalnNSb, GalnPAs, GalnPSb, InAlNP, InAINAs, InAlNSb, InAlPAs, InAlPSb, and/or the like; or any combination thereof. In one or more embodiments, the Group III-V semiconductor compound may further include a Group II element. Non-limiting examples of the Group III-V semiconductor compound further including a Group II element may include (e.g., be) InZnP, InGaZnP, InAlZnP, and/or the like.

Non-limiting examples of the Group III-VI semiconductor compound may include (e.g., be): a binary compound, such as GaS, GaSe, Ga₂Se₃, GaTe, InS, InSe, In₂S₃, In₂Se₃, and/or InTe; a ternary compound, such as InGaS₃ and/or InGaSe₃; and/or a (e.g., any suitable) combination thereof.

Non-limiting examples of the Group I-III-VI semiconductor compound may include (e.g., be): a ternary compound, such as AgInS, AgInS₂, CuInS, CuInS₂, CuGaO₂, AgGaO₂, AgAlO₂, and/or the like; or any combination thereof.

Non-limiting examples of the Group IV-VI semiconductor compound may include (e.g., be): a binary compound, such as SnS, SnSe, SnTe, PbS, PbSe, and/or PbTe; a ternary compound, such as SnSeS, SnSeTe, SnSTe, PbSeS, PbSeTe, PbSTe, SnPbS, SnPbSe, and/or SnPbTe; a quaternary compound, such as SnPbSSe, SnPbSeTe, and/or SnPbSTe; and/or a (e.g., any suitable)combination thereof.

The Group IV element or compound may include: a single element compound, such as Si and/or Ge; a binary compound, such as SiC and/or SiGe; and/or a (e.g., any suitable) combination thereof.

Each element included in a multi-element compound such as the binary compound, the ternary compound, and the quaternary compound may be present at a substantially uniform concentration or non-uniform concentration in a particle.

In one or more embodiments, the quantum dot may have a single structure in which the concentration of each element in the quantum dot is substantially uniform, or a core-shell dual structure. For example, a material included in the core and a material included in the shell may be different from each other.

The shell of the quantum dot may act as a protective layer that prevents chemical degeneration of the core to maintain semiconductor characteristics, and/or as a charging layer that imparts electrophoretic characteristics to the quantum dot. The shell may be a single layer or a multi-layer. In one or more embodiments, an interface between the core and the shell may have a concentration gradient in which the concentration of an element existing in the shell decreases toward the center of the core.

Examples of the shell of the quantum dot may be an oxide of metal, metalloid, or non-metal, a semiconductor compound, and/or a (e.g., any suitable) combination thereof. Non-limiting examples of the oxide of metal, metalloid, or non-metal may include (e.g., be) a binary compound, such as SiO₂, Al₂O₃, TiO₂, ZnO, MnO, Mn₂O₃, Mn₃O₄, CuO, FeO, Fe₂O₃, Fe₃O₄, CoO, Co₃O₄, and/or NiO; a ternary compound, such as MgAl₂O₄, CoFe₂O₄, NiFe₂O₄, and/or CoMn₂O₄; and/or a (e.g., any suitable) combination thereof. Examples of the semiconductor compound are, as described herein, a Group II-VI semiconductor compound; a Group III-V semiconductor compound; a Group III-VI semiconductor compound; a Group I-III-VI semiconductor compound; a Group IV-VI semiconductor compound; and/or a (e.g., any suitable) combination thereof. For example, the semiconductor compound suitable as a shell may include CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, and/or a (e.g., any suitable) combination thereof.

A full width at half maximum (FWHM) of the emission spectrum of the quantum dot may be about 45 nm or less, for example, about 40 nm or less, for example, about 30 nm or less, and within these ranges, color purity or color reproducibility of the quantum dot may be increased. In addition, because the light emitted through the quantum dot is emitted in all directions, the wide viewing angle may be improved.

In addition, the quantum dot may be in the form of a spherical particle, a pyramidal particle, a multi-arm particle, a cubic nanoparticle, a nanotube particle, a nanowire particle, a nanofiber particle, or a nanoplate particle.

Because an energy band gap of the quantum dot may be adjusted by controlling the size of the quantum dot, light having one or more suitable wavelength bands may be obtained from a quantum dot emission layer. Accordingly, by using quantum dots of different sizes, a light-emitting device that emits light of one or more suitable wavelengths may be implemented. In one or more embodiments, the size of the quantum dots may be selected to enable the quantum dots to emit red, green and/or blue light. In addition, the quantum dots with suitable size may be configured to emit white light by combination of light of one or more suitable colors.

### Electron transport region in interlayer 130

The electron transport region may have: i) a single-layered structure including (e.g., consisting of) a single layer including (e.g., consisting of) a single material, ii) a single-layered structure including (e.g., consisting of) a single layer including multiple different materials, or iii) a multilayer structure including multiple layers including multiple different materials.

The electron transport region may include a buffer layer, a hole-blocking layer, an electron control layer, an electron transport layer, an electron injection layer, or any combination thereof.

For example, in one or more embodiments, the electron transport region may have an electron transport layer/electron injection layer structure, a hole-blocking layer/electron transport layer/electron injection layer structure, an electron control layer/electron transport layer/electron injection layer structure, or a buffer layer/electron transport layer/electron injection layer structure, wherein constituent layers in each structure are sequentially stacked from the emission layer in the stated order.

The electron transport region (e.g., the buffer layer, the hole-blocking layer, the electron control layer, or the electron transport layer in the electron transport region) may include a metal-free compound including at least one π electron-deficient nitrogen-containing C₁-C₆₀ cyclic group, for example, a heterocyclic compound represented by Formula 1.

For example, in one or more embodiments, the electron transport region may further include, in addition to the heterocyclic compound represented by Formula 1, a compound represented by Formula 601.

**Formula 601** **[Ar₆₀₁]ₓₑ₁₁-[(L₆₀₁)ₓₑ₁-R₆₀₁]ₓₑ₂₁**

In Formula 601,
Ar₆₀₁ and L₆₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xe11 may be 1, 2, or 3,
xe1 may be 0, 1, 2, 3, 4, or 5,
R₆₀₁ may be a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₆₀₁)(Q₆₀₂)(Q₆₀₃), -C(=O)(Q₆₀₁), -S(=O)₂(Q₆₀₁), or -P(=O)(Q₆₀₁)(Q₆₀₂),
Q₆₀₁ to Q₆₀₃ are each the same as described with respect to Q₁,
xe21 may be 1, 2, 3, 4, or 5, and
at least one selected from among Ar₆₀₁, L₆₀₁, and R₆₀₁ may each independently be a π electron-deficient nitrogen-containing C₁-C₆₀ cyclic group unsubstituted or substituted with at least one R₁₀ₐ.

In one or more embodiments, if (e.g., when) xe11 in Formula 601 is 2 or more, two or more of Ar₆₀₁(s) may be linked together via a single bond.

In one or more embodiments, Ar₆₀₁ in Formula 601 may be a substituted or unsubstituted anthracene group.

In one or more embodiments, the electron transport region may include a compound represented by Formula 601-1: 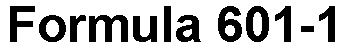 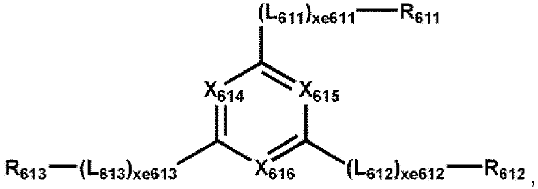 wherein, in Formula 601-1,
X₆₁₄ may be N or C(R₆₁₄), X₆₁₅ may be N or C(R₆₁₅), X₆₁₆ may be N or C(R₆₁₆), and at least one selected from among X₆₁₄ to X₆₁₆ may be N,
L₆₁₁ to L₆₁₃ are each the same as described with respect to L₆₀₁,
xe611 to xe613 are each the same as described with respect to xe1,
R₆₁₁ to R₆₁₃ are each the same as described with respect to R₆₀₁, and
R₆₁₄ to R₆₁₆ may each independently be hydrogen, deuterium, -F, -Cl, -Br, -I, a hydroxyl group, a cyano group, a nitro group, a C₁-C₂₀ alkyl group, a C₁-C₂₀ alkoxy group, a C₃-C₆₀ carbocyclic group unsubstituted or substituted with at least one R₁₀ₐ, or a C₁-C₆₀ heterocyclic group unsubstituted or substituted with at least one R₁₀ₐ.

In one or more embodiments, xe1 and xe611 to xe613 in Formulae 601 and 601-1 may each independently be 0, 1, or 2.

In one or more embodiments, the electron transport region may include one of (e.g., at least one selected from among) Compounds ET1 to ET45, 2,9-dimethyl-4,7-diphenyl-1,10-phenanthroline (BCP), 4,7-diphenyl-1,10-phenanthroline (Bphen), tris(8-hydroxyquinolinato)aluminium (Alq₃), bis(2-methyl-8-quinolinolato-N1,08)-(1,1'-biphenyl-4-olato)aluminium (BAlq), 3-(4-biphenyl)-4-phenyl-5-tert-butylphenyl-1,2,4-triazole (TAZ), 4-(naphthalen-1-yl)-3,5-diphenyl-4H-1,2,4-triazole (NTAZ), or any combination thereof:

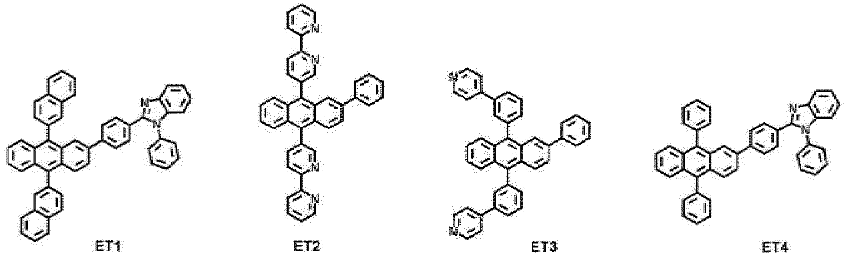

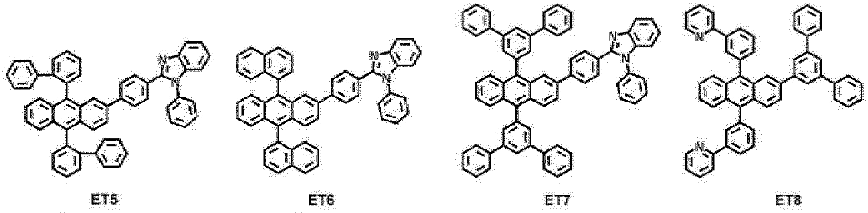

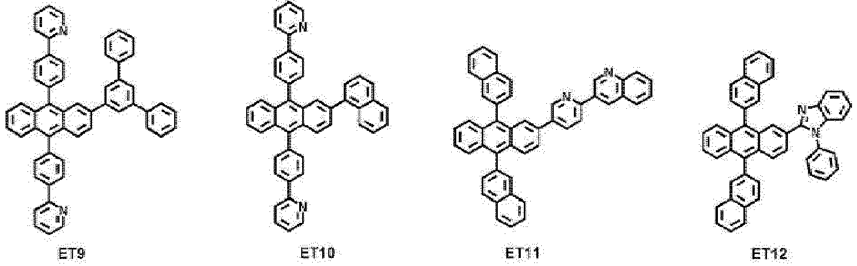

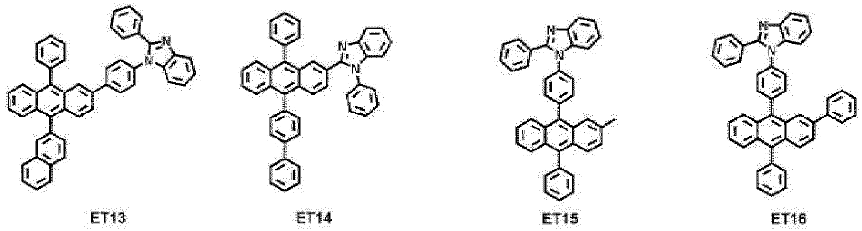

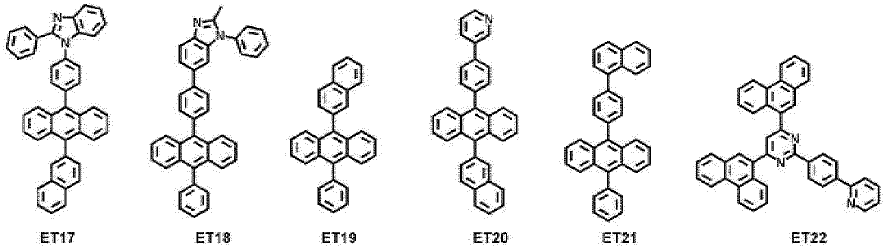

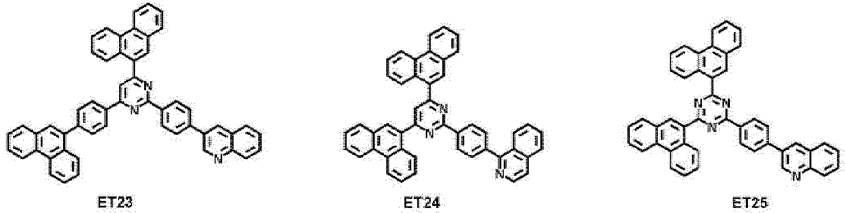

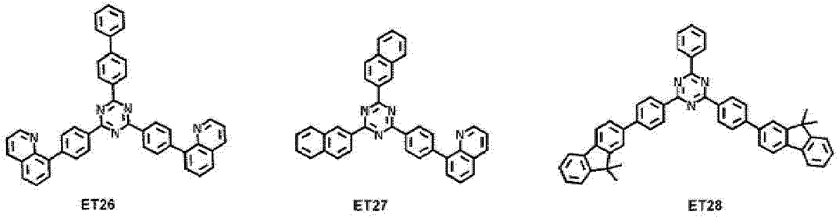

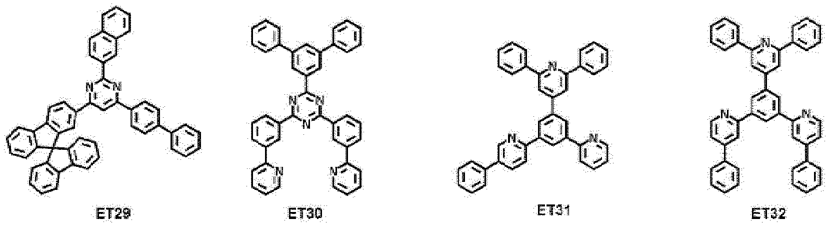

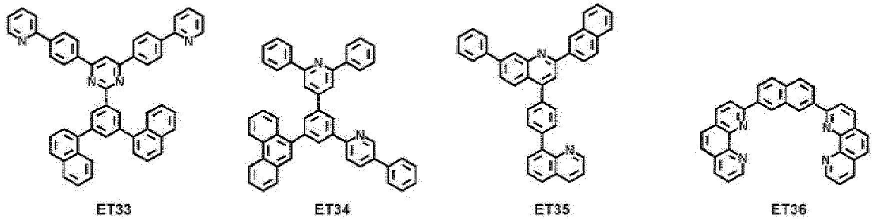

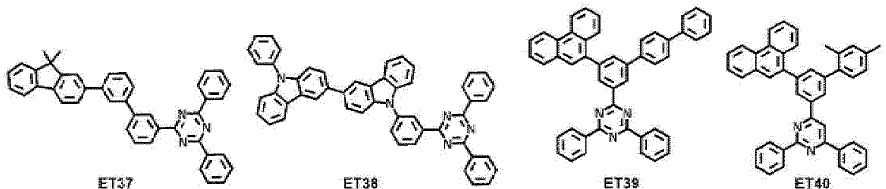

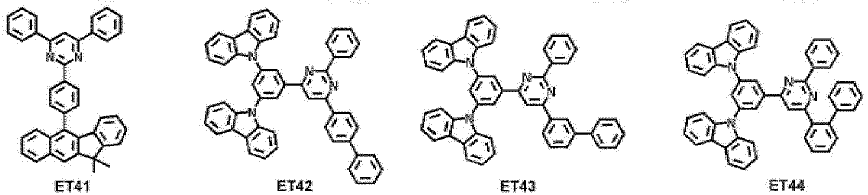

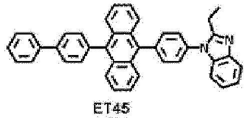

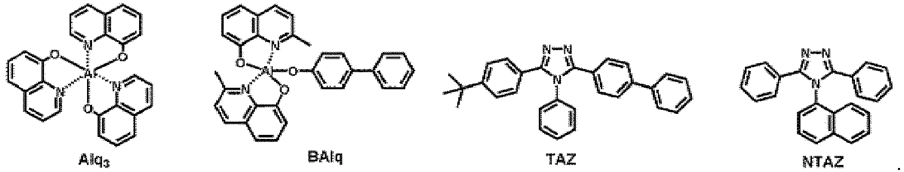

A thickness of the electron transport region may be about 100 Å to about 5,000 Å, for example, about 160 Å to about 4,000 Å. When the electron transport region includes a buffer layer, a hole-blocking layer, an electron control layer, an electron transport layer, or any combination thereof, a thickness of the buffer layer, the hole-blocking layer, or the electron control layer may each independently be in a range of about 20 Å to about 1,000 Å, for example, about 30 Å to about 300 Å, and a thickness of the electron transport layer may be in a range of about 100 Å to about 1,000 Å, for example, about 150 Å to about 500 Å. When the thicknesses of the buffer layer, the hole-blocking layer, the electron control layer, the electron transport layer, and/or the electron transport region are within the ranges described above, satisfactory electron transporting characteristics may be obtained without a substantial increase in driving voltage.

In one or more embodiments, the electron transport region (for example, the electron transport layer in the electron transport region) may further include, in addition to one or more of the materials described above, a metal-containing material.

The metal-containing material may include an alkali metal complex, an alkaline earth metal complex, or any combination thereof. A metal ion of the alkali metal complex may be a Li ion, a Na ion, a K ion, a Rb ion, or a Cs ion, and a metal ion of the alkaline earth metal complex may be a Be ion, a Mg ion, a Ca ion, a Sr ion, or a Ba ion. A ligand coordinated with the metal ion of the alkali metal complex or the metal ion of the alkaline earth-metal complex may include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

In one or more embodiments, the metal-containing material may include a Li complex. The Li complex may include, for example, Compound ET-D1 (LiQ) or ET-D2:

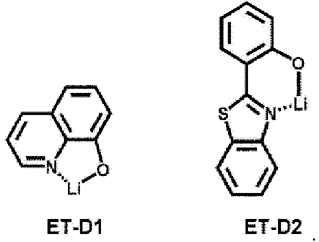

In one or more embodiments, the electron transport region may include an electron injection layer that facilitates the injection of electrons from the second electrode 150. The electron injection layer may directly contact the second electrode 150.

The electron injection layer may have: i) a single-layered structure including (e.g., consisting of) a single layer including (e.g., consisting of) a single material, ii) a single-layered structure including (e.g., consisting of) a single layer including multiple different materials, or iii) a multilayer structure including multiple layers including multiple different materials.

The electron injection layer may include an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof.

The alkali metal may include Li, Na, K, Rb, Cs, or any combination thereof. The alkaline earth metal may include Mg, Ca, Sr, Ba, or any combination thereof. The rare earth metal may include Sc, Y, Ce, Tb, Yb, Gd, or any combination thereof.

The alkali metal-containing compound, the alkaline earth metal-containing compound, and the rare earth metal-containing compound may include oxides, halides (for example, fluorides, chlorides, bromides, iodides, and/or the like), or tellurides of the alkali metal, the alkaline earth metal, and the rare earth metal, respectively, or any combination thereof.

The alkali metal-containing compound may include: alkali metal oxides, such as Li₂O, Cs₂O, and/or K₂O; alkali metal halides, such as LiF, NaF, CsF, KF, Lil, Nal, CsI, and/or KI; or any combination thereof. The alkaline earth metal-containing compound may include an alkaline earth metal oxide, such as BaO, SrO, CaO, BaₓSr₁₋ₓO (x is a real number satisfying 0<x<1), and/or BaₓCa₁₋ₓO (x is a real number satisfying 0<x<1). The rare earth metal-containing compound may include YbF₃, ScF₃, Sc₂O₃, Y₂O₃, Ce₂O₃, GdF₃, TbF₃, YbI₃, ScI₃, TbI₃, or any combination thereof. In one or more embodiments, the rare earth metal-containing compound may include a lanthanide metal telluride. Non-limiting examples of the lanthanide metal telluride may include LaTe, CeTe, PrTe, NdTe, PmTe, SmTe, EuTe, GdTe, TbTe, DyTe, HoTe, ErTe, TmTe, YbTe, LuTe, La₂Te₃, Ce₂Te₃, Pr₂Te₃, Nd₂Te₃, Pm₂Te₃, Sm₂Te₃, Eu₂Te₃, Gd₂Te₃, Tb₂Te₃, Dy₂Te₃, Ho₂Te₃, Er₂Te₃, Tm₂Te₃, Yb₂Te₃, and Lu₂Te₃.

The alkali metal complex, the alkaline earth-metal complex, and the rare earth metal complex may include i) one of metal ions of the alkali metal, one of metal ions of the alkaline earth metal, and one of metal ions of the rare earth metal, respectively, and ii) a ligand bonded to the respective metal ion, for example, hydroxyquinoline, hydroxyisoquinoline, hydroxybenzoquinoline, hydroxyacridine, hydroxyphenanthridine, hydroxyphenyloxazole, hydroxyphenylthiazole, hydroxyphenyloxadiazole, hydroxyphenylthiadiazole, hydroxyphenylpyridine, hydroxyphenyl benzimidazole, hydroxyphenylbenzothiazole, bipyridine, phenanthroline, cyclopentadiene, or any combination thereof.

In one or more embodiments, the electron injection layer may include (e.g., consist of) an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof, as described above. In one or more embodiments, the electron injection layer may further include an organic material (for example, a compound represented by Formula 601).

In one or more embodiments, the electron injection layer may include (e.g., consist of) i) an alkali metal-containing compound (for example, alkali metal halide), or ii) a) an alkali metal-containing compound (for example, alkali metal halide); and b) an alkali metal, an alkaline earth metal, a rare earth metal, or any combination thereof. In one or more embodiments, the electron injection layer may be a KI:Yb co-deposited layer, an RbI:Yb co-deposited layer, a LiF:Yb co-deposited layer, and/or the like.

When the electron injection layer further includes an organic material, the alkali metal, the alkaline earth metal, the rare earth metal, the alkali metal-containing compound, the alkaline earth metal-containing compound, the rare earth metal-containing compound, the alkali metal complex, the alkaline earth-metal complex, the rare earth metal complex, or any combination thereof may be uniformly (e.g., substantially uniformly) or non-uniformly dispersed in a matrix including the organic material.

A thickness of the electron injection layer may be about 1 Å to about 100 Å, for example, about 3 Å to about 90 Å. When the thickness of the electron injection layer is within these range, satisfactory electron injection characteristics may be obtained without a substantial increase in driving voltage.

### Second electrode 150

The second electrode 150 may be arranged on the interlayer 130. The second electrode 150 may be a cathode, which is an electron injection electrode, and as a material for forming the second electrode 150, a metal, an alloy, an electrically conductive compound, or any combination thereof, each having a low-work function, may be used.

The second electrode 150 may include lithium (Li), silver (Ag), magnesium (Mg), aluminium (Al), aluminium-lithium (Al-Li), calcium (Ca), magnesium-indium (Mg-In), magnesium-silver (Mg-Ag), ytterbium (Yb), silver-ytterbium (Ag-Yb), ITO, IZO, or any combination thereof. The second electrode 150 may be a transmissive electrode, a transflective electrode, or a reflective electrode.

The second electrode 150 may have a single-layer structure or a multilayer structure including a plurality of layers.

### Capping layer

In one or more embodiments, a first capping layer may be arranged outside (e.g., on) the first electrode 110, and/or a second capping layer may be arranged outside (e.g., on) the second electrode 150. In one or more embodiments, the light-emitting device 10 may have a structure in which the first capping layer, the first electrode 110, the interlayer 130, and the second electrode 150 are sequentially stacked in the stated order, a structure in which the first electrode 110, the interlayer 130, the second electrode 150, and the second capping layer are sequentially stacked in the stated order, or a structure in which the first capping layer, the first electrode 110, the interlayer 130, the second electrode 150, and the second capping layer are sequentially stacked in the stated order.

In one or more embodiments, light generated in the emission layer of the interlayer 130 of the light-emitting device 10 may be extracted toward the outside through the first electrode 110 which is a transflective electrode or a transmissive electrode, and the first capping layer. In one or more embodiments, light generated in the emission layer of the interlayer 130 of the light-emitting device 10 may be extracted toward the outside through the second electrode 150 which is a transflective electrode or a transmissive electrode, and the second capping layer.

The first capping layer and the second capping layer may increase external emission efficiency according to the principle of constructive interference. Accordingly, the light extraction efficiency of the light-emitting device 10 is increased; as a result, the luminescence efficiency of the light-emitting device 10 may be increased.

Each of the first capping layer and the second capping layer may include a material having a refractive index of 1.6 or more (at 589 nm).

The first capping layer and the second capping layer may each independently be an organic capping layer including an organic material, an inorganic capping layer including an inorganic material, or an organic-inorganic composite capping layer including an organic material and an inorganic material.

At least one of the first capping layer and/or the second capping layer may (e.g., the first capping layer and the second capping layer may each independently) include a carbocyclic compound, a heterocyclic compound, an amine group-containing compound, a porphine derivative, a phthalocyanine derivative, a naphthalocyanine derivative, an alkali metal complex, an alkaline earth metal complex, or any combination thereof. The carbocyclic compound, the heterocyclic compound, and the amine group-containing compound may each optionally be substituted with a substituent including O, N, S, Se, Si, F, Cl, Br, I, or any combination thereof. In one or more embodiments, at least one of the first capping layer and/or the second capping layer may (e.g., the first capping layer and the second capping layer may each independently) include an amine group-containing compound.

In one or more embodiments, at least one of the first capping layer and/or the second capping layer may (e.g., the first capping layer and the second capping layer may each independently) include a compound represented by Formula 201, a compound represented by Formula 202, or any combination thereof.

In one or more embodiments, at least one of the first capping layer and/or the second capping layer may (e.g., the first capping layer and the second capping layer may each independently) include one of (e.g., at least one selected from among) Compounds HT28 to HT33, one of (e.g., at least one selected from among) Compounds CP1 to CP6, β-NPB, or any combination thereof:

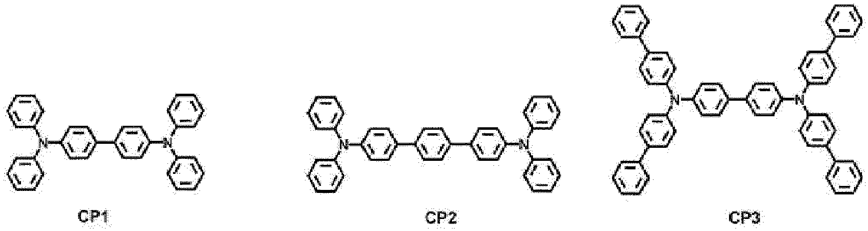

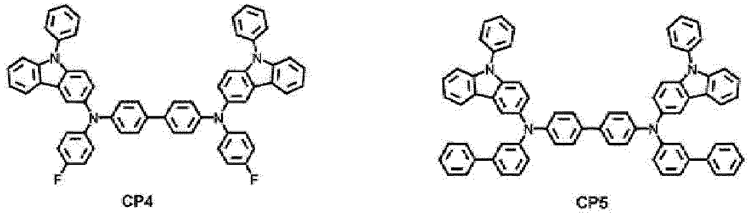

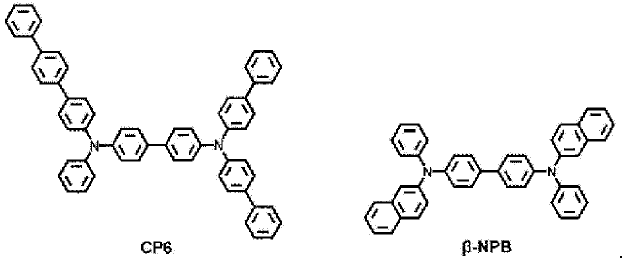

### Film

The heterocyclic compound represented by Formula 1 may be included in one or more suitable films. Accordingly, one or more aspects of embodiments of the disclosure are directed toward a film including the heterocyclic compound represented by Formula 1. The film may be, for example, an optical member (or a light control element) (e.g., a color filter, a color conversion member, a capping layer, a light extraction efficiency enhancement layer, a selective light absorbing layer, a polarizing layer, a quantum dot-containing layer, and/or the like), a light-blocking member (e.g., a light reflective layer, a light absorbing layer, and/or the like), a protective member (e.g., an insulating layer, a dielectric layer, and/or the like), and/or the like.

### Electronic apparatus

The light-emitting device may be included in one or more suitable electronic apparatuses. For example, the electronic apparatus including the light-emitting device may be a light-emitting apparatus, an authentication apparatus, and/or the like.

In one or more embodiments, the electronic apparatus (for example, a light-emitting apparatus) may further include, in addition to the light-emitting device, i) a color filter, ii) a color conversion layer, or iii) a color filter and a color conversion layer. The color filter and/or the color conversion layer may be arranged in at least one travel direction of light emitted from the light-emitting device. For example, in one or more embodiments, the light emitted from the light-emitting device may be blue light or white light (e.g., combined white light). A detailed description of the light-emitting device is provided above. In one or more embodiments, the color conversion layer may include quantum dots. The quantum dot may be, for example, a quantum dot as described herein.

The electronic apparatus may include a first substrate. The first substrate may include a plurality of subpixel areas, the color filter may include a plurality of color filter areas respectively corresponding to the subpixel areas, and the color conversion layer may include a plurality of color conversion areas respectively corresponding to the subpixel areas.

A pixel-defining film may be arranged among the subpixel areas to define each of the subpixel areas.

The color filter may further include a plurality of color filter areas and light-shielding patterns arranged among the color filter areas, and the color conversion layer may further include a plurality of color conversion areas and light-shielding patterns arranged among the color conversion areas.

The plurality of color filter areas (or the plurality of color conversion areas) may include a first area configured to emit first color light, a second area configured to emit second color light, and/or a third area configured to emit third color light, wherein the first color light, the second color light, and/or the third color light may have different maximum emission wavelengths. In one or more embodiments, the first color light may be red light, the second color light may be green light, and the third color light may be blue light. In one or more embodiments, the plurality of color filter areas (or the plurality of color conversion areas) may include quantum dots. For example, the first area may include red quantum dots to emit red light, the second area may include green quantum dots to emit green light, and the third area may not include (e.g., may exclude) quantum dots. A detailed description of the quantum dots may refer to the descriptions provided herein. The first area, the second area, and/or the third area may each further include a scatterer.

In one or more embodiments, the light-emitting device may be to emit first light, the first area may be to absorb the first light to emit first-first color light, the second area may be to absorb the first light to emit second-first color light, and the third area may be to absorb the first light to emit third-first color light. In this regard, the first-first color light, the second-first color light, and the third-first color light may have different maximum emission wavelengths. For example, the first light may be blue light, the first-first color light may be red light, the second-first color light may be green light, and the third-first color light may be blue light.

In one or more embodiments, the electronic apparatus may further include a thin-film transistor, in addition to the light-emitting device as described above. The thin-film transistor may include a source electrode, a drain electrode, and an activation layer, wherein one selected from among the source electrode and the drain electrode may be electrically connected to the first electrode or the second electrode of the light-emitting device.

The thin-film transistor may further include a gate electrode, a gate insulating film, and/or the like.

The activation layer may include crystalline silicon, amorphous silicon, an organic semiconductor, an oxide semiconductor, and/or the like.

In one or more embodiments, the electronic apparatus may further include a sealing portion for sealing the light-emitting device. The sealing portion may be arranged between the color filter and/or the color conversion layer and the light-emitting device. The sealing portion allows light from the light-emitting device to be extracted to the outside, and concurrently (e.g., simultaneously) prevents ambient air and moisture from penetrating into the light-emitting device. The sealing portion may be a sealing substrate including a transparent glass substrate or a plastic substrate. The sealing portion may be a thin-film encapsulation layer including at least one layer of an organic layer and/or an inorganic layer. When the sealing portion is a thin film encapsulation layer, the electronic apparatus may be flexible.

In one or more embodiments, various functional layers may be additionally arranged on the sealing portion, in addition to the color filter and/or the color conversion layer, according to the use of the electronic apparatus. Non-limiting examples of the functional layers may include a touch screen layer and a polarizing layer. The touch screen layer may be a pressure-sensitive touch screen layer, a capacitive touch screen layer, or an infrared touch screen layer.

The authentication apparatus may further include, in addition to the light-emitting device as described above, a biometric information collector. The authentication apparatus may be, for example, a biometric authentication apparatus that authenticates an individual by using biometric information of a living body (for example, fingertips, pupils, and/or the like).

The electronic apparatus may be applied to one or more of displays, light sources, lighting, personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram displays, ultrasonic diagnostic devices, or endoscope displays), fish finders, one or more suitable measuring instruments, meters (for example, meters for a vehicle, an aircraft, and a vessel), projectors, and/or the like.

### Electronic equipment

The light-emitting device may be included in one or more suitable electronic equipment.

In one or more embodiments, the electronic equipment including the light-emitting device may be at least one of a flat panel display, a curved display, a computer monitor, a medical monitor, a television, a billboard, an indoor light, an indoor light, a light for signaling, a head-up display, a fully transparent display, a partially transparent display, a flexible display, a rollable display, a foldable display, a stretchable display, a laser printer, a telephone, a mobile phone, a tablet, a phablet, a personal digital assistant (PDA), a wearable device, a laptop computer, a digital camera, a camcorder, a viewfinder, a micro display, a 3D display, a virtual reality display, an augmented reality display, a vehicle, a video wall including multiple displays tiled together, a theater screen, a stadium screen, a phototherapy device, or a signboard.

Because the light-emitting device has excellent or suitable effects in terms of luminescence efficiency long lifespan, the electronic equipment including the light-emitting device may have characteristics with high luminance, high resolution, and low power consumption.

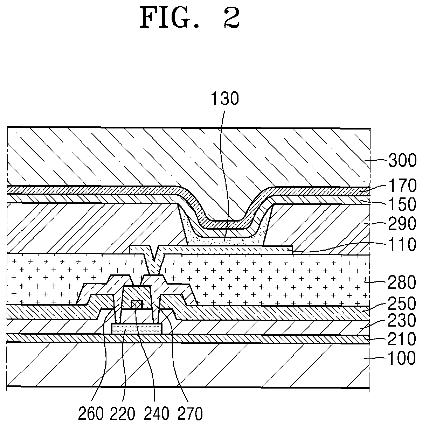

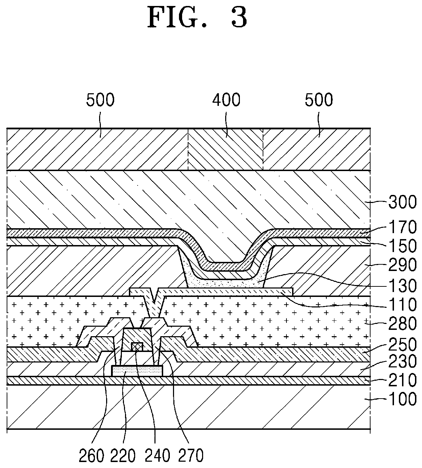

### Description of FIG. 2 and FIG. 3

FIG. 2 is a cross-sectional view showing a light-emitting apparatus according to one or more embodiments of the present disclosure; and

The light-emitting apparatus of FIG. 2 may include a substrate 100, a thin-film transistor (TFT), a light-emitting device, and an encapsulation portion 300 that seals the light-emitting device.

The substrate 100 may be a flexible substrate, a glass substrate, or a metal substrate. A buffer layer 210 may be on the substrate 100. The buffer layer 210 may prevent or reduce penetration of impurities through the substrate 100 and may provide a flat surface on the substrate 100.

The TFT may be on the buffer layer 210. The TFT may include an activation layer 220, a gate electrode 240, a source electrode 260, and a drain electrode 270.

The activation layer 220 may include an inorganic semiconductor, such as silicon or polysilicon, an organic semiconductor, or an oxide semiconductor, and may include a source region, a drain region, and a channel region.

A gate insulating film 230 for insulating the activation layer 220 from the gate electrode 240 may be on the activation layer 220, and the gate electrode 240 may be on the gate insulating film 230.

An interlayer insulating film 250 may be on the gate electrode 240. The interlayer insulating film 250 may be arranged between the gate electrode 240 and the source electrode 260 and between the gate electrode 240 and the drain electrode 270, to insulate one another.

The source electrode 260 and the drain electrode 270 may be on the interlayer insulating film 250. The interlayer insulating film 250 and the gate insulating film 230 may be formed to expose the source region and the drain region of the activation layer 220, and the source electrode 260 and the drain electrode 270 may be arranged in contact with the exposed portions of the source region and the drain region of the activation layer 220, respectively.

The TFT may be electrically connected to the light-emitting device to drive the light-emitting device, and may be covered and protected by a passivation layer 280. The passivation layer 280 may include an inorganic insulating film, an organic insulating film, or any combination thereof. The light-emitting device may be provided on the passivation layer 280. The light-emitting device may include a first electrode 110, an interlayer 130, and a second electrode 150.

The first electrode 110 may be on the passivation layer 280. The passivation layer 280 may be arranged to expose a portion of the drain electrode 270, not fully covering the drain electrode 270, and the first electrode 110 may be arranged to be connected to the exposed portion of the drain electrode 270.

A pixel-defining film 290 including an insulating material may be on the first electrode 110. The pixel-defining film 290 may expose a certain region of the first electrode 110, and the interlayer 130 may be formed in the exposed region of the first electrode 110. The pixel-defining film 290 may be a polyimide-based organic film or a polyacrylic organic film. In one or more embodiments, at least some layers of the interlayer 130 may extend beyond the upper portion of the pixel-defining film 290 to be arranged in the form of a common layer.

The second electrode 150 may be on the interlayer 130, and a capping layer 170 may be additionally formed on the second electrode 150. The capping layer 170 may be formed to cover the second electrode 150.

The encapsulation portion 300 may be located on the capping layer 170. The encapsulation portion 300 may be arranged on the light-emitting device to protect the light-emitting device from moisture and/or oxygen. The encapsulation portion 300 may include: an inorganic film including silicon nitride (SiNₓ), silicon oxide (SiOₓ), indium tin oxide, indium zinc oxide, or any combination thereof; an organic film including polyethylene terephthalate, polyethylene naphthalate, polycarbonate, polyimide, polyethylene sulfonate, polyoxymethylene, polyarylate, hexamethyldisiloxane, an acrylic-based resin (for example, polymethyl methacrylate, polyacrylic acid, and/or the like), an epoxy-based resin (for example, aliphatic glycidyl ether (AGE), and/or the like), or any combination thereof; and/or a (e.g., any suitable) combination of the inorganic film and the organic film.

FIG. 3 shows a cross-sectional view showing a light-emitting apparatus according to one or more embodiments of the present disclosure.

The light-emitting apparatus of FIG. 3 is substantially the same as the light-emitting apparatus of FIG. 2, except that a light-shielding pattern 500 and a functional region 400 are additionally arranged on the encapsulation portion 300. The functional region 400 may be i) a color filter area, ii) a color conversion area, or iii) a combination of the color filter area and the color conversion area. In one or more embodiments, the light-emitting device included in the light-emitting apparatus of FIG. 3 may be a tandem light-emitting device.

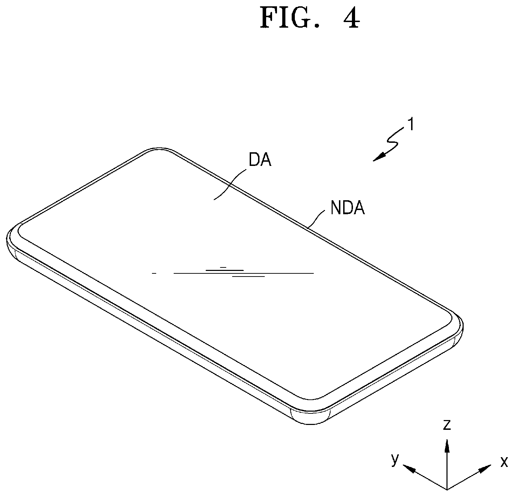

### Description of FIG. 4

FIG. 4 is a schematic perspective view of electronic equipment 1 including the light-emitting device according to one or more embodiments of the present disclosure. The electronic equipment 1 may be, as an electronic apparatus that displays a moving image or a still image, a portable electronic equipment, such as a mobile phone, a smartphone, a tablet personal computer (PC), a mobile communication terminal, an electronic notebook, an electronic book, a portable multimedia player (PMP), a navigation, or an ultra-mobile PC (UMPC), as well as one or more suitable products, such as a television, a laptop, a monitor, a billboard, or an Internet of things (IoT) device. The electronic equipment 1 may be such a product above or a part thereof. In one or more embodiments, the electronic equipment 1 may be a wearable device, such as a smart watch, a watch phone, a glasses-type (kind) display, or a head mounted display (HMD), or a part of the wearable device. However, embodiments of the present disclosure are not limited thereto. In one or more embodiments, the electronic device 1 may be a dashboard of a vehicle, a center information display (CID) arranged on a center fascia or dashboard of a vehicle, a room mirror display instead of a side-view mirror of a vehicle, an entertainment for a back seat of a vehicle, or a display arranged on the back of a front seat of a vehicle, a head up display (HUD) installed on the front of a vehicle or projected on a front window glass thereof, or a computer generated hologram augmented reality head up display (CGH AR HUD). FIG. 4 illustrates one or more embodiments in which the electronic equipment 1 is a smartphone for convenience of explanation.

The electronic equipment 1 may include a display area DA and a non-display area NDA outside the display area DA. A display apparatus may implement an image through an array of a plurality of pixels that are two-dimensionally arranged in the display area DA.

The non-display area NDA is an area that does not display an image, and may entirely be around (e.g., surround) the display area DA. On the non-display area NDA, a driver for providing electrical signals or power to display devices arranged on the display area DA may be arranged. On the non-display area NDA, a pad, which is an area to which an electronic element or a printed circuit board, may be electrically connected may be arranged.

In the electronic equipment 1, a length in an x-axis direction and a length (e.g., a width) in a y-axis direction may be different from each other. In one or more embodiments, as shown in FIG. 4, the length in the x-axis direction may be less than the length (e.g., the width) in the y-axis direction. In one or more embodiments, the length in the x-axis direction may be substantially the same as the length (e.g., the width) in the y-axis direction. In one or more embodiments, the length in the x-axis direction may be greater than the length (e.g., the width) in the y-axis direction.

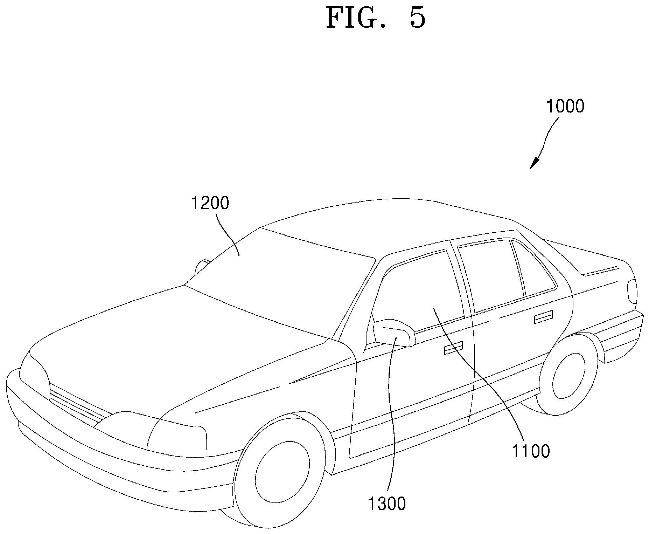

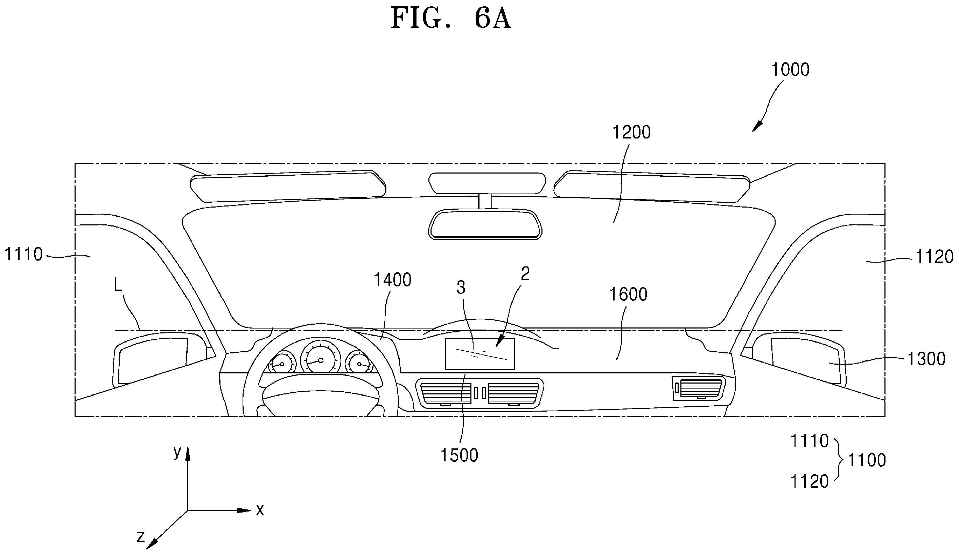

### Descriptions of FIGS. 5 and 6A to 6C

FIG. 5 is a schematic view of an exterior of a vehicle 1000 as electronic equipment including the light-emitting device according to one or more embodiments of the present disclosure. FIGS. 6A to 6C are each a schematic view of an interior of the vehicle 1000 according to one or more embodiments.

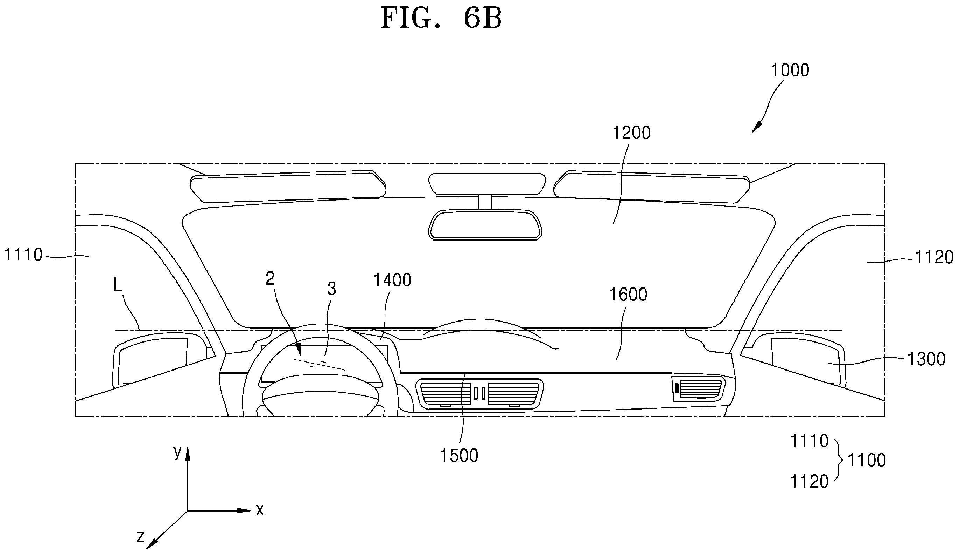

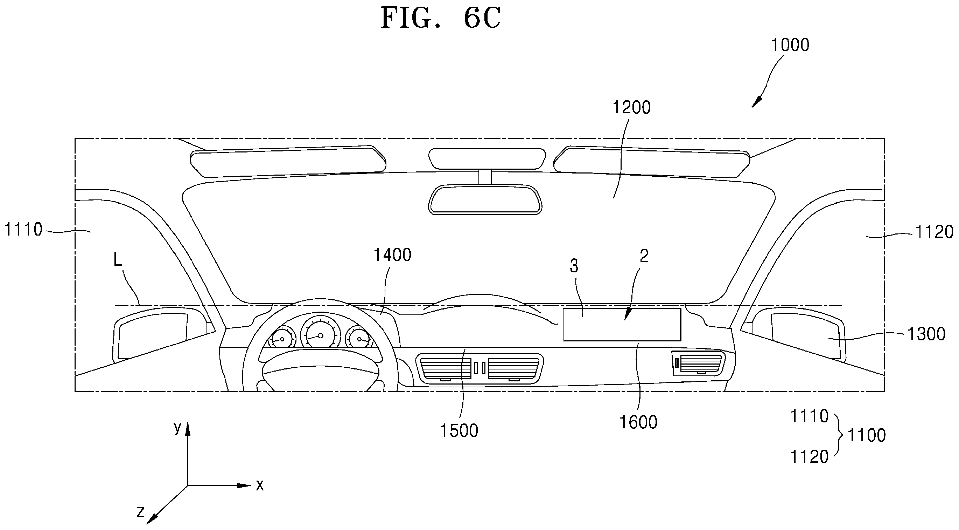

Referring to FIGS. 5, 6A, 6B, and 6C, the vehicle 1000 may refer to one or more suitable apparatuses for moving an object to be transported, such as a human, an object, or an animal, from a departure point to a destination point. The vehicle 1000 may include a vehicle traveling on a road or a track, a vessel moving over the sea or a river, an airplane flying in the sky using the action of air, and/or the like.

In one or more embodiments, the vehicle 1000 may travel on a road or a track. The vehicle 1000 may move in a certain direction according to rotation of at least one wheel thereof. In one or more embodiments, the vehicle 1000 may include a three-wheeled or four-wheeled vehicle, a construction machine, a two-wheeled vehicle, a prime mover device, a bicycle, or a train running on a track.

The vehicle 1000 may include a body having an interior and an exterior, and a chassis in which mechanical apparatuses necessary for driving are installed as other parts except for the body of the vehicle 1000. The exterior of the body of the vehicle may include a front panel, a bonnet, a roof panel, a rear panel, a trunk, a pillar provided at a boundary between doors, and/or the like. The chassis of the vehicle 1000 may include a power generating device, a power transmitting device, a driving device, a steering device, a braking device, a suspension device, a transmission device, a fuel device, front and rear wheels, left and right wheels, and/or the like.

The vehicle 1000 may include a side window glass 1100, a front window glass 1200, a side-view mirror 1300, a cluster 1400, a center fascia 1500, a passenger seat dashboard 1600, and a display apparatus 2.

The side window glass 1100 and the front window glass 1200 may be partitioned by a pillar arranged between the side window glass 1100 and the front window glass 1200.

The side window glass 1100 may be installed on a side of the vehicle 1000. In one or more embodiments, the side window glass 1100 may be installed on a door of the vehicle 1000. A plurality of side window glasses 1100 may be provided and may face each other. In one or more embodiments, the side window glass 1100 may include a first side window glass 1110 and a second side window glass 1120. In one or more embodiments, the first side window glass 1110 may be arranged adjacent to the cluster 1400. The second side window glass 1120 may be arranged adjacent to the passenger seat dashboard 1600.

In one or more embodiments, the side window glasses 1100 may be spaced and/or apart (e.g., spaced apart or separated) from each other in an x direction or a -x direction (the direction opposite the x-direction). In one or more embodiments, the first side window glass 1110 and the second side window glass 1120 may be spaced and/or apart (e.g., spaced apart or separated) from each other in the x direction or the - x direction. For example, an imaginary straight line L connecting the side window glasses 1100 may extend in the x direction or the -x direction. In one or more embodiments, an imaginary straight line L connecting the first side window glass 1110 and the second side window glass 1120 to each other may extend in the x direction or the -x direction.

The front window glass 1200 may be installed in the front of the vehicle 1000. The front window glass 1200 may be arranged between the side window glasses 1100 opposite to (e.g., facing) each other.

The side-view mirror 1300 may provide a rear view of the vehicle 1000. The side-view mirror 1300 may be installed on the exterior of the body of the vehicle. In one or more embodiments, a plurality of side-view mirrors 1300 may be provided. Any one of the plurality of side-view mirrors 1300 may be arranged outside the first side window glass 1110. The other one of the plurality of side-view mirrors 1300 may be arranged outside the second side window glass 1120.

The cluster 1400 may be arranged in front of the steering wheel. The cluster 1400 may include a tachometer, a speedometer, a coolant thermometer, a fuel gauge, a turn signal indicator, a high beam indicator, a warning light, a seat belt warning light, an odometer, a tachograph, an automatic shift selector indicator, a door open warning light, an engine oil warning light, and/or a low fuel warning light.

The center fascia 1500 may include a control panel on which a plurality of buttons for adjusting an audio device, an air conditioning device, and/or a seat heater are arranged. The center fascia 1500 may be arranged on one side of the cluster 1400.

The passenger seat dashboard 1600 may be spaced and/or apart (e.g., spaced apart or separated) from the cluster 1400, and the center fascia 1500 may be arranged between the cluster 1400 and the passenger seat dashboard 1600. In one or more embodiments, the cluster 1400 may be arranged to correspond to a driver seat, and the passenger seat dashboard 1600 may be arranged to correspond to a passenger seat. In one or more embodiments, the cluster 1400 may be adjacent to the first side window glass 1110, and the passenger seat dashboard 1600 may be adjacent to the second side window glass 1120.

In one or more embodiments, the display apparatus 2 may include a display panel 3, and the display panel 3 may display an image. The display apparatus 2 may be arranged inside the vehicle 1000. In one or more embodiments, the display apparatus 2 may be arranged between the side window glasses 1100 opposite to (e.g., facing) each other. The display apparatus 2 may be arranged on at least one of the cluster 1400, the center fascia 1500, or the passenger seat dashboard 1600.

The display apparatus 2 may include an organic light-emitting display apparatus, an inorganic electroluminescent display apparatus, a quantum dot display apparatus, and/or the like. Hereinafter, as the display apparatus 2 according to one or more embodiments, an organic light-emitting display apparatus including the light-emitting device will be described as an example, but one or more suitable types (kinds) of display apparatuses as described above may be used in embodiments.

Referring to FIG. 6A, in one or more embodiments, the display apparatus 2 may be arranged on the center fascia 1500. In one or more embodiments, the display apparatus 2 may display navigation information. In one or more embodiments, the display apparatus 2 may display information regarding audio settings, video setting, and/or vehicle settings.

Referring to FIG. 6B, in one or more embodiments, the display apparatus 2 may be arranged on the cluster 1400. In these embodiments, the cluster 1400 may display driving information and/or the like through the display apparatus 2. For example, the cluster 1400 may be implemented in a digital manner. The digital cluster 1400 implemented in a digital manner may display vehicle information and driving information in the form of images. In one or more embodiments, a needle and a gauge of a tachometer and one or more suitable warning light icons may be displayed by a digital signal.

Referring to FIG. 6C, in one or more embodiments, the display apparatus 2 may be arranged on the passenger seat dashboard 1600. The display apparatus 2 may be embedded in the passenger seat dashboard 1600 or arranged on the passenger seat dashboard 1600. In one or more embodiments, the display apparatus 2 arranged on the passenger seat dashboard 1600 may display an image related to information displayed on the cluster 1400 and/or information displayed on the center fascia 1500. In one or more embodiments, the display apparatus 2 arranged on the passenger seat dashboard 1600 may display information different from information displayed on the cluster 1400 and/or information displayed on the center fascia 1500.

### Manufacturing method

Layers constituting the hole transport region, the emission layer, and layers constituting the electron transport region may be formed in a certain region by using one or more suitable methods such as vacuum deposition, spin coating, casting, Langmuir-Blodgett (LB) deposition, ink-jet printing, laser-printing, laser-induced thermal imaging, and/or the like.

When the layers constituting the hole transport region, the emission layer, and the layers constituting the electron transport region are formed by vacuum deposition, the deposition may be performed at a deposition temperature in a range of about 100 °C to about 500 °C, at a vacuum degree in a range of about 10⁻⁸ torr to about 10⁻³ torr, and at a deposition speed in a range of about 0.01 Å/sec to about 100 Å/sec, depending on a material to be included in a layer to be formed and the structure of a layer to be formed.

### Definition of Terms

The term "C₃-C₆₀ carbocyclic group" as used herein refers to a cyclic group including carbon atoms as the only ring-forming atoms and having three to sixty carbon atoms, and the term "C₁-C₆₀ heterocyclic group" as used herein refers to a cyclic group that has one to sixty carbon atoms and further includes, in addition to carbon atom(s), a heteroatom as a ring-forming atom. The C₃-C₆₀ carbocyclic group and the C₁-C₆₀ heterocyclic group may each be a monocyclic group including (e.g., consisting of) one (exactly one) ring or a polycyclic group in which two or more rings are condensed with each other. In one or more embodiments, the number of ring-forming atoms of the C₁-C₆₀ heterocyclic group may be 3 to 61.

The term "cyclic group" as used herein may include both (e.g., simultaneously) the C₃-C₆₀ carbocyclic group and the C₁-C₆₀ heterocyclic group.

The term "π electron-rich C₃-C₆₀ cyclic group" as used herein refers to a cyclic group that has three to sixty carbon atoms and does not include *-N=*' as a ring-forming moiety, and the term "π electron-deficient nitrogen-containing C₁-C₆₀ cyclic group" as used herein refers to a heterocyclic group that has one to sixty carbon atoms and includes *-N=*' as a ring-forming moiety.

In one or more embodiments,
the C₃-C₆₀ carbocyclic group may be i) Group T1 or ii) a condensed cyclic group in which two or more of Group T1 are condensed with each other (for example, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, or an indenoanthracene group),
the C₁-C₆₀ heterocyclic group may be i) Group T2, ii) a condensed cyclic group in which two or more of Group T2 are condensed with each other, or iii) a condensed cyclic group in which at least one Group T2 and at least one Group T1 are condensed with each other (for example, a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and/or the like),
the π electron-rich C₃-C₆₀ cyclic group may be i) Group T1, ii) a condensed cyclic group in which two or more of Group T1 are condensed with each other, iii) Group T3, iv) a condensed cyclic group in which two or more of Group T3 are condensed with each other, or v) a condensed cyclic group in which at least one Group T3 and at least one Group T1 are condensed with each other (for example, the C₃-C₆₀ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, and/or the like),
the π electron-deficient nitrogen-containing C₁-C₆₀ cyclic group may be i) Group T4, ii) a condensed cyclic group in which two or more of Group T4 are condensed with each other, iii) a condensed cyclic group in which at least one Group T4 and at least one Group T1 are condensed with each other, iv) a condensed cyclic group in which at least one Group T4 and at least one Group T3 are condensed with each other, or v) a condensed cyclic group in which at least one Group T4, at least one Group T1, and at least one Group T3 are condensed with one another (for example, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and/or the like),
Group T1 may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or bicyclo[2.2.1]heptane) group, a norbornene group, a bicyclo[1.1.1]pentane group, a bicyclo[2.1.1]hexane group, a bicyclo[2.2.2]octane group, or a benzene group,
Group T2 may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group,
Group T3 may be a furan group, a thiophene group, a 1H-pyrrole group, a silole group, or a borole group, and
Group T4 may be a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, or a tetrazine group.

The terms "cyclic group", "C₃-C₆₀ carbocyclic group", "C₁-C₆₀ heterocyclic group", "π electron-rich C₃-C₆₀ cyclic group", or "π electron-deficient nitrogen-containing C₁-C₆₀ cyclic group" as used herein may refer to a group condensed to any cyclic group, a monovalent group, or a polyvalent group (for example, a divalent group, a trivalent group, a tetravalent group, and/or the like) according to the structure of a formula for which the corresponding term is used. In one or more embodiments, "a benzene group" may be a benzo group, a phenyl group, a phenylene group, and/or the like, which may be easily understand by one of ordinary skill in the art according to the structure of a formula including the "benzene group."

Non-limiting examples of the monovalent C₃-C₆₀carbocyclic group and the monovalent C₁-C₆₀ heterocyclic group may include a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group. Non-limiting examples of the divalent C₃-C₆₀ carbocyclic group and the divalent C₁-C₆₀ heterocyclic group may include a C₃-C₁₀ cycloalkylene group, a C₁-C₁₀ heterocycloalkylene group, a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a divalent non-aromatic condensed heteropolycyclic group.

The term "C₁-C₆₀ alkyl group" as used herein refers to a linear or branched aliphatic hydrocarbon monovalent group that has one to sixty carbon atoms, and non-limiting examples thereof include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, and a tert-decyl group. The term "C₁-C₆₀ alkylene group" as used herein refers to a divalent group having substantially the same structure as the C₁-C₆₀ alkyl group.

The term "C₂-C₆₀ alkenyl group" as used herein refers to a monovalent hydrocarbon group having at least one carbon-carbon double bond in the middle or at the terminus of a C₂-C₆₀ alkyl group, and non-limiting examples thereof include an ethenyl group, a propenyl group, and a butenyl group. The term "C₂-C₆₀ alkenylene group" as used herein refers to a divalent group having substantially the same structure as the C₂-C₆₀ alkenyl group.

The term "C₂-C₆₀ alkynyl group" as used herein refers to a monovalent hydrocarbon group having at least one carbon-carbon triple bond in the middle or at the terminus of a C₂-C₆₀ alkyl group, and non-limiting examples thereof include an ethynyl group and a propynyl group. The term "C₂-C₆₀ alkynylene group" as used herein refers to a divalent group having substantially the same structure as the C₂-C₆₀ alkynyl group.

The term "C₁-C₆₀ alkoxy group" as used herein refers to a monovalent group represented by -OA₁₀₁ (wherein A₁₀₁ is a C₁-C₆₀ alkyl group), and non-limiting examples thereof include a methoxy group, an ethoxy group, and an isopropyloxy group.

The term "C₃-C₁₀ cycloalkyl group" as used herein refers to a monovalent saturated hydrocarbon cyclic group having 3 to 10 carbon atoms, and non-limiting examples thereof may include a cyclopropyl group, a cyclobutyl group, a cyclopentyl group, a cyclohexyl group, a cycloheptyl group, a cyclooctyl group, an adamantanyl (i.e., adamantyl) group, a norbornanyl (i.e., norbornyl) group (or bicyclo[2.2.1]heptyl group), a bicyclo[1.1.1]pentyl group, a bicyclo[2.1.1]hexyl group, a bicyclo[2.2.2]octyl group, and/or the like. The term "C₃-C₁₀ cycloalkylene group" as used herein refers to a divalent group having substantially the same structure as the C₃-C₁₀ cycloalkyl group.

The term "C₁-C₁₀ heterocycloalkyl group" as used herein refers to a monovalent cyclic group that has one to ten carbon atoms and further includes, in addition to the carbon atoms, at least one heteroatom as a ring-forming atom, and non-limiting examples thereof include a 1,2,3,4-oxatriazolidinyl group, a tetrahydrofuranyl group, and a tetrahydrothiophenyl group. The term "C₁-C₁₀ heterocycloalkylene group" as used herein refers to a divalent group having substantially the same structure as the C₁-C₁₀ heterocycloalkyl group.

The term "C₃-C₁₀ cycloalkenyl group" as used herein refers to a monovalent cyclic group that has three to ten carbon atoms and at least one carbon-carbon double bond in the ring thereof and no aromaticity, and non-limiting examples thereof include a cyclopentenyl group, a cyclohexenyl group, and a cycloheptenyl group. The term "C₃-C₁₀ cycloalkenylene group" as used herein refers to a divalent group having substantially the same structure as the C₃-C₁₀ cycloalkenyl group.

The term "C₁-C₁₀ heterocycloalkenyl group" as used herein refers to a monovalent cyclic group that has one to ten carbon atoms, further includes, in addition to the carbon atoms, at least one heteroatom as a ring-forming atom, and has at least one double bond in the ring thereof. Non-limiting examples of the C₁-C₁₀ heterocycloalkenyl group include a 4,5-dihydro-1,2,3,4-oxatriazolyl group, a 2,3-dihydrofuranyl group, and a 2,3-dihydrothiophenyl group. The term "C₁-C₁₀ heterocycloalkenylene group" as used herein refers to a divalent group having substantially the same structure as the C₁-C₁₀ heterocycloalkenyl group.

The term "C₆-C₆₀ aryl group" as used herein refers to a monovalent group having a carbocyclic aromatic system of six to sixty carbon atoms, and the term "C₆-C₆₀ arylene group" as used herein refers to a divalent group having a carbocyclic aromatic system of six to sixty carbon atoms. Non-limiting examples of the C₆-C₆₀ aryl group include a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, and an ovalenyl group. When the C₆-C₆₀ aryl group and the C₆-C₆₀ arylene group each include two or more rings, the two or more rings may be condensed with each other.

The term "C₁-C₆₀ heteroaryl group" as used herein refers to a monovalent group having a heterocyclic aromatic system that has one to sixty carbon atoms and further includes, in addition to the carbon atoms, at least one heteroatom as a ring-forming atom. The term "C₁-C₆₀ heteroarylene group" as used herein refers to a divalent group having a heterocyclic aromatic system that has one to sixty carbon atoms and further includes, in addition to the carbon atoms, at least one heteroatom as a ring-forming atom. non-limiting examples of the C₁-C₆₀ heteroaryl group include a pyridinyl group, a pyrimidinyl group, a pyrazinyl group, a pyridazinyl group, a triazinyl group, a quinolinyl group, a benzoquinolinyl group, an isoquinolinyl group, a benzoisoquinolinyl group, a quinoxalinyl group, a benzoquinoxalinyl group, a quinazolinyl group, a benzoquinazolinyl group, a cinnolinyl group, a phenanthrolinyl group, a phthalazinyl group, and a naphthyridinyl group. When the C₁-C₆₀ heteroaryl group and the C₁-C₆₀ heteroarylene group each include two or more rings, the two or more rings may be condensed with each other.

The term "monovalent non-aromatic condensed polycyclic group" as used herein refers to a monovalent group having two or more rings condensed with each other, only carbon atoms (for example, eight to sixty carbon atoms) as ring-forming atoms, and no aromaticity in its molecular structure if (e.g., when) considered as a whole. Non-limiting examples of the monovalent non-aromatic condensed polycyclic group include an indenyl group, a fluorenyl group, a spiro-bifluorenyl group, a benzofluorenyl group, an indenophenanthrenyl group, and an indeno anthracenyl group. The term "divalent non-aromatic condensed polycyclic group" as used herein refers to a divalent group having substantially the same structure as the monovalent non-aromatic condensed polycyclic group.

The term "monovalent non-aromatic condensed heteropolycyclic group" as used herein refers to a monovalent group that has two or more rings condensed with each other, further includes, in addition to carbon atoms (for example, one to sixty carbon atoms), at least one heteroatom as a ring-forming atom, and has no aromaticity in its molecular structure if (e.g., when) considered as a whole. Non-limiting examples of the monovalent non-aromatic condensed heteropolycyclic group include a pyrrolyl group, a thiophenyl group, a furanyl group, an indolyl group, a benzoindolyl group, a naphthoindolyl group, an isoindolyl group, a benzoisoindolyl group, a naphthoisoindolyl group, a benzosilolyl group, a benzothiophenyl group, a benzofuranyl group, a carbazolyl group, a dibenzosilolyl group, a dibenzothiophenyl group, a dibenzofuranyl group, an azacarbazolyl group, an azafluorenyl group, an azadibenzosilolyl group, an azadibenzothiophenyl group, an azadibenzofuranyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, a benzoxazolyl group, a benzothiazolyl group, a benzoxadiazolyl group, a benzothiadiazolyl group, an imidazopyridinyl group, an imidazopyrimidinyl group, an imidazotriazinyl group, an imidazopyrazinyl group, an imidazopyridazinyl group, an indeno carbazolyl group, an indolocarbazolyl group, a benzofurocarbazolyl group, a benzothienocarbazolyl group, a benzosilolocarbazolyl group, a benzoindolocarbazolyl group, a benzocarbazolyl group, a benzonaphthofuranyl group, a benzonaphthothiophenyl group, a benzonaphthosilolyl group, a benzofurodibenzofuranyl group, a benzofurodibenzothiophenyl group, and a benzothienodibenzothiophenyl group. The term "divalent non-aromatic condensed heteropolycyclic group" as used herein refers to a divalent group having substantially the same structure as the monovalent non-aromatic condensed heteropolycyclic group.

The term "C₆-C₆₀ aryloxy group" as used herein refers to -OA₁₀₂ (wherein A₁₀₂ is a C₆-C₆₀ aryl group), and the term "C₆-C₆₀ arylthio group" as used herein refers to -SA₁₀₃ (wherein A₁₀₃ is a C₆-C₆₀ aryl group).

The term "C₇-C₆₀ arylalkyl group" as used herein refers to -A₁₀₄A₁₀₅ (wherein A₁₀₄ is a C₁-C₅₄ alkylene group, and A₁₀₅ is a C₆-C₅₉ aryl group), and the term "C₂-C₆₀ heteroarylalkyl group" as used herein refers to -A₁₀₆A₁₀₇ (wherein A₁₀₆ is a C₁-C₅₉ alkylene group, and A₁₀₇ is a C₁-C₅₉ heteroaryl group).

The term "R₁₀₂" as used herein refers to:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, or a C₂-C₆₀ heteroaryl alkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₂₁)(Q₂₂)(Q₂₃), -N(Q₂₁)(Q₂₂), -B(Q₂₁)(Q₂₂), - C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ used herein may each independently be: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof; a C₇-C₆₀ arylalkyl group; or a C₂-C₆₀ heteroarylalkyl group.

The term "heteroatom" as used herein refers to any atom other than a carbon atom or a hydrogen atom. Non-limiting examples of the heteroatom include O, S, N, P, Si, B, Ge, Se, or any combination thereof.

The term "transition metal" used herein may include hafnium (Hf), tantalum (Ta), tungsten (W), rhenium (Re), osmium (Os), iridium (Ir), platinum (Pt), gold (Au), and/or the like.

The term "Ph" as used herein refers to a phenyl group, the term "Me" as used herein refers to a methyl group, the term "Et" as used herein refers to an ethyl group, the term "tert-Bu" or "Bu^{t}" as used herein refers to a tert-butyl group, and the term "OMe" as used herein refers to a methoxy group.

The term "biphenyl group" as used herein refers to "a phenyl group substituted with a phenyl group." For example, the "biphenyl group" is a substituted phenyl group having a C₆-C₆₀ aryl group as a substituent.

The term "terphenyl group" as used herein refers to "a phenyl group substituted with a biphenyl group." The "terphenyl group" refers to "a substituted phenyl group" having, as a substituent, "a C₆-C₆₀ aryl group substituted with a C₆-C₆₀ aryl group," and may include an o-terphenyl group, a m-terphenyl group, and a p-terphenyl group.
* and *' as used herein, unless defined otherwise, each refer to a binding site to a neighboring atom in a corresponding formula or moiety.

The terms "x-axis", "y-axis", and "z-axis" as used herein are not limited to three axes in an orthogonal coordinate system, and may be interpreted in a broader sense than the aforementioned three axes in an orthogonal coordinate system. For example, the x-axis, y-axis, and z-axis may describe axes that are orthogonal to each other, or may describe axes that are in different directions that are not orthogonal to each other.

Hereinafter, compounds according to one or more embodiments and light-emitting devices according to one or more embodiments will be described in more detail with reference to the following Synthesis Examples and Examples. The wording "B was used instead of A" used in describing Synthesis Examples refers to that an substantially identical molar equivalent of B was used in place of A.

### Examples

### Synthesis Example 1 (Compound 31)

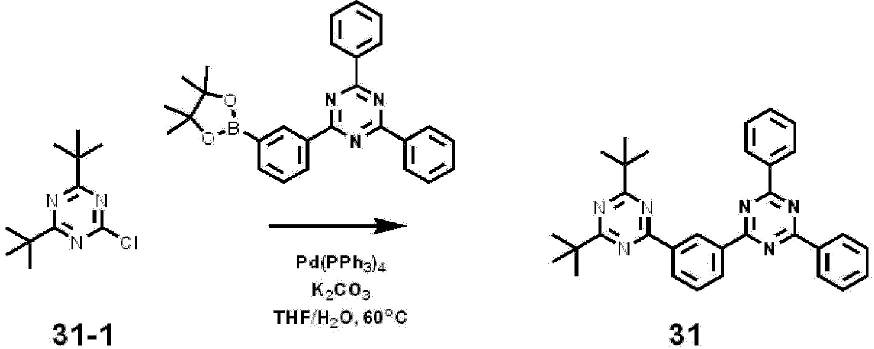

Intermediate 31-1 (2.27 g), Pd(PPh₃)₄ (0.56 g), K₂CO₃ (3.45 g), and 2,4-diphenyl-6-(3-(4,4,5,5-tetramethyl-1,3,2-dioxaborolan-2-yl)phenyl)-1,3,5-triazine (4.35 g) were dissolved in tetrahydrofuran (THF)/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Compound 31 (3.50 g, yield: 70%).

### Synthesis Example 2 (Compound 59)

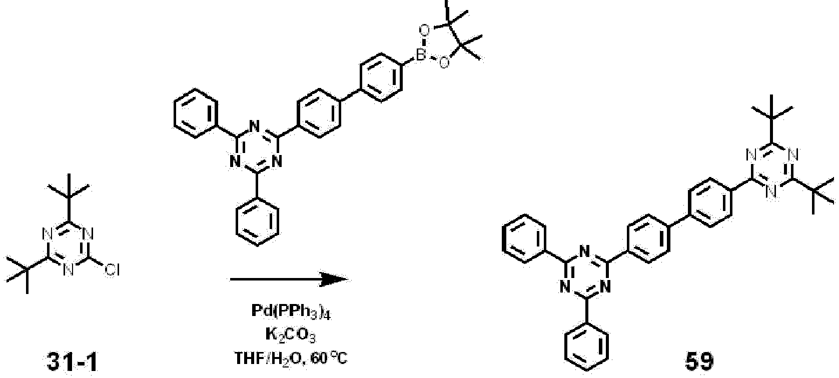

Intermediate 31-1 (2.27 g), Pd(PPh₃)₄ (0.56 g), K₂CO₃ (3.45 g), and 2,4-diphenyl-6-(4'-(4,4,5,5-tetramethyl-1,3,2-dioxaborolan-2-yl)-[1,1'-biphenyl]-4-yl)-1,3,5-triazine (5.11 g) were dissolved in THF/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Compound 59 (4.03 g, yield: 70%).

### Synthesis Example 3 (Compound 135)

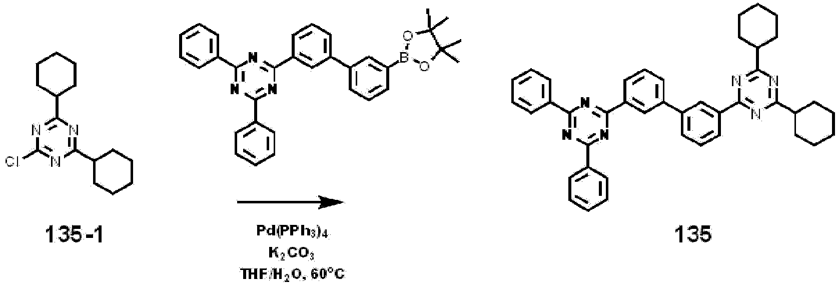

Intermediate 135-1 (2.79 g), Pd(PPh₃)₄ (0.56 g), K₂CO₃ (3.45 g), and 2,4-diphenyl-6-(3'-(4,4,5,5-tetramethyl-1,3,2-dioxaborolan-2-yl)-[1,1'-biphenyl]-3-yl)-1,3,5-triazine (5.11 g) were dissolved in THF/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Compound 135 (4.39 g, yield: 70%).

### Synthesis Example 4 (Compound 143)

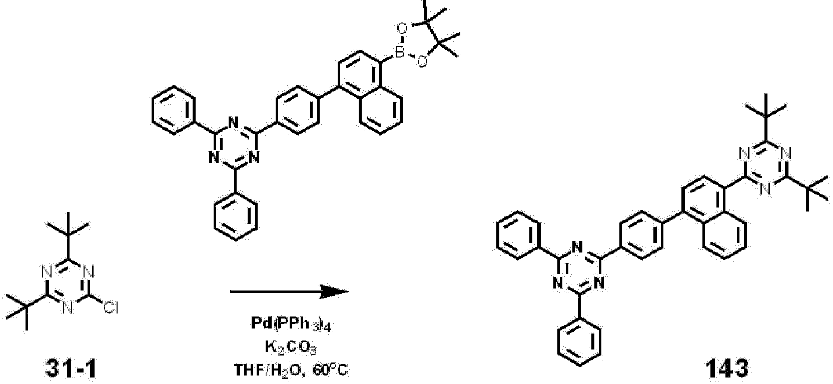

Intermediate 31-1 (2.27 g), Pd(PPh₃)₄ (0.56 g), K₂CO₃ (3.45 g), and 2,4-diphenyl-6-(4-(4-(4,4,5,5-tetramethyl-1,3,2-dioxaborolan-2-yl)naphthalen-1-yl)phenyl)-1,3,5-triazine (5.61 g) were dissolved in THF/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Compound 143 (4.00 g, yield: 64%).

### Synthesis Example 5 (Compound 171)

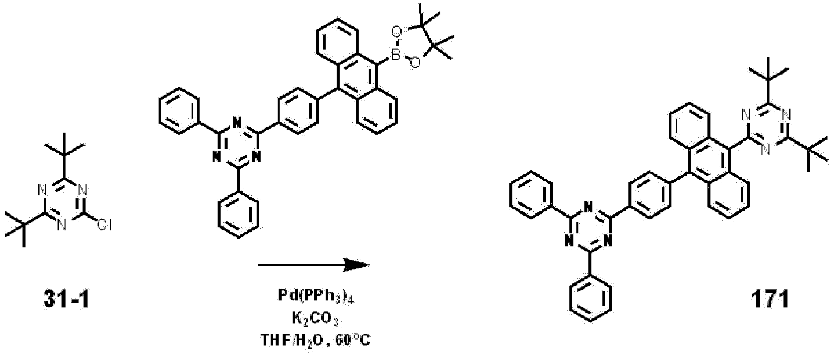

Intermediate 31-1 (2.27 g), Pd(PPh₃)₄ (0.56 g), K₂CO₃ (3.45 g), and 2,4-diphenyl-6-(4-(10-(4,4,5,5-tetramethyl-1,3,2-dioxaborolan-2-yl)anthracen-9-yl)phenyl)-1,3,5-triazine (6.11 g) were dissolved in THF/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Compound 171 (4.46 g, yield: 66%).

### Synthesis Example 6 (Compound 265)

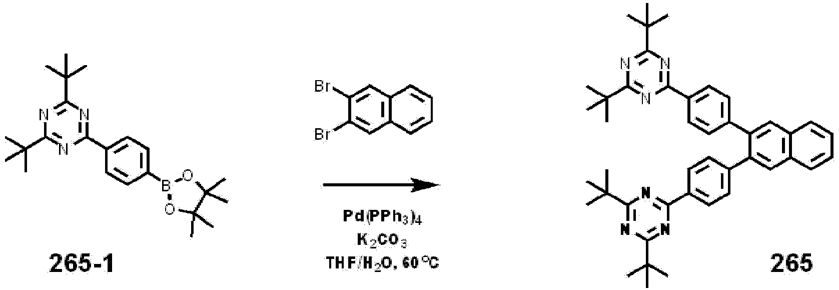

Intermediate 265-1 (3.95 g), Pd(PPh₃)₄ (0.56 g), K₂CO₃ (3.45 g), and 2,3-dibromonaphthalene (2.85 g) were dissolved in THF/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Compound 265 (4.43 g, yield: 67%).

### Synthesis Example 7 (Compound 277)

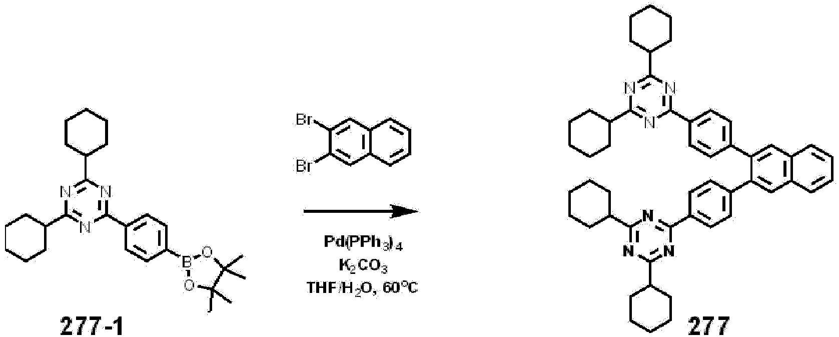

Intermediate 277-1 (4.47 g), Pd(PPh₃)₄ (0.56 g), K₂CO₃ (3.45 g), and 2,3-dibromonaphthalene (2.85 g) were dissolved in THF/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Compound 277 (5.44 g, yield: 71%).

### Synthesis Example 8 (Compound 410)

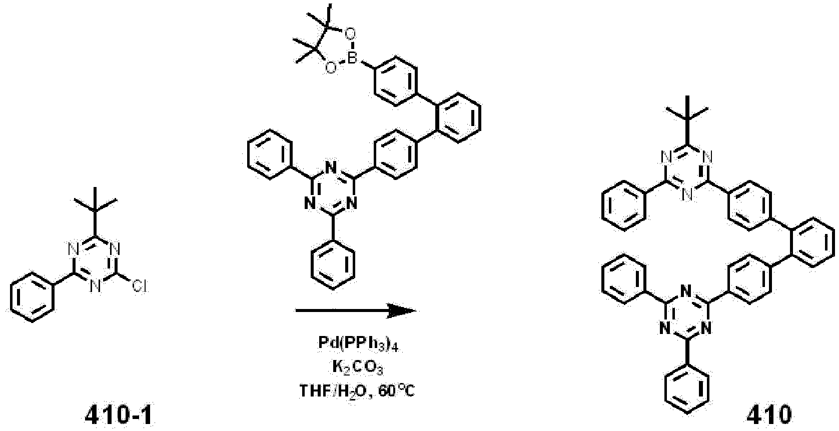

Intermediate 410-1 (2.47 g), Pd(PPh₃)₄ (0.56 g), K₂CO₃ (3.45 g), and 2,4-diphenyl-6-(4"-(4,4,5,5-tetramethyl-1,3,2-dioxaborolan-2-yl)-[1,1':2',1"-terphenyl]-4-yl)-1,3,5-triazine (5.87 g) were dissolved in THF/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Compound 410 (5.04 g, yield: 75%).

### Synthesis Example 9 (Compound 436)

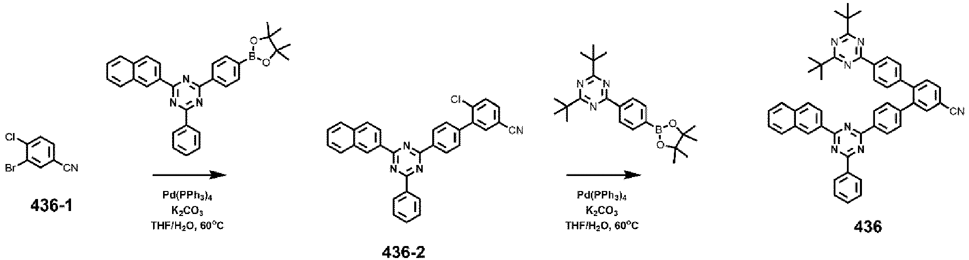

### Synthesis of Intermediate 436-2

Intermediate 436-1 (2.16 g), Pd(PPh₃)₄ (0.56 g), K₂CO₃ (3.45 g), and 2-(naphthalen-2-yl)-4-phenyl-6-(4-(4,4,5,5-tetramethyl-1,3,2-dioxaborolan-2-yl)phenyl)-1,3,5-triazine (4.85 g) were dissolved in THF/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Intermediate 436-2 (5.01 g, yield: 69%).

### Synthesis of Compound 436

Intermediate 436-2 (7.27 g), Pd(PPh₃)₄ (0.56 g), K₂CO₃ (3.45 g), and 2,4-di-tert-butyl-6-(4-(4,4,5,5-tetramethyl-1,3,2-dioxaborolan-2-yl)phenyl)-1,3,5-triazine (3.95 g) were dissolved in THF/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Compound 436 (4.58 g, yield: 63%).

### Synthesis Example 10 (Compound 465)

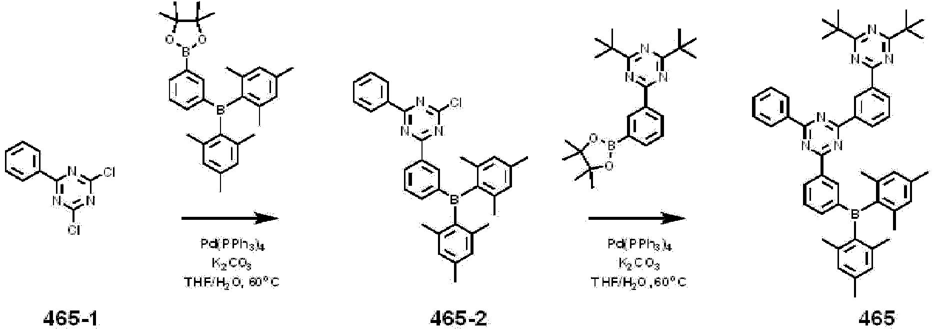

### Synthesis of Intermediate 465-2

Intermediate 465-1 (2.26 g), Pd(PPh₃)₄ (0.56 g), K₂CO₃ (3.45 g), and 2-(3-(dimesitylboranyl)phenyl)-4,4,5,5-tetramethyl-1,3,2-dioxaborolane (4.52 g) were dissolved in THF/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Intermediate 465-2 (3.39 g, yield: 66%).

### Synthesis of Compound 465

Intermediate 465-2 (5.15 g), Pd(PPh₃)₄ (0.56 g), K₂CO₃ (3.45 g), and 2,4-di-tert-butyl-6-(3-(4,4,5,5-tetramethyl-1,3,2-dioxaborolan-2-yl)phenyl)-1,3,5-triazine (3.95 g) were dissolved in THF/H₂O (80 mL//20 mL), and then stirred at a temperature of 60 °C for 12 hours. The reaction temperature was lowered to room temperature, and the reaction was terminated by using water. Then, an extraction process was performed thereon three times by using ethyl ether. The separated organic layer was dried over anhydrous magnesium sulfate and concentrated under reduced pressure. The resulting residue was separated and purified by column chromatography to obtain Compound 465 (5.16 g, yield: 69%).

For each of the compounds synthesized according to Synthesis Examples 1 to 10, the proton nuclear magnetic resonance (¹H NMR) data and high-resolution mass (HR-MS) were measured. Results thereof are shown in Table 1. Synthesis methods for compounds other than those synthesized in Synthesis Examples 1 to 10 can be readily recognized by those skilled in the art by referring to the synthesis routes and starting materials.

**Table 1**

| Compound | ¹H NMR (CDCl₃, 500 MHz) | HR-MS (m/z) [M⁺] | |
|---|---|---|---|
| | | found | calc. |
| Synthesis Example 1 | 8.36-8.38(m, 4H), 7.94(s, 1H), 7.73(t, 1H), 7.50(m, 6H), 1.35 (s, 18H) | 499.27 | 500.27 |
| Synthesis Example 2 | 8.36 (d, 4H), 7.96(d, 4H), 7.50(m, 6H), 7.25(d, 4H), 1.35 (s, 18H) | 575.30 | 576.30 |
| Synthesis Example 3 | 8.36-8.38 (m, 6H), 7.94(s, 2H), 7.73(t, 2H), 7.61(d, 2H), 7.50(m, 6H), 2.72 (q, 2H), 1.43-1.85(m, 20H) | 627.33 | 628.33 |
| Synthesis Example 4 | 9.02 (d, 1H), 8.95(d, 1H), 8.36(d, 4H), 8.06(d, 1H), 7.96(d, 2H) 7.84 (d, 2H), 7.50(m, 6H), 7.46-7.52(m, 8H), 7.25(d, 2H), 1.35(s, 18H) | 625.32 | 626.32 |
| Synthesis Example 5 | 8.36 (m, 4H), 8.19-8.22(m, 4H), 7.96(d, 2H), 7.40-7.50(m, 10H), 7.25(d, 2H), 1.35(s, 18H) | 675.33 | 676.33 |
| Synthesis Example 6 | 8.57 (s, 2H), 8.15(d, 2H), 7.96(d, 4H), 7.64(d, 2H), 7.25(d, 4H), 1.35(s, 36H) | 661.42 | 662.42 |
| Synthesis Example 7 | 8.57 (s, 2H), 8.15(d, 2H), 7.96(d, 4H), 7.64(d, 2H), 7.25(d, 4H), 2.72 (q, 2H), 1.43-1.85(m, 20H) | 765.47 | 766.47 |
| Synthesis Example 8 | 8.36 (m, 4H), 7.96(d, 6H), 7.60(d, 2H), 7.50(m, 9H), 7.25(d, 4H), 1.35(s, 18H) | 671.30 | 672.30 |
| Synthesis Example 9 | 9.09 (s, 1H), 8.49(d, 1H), 8.36(d, 2H), 7.92-8.16(m, 10H), 7.59-7.61 (m, 2H), 7.50(m, 3H), 7.25(d, 4H), 1.35(s, 18H) | 726.34 | 727.34 |
| Synthesis Example 10 | 8.36-8.38(m, 4H), 8.28(d, 1H), 7.94(s, 1H), 7.85(d, 1H), 7.73(t, 1H), 7.67(s, 1H), 7.50-7.54(m, 4H), 6.97(s, 4H), 2.33(s, 12H), 2.18(s, 6H), 1.35(s, 18H) | 747.44 | 748.44 |

### Example 1

As an anode, a glass substrate (product of Corning Inc.) with a 15 Ω/cm² (1,200 Å) ITO electrode formed thereon was cut to a size of 50 mm x 50 mm x 0.7 mm, sonicated with isopropyl alcohol and (then with) pure water for 5 minutes each, and then cleaned by irradiation of ultraviolet rays and exposure of ozone thereto for 30 minutes. Then, the resultant ITO glass substrate was mounted on a vacuum deposition apparatus.

NPD was deposited on the anode to form a hole injection layer having a thickness of 300 Å, and then HTL3 was deposited on this hole injection layer to form a hole transport layer having a thickness of 200 Å. CzSi was deposited on the hole transport layer to form an emission auxiliary layer having a thickness of 100 Å.

A host mixture of HT-3 and ET-2 mixed in a 5:5 ratio, PS-2 (sensitizer), and t-DABNA (dopant) were co-deposited on the emission auxiliary layer in a weight ratio of 84:15:1 to form an emission layer having a thickness of 200 Å, and TSPO1 was deposited on the emission layer to form a hole-blocking layer having a thickness of 200 Å. Subsequently, Compound 31 was deposited on the hole-blocking layer to form an electron transport layer having a thickness of 300 Å, and then LiF was deposited on the electron transport layer to form an electron injection layer having a thickness of 10 Å. Subsequently, a cathode having a thickness of 3000 Å was formed using Al.

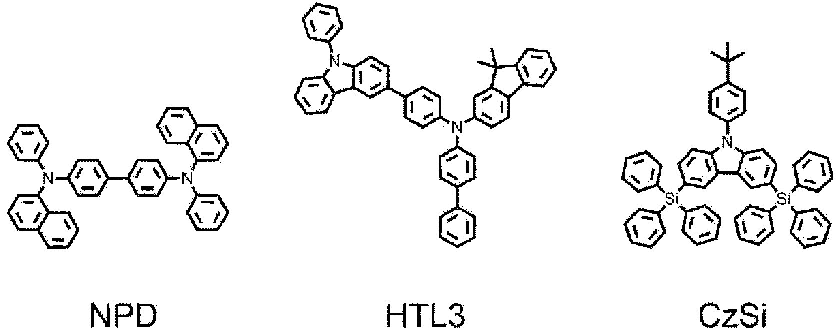

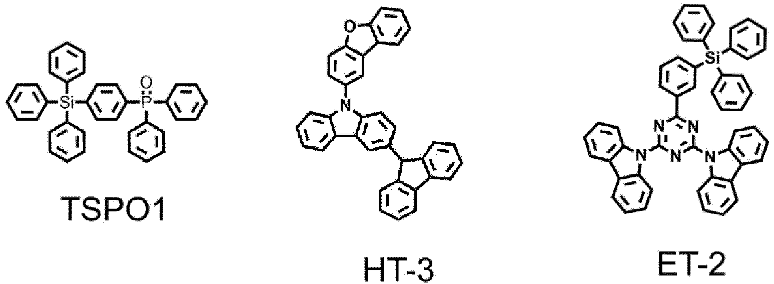

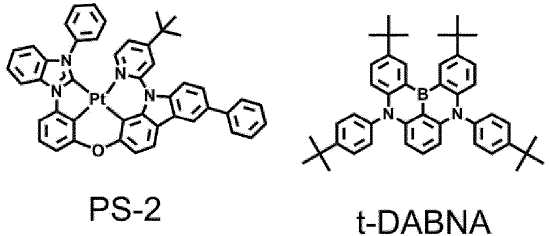

### Examples 2 to 10 and Comparative Examples 1 to 6

Instead of using Compound 31 in Example 1, light-emitting devices of Examples 2 to 10 and Comparative Examples 1 to 6 were each fabricated in substantially the same manner as Example 1, except for using Compound 59, Compound 135, Compound 143, Compound 171, Compound 265, Compound 277, Compound 410, Compound 436, Compound 465, and Comparative Example Compounds A to F, respectively.

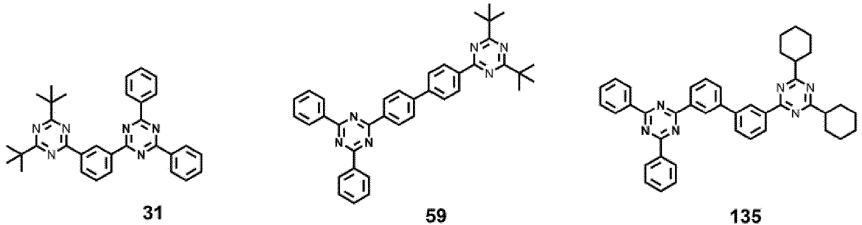

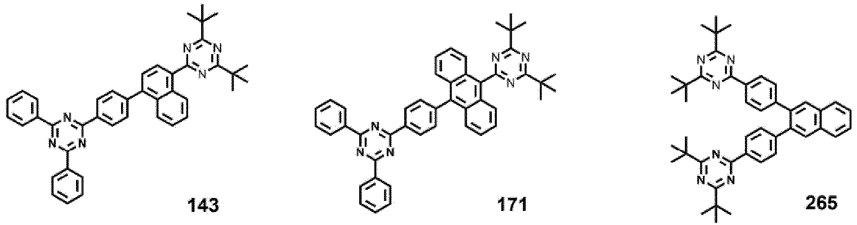

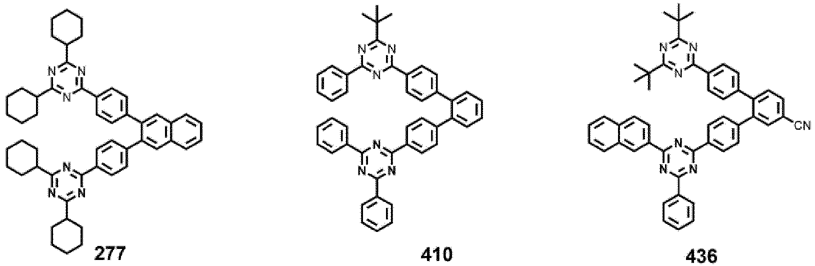

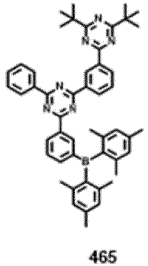

### Comparative Example Compound A

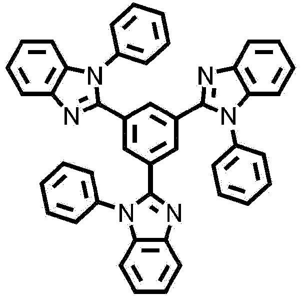

### Comparative Example Compound B

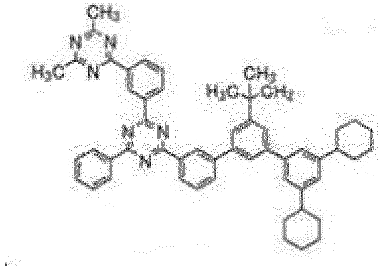

### Comparative Example Compound C

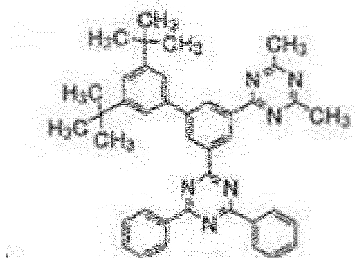

### Comparative Example Compound D

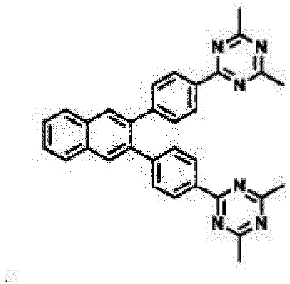

### Comparative Example Compound E

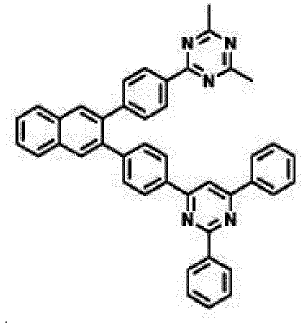

### Comparative Example Compound F

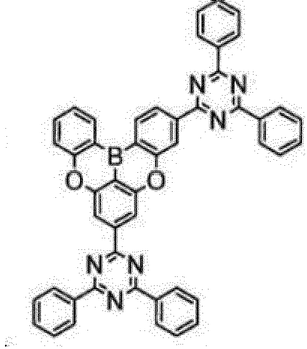

### Evaluation Example 1

The characteristics of each of the light-emitting devices manufactured in Examples 1 to 10 and Comparative Examples 1 to 6 were evaluated by measuring the driving voltage at a current density of 10 mA/cm², luminescence efficiency, and device lifespan. Results thereof are shown in Table 2. The driving voltage and luminescence efficiency of the organic light-emitting devices were measured using a V7000 OLED IVL test system (Polaronix). To measure the device lifespan, the time taken to reach 95% of the initial luminance for each of Examples 1 to 10 and Comparative Examples 1 to 6 was measured, and a relative lifespan (T₉₅) expressed by a ratio of the time taken to reach 95% of the initial luminance in each of Examples 1 to 10 and Comparative Example 2 and 6 to the time taken to reach 95% of the initial luminance in Comparative Example 1 was calculated.

**Table 2**

| | Driving voltage (V) | Luminescence efficiency (cd/A) | Relative lifespan (T₉₅) |
|---|---|---|---|
| Example 1 | 3.94 | 26.8 | 6.3 |
| Example 2 | 4.09 | 25.3 | 6.1 |
| Example 3 | 3.96 | 25.3 | 5.8 |
| Example 4 | 4.11 | 25.2 | 5.9 |
| Example 5 | 4.23 | 25.9 | 5.7 |
| Example 6 | 4.34 | 26.7 | 6.3 |
| Example 7 | 4.21 | 26.9 | 6.4 |
| Example 8 | 4.03 | 27.4 | 6.5 |
| Example 9 | 4.19 | 25.0 | 6.7 |
| Example 10 | 4.10 | 26.0 | 7.7 |
| Comparative Example 1 | 5.6 | 18.8 | 1 |
| Comparative Example 2 | 5.42 | 20.3 | 3.9 |
| Comparative Example 3 | 5.31 | 21.3 | 4.1 |
| Comparative Example 4 | 4.92 | 20.3 | 5.0 |
| Comparative Example 5 | 4.80 | 21.1 | 5.1 |
| Comparative Example 6 | 5.44 | 21.5 | 5.0 |

As can be seen in Table 2, the light-emitting devices of Examples 1 to 10 each show lower driving voltage, higher luminescence efficiency, and longer lifespan compared to the light-emitting devices of Comparative Examples 1 to 6, indicating overall superior device characteristics.

By using the heterocyclic compound, a light-emitting device having high efficiency and a long lifespan and a high-quality electronic apparatus including the light-emitting device may be manufactured.

In the present disclosure, it will be understood that the term "comprise(s)/comprising," "include(s)/including," or "have/has/having" specifies the presence of stated features, integers, steps, operations, elements, and/or components, but do not preclude the presence or addition of one or more other features, integers, steps, operations, elements, components, and/or groups thereof. Additionally, the terms "comprise(s)/comprising," "include(s)/including," "have/has/having", or other similar terms include or support the terms "consisting of" and "consisting essentially of," indicating the presence of stated features, integers, steps, operations, elements, and/or components, without or essentially without the presence of other features, integers, steps, operations, elements, components, and/or groups thereof.

In the context of the present application and unless otherwise defined, the terms "use," "using," and "used" may be considered synonymous with the terms "utilize," "utilizing," and "utilized," respectively.

Throughout the present disclosure, when a component such as a layer, a film, a region, or a plate is mentioned to be placed "on" another component, it will be understood that it may be directly on another component or that another component may be interposed therebetween. In some embodiments, "directly on" may refer to that there are no additional layers, films, regions, plates, etc., between a layer, a film, a region, a plate, etc. and the other part. For example, "directly on" may refer to two layers or two members are disposed without utilizing an additional member such as an adhesive member therebetween.

In the present disclosure, although the terms "first," "second," etc., may be utilized herein to describe one or more elements, components, regions, and/or layers, these elements, components, regions, and/or layers should not be limited by these terms. These terms are only utilized to distinguish one component from another component.

As utilized herein, the singular forms "a," "an," "one," and "the" are intended to include the plural forms as well, unless the context clearly indicates otherwise. Further, the use of "may" when describing embodiments of the present disclosure refers to "one or more embodiments of the present disclosure".

As utilized herein, the terms "substantially," "about," or similar terms are used as terms of approximation and not as terms of degree, and are intended to account for the inherent deviations in measured or calculated values that would be recognized by those of ordinary skill in the art. "About" as used herein, is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, or 5% of the stated value.

Any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in the present disclosure is intended to include all higher numerical limitations subsumed therein.

The light-emitting device, the light-emitting apparatus, the display device, the electronic apparatus, the electronic device/equipment, the manufacturing apparatus thereof, or any other relevant devices or components according to embodiments of the present disclosure described herein may be implemented utilizing any suitable hardware, firmware (e.g., an application-specific integrated circuit), software, or a combination of software, firmware, and hardware. For example, the various components of the device may be formed on one integrated circuit (IC) chip or on separate IC chips. Further, the various components of the device may be implemented on a flexible printed circuit film, a tape carrier package (TCP), a printed circuit board (PCB), or formed on one substrate. Further, the various components of the device may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random access memory (RAM). The computer program instructions may also be stored in other non-transitory computer readable media such as, for example, a CD-ROM, flash drive, or the like. Also, a person of skill in the art should recognize that the functionality of various computing devices may be combined or integrated into a single computing device, or the functionality of a particular computing device may be distributed across one or more other computing devices without departing from the scope of the embodiments of the present disclosure.

It should be understood that embodiments described herein should be considered in a descriptive sense only and not for purposes of limitation. Descriptions of features or aspects within each embodiment should typically be considered as available for other similar features or aspects in one or more embodiments. While one or more embodiments have been described with reference to the drawings, it will be understood by those of ordinary skill in the art that one or more suitable changes in form and details may be made therein without departing from the scope as defined by the appended claims and equivalents thereof.

## Claims

1. A light-emitting device comprising:
a first electrode;
a second electrode opposite to the first electrode;
an interlayer between the first electrode and the second electrode and comprising an emission layer; and
a heterocyclic compound represented by Formula 1: 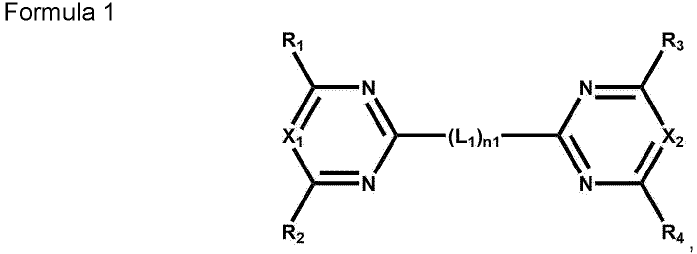 wherein, in Formula 1,
X₁ is C(R₅) or N,
X₂ is C(R₆) or N,
L₁ is a C₃-C₆₀ carbocyclic group unsubstituted or substituted with at least one R₁₀ₐ or a C₁-C₆₀ heterocyclic group unsubstituted or substituted with at least one R₁₀ₐ,
n1 is an integer from 1 to 5,
R₁ to R₆ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -C(Q₁)(Q₂)(Q₃), - Si(Q₁)(Q₂)(Q₃), -N(Q₁)(Q₂), -B(Q₁)(Q₂), -C(=O)(Q₁), -S(=O)₂(Q₁), -P(=O)(Q₁)(Q₂), or a group represented by Formula 2, 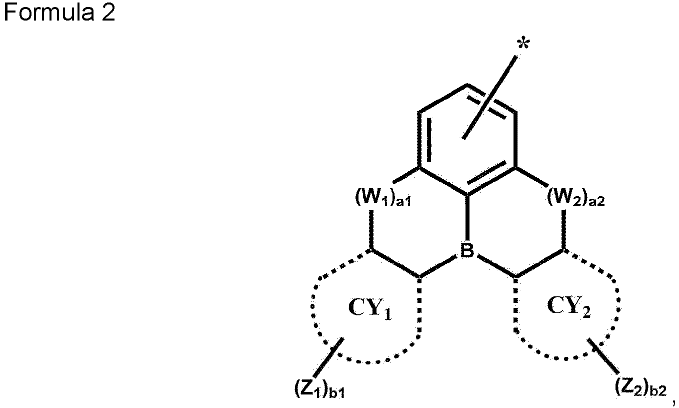 in Formula 2,
ring CY₁ and ring CY₂ are each independently a C₃-C₆₀ carbocyclic group unsubstituted or substituted with at least one R₁₀ₐ or a C₁-C₆₀ heterocyclic group unsubstituted or substituted with at least one R₁₀ₐ,
W₁ is a single bond, O, S, N(Z₃), C(Z₃)(Z₄), or Si(Z₃)(Z₄),
W₂ is a single bond, O, S, N(Zs), C(Z₅)(Z₆), or Si(Z₅)(Z₆),
a1 and a2 are each independently 0 or 1,
when a1 is 0, then *-(W₁)ₐ₁-*' is not present,
when a2 is 0, then *-(W₂)ₐ₂-*' is not present,
b1 and b2 are each independently an integer from 0 to 10,
Z₁ to Z₆ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -C(Q₁)(Q₂)(Q₃), - Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂),
* indicates a binding site to a neighboring carbon atom,
at least one selected from among R₁ to R₄ in Formula 1 is a C₃-C₁₀ alkyl group unsubstituted or substituted with at least one R₁₀ₐ, a C₃-C₁₀ cycloalkyl group unsubstituted or substituted with at least one R₁₀ₐ, or a group represented by Formula 2,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)2(Q31), or - P(=O)(Q₃₁)(Q₃₂), and
Q₁ to Q₃, Q₁₁ to Q₁₃, Q₂₁ to Q₂₃, and Q₃₁ to Q₃₃ are each independently: hydrogen; deuterium; -F; -Cl; -Br; -I; a hydroxyl group; a cyano group; a nitro group; or a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

2. The light-emitting device of claim 1, wherein
the first electrode is an anode,
the second electrode is a cathode,
the interlayer further comprises a hole transport region between the first electrode and the emission layer and an electron transport region between the emission layer and the second electrode,
the hole transport region comprises a hole injection layer, a hole transport layer, an emission auxiliary layer, an electron-blocking layer, or any combination thereof, and
the electron transport region comprises a hole-blocking layer, an electron transport layer, an electron injection layer, an electron control layer, or any combination thereof.

3. The light-emitting device of claim 1 or claim 2, wherein
the heterocyclic compound represented by Formula 1 is in the interlayer.

4. The light-emitting device of claim 2 or claim 3, wherein
the heterocyclic compound represented by Formula 1 is in the electron transport region.

5. The light-emitting device of any one of claims 1 to 4, wherein
the light-emitting device further comprises a first host and a second host,
the first host is a hole-transporting compound comprising at least one electron-donating group, and
the second host is an electron-transporting compound comprising at least one electron-withdrawing group.

6. An electronic apparatus comprising the light-emitting device of any one of claims 1 to 5, optionally further comprising
a color filter, a color conversion layer, a touch screen layer, a polarizing layer, or any combination thereof.

7. An electronic equipment comprising a light-emitting device, wherein, the light-emitting device comprises:
a first electrode;
a second electrode opposite to the first electrode;
an interlayer between the first electrode and the second electrode and comprising an emission layer; and
a heterocyclic compound represented by Formula 1: 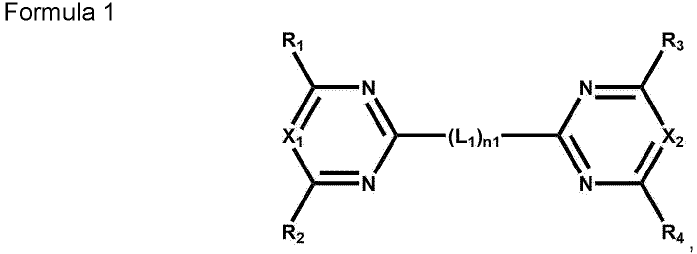 wherein, in Formula 1,
X₁ is C(R₅) or N,
X₂ is C(R₆) or N,
L₁ is a C₃-C₆₀ carbocyclic group unsubstituted or substituted with at least one R₁₀ₐ or a C₁-C₆₀ heterocyclic group unsubstituted or substituted with at least one R₁₀ₐ,
n1 is an integer from 1 to 5,
R₁ to R₆ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -C(Q₁)(Q₂)(Q₃), - Si(Q₁)(Q₂)(Q₃), -N(Q₁)(Q₂), -B(Q₁)(Q₂), -C(=O)(Q₁), -S(=O)₂(Q₁), -P(=O)(Q₁)(Q₂), or a group represented by Formula 2, 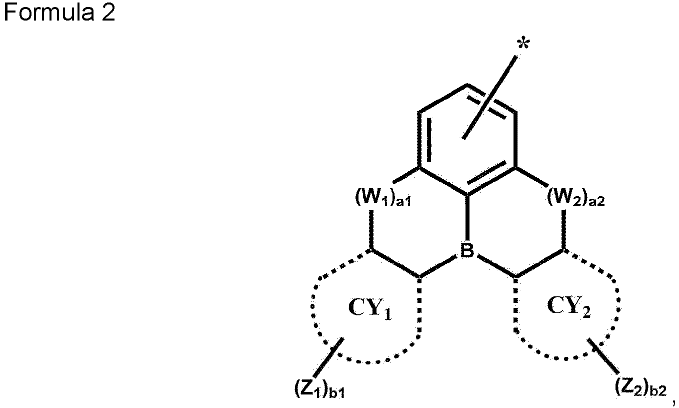 in Formula 2,
ring CY₁ and ring CY₂ are each independently a C₃-C₆₀ carbocyclic group unsubstituted or substituted with at least one R₁₀ₐ or a C₁-C₆₀ heterocyclic group unsubstituted or substituted with at least one R₁₀ₐ,
W₁ is a single bond, O, S, N(Z₃), C(Z₃)(Z₄), or Si(Z₃)(Z₄),
W₂ is a single bond, O, S, N(Zs), C(Z₅)(Z₆), or Si(Z₅)(Z₆),
a1 and a2 are each independently 0 or 1,
when a1 is 0, then *-(W₁)ₐ₁-*' is not present,
when a2 is 0, then *-(W₂)ₐ₂-*' is not present,
b1 and b2 are each independently an integer from 0 to 10,
Z₁ to Z₆ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -C(Q₁)(Q₂)(Q₃), - Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂),
* indicates a binding site to a neighboring carbon atom,
at least one selected from among R₁ to R₄ in Formula 1 is a C₃-C₁₀ alkyl group unsubstituted or substituted with at least one R₁₀ₐ, a C₃-C₁₀ cycloalkyl group unsubstituted or substituted with at least one R₁₀ₐ, or a group represented by Formula 2,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently: hydrogen; deuterium; -F; -Cl; -Br; -I; a hydroxyl group; a cyano group; a nitro group; or a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof, optionally wherein
the electronic equipment is at least one of a flat panel display, a curved display, a computer monitor, a medical monitor, a television, a billboard, an indoor light, an outdoor light, a light for signaling, a head-up display, a fully transparent display, a partially transparent display, a flexible display, a rollable display, a foldable display, a stretchable display, a laser printer, a telephone, a portable phone, a tablet personal computer, a phablet, a personal digital assistant, a wearable device, a laptop computer, a digital camera, a camcorder, a viewfinder, a micro display, a three-dimensional (3D) display, a virtual reality display, an augmented reality display, a vehicle, a video wall with multiple displays tiled together, a theater screen, a stadium screen, a phototherapy device, or a signboard.

8. A heterocyclic compound represented by Formula 1: 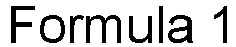 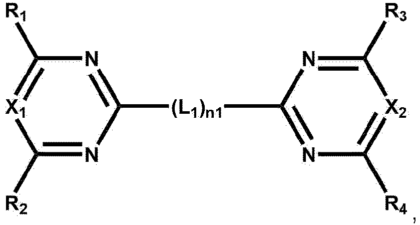 wherein, in Formula 1,
X₁ is C(R₅) or N,
X₂ is C(R₆) or N,
L₁ is a C₃-C₆₀ carbocyclic group unsubstituted or substituted with at least one R₁₀ₐ or a C₁-C₆₀ heterocyclic group unsubstituted or substituted with at least one R₁₀ₐ,
n1 is an integer from 1 to 5,
R₁ to R₆ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -C(Q₁)(Q₂)(Q₃), - Si(Q₁)(Q₂)(Q₃), -N(Q₁)(Q₂), -B(Q₁)(Q₂), -C(=O)(Q₁), -S(=O)₂(Q₁), -P(=O)(Q₁)(Q₂), or a group represented by Formula 2, 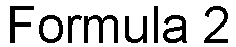 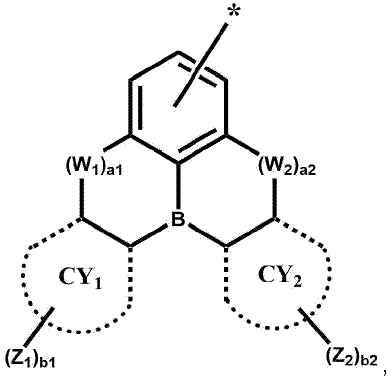 in Formula 2,
ring CY₁ and ring CY₂ are each independently a C₃-C₆₀ carbocyclic group unsubstituted or substituted with at least one R₁₀ₐ or a C₁-C₆₀ heterocyclic group unsubstituted or substituted with at least one R₁₀ₐ,
W₁ is a single bond, O, S, N(Z₃), C(Z₃)(Z₄), or Si(Z₃)(Z₄),
W₂ is a single bond, O, S, N(Zs), C(Z₅)(Z₆), or Si(Z₅)(Z₆),
a1 and a2 are each independently 0 or 1,
when a1 is 0, then *-(W₁)ₐ₁-*' is not present,
when a2 is 0, then *-(W₂)ₐ₂-*' is not present,
b1 and b2 are each independently an integer from 0 to 10,
Z₁ to Z₆ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -C(Q₁)(Q₂)(Qs₃), - Si(Q₁)(Q₂)(Q ₃), -N(Q₁)(Q₂), -B(Q₁)(Q₂), -C(=O)(Q₁), -S(=O)₂(Q₁), or -P(=O)(Q₁)(Q₂),
* indicates a binding site to a neighboring carbon atom,
at least one selected from among R₁ to R₄ in Formula 1 is a C₃-C₁₀ alkyl group unsubstituted or substituted with at least one R₁₀ₐ, a C₃-C₁₀ cycloalkyl group unsubstituted or substituted with at least one R₁₀ₐ, or a group represented by Formula 2,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently: hydrogen; deuterium; -F; -Cl; -Br; -I; a hydroxyl group; a cyano group; a nitro group; or a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

9. The heterocyclic compound of claim 8, wherein
the heterocyclic compound comprises at least one of deuterium, -F, a cyano group, a methyl group, a tert-butyl group, a carbazolyl group, a norbornanyl group, or any combination thereof.

10. The heterocyclic compound of claim 8 or claim 9, wherein
X₁ and X₂ are each N.

11. The heterocyclic compound of any one of claims 8 to 10, wherein:
(i) L₁ is a C₆-C₃₀ arylene group unsubstituted or substituted with at least one R₁₀ₐ; or
(ii) L₁ is a group represented by any one selected from among Formulae 3-1 to 3-24: 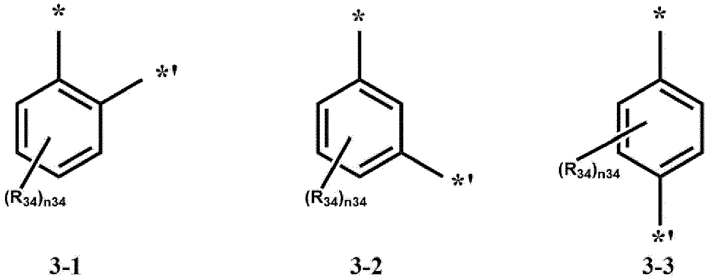 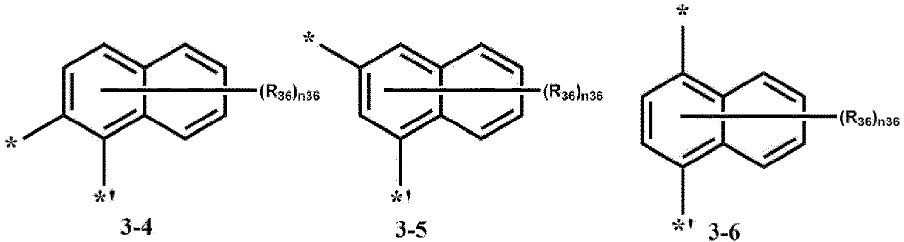 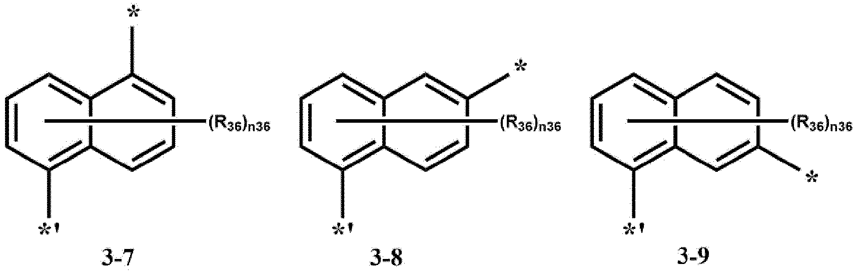 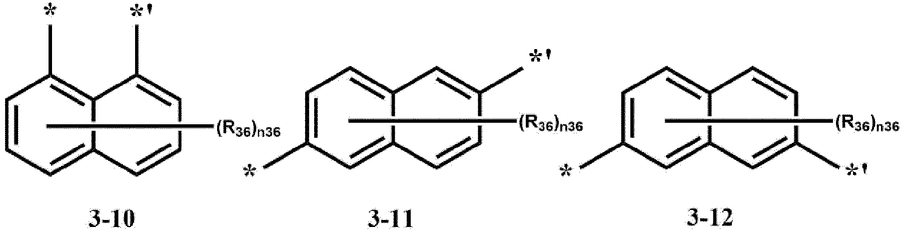 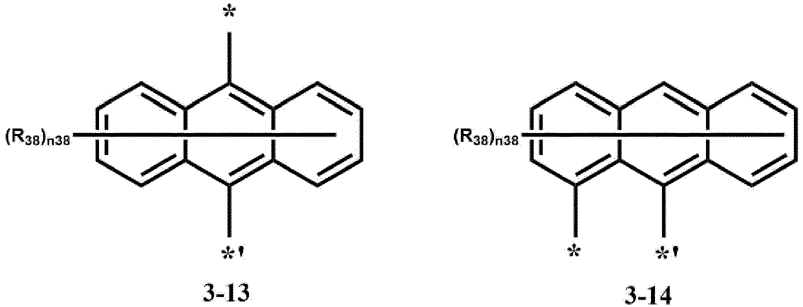 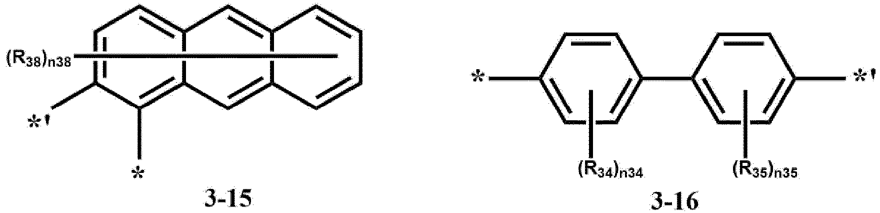 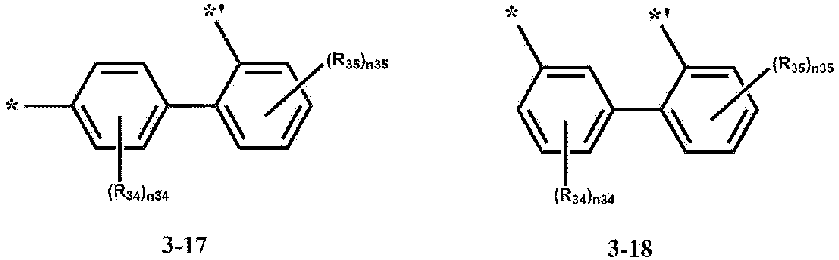 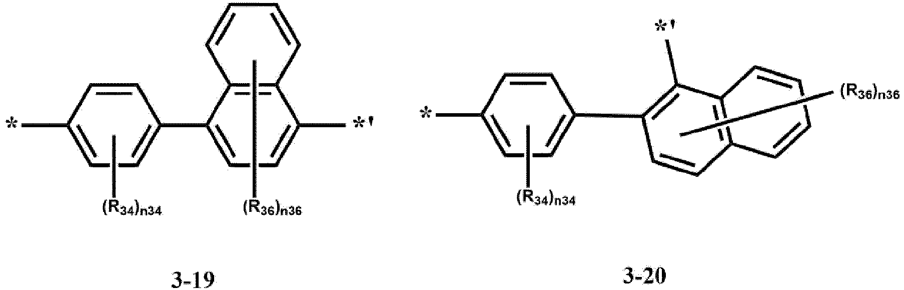 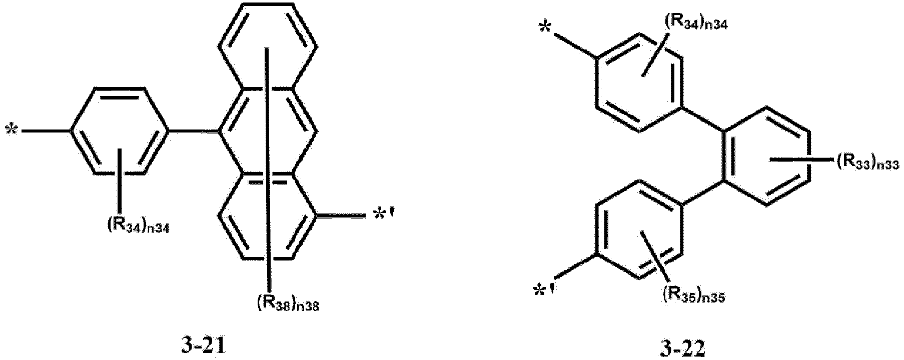 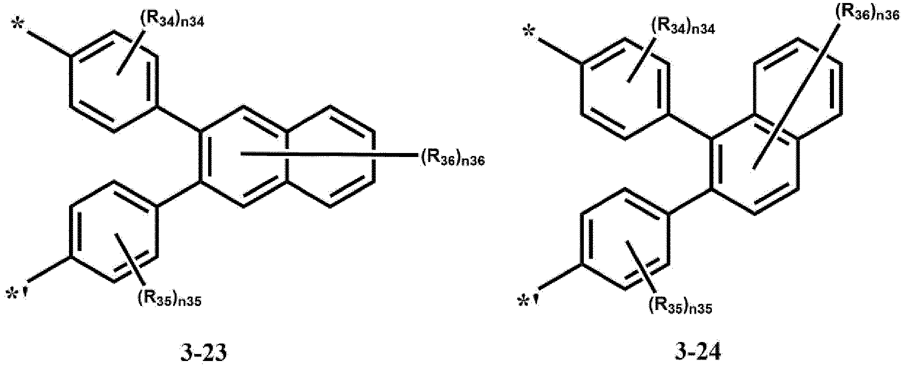 in Formulae 3-1 to 3-24,
R₃₃ to R₃₆ and R₃₈ being each the same as defined with respect to R₁ in Formula 1,
n33 to n35 being each independently an integer from 0 to 4,
n36 being an integer from 0 to 6,
n38 being an integer from 0 to 8, and
* and *' each indicating a binding site to a neighboring carbon atom.

12. The heterocyclic compound of any one of claims 8 to 11, wherein:
(i) n1 is an integer from 1 to 3; and/or
(ii) a1 and a2 are each 0, or
a1 and a2 are each 1.

13. The heterocyclic compound of any one of claims 8 to 12, wherein
the group represented by Formula 2 is a group represented by any one selected from among Formulae 2-1 to 2-4: 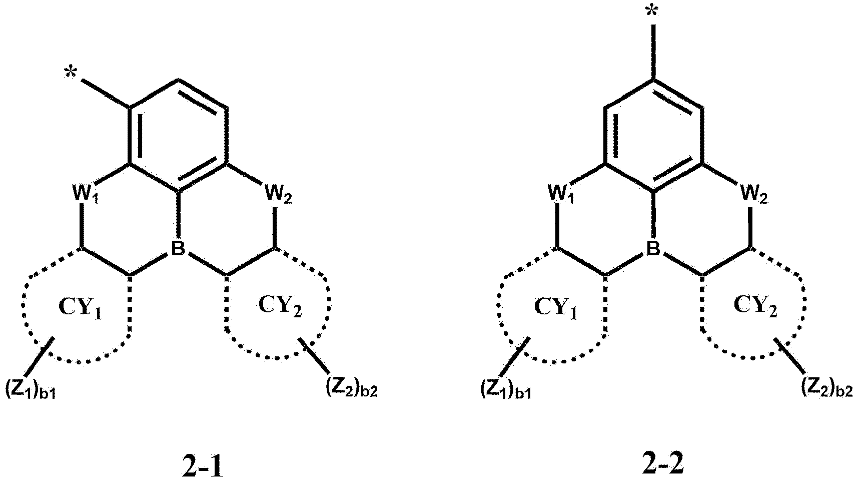 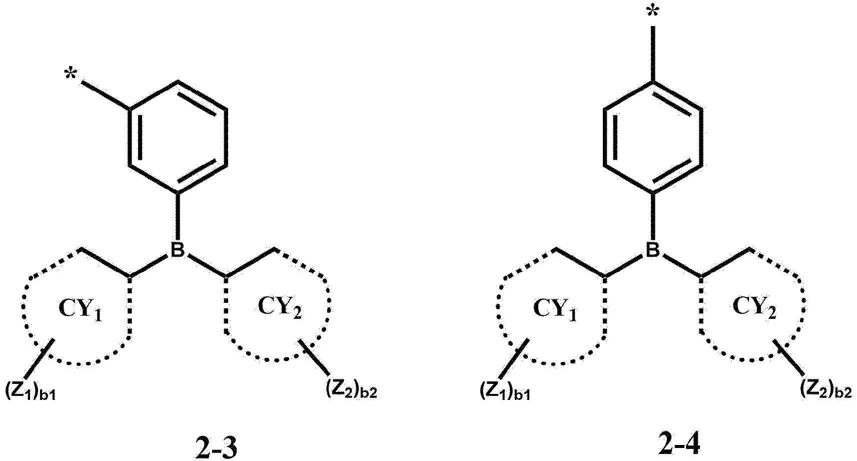 in Formulae 2-1 to 2-4, ring CY₁, ring CY₂, W₁, W₂, Z₁, Z₂, b1, and b2 are each the same as defined in Formula 2.

14. The heterocyclic compound of any one of claims 8 to 13, wherein:
(i) at least one selected from among R₁ to R₄ is: an iso-propyl group, a sec-butyl group, an isobutyl group, a tert-butyl group, a tert-pentyl group, a neopentyl group, an isopentyl group, a sec-pentyl group, a 3-pentyl group, a sec-isopentyl group, an isohexyl group, a sec-hexyl group, a tert-hexyl group, an iso-heptyl group, a sec-heptyl group, a tert-heptyl group, an iso-octyl group, a sec-octyl group, a tert-octyl group, an iso-nonyl group, a sec-nonyl group, a tert-nonyl group, an iso-decyl group, a sec-decyl group, a tert-decyl group, a cyclohexyl group, a cycloheptyl group, a cyclooctyl group, or an adamantanyl group, each unsubstituted or substituted with at least one R₁₀ₐ; or a group represented by Formula 2; or
(ii) at least one selected from among R₁ to R₄ is: an iso-propyl group, a sec-butyl group, an isobutyl group, a tert-butyl group, or a cyclohexyl group, each unsubstituted or substituted with at least one R₁₀ₐ; or a group represented by Formula 2.

15. The heterocyclic compound of claim 8, wherein
the heterocyclic compound is any one of Compounds 1 to 468:
